# EUROPEAN PATENT APPLICATION

(11) **EP 4 818 170 A2**
(43) Date of publication of application: **07.10.2026**
(21) Application number: 26182138.3
(22) Date of filing: 26.08.2022
(51) Int. Cl.: C07D 487/04

(54) **SUBSTITUTED TRICYCLIC COMPOUNDS AS PARP INHIBITORS AND USE THEREOF**

(30) Priority: 27.08.2021 CN 202111000443; 30.11.2021 CN 202111447991; 18.03.2022 CN 202210274490
(62) Document number(s) of the earlier application(s) in accordance with Art. 76 EPC: 22860653.9 / 4 392 425
(71) Applicant: IMPACT THERAPEUTICS (SHANGHAI), INC., Shanghai 201210 (CN)
(72) Inventor: CAI, Sui Xiong, Shanghai 200124 (CN); TIAN, Ye Edward, Shanghai 200124 (CN); WANG, Xiaozhu, Nanjing, Jiangsu 211106 (CN)
(74) Representative: Snaith, James Michael

(57) **Abstract**

Provided are substituted tricyclic compounds as PARP inhibitors and the use thereof. The compounds are represented by Formula (I) as below, wherein, the ring Z, Z1, Z2, A1, A2, A3, L and Cy are defined herein. The compounds of Formula (I) are PARP inhibitors and thus are useful in the treatment of diseases, disorders and conditions, such as cancer, responsive to the inhibition of PARP activity. Provided are also a pharmaceutical composition comprising the compound of Formula (I) and the use of the compound of Formula (I) in the preparation of a medicament for the treatment or prevention of diseases or conditions responsive to the inhibition of PARP activity.

## Description

### Field of the Disclosure

This disclosure is in the field of medicinal chemistry. In particular, the disclosure relates to substituted tricyclic compounds, and the use of these compounds as therapeutically effective PARP inhibitors and anticancer drugs.

### Background of the Invention

Poly (ADP-ribose) polymerase (PARP) is a family of proteins, which transfer negatively charged ADP-ribose groups from donor NAD⁺ onto target proteins. That is one of many post-transcriptional modifications. Therefore, PARP also is termed as ADP-ribose transferase.

Humans are thought to express 17 PARPs identified based on amino acid sequence homology to the catalytic domain (Vyas et al., 2013 Nature Communication, 4: 3240/1-3240/13). PARPs either catalyze the addition of a single ADP-ribose unit on target proteins or catalyze the polymerization of ADP-ribose units to form poly ADP-ribose, also known as poly (ADP-ribose) modification. As a result, the PARP family is further grouped into two subfamilies accordingly. Post-translational modification of poly (ADP-ribose) regulate many aspects of protein function and the physiological function of many PARPs have not been established.

The most characteristic member of the PARP family is PARP1, which was found to have the highest intracellular levels. PARP1 consists of 1014 amino acids (NCBI Accession P09874) with a total molecular weight of approximately 116 kDa. Structurally, this enzyme is composed of two main domains including an N-terminal DNA-binding domain and a catalytic domain. PARP1 is known to play an important role in many cellular functions, including gene expression, transcription, cell division, cell differentiation, cell apoptosis, DNA damage response and repair. PARP1 is activated when DNA damage occurs and is involved in base excision repair (BER) which is a major mechanism of DNA single-strand damage repair. PARP1 binds to the site of Single Strand Break (SSB), and subsequently repair DNA via BER. In response to DNA damage, cells also have evolved two main repair pathways: Homologous Recombination (HR) and Non-Homologous End Joining (NHEJ), in addition to BER repair mechanisms. HR deficient tumors have been found to be sensitive to PARP inhibitors, indicating homologous recombination defects and PARP1 inhibition formed a pair of synthetic lethality, which has been validated by clinical studies. Several PARP inhibitors are currently approved for the treatment of breast, ovarian, pancreatic and prostate cancers with DNA damage repair deficient such as BRCA1/2 mutation.

PARP2 is a protein of 559 amino acids with molecular mass of approximately 62 kDa and composed of DNA binding domain and catalytic domain (Ame et al., 1999 J Biol Chem 274: 17860-17868). The catalytic domain of PARP2 is very similar to that of PARP1. PARP2 is also found to have similar functions to PARP1 and is involved in the repair of DNA damage through BER mechanism (Schreiber et al., 2002 J Biol Chem 277: 23028-23036). Marketed PARP inhibitors, such as Olaparib, Niraparib, Talazoparib and Rucaparib, not only have inhibitory activities against PARP1, but also have similar inhibitory activities against PARP2. Based on the results of clinical trials, the therapeutic effects of these marketed PARP inhibitors are comparable whereas their toxicity profiles are quite different. For example, Talazoparib has toxicity similar to chemotherapy drugs such as hair loss. Talazoparib also shows more potent inhibitory activity against TNKS1/2 (Tankyrase 1 or Tankyrase 2) than other PARP inhibitors (PARPi) in biochemical assay (Ryan et al., 2021, J Biol Chem 296:100251/1-100251/13). TNKS1 and TNKS2 share 83% sequence identity overall, and their catalytic domain sequences are 89% identical. They play roles in DNA repair, telomere maintenance, and Wnt/β-catenin signaling. Targeting PARPs other than PARP1 may be the reason why PARP inhibitors cause off-targeted toxicity, such as hair loss and diarrhea. In addition, inhibition of PARP2 activity has been found to lead to hematotoxicity (Farrés et al., 2013 Blood 122: 44-54; Farrés et al., 2015 Cell Death and Differentiation 22: 1144-1157). The toxicity of these PARP inhibitors limits their clinical application as well as in combination with other targeted drugs.

Therefore, to improve, enhance and expand the clinical application of PARP inhibitors, it is important to explore highly selective PAPR1 inhibitor to reduce mechanism - related or mechanism - independent toxicity.

Various PARP1 inhibitors have been disclosed in, for example, WO2011006803, WO2013014038, WO2021013735 and WO2021260092.

### Summary of the Disclosure

The disclosure provides compounds and analogues thereof as represented by Formula I (including Formulae II, III and IV). The compounds can be used as PARP inhibitors. In particular, the compounds of the disclosure are selective PARP1 inhibitors relative to PARP2.

The disclosure also provides pharmaceutical compositions comprising an effective amount of the compound of Formula I (including Formulae II, III and IV). The pharmaceutical compositions can be used for the treatment of cancer.

In a specific embodiment, the pharmaceutical composition may further contain one or more pharmaceutically acceptable carriers, excipients or diluents.

In a specific embodiment, the pharmaceutical composition may further contain at least one known anticancer drug or pharmaceutically acceptable salts thereof.

The disclosure is also directed to methods for the preparation of novel compounds of Formula I (including Formulae II, III and IV).

The disclosure also provides a method for treating or preventing a diseases or conditions responsive to the inhibition of PARP activity (especially PARP1 activity), comprising administering to a subject in need thereof an effective amount of the compound of Formula I (including Formulae II, III and IV) or a pharmaceutically acceptable salt thereof.

### Detailed Description of the Disclosure

It should be understood that the characteristics of the embodiments described herein can be arbitrarily combined to form the technical solution of this disclosure. The definition of each group herein can apply to any of the embodiments described herein. For example, the definitions of the substituents of alkyl herein apply to any of the embodiments described herein unless the substituents of alkyl are clearly defined in the embodiment.

The term "hydrogen (H)" as empolyed herein includes its isotopes D and T.

The term "heteroatoms" as empolyed herein includes O, S and N.

The term "alkyl" as used herein refers to alkyl itself or a straight or branched chain radical of up to ten carbons. Useful alkyl groups include straight-chain or branched C₁₋₁₀ alkyl groups, preferably C₁₋₆ alkyl groups. In some embodiments, alkyl is C₁₋₄ alkyl. In some embodiments, alkyl is C₁₋₃ alkyl. In some embodiments, alkyl is deuterated C₁₋₃ alkyl. Typical C₁₋₁₀ alkyl groups include methyl, methyl-d3, ethyl, propyl, isopropyl, butyl, sec-butyl, *tert*-butyl, pentyl (such as 3-pentyl), hexyl and octyl groups, which may be optionally substituted.

The term "alkenyl" as used herein refers to a straight or branched chain radical of 2-10 carbon atoms, unless the chain length is limited thereto, wherein there is at least one double bond between two of the carbon atoms in the chain; preferably, C₂₋₆ alkenyl. Typical alkenyl groups include ethenyl, 1-propenyl, 2-propenyl, 2-methyl-1-propenyl, 1-butenyl and 2-butenyl.

The term "alkynyl" as used herein refers to a straight or branched chain radical of 2-10 carbon atoms, unless the chain length is limited thereto, wherein there is at least one triple bond between two of the carbon atoms in the chain; preferably, C₂₋₆ alkynyl. Typical alkynyl groups include ethynyl, 1-propynyl, 1-methyl-2-propynyl, 2-propynyl, 1-butynyl and 2-butynyl.

Useful alkoxy groups include oxygen substituted by the above mentioned C₁₋₁₀ alkyl groups, preferred C₁₋₆ alkyl groups or C₁₋₄ alkyl groups, e.g., methoxy, ethoxy, etc. The alkyl in the alkoxy groups may be optionally substituted. Substituents of alkoxy groups include, without limitation, halogen, morpholino, amino (including alkylamino and dialkylamino), and carboxy (including esters thereof).

Useful amino and optionally substituted amino groups are -NR'R", wherein R' and R" each are independently hydrogen, an optionally substituted C₁₋₁₀ alkyl, an optionally substituted C₃₋₈ cycloalkyl, an optionally substituted aryl or an optionally substituted heteroaryl. Preferably, R' and R" each are independently hydrogen, an optionally substituted C₁₋₄ alkyl, an optionally substituted C₃₋₆ cycloalkyl, or R' and R" together with the N to which they are attached form an optionally substituted 4-7 membered cyclic amino group, which optionally comprises one or more (such as 2, 3) additional heteroatoms selected from a group consisting of O, N and S. Preferred amino groups include NH₂, and at least one of R' and R" is a C₁₋₆ alkyl in -NR'R".

The term "oxo" as used herein refers to =O.

The term "aryl" as used herein by itself or as part of another group refers to monocyclic, bicyclic or tricyclic aromatic groups containing 6 to 14 carbon atoms. Aryl may be substituted by one or more substituents as described herein.

Useful aryl groups include C₆₋₁₄ aryl groups, preferably C₆₋₁₀ aryl groups. Typical C₆₋₁₄ aryl groups include phenyl, naphthyl, phenanthryl, anthracyl, indenyl, azulyl, biphenyl, biphenylene and fluorenyl.

The term "carbocyclic group" as used herein include cycloalkyl and partially saturated carbocyclic groups. Useful cycloalkyl groups are C₃₋₈ cycloalkyl. In some preferred embodiments, cycloalkyl groups are C₃₋₆ cycloalkyl. Typical cycloalkyl groups include cyclopropyl, cyclobutyl, cyclopentyl, cyclohexyl and cycloheptyl. Useful partially saturated carbocyclic groups are cycloalkenyl, such as C₃₋₈ cycloalkenyl, which include cyclopentenyl, cycloheptenyl and cyclooctenyl. Carbocyclic group may be substituted by one or more substituents as described herein.

Useful halo or halogen groups include fluoro, chloro, bromo and iodo.

Useful acylamino (amido) groups are any C₁₋₆ acyl (alkanoyl) attached to an amino nitrogen, e.g., acetamino, propionamido, butanoylamido, pentanoylamido and hexanoylamido, as well as aryl-substituted C₁₋₆ acylamino groups, e.g., benzoylamido.

Useful acyl groups include C₁₋₆ acyl groups, such as acetyl. Acyl may be optionally substituted by group selected from halo, amino and aryl, wherein the amino and aryl may be optionally substituted. When acyl is substituted by halo, the number of halogen substituents may be in the range of 1-5. Examples of substituted acyls include chloroacetyl and pentafluorobenzoyl. When acyl is substituted by amino, amino group may be substituted by one or two substituents as described herein. In some embodiments, aminoacyl is -C(O)-NR'R", wherein R' and R" each are independently hydrogen, an optionally substituted C₁₋₁₀ alkyl, an optionally substituted C₃₋₈ cycloalkyl, an optionally substituted aryl or an optionally substituted heteroaryl. Preferably, R' and R" each are independently hydrogen, an optionally substituted C₁₋₄ alkyl, or an optionally substituted C₃₋₆ cycloalkyl.

The term "heterocyclic group" as used herein refers to a saturated or partially saturated 3-7 membered monocyclic, or 7-10 membered bicyclic ring, spirocyclic ring or bridged ring system, which consists of carbon atoms and one to four heteroatoms independently selected from a group consisting of O, N, and S, wherein the nitrogen and/or sulfur heteroatoms can be optionally oxidized and the nitrogen can be optionally quaternized, and the term also includes any bicyclic ring system in which any of the above-defined heterocyclic rings is fused to a benzene ring. The heterocyclic group can be substituted on carbon atom or nitrogen atom if the resulting compound is stable. The heterocyclic group may be substituted by one or more substituents as described herein.

Useful saturated or partially saturated heterocyclic groups include tetrahydrofuranyl, tetrahydropyranyl, piperidinyl, piperazinyl, 1,4-diazepanyl, azetidinyl, oxetanyl, pyrrolidinyl, imidazolidinyl, imidazolinyl, indoline, isoindoline, quinuclidinyl, morpholinyl, isochromanyl, chromanyl, pyrazolidine, pyrazolinyl, tetrahydroisoquinolyl, tetronoyl and tetramoyl, which may be optionally substituted by one or more substituents as described herein.

The term "heteroaryl" as used herein refers to a group having 5 to 14 ring atoms, preferably 5 to 10 ring atoms, with 6, 10 or 14 electrons shared in a cyclic array. Ring atoms are carbon atoms and 1-3 heteroatoms selected from a group consisting of oxygen, nitrogen and sulfur. Heteroaryl may be optionally substituted by one or more substituents as described herein.

Useful heteroaryl groups include thienyl (thiophenyl), benzo[d]isothiazol-3-yl, benzo[b]thienyl, naphtho[2,3-b]thienyl, thianthrenyl, furyl (furanyl), pyranyl, isobenzofuranyl, chromenyl, xanthenyl, phenoxanthiinyl, pyrrolyl, imidazolyl, pyrazolyl, pyridyl (pyridinyl, including without limitation 2-pyridyl, 3-pyridyl, and 4-pyridyl), pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalzinyl, naphthyridinyl, quinozalinyl, cinnolinyl, pteridinyl, carbazolyl, β-carbolinyl, phenanthridinyl, acridinyl, perimidinyl, phenanthrolinyl, phenazinyl, isothiazolyl, phenothiazinyl, isoxazolyl, furazanyl, phenoxazinyl, tetrahydrocyclopenta[c]pyrazol-3-yl, benzoisoxazolyl such as 1,2-benzoisoxazol-3-yl, benzimidazolyl, 2-oxindolyl, thiadiazolyl, 2-oxobenzimidazolyl, imidazopyridazinyl, imidazopyridyl, triazolopyridazinyl, pyrazolopyrimidinyl, pyrrolopyrimidinyl, pyrrolopyridyl, pyrrolopyrazinyl or triazolopyrazinyl. Where the heteroaryl group contains a nitrogen atom in a ring, such nitrogen atom may be in the form of an N-oxide, e.g., a pyridyl N-oxide, pyrazinyl N-oxide and pyrimidinyl N-oxide.

In this disclosure, unless otherwise described, when substituted, the alkyl, cycloalkyl, heterocycloalkyl, alkoxy, heterocycloalkoxy, alkenyl, heterocycloalkenyl, alkynyl, amino, amido, acyloxy, carboxyl, hydroxyl, mercapto, alkylthio sulfonyl, sulfonyl, sulfinyl, aminoacyl, silyl, phosphinecarboxy, phosphono, carbocyclic group, heterocyclic group, aryl or heteroaryl as described in any embodiment herein may be substituted by one or more (such as 1, 2, 3, 4, 5 or 6) substituents selected from the group consisting of halogen, hydroxyl, carboxyl, amino, nitro, cyano, C₁₋₆ amido, C₁₋₆ acyloxy, C₁₋₆ alkoxy, aryloxy, alkylthio, C₁₋₆ alkyl, C₁₋₆ acyl, C₆₋₁₀ aryl, C₃₋₈ cycloalkyl, C₂₋₆ alkenyl, C₂₋₆ alkynyl, heterocyclic or heteroaryl, methylenedioxy, urea group, mercapto group, azide group, carbonyl, alkanesulfonyl, sulfamoyl, dialkylsulfamoyl and alkylsulfinyl, etc. The substituent itself may also be optionally substituted. Preferred substituents include without limitation halogen, hydroxyl, carboxyl, amino, C₁₋₆ amido, C₁₋₆ acyloxy, C₁₋₆ alkoxy, C₁₋₆ alkyl, C₁₋₆ acyl and alkanesulfonyl.

It should be understood that in each embodiment, when the substituent is a heterocyclic group, aryl or heteroaryl, the number thereof is usually 1.

Specifically, the disclosure provides compounds represented by Formula I: 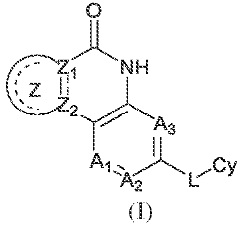 or stereoisomers, tautomers, N-oxides, hydrates, solvates, isotope-substituted derivatives, or pharmaceutically acceptable salts thereof, or mixtures thereof, or prodrugs thereof, wherein:
A₁, A₂ and A₃ are each independently selected from N and CRi;
the Z ring shown as 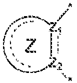 is an optionally substituted 5-7 membered carbocyclic group, an optionally substituted 5-7 membered heterocyclic group or an optionally substituted 5 membered heteroaryl group, * indicates the position at which the Z ring is attached to the rest of the compound, the dashed lines indicate optional presence of unsaturated bond(s), wherein Z₁ and Z₂ are each independently C or N, wherein Z₁ and Z₂ are not N at the same time; in addition, Z₁ and Z₂ satisfy the following conditions: (1) when the Z ring is an optionally substituted 5-7 membered carbocyclic group or an optionally substituted 5-7 membered heterocyclic group, both of Z₁ and Z₂ are C, and among the bonds of the Z ring, only the bond between Z₁ and Z₂ is a double bond; (2) when the Z ring is an optionally substituted 5-7 membered heterocyclic group and Z₂ is C, the ring atom connected to Z₂ of the Z ring is not N; (3) when the Z ring is an optionally substituted 5 membered heteroaryl group and Z₁ is N, in addition to the N at the Z₁ position, the 5 membered heteroaryl further contains 1-3 N heteroatoms; or when Z ring is an optionally substituted 5 membered heteroaryl, Z₁ is N, and there are no other heteroatoms in the Z ring, A₁ is CRi;
L is selected from a bond and an alkylene optionally substituted by R₂ and/or R₃;
Cy is selected from a group consisting of an optionally substituted heterocyclic group, an optionally substituted aryl, and an optionally substituted heteroaryl;
R₁ is selected from a group consisting of hydrogen, halogen, an optionally substituted alkyl, an optionally substituted alkoxy and an optionally substituted carbocyclic group;
R₂ and R₃ are each independently selected from a group consisting of halogen, cyano, an optionally substituted alkyl, an optionally substituted alkoxy, an optionally substituted cycloalkyl, an optionally substituted alkenyl, and an optionally substituted alkynyl; or R₂ and R₃ together with the attached C form a ring.

In Formula I and each formula of the disclosure, unless otherwise described, each alkyl is independently a C₁₋₆ alkyl, preferably a C₁₋₄ alkyl; each alkylene is a C₁₋₆ alkylene, preferably a C₁₋₃ alkylene; each alkenyl is independently a C₂₋₆ alkenyl, preferably C₂₋₄ alkenyl; each alkynyl is independently C₂₋₆ alkynyl, preferably C₂₋₄ alkynyl; each alkoxy is independently C₁₋₆ alkoxy, preferably C₁₋₄ alkoxy. Preferably, when the alkyl, alkenyl, alkynyl and alkoxy are substituted, the substituents can be selected from a group consisting of cyano, hydroxyl, nitro, amino (-NR'R"), aryl, heterocyclic group, heteroaryl, halogen and carboxyl, etc. The number of substituents may be 1-5, R' and R" are preferably each independently H, an optionally substituted C₁₋₄ alkyl or an optionally substituted C₃₋₆ cycloalkyl. For example, the substituted alkyl *per se* or as a substituent of other groups may be hydroxyalkyl, dihydroxyalkyl, alkylaminoalkyl, dialkylaminoalkyl, heterocyclic alkyl, aralkyl, heteroarylalkyl and haloalkyl, etc. It should be understood, when the substituent is aryl, heteroaryl, heterocyclic group, cyano, nitro and carboxyl, the number thereof is usually 1. When the substituent is halogen, the number of substituents can be up to 5 depending on the carbon chain length of the alkyl, alkenyl, alkynyl and alkoxy groups; exemplary substituents are trifluoromethyl and pentafluoroethyl, etc.

In Formula I and each formula of the disclosure, unless otherwise described, the number of ring carbon atoms of each carbocyclic group is preferably 3-8. Preferred carbocyclic groups are C₃₋₈ cycloalkyl groups or C₃₋₈ cycloalkenyl. The substituents on the carbocyclic group are preferably C₁₋₄ alkyl, halogenated C₁₋₄ alkyl, C₁₋₄ alkoxy, halogen, hydroxyl, carboxyl, amino (-NR'R"), aryl, heterocyclic group, heteroaryl and carboxyl, etc. The number of substituents may be 1-5, R' and R" are preferably each independently H, an optionally substituted C₁₋₄ alkyl or an optionally substituted C₃₋₆ cycloalkyl. It should be understood, when the substituent is aryl, heteroaryl, heterocyclic group, cyano, nitro and carboxyl, the number thereof is usually 1. When the substituent is halogen, the number of substituents can be up to 5.

In Formula I and each formula of the disclosure, unless otherwise described, the aryl refers to C₆₋₁₄ aryl, the heteroaryl refers to 5-10 membered heteroaryl, and the heterocyclic group refers to 4-10 membered heterocyclic group. The substituents on each of the aryl, heteroaryl and heterocyclic group can each be independently selected from 1-5 groups consisting of C₁₋₄ alkyl, halogenated C₁₋₄ alkyl, C₁₋₄ alkoxy, halogen, hydroxyl, carboxyl, amino (-NR'R"), an optionally substituted aryl, an optionally substituted heteroaryl, an optionally substituted heterocyclic group, halogen, amido, aminoacyl (-C(O)-NR'R") and carboxyl, etc.; wherein R' and R" each are independently hydrogen, an optionally substituted C₁₋₁₀ alkyl, an optionally substituted C₃₋₈ cycloalkyl, an optionally substituted aryl or an optionally substituted heteroaryl. Preferably, R' and R" each are independently hydrogen, an optionally substituted C₁₋₄ alkyl, an optionally substituted C₃₋₆ cycloalkyl. The number of substituents may be 1-5. The said optionally substituted aryl, optionally substituted heteroaryl and optionally substituted heterocyclic group may be optionally substituted by 1-5 groups selected from C₁₋₄ alkyl, halogenated C₁₋₄ alkyl, C₁₋₄ alkoxy, halogen, hydroxyl, carboxyl, amino (-NR'R"), aminoacyl (-C(O)-NR'R") and carboxyl, wherein, the said R' and R" are preferably each independently H, optionally substituted C₁₋₄ alkyl or optionally substituted C₃₋₆ cycloalkyl. It should be understood, when the substituent is aryl, heteroaryl, heterocyclic group, cyano, nitro and carboxyl, the number thereof is usually 1. When the substituent is halogen, the number of substituents can be up to 5.

In one or more embodiments of the compound of Formula I, the Z ring is selected from the following groups: 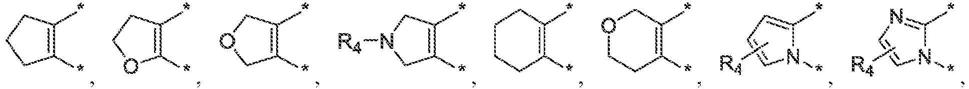 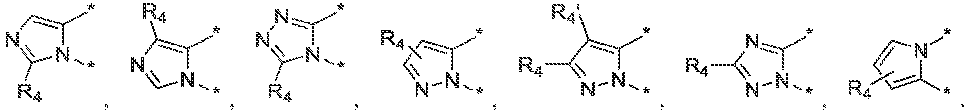 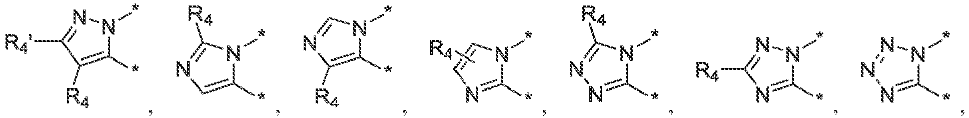 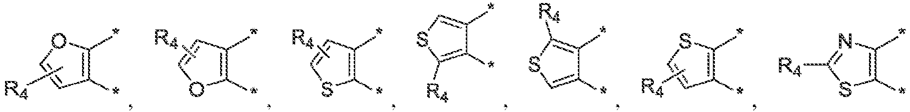 and 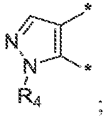 preferably the Z ring is selected from the following groups: 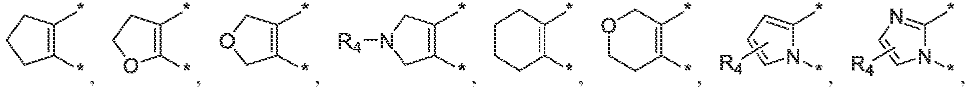 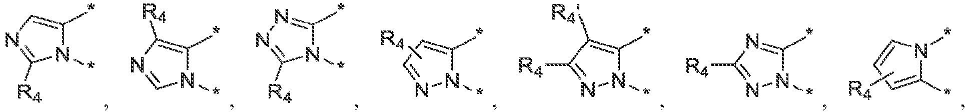 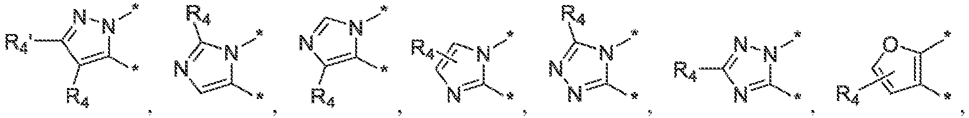 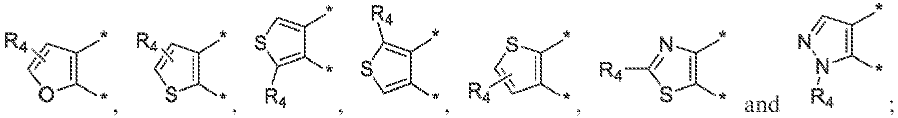 more preferably the Z ring is selected from the following groups: 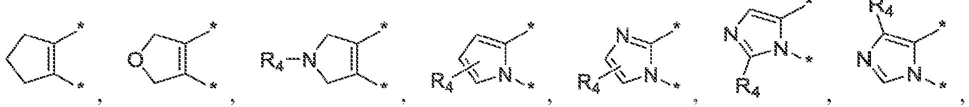 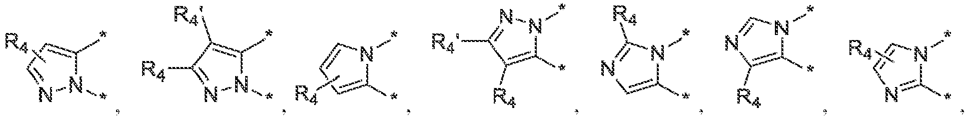 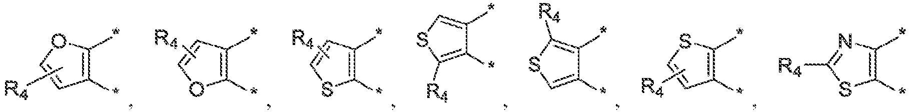 and 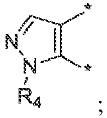 wherein * indicates the position at which the Z ring is attached to the rest of the compound; each R₄ is independently selected from a group consisting of hydrogen, halogen and an optionally substituted alkyl, preferably hydrogen, halogen and an optionally substituted C₁₋₃ alkyl; each R₄' is independently selected from a group consisting of hydrogen, halogen and an optionally substituted alkyl, preferably hydrogen, halogen and an optionally substituted C₁₋₃ alkyl. In some embodiments, each R₄ is independently selected from a group consisting of hydrogen and an optionally substituted alkyl, preferably hydrogen and an optionally substituted C₁₋₃ alkyl.

In one or more embodiments of the compound of Formula I, the Z ring is selected from the following groups:

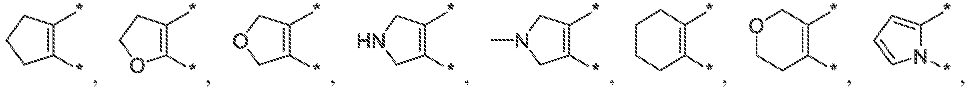

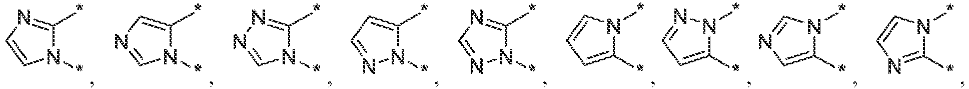

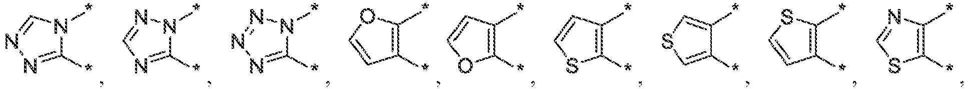

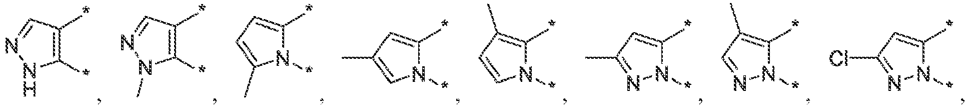

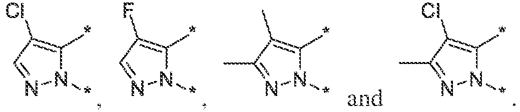

In one or more embodiments of the compound of Formula I, the Z ring is selected from the following groups:

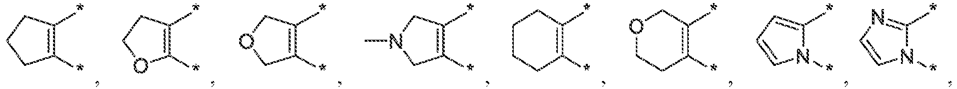

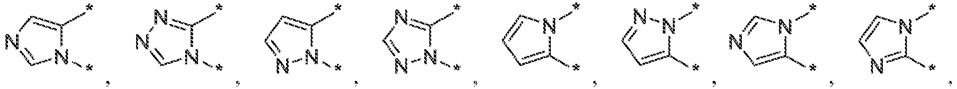

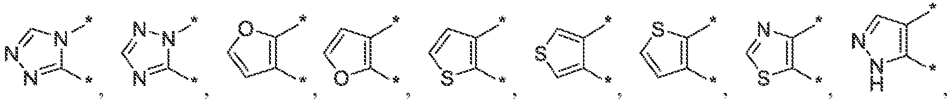

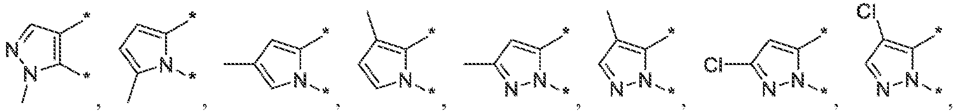

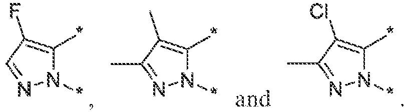

In one or more embodiments of the compound of Formula I, the Z ring is selected from the following groups: 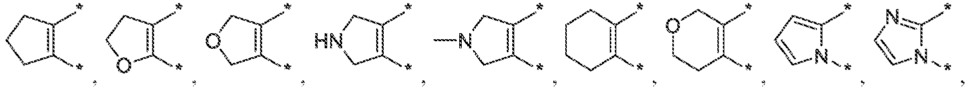 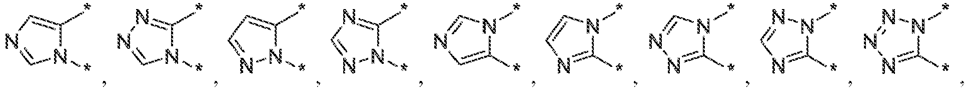 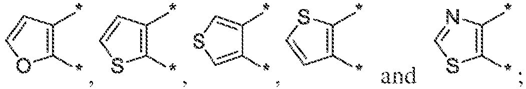 preferably the Z ring is selected from the following groups:

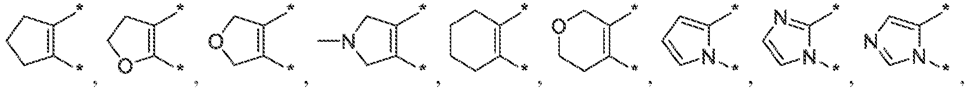

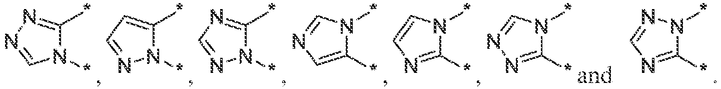

In one or more embodiments of the compound of Formula I, A₁, A₂ and A₃ are each independently selected from N and CR₁, wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. In some embodiments, only one of A₁, A₂ and A₃ is N, and the other two are independently CR₁, preferably, R₁ is independently H, C₁₋₃ alkyl or halogen. In more preferred embodiment, A₃ is CH, one of A₁ and A₂ is N and the other is CRi, wherein, R₁ is H, C₁₋₃ alkyl or halogen. In some embodiments, A₁ is N, both of A₂ and A₃ are CH. In some embodiments, A₂ is N, both A₁ and A₃ are CH. In some embodiments, all of A₁, A₂ and A₃ are CR₁, each R₁ is independently H, C₁₋₃ alkyl or halogen. Preferably, A₃ is CH, one of A₁ and A₂ is CR₁, wherein R₁ is halogen; more preferably, both of A₂ and A₃ are CH, A₁ is CR₁, wherein R₁ is halogen.

In one or more embodiments of the compound of Formula I, when the Z ring is not 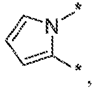 A₁, A₂ and A₃ are each independently selected from N and CR₁, wherein R₁ is preferably hydrogen, halogen, an optionally substituted C₁₋₃ alkyl or an optionally substituted C₁. ₃ alkoxy, more preferably, R₁ is hydrogen, C₁₋₃ alkyl or halogen. In some embodiments, only one of A₁, A₂ and A₃ is N, and the other two are independently CR₁, preferably, R₁ is independently H, C₁₋₃ alkyl or halogen. In more preferred embodiment, A₃ is CH, one of A₁ and A₂ is N and the other is CR₁, wherein, R₁ is H, C₁₋₃ alkyl or halogen. In some embodiments, A₁ is N, both of A₂ and A₃ are CH. In some embodiments, A₂ is N, both A₁ and A₃ are CH. In some embodiments, all of A₁, A₂ and A₃ are CR₁, each R₁ is independently H, C₁₋₃ alkyl or halogen. Preferably, A₃ is CH, one of A₁ and A₂ is CR₁, wherein R₁ is C₁₋₃ alkyl or halogen; more preferably, both of A₂ and A₃ are CH, A₁ is CR₁, wherein R₁ is C₁₋₃ alkyl or halogen. Preferably, A₂ is CH, one of A₁ and A₃ is CR₁, wherein R₁ is C₁₋₃ alkyl or halogen; more preferably, both of A₁ and A₂ are CH, A₃ is CR₁, wherein R₁ is C₁₋₃ alkyl or halogen.

In one or more embodiments of the compound of Formula I, when the Z ring is 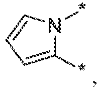 A₁ is CR₁, A₂ and A₃ each are independently N or CR₁, wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. Preferably, A₁, A₂ and A₃ each are independently CR₁, wherein R₁ is independently H, halogen or C₁₋₃ alkyl. In some embodiments, A₁ is CR₁, both of A₂ and A₃ are CH, wherein R₁ is C₁₋₃ alkyl or halogen. In some embodiments, A₂ is CR₁, both of A₁ and A₃ are CH, wherein R₁ is C₁₋₃ alkyl or halogen. In some embodiments, A₃ is CR₁, both of A₁ and A₂ are CH, wherein R₁ is C₁₋₃ alkyl or halogen. In some embodiments, all of A₁, A₂ and A₃ are CH. Preferably, A₁ is CR₁, both of A₂ and A₃ are CH; or both of A₁ and A₂ are CH, A₃ is CR₁, wherein R₁ is C₁₋₃ alkyl or halogen.

The substituents on the Z ring can be 1-3 groups selected from a group consisting of hydroxy, halogen, C₁₋₄ alkyl, C₁₋₄ alkoxy, halogenated C₁₋₄ alkyl, halogenated C₁₋₄ alkoxy, C₁₋₄ alkyl substituted with hydroxy, C₁₋₄ alkoxy substituted with hydroxy and amino (-NR'R"), wherein R' and R" each are preferably independently H or C₁₋₄ alkyl. In some embodiments, when the Z ring is substituted, the substituents can be 1-3 groups selected from halogen or C₁₋₄ alkyl.

In one or more embodiments of the compound of Formula I, each R₁ is preferably independently hydrogen, halogen, an optionally substituted C₁₋₃ alkyl or an optionally substituted C₁₋₃ alkoxy. In some embodiments, R₁ is an optionally substituted C₁₋₃ alkyl. Preferably, when R₁ is substituted, the substituents can be 1-5 groups selected from halogen, hydroxy, amino (-NR'R"), etc., wherein R' and R" each are preferably independently H, an optionally substituted C₁₋₄ alkyl or an optionally substituted C₃₋₆ cycloalkyl. Preferably, R₁ is halogen, C₁₋₃ alkyl or halogenated C₁₋₃ alkyl. In some embodiments, R₁ is hydrogen, C₁₋₃ alkyl or halogen. In some embodiments, R₁ is hydrogen or halogen.

In one or more embodiments of the compound of Formula I, preferably, R₂ and R₃ are each independently halogen or C₁₋₃ alkyl. In some embodiments, R₂ and R₃ together with the attached C form a 3-6 membered cycloalkyl.

In one or more embodiments of the compound of Formula I, L is an unsubstituted alkylene, preferably an unsubstituted C₁₋₃ alkylene, more preferably methylene.

In one or more embodiments of the compound of Formula I, the said aryl is preferably a phenyl. The said heteroaryl is a 5-10-membered heteroaryl containing 1 or 2 heteroatoms selected from oxygen, sulfur and nitrogen atoms. In some embodiments, the said heteroaryl is a 5-10-membered heteroaryl containing 1 or 2 nitrogen atoms. The said heteroaryl includes but is not limited to pyridyl, pyrazinyl, pyrrolyl, imidazolyl, pyrazolyl, pyrimidinyl, pyridazinyl, indolizinyl, thienyl, furyl, and thiazolyl, etc. The said carbocyclic group is preferably a C₃₋₈ cycloalkyl or a C₃₋₈ cycloalkenyl. The said heterocyclic group is preferably a 4-10 membered heterocyclic group containing O, S and/or N, including but not limited to azetidinyl, oxetanyl, pyrrolidinyl, piperazinyl, piperidinyl, dihydrofuranyl, tetrahydrofuranyl, tetrahydroisoquinolyl and morpholinyl, etc.

In one or more embodiments of the compound of Formula I, Cy is an optionally substituted 5-7 membered nitrogen-containing heterocyclic group. Preferably, the 5-7 membered nitrogen-containing heterocyclic group is covalently attached to L through its ring nitrogen atom. Further preferably, Cy is an optionally substituted piperazinyl. Preferably, when Cy is substituted, the substituents on Cy can be 1-3 groups selected from a group consisting of halogen, C₁₋₄ alkyl, C₁₋₄ alkoxy, halogenated C₁₋₄ alkyl, halogenated C₁₋₄ alkoxy, an optionally substituted 6-14 membered aryl, an optionally substituted 5-10 membered heteroaryl, an optionally substituted 4-10 membered heterocyclic group and an optionally substituted C₃₋₈ cycloalkyl. The optionally substituted 6-14 membered aryl, the optionally substituted 5-10 membered heteroaryl, the optionally substituted 4-10 membered heterocyclic group and the optionally substituted C₃₋₈ cycloalkyl each can be independently substituted by 1-5 substituents selected from a group consisting of halogen, an optionally substituted alkyl (such as an optionally substituted C₁₋₄ alkyl), an optionally substituted alkoxy (such as an optionally substituted C₁₋₄ alkoxy), an optionally substituted carbocyclic group (such as an optionally substituted C₃₋₈ cycloalkyl), an optionally substituted alkenyl (such as an optionally substituted C₂₋₄ alkenyl), an optionally substituted alkynyl (such as an optionally substituted C₂₋₄ alkynyl), an amino (-NR'R"), an aminoacyl (-C(O)-NR'R") and carboxyl, wherein the said R' and R" each are preferably independently H, an optionally substituted C₁₋₁₀ alkyl, an optionally substituted C₃₋₈ cycloalkyl, an optionally substituted aryl or an optionally substituted heteroaryl; preferably H, an optionally substituted C₁₋₄ alkyl or an optionally substituted C₃₋₈ cycloalkyl. In some preferred embodiments, the substituent(s) include at least -C(O)-NR'R", and optionally include one or two of substituents selected from a group consisting of halogen, C₁₋₄ alkyl and halogenated C₁₋₄ alkyl. Preferably, the said an optionally substituted alkyl, an optionally substituted alkoxy, an optionally substituted carbocyclic group, an optionally substituted alkenyl and an optionally substituted alkynyl each can be independently substituted by 1-5 substituents selected from a group consisting of halogen, hydroxy and amino, such as halogenated C₁₋₄ alkyl and halogenated C₁₋₄ alkoxy, etc.

In some preferred embodiments, Cy is substituted by an optionally substituted 5-10 membered heteroaryl, preferably an optionally substituted 5-10 membered nitrogen-containing heteroaryl. Preferably, the 5-10 membered heteroaryl or 5-10 membered nitrogen-containing heteroaryl is at least substituted by -C(O)-NR'R" or -COOH and optionally further substituted by one or two substituents selected from a group consisting of halogen, C₁₋₄ alkyl and halogenated C₁₋₄ alkyl. In some particularly preferred embodiments, Cy is piperazinyl substituted with an optionally substituted pyridyl, and the said pyridyl is at least substituted with -C(O)-NR'R". Preferably, in the embodiments as described herein, when the said R' and R" are substituted, the substituents can be 1-5 groups selected from a group consisting of halogen, hydroxy and amino.

In one or more embodiments of the compound of Formula I, A₁, A₂ and A₃ are each independently CRi; each R₁ is independently H, C₁₋₃ alkyl or halogen; L is -CH₂- or - CH₂CH₂-; Cy is piperazinyl substituted with pyridyl, and the said pyridyl is substituted with - C(O)-NR'R" and optionally further substituted by one or two substituents selected from a group consisting of halogen, C₁₋₄ alkyl and halogenated C₁₋₄ alkyl; R' and R" each are independently H, C₁₋₄ alkyl optionally substituted by hydroxy, or C₃₋₈ cycloalkyl.

In one or more embodiments of the compound of Formula I, A₁, A₂ and A₃ are each independently CR₁; each R₁ is independently H, C₁₋₃ alkyl or halogen; L is -CH₂-; Cy is piperazinyl substituted with pyridyl, and the said pyridyl is substituted with -C(O)-NR'R" and optionally further substituted by one or two substituents selected from a group consisting of halogen and C₁₋₄ alkyl; R' and R" each are independently H, C₁₋₄ alkyl optionally substituted by hydroxy, or C₃₋₈ cycloalkyl.

One group of preferred compounds of Formula I in this disclosure is represented by compounds of Formula II (including Formulae IIa and IIb): 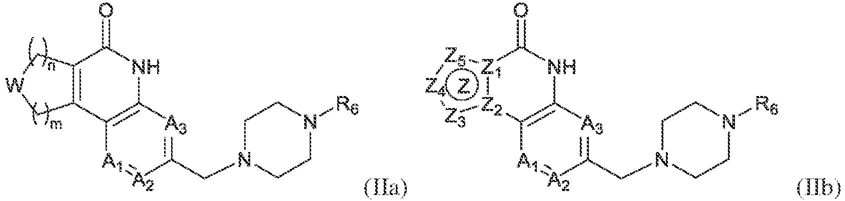 or stereoisomers, tautomers, N-oxides, hydrates, solvates, isotope-substituted derivatives, or pharmaceutically acceptable salts thereof, or mixtures thereof, or prodrugs thereof, wherein:
A₁, A₂ and A₃ are as defined in any embodiments of Formula I;
W is selected form a group consisting of CR₄R₅, O, S and NR₄;
Z₁ and Z₂ each are independently C or N, with the proviso that Z₁ and Z₂ are not N at the same time;
Z₃, Z₄ and Z₅ are independently selected from a group consisting of CR₄, O, S, N, and NR₄; and when Z₁ is N, at least one of Z₃, Z₄ and Z₆ is N; or when Z₁ is N and all of Z₃, Z₄ and Z₅ are CR₄, A₁ is CR₁;
R₁ is selected from a group consisting of hydrogen, halogen, an optionally substituted alkyl, an optionally substituted alkoxy and an optionally substituted carbocyclic group;
R₄ and R₅ each are independently selected from a group consisting of hydrogen, halogen and an optionally substituted alkyl;
R₆ is selected from an optionally substituted aryl and an optionally substituted heteroaryl;
n and m each are independently selected from a group consisting of 0, 1, 2 and 3; and 2≤n+m≤4;
When m is 0 and n is 3, W is not NH.

In one or more embodiments of the compound of Formula IIa, W is CH₂, O or N-C₁₋₃ alkyl, preferably O, CH₂ or N-CH₃.

In one or more embodiments of the compound of Formula IIb, Z₃, Z₄ and Z₆ are independently selected from a group consisting of CR₄, O, Sand N; and when Z₁ is N, at least one of Z₃, Z₄ and Z₅ is N; or when Z₁ is N and all of Z₃, Z₄ and Z₅ are CR₄, A₁ is CR₁.

In one or more embodiments of the compound of Formula IIb, the Z ring is selected from the following groups: 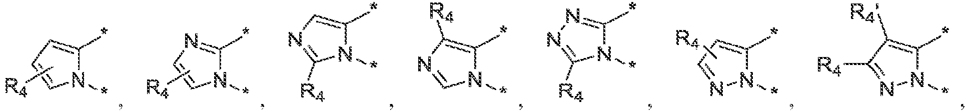 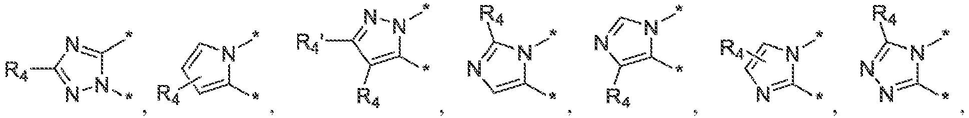 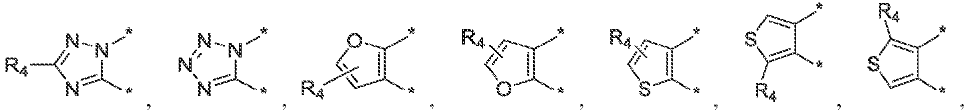 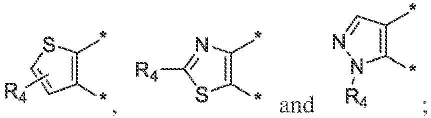 preferably the Z ring is selected from following groups: 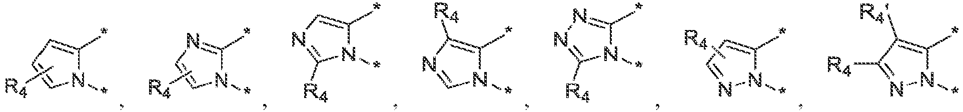 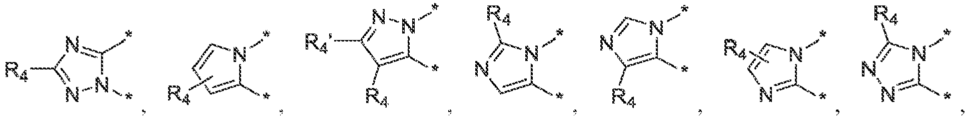 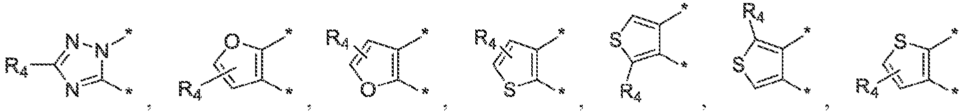 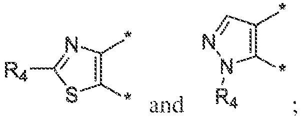 more preferably the Z ring is selected from following groups: 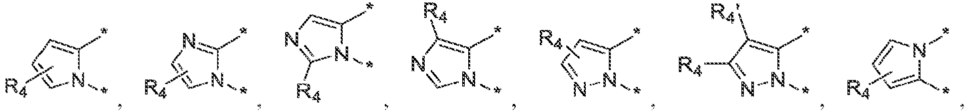 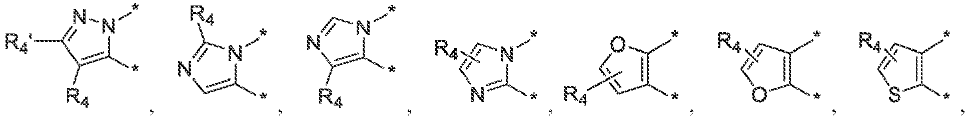 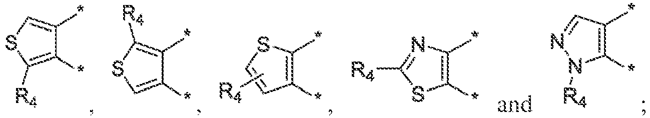 wherein * indicates the position at which the Z ring is attached to the rest of the compound; each R₄ is independently selected from hydrogen, halogen and an optionally substituted alkyl, preferably hydrogen, halogen and an optionally substituted C₁₋₃ alkyl; each R₄' is independently selected from a group consisting of hydrogen, halogen and an optionally substituted alkyl, preferably hydrogen, halogen and an optionally substituted C₁₋₃ alkyl. In some embodiments, each R₄ is independently selected from a group consisting of hydrogen and an optionally substituted alkyl, preferably hydrogen and an optionally substituted C₁₋₃ alkyl.

In one or more embodiments of the compound of Formula II (including Formulae IIa and IIb), the Z ring is selected from the following groups: 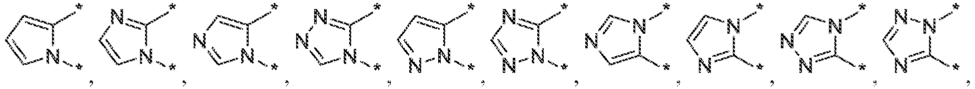 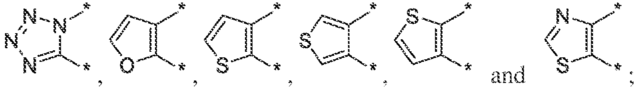 more preferably, the Z ring is selected from the following groups: 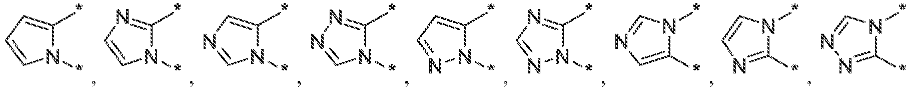 and

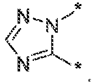

In one or more embodiments of the compound of Formula II (including Formulae IIa and IIb), A₁, A₂ and A₃ are each independently selected from N and CR₁, wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. In some embodiments, only one of A₁, A₂ and A₃ is N, and the other two are independently CR₁, preferably, R₁ is independently H, C₁₋₃ alkyl or halogen. In more preferred embodiment, A₃ is CH, one of A₁ and A₂ is N and the other is CR₁, wherein, R₁ is H, C₁₋₃ alkyl or halogen. In some embodiments, A₁ is N, both of A₂ and A₃ are CH. In some embodiments, A₂ is N, both A₁ and A₃ are CH. In some embodiments, all of A₁, A₂ and A₃ are CR₁, each R₁ is independently H, C₁₋₃ alkyl or halogen. Preferably, A₃ is CH, one of A₁ and A₂ is CR₁, wherein R₁ is halogen; more preferably, both of A₂ and A₃ are CH, A₁ is CR₁, wherein R₁ is halogen.

In one or more embodiments of the compound of Formula II (including Formulae IIa and IIb), each R₁ is preferably independently hydrogen, halogen, an optionally substituted C₁₋₃ alkyl or an optionally substituted C₁₋₃ alkoxy. In some embodiments, R₁ is an optionally substituted C₁₋₃ alkyl. Preferably, when R₁ is substituted, the substituents can be 1-5 groups selected from halogen, hydroxy, and amino (-NR'R"), etc. wherein, R' and R" each are preferably independently H, an optionally substituted C₁₋₄ alkyl or an optionally substituted C₃₋₆ cycloalkyl. Preferably, R₁ is C₁₋₃ alkyl or halogenated C₁₋₃ alkyl. In some embodiments, R₁ is hydrogen, C₁₋₃ alkyl or halogen. In some embodiments, R₁ is hydrogen or halogen.

In one or more embodiments of the compound of Formula II (including Formulae IIa and IIb), when the Z ring is not 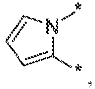 A₁, A₂ and A₃ are each independently selected from N and CR₁, wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. In preferred embodiments, only one of A₁, A₂ and A₃ is N, and the other two are independently CR₁, preferably, R₁ is independently H, C₁₋₃ alkyl or halogen. In more preferred embodiment, one of A₁ and A₂ is N and the other is CR₁, A₃ is CH, wherein, R₁ is H, C₁₋₃ alkyl or halogen. In some embodiments, A₁ is N, both of A₂ and A₃ are CH. In some embodiments, A₂ is N, both A₁ and A₃ are CH. In some embodiments, all of A₁, A₂ and A₃ are CR₁, each R₁ is independently H, Ci. ₃ alkyl or halogen. Preferably, A₃ is CH, one of A₁ and A₂ is CR₁, wherein, R₁ is C₁₋₃ alkyl or halogen; more preferably, both of A₂ and A₃ are CH, A₁ is CR₁, wherein, R₁ is C₁₋₃ alkyl or halogen. Preferably, A₂ is CH, one of A₁ and A₃ is CR₁, wherein, R₁ is C₁₋₃ alkyl or halogen; more preferably, both of A₁ and A₂ are CH, A₃ is CR₁, wherein, R₁ is C₁₋₃ alkyl or halogen.

In one or more embodiments of the compound of Formula IIb, when the Z ring is 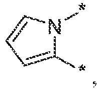 A₁ is CR₁, A₂ and A₃ each are independently N or CR₁, wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. Preferably, A₁, A₂ and A₃ each are independently CR₁, wherein R₁ is independently H, halogen or C₁₋₃ alkyl. In some embodiments, A₁ is CR₁, both of A₂ and A₃ are CH, wherein, R₁ is C₁₋₃ alkyl or halogen. In some embodiments, A₂ is CR₁, both A₁ and A₃ are CH, wherein, R₁ is C₁₋₃ alkyl or halogen. In some embodiments, A₃ is CR₁, both A₁ and A₂ are CH, wherein, R₁ is C₁₋₃ alkyl or halogen. In some embodiments, all of A₁, A₂ and A₃ are CH. Preferably, A₁ is CR₁, both of A₂ and A₃ are CH; or both of A₁ and A₂ are CH, A₃ is CR₁, wherein, R₁ is C₁₋₃ alkyl or halogen.

In one or more embodiments of the compound of Formula II (including Formulae IIa and IIb), R₆ is an optionally substituted 6-14 membered aryl or an optionally substituted 5-10 membered heteroaryl. An exemplary 6-14 membered aryl group is phenyl. The said 5-10 membered heteroaryl is preferably a 5-10 membered nitrogen-containing heteroaryl, including but is not limited to pyridyl, pyrazinyl, pyrrolyl, imidazolyl, pyrazolyl, pyrimidinyl, pyridazinyl and indolizinyl, preferably pyridyl, pyrimidinyl and pyridazinyl. Preferably, when R₆ is substituted, the substituents can be 1-5 groups selected from a group consisting of halogen, an optionally substituted alkyl (such as C₁₋₄ alkyl and halogenated C₁₋₄ alkyl), an optionally substituted alkoxy (such as C₁₋₄ alkoxy and halogenated C₁₋₄ alkoxy), aminoacyl (-C(O)-NR'R") and carboxyl. More preferably, R₆ is substituted with at least one aminoacyl group, preferably, R₆ is substituted in the para position with an optionally substituted aminoacyl group. Preferably, the said optionally substituted aminoacyl is -C(O)-NR'R", wherein the R' and R" each are preferably independently H, an optionally substituted C₁₋₁₀ alkyl, an optionally substituted C₃₋₈ cycloalkyl, an optionally substituted aryl or an optionally substituted heteroaryl, preferably H, an optionally substituted C₁₋₄ alkyl or an optionally substituted C₃₋₆ cycloalkyl. Preferably, when the said R' and R" are substituted, the substituents can be 1-5 groups selected from a group consisting of halogen, hydroxy, oxygen and amino. Preferably, the number of carbon atoms of the optionally substituted alkyl and optionally substituted alkoxy is 1-4, preferably, the substituents can be 1-5 groups selected from a group consisting of halogen, hydroxyl, oxygen and amino. In some preferred embodiments, the substituents on R₆ include at least aminoacyl (-C(O)-NR'R"), and optionally include any one or two groups of halogen, C₁₋₄ alkyl and halogenated C₁₋₄ alkyl.

In one or more embodiments of the compound of Formula II, R₆ is: 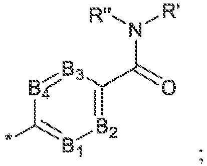 wherein, B₁, B₂, B₃ and B₄ are independently selected from a group consisting of N and CR₇; R₇ is selected from a group consisting of hydrogen, halogen, an optionally substituted alkyl, an optionally substituted alkoxy, an optionally substituted carbocyclic group, an optionally substituted alkenyl and an optionally substituted alkynyl; wherein R' and R" each are independently hydrogen, an optionally substituted C₁₋₁₀ alkyl, an optionally substituted C₃₋₈ cycloalkyl, an optionally substituted aryl or an optionally substituted heteroaryl. Preferably, R' and R" each are independently hydrogen, an optionally substituted C₁₋₄ alkyl (including deuterated C₁₋₄ alkyl), an optionally substituted C₃₋₆ cycloalkyl. * indicates the position at which the said group is attached to the rest of the compound. Preferably, the group containing B₁, B₂, B₃ and B₄ is phenyl, pyridyl, pyrimidinyl or pyridazinyl. Preferably, R₇ is H, halogen, C₁₋₃ alkyl, C₁₋₃ alkoxy or halogenated C₁₋₃ alkyl. Preferably, B₃ is N, B₄ is CR₇, both of B₁ and B₂ are CH, wherein R₇ is H, halogen, C₁₋₃ alkyl, C₁₋₃ alkoxy or halogenated C₁₋₃ alkyl. Preferably, when the said R' and R" are substituted, the substituents can be 1-5 groups selected from a group consisting of halogen, hydroxy and amino. Preferably, R' is hydrogen, R" is hydrogen, C₁₋₃ alkyl, C₃₋₆ cycloalkyl, halogenated C₁₋₃ alkyl, deuterated C₁₋₃ alkyl or hydroxy C₁₋₃ alkyl. In some embodiments, R' is hydrogen, R" is hydrogen, C₁₋₃ alkyl, deuterated C₁₋₃ alkyl, C₃₋₆ cycloalkyl, or halogenated C₁₋₃ alky.

Preferably, in the embodiments as described herein, the said R₇ is an optionally substituted alkyl, an optionally substituted alkoxy, an optionally substituted carbocyclic group, an optionally substituted alkenyl or an optionally substituted alkynyl, which can be independently substituted by 1-5 substituents selected from a group consisting of halogen, hydroxy and amino.

In one or more embodiments of the compound of Formula Ila, n is 1 or 2.

In one or more embodiments of the compound of Formula IIa, m is 1 or 2.

In one or more embodiments of the compound of Formula IIa, when m is 0, W is O or CH₂.

In one or more embodiments of the compound of Formula IIa, W is selected from O, CH₂ and N-CH₃; A₁, A₂ and A₃ each are independently selected from N and CR₁; R₆ is: 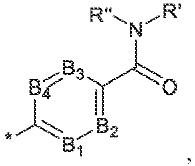 wherein, B₁, B₂, B₃ and B₄ are independently selected from a group consisting of N and CR₇; R' and R" each are independently hydrogen, an optionally substituted C₁₋₁₀ alkyl, an optionally substituted C₃₋₈ cycloalkyl, an optionally substituted aryl or an optionally substituted heteroaryl. Preferably, R' and R" each are independently hydrogen, an optionally substituted C₁₋₄ alkyl, an optionally substituted C₃₋₆ cycloalkyl. R₁ is selected from hydrogen, halogen, an optionally substituted alkyl, an optionally substituted alkoxy and an optionally substituted carbocyclic group, preferably H, halogen and C₁₋₃ alkyl. R₇ is selected from a group consisting of hydrogen, halogen, an optionally substituted alkyl, an optionally substituted alkoxy, an optionally substituted carbocyclic group, an optionally substituted alkenyl and an optionally substituted alkynyl; preferably hydrogen, C₁₋₃ alkyl, halogenated C₁₋₃ alkyl or halogen. n is 1 or 2. Preferably, A₁, A₂ and A₃ each are independently selected from N and CRi; wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. In some embodiments, only one of A₁, A₂ and A₃ is N, and the other two are independently CR₁, preferably, R₁ is independently H, C₁₋₃ alkyl or halogen. In some embodiments, one of A₁ and A₂ is N and the other is CRi, A₃ is CH, wherein, R₁ is H, C₁₋₃ alkyl or halogen. In some embodiments, A₁ is N, both of A₂ and A₃ are CH. In some embodiments, A₂ is N, both of A₁ and A₃ are CH. In some embodiments, all of A₁, A₂ and A₃ are CR₁, each R₁ is independently H, C₁₋₃ alkyl or halogen. Preferably, A₃ is CH, one of A₁ and A₂ is CR₁, wherein, R₁ is C₁₋₃ alkyl or halogen; more preferably, both of A₂ and A₃ are CH, A₁ is CR₁, wherein, R₁ is C₁₋₃ alkyl or halogen. Preferably, A₂ is CH, one of A₁ and A₃ is CR₁, wherein, R₁ is C₁₋₃ alkyl or halogen; more preferably, both of A₁ and A₂ are CH, A₃ is CR₁, wherein, R₁ is C₁₋₃ alkyl or halogen. In some embodiments, A₂ is CR₁, both A₁ and A₃ are CH, wherein, R₁ is C₁₋₃ alkyl or halogen. In some embodiments, A₃ is CR₁, both A₁ and A₂ are CH, wherein, R₁ is C₁₋₃ alkyl or halogen. In some embodiments, all of A₁, A₂ and A₃ are CH. Preferably, A₁ is CR₁, both of A₂ and A₃ are CH; or both of A₁ and A₂ are CH, A₃ is CR₁, wherein, R₁ is C₁₋₃ alkyl or halogen. Preferably, B₁, B₂, B₃ and B₄ are independently selected from a group consisting of N and CR₇; wherein R₇ is preferably hydrogen, halogen, an optionally substituted C₁₋₃ alkyl or an optionally substituted C₁₋₃ alkoxy, more preferably, R₇ is hydrogen, C₁₋₃ alkyl, halogenated C₁₋₃ alkyl or halogen. Preferably, both of B₁ and B₂ are CH, B₃ is N, B₄ is CR₇, wherein R₇ is H, halogen, an optionally substituted C₁₋₃ alkyl or an optionally substituted C₁₋₃ alkoxy, more preferably, R₇ is hydrogen, C₁₋₃ alkyl, halogenated C₁₋₃ alkyl or halogen. Preferably, R' and R" each are independently hydrogen, an optionally substituted C₁₋₄ alkyl, an optionally substituted C₃₋₆ cycloalkyl. Preferably, when the said R' and R" are substituted, the substituents can be 1-5 groups selected from a group consisting of halogen, hydroxy and amino. Preferably, R' is hydrogen, R" is hydrogen, C₁₋₃ alkyl, C₃₋₆ cycloalkyl, halogenated C₁₋₃ alkyl, deuterated C₁₋₃ alkyl or hydroxy C₁₋₃ alkyl. Preferably, R₇ is hydrogen, halogen or an optionally substituted C₁₋₃ alkyl. Preferably, R₇ is hydrogen, halogen, C₁₋₃ alkyl or halogenated C₁₋₃ alkyl. Preferably, n is 1 or 2; m is 1 or 2. More preferably, n is 1; m is 1 or 2.

In one or more embodiments of the compound of Formula IIb, the Z ring is selected from: 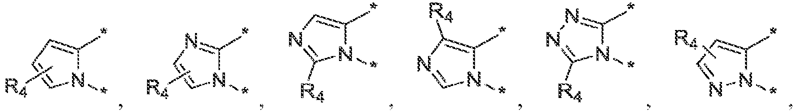 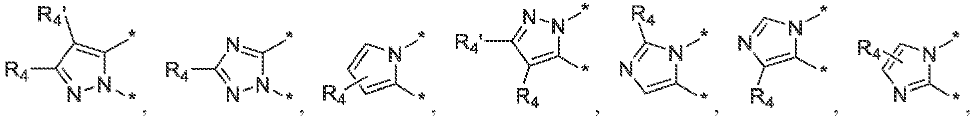 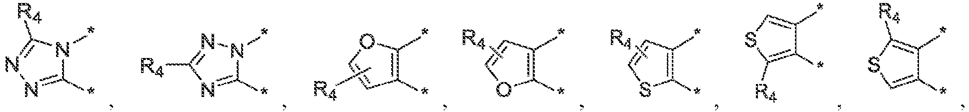 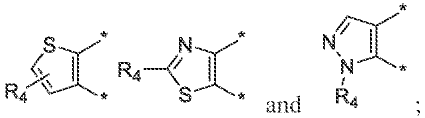 A₁, A₂ and A₃ each are independently N or CRi; wherein each R₄ is independently selected from a group consisting of hydrogen, halogen and an optionally substituted alkyl, preferably hydrogen, halogen and an optionally substituted C₁₋₃ alkyl; each R₄' is independently selected from a group consisting of hydrogen, halogen and an optionally substituted alkyl, preferably hydrogen, halogen and an optionally substituted C₁₋₃ alkyl. In some embodiments, each R₄ is independently selected from a group consisting of hydrogen and an optionally substituted alkyl, preferably hydrogen and an optionally substituted C₁₋₃ alkyl; preferably, the Z ring is selected from: 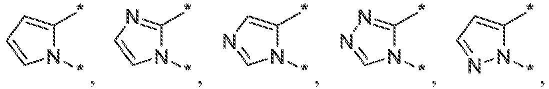 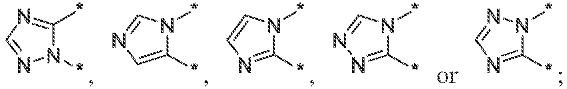
or the Z ring is 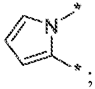 A₁ is selected from CRi; A₂ and A₃ are independently N or CRi;
R₆ is selected from the following groups: 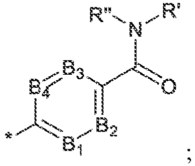 wherein, B₁, B₂, B₃ and B₄ are independently selected from a group consisting of N and CR₇; R' and R" each are independently hydrogen, an optionally substituted C₁₋₁₀ alkyl, an optionally substituted C₃₋₈ cycloalkyl, an optionally substituted aryl or an optionally substituted heteroaryl. Preferably, R' and R" each are independently hydrogen, an optionally substituted C₁₋₄ alkyl, an optionally substituted C₃₋₆ cycloalkyl. R₁ is selected from a group consisting of hydrogen, halogen, an optionally substituted alkyl, an optionally substituted alkoxy and an optionally substituted carbocyclic group. R₇ is selected from a group consisting of hydrogen, halogen, an optionally substituted alkyl, an optionally substituted alkoxy, an optionally substituted carbocyclic group, an optionally substituted alkenyl and an optionally substituted alkynyl. Preferably, A₁, A₂ and A₃ each are independently selected from N and CR₁; wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. Preferably, only one of A₁, A₂ and A₃ is N, and the other two are independently CR₁, preferably, R₁ is independently H, C₁₋₃ alkyl or halogen. Preferably, one of A₁ and A₂ is N and the other is CR₁, A₃ is CH, wherein, R₁ is H, C₁₋₃ alkyl or halogen. Preferably, A₁ is N, and both of A₂ and A₃ are CH. In some embodiments, A₂ is N, and both of A₁ and A₃ are CH. In some embodiments, all of A₁, A₂ and A₃ are CR₁, each R₁ is independently H, C₁₋₃ alkyl or halogen. Preferably, A₃ is CH, one of A₁ and A₂ is CR₁, wherein, R₁ is C₁₋₃ alkyl or halogen; more preferably, both of A₂ and A₃ are CH, A₁ is CR₁, wherein, R₁ is C₁₋₃ alkyl or halogen. Preferably, A₂ is CH, one of A₁ and A₃ is CR₁, wherein, R₁ is C₁₋₃ alkyl or halogen; more preferably, both of A₁ and A₂ are CH, A₃ is CR₁, wherein, R₁ is C₁₋₃ alkyl or halogen. Preferably, B₁, B₂, B₃ and B₄ are independently selected from a group consisting of N and CR₇; wherein R₇ is preferably hydrogen, halogen, an optionally substituted C₁₋₃ alkyl or an optionally substituted C₁₋₃ alkoxy, more preferably, R₇ is hydrogen, C₁₋₃ alkyl, halogenated C₁₋₃ alkyl or halogen. Preferably, both of B₁ and B₂ are CH, B₃ is N, B₄ is CR₇, wherein R₇ is H, halogen, an optionally substituted C₁₋₃ alkyl or an optionally substituted C₁₋₃ alkoxy, more preferably, R₇ is hydrogen, C₁₋₃ alkyl, halogenated C₁₋₃ alkyl or halogen. Preferably, R' and R" each are independently hydrogen, an optionally substituted C₁₋₃ alkyl, an optionally substituted C₃₋₆ cycloalkyl. Preferably, when the said R' and R" are substituted, the substituents can be 1-5 groups selected from a group consisting of halogen, hydroxy and amino. Preferably, R' is hydrogen, R" is hydrogen, C₁₋₃ alkyl, C₃₋₆ cycloalkyl, halogenated C₁₋₃ alkyl, deuterated C₁₋₃ alkyl, or hydroxy C₁₋₃ alkyl. Preferably, R₇ is hydrogen, halogen, C₁₋₃ alkyl, or halogenated C₁₋₃ alkyl.

In one or more embodiments of the compound of Formula II, A₁, A₂ and A₃ are each independently CRi; each R₁ is independently H, C₁₋₃ alkyl or halogen; R₆ is pyridyl substituted with -C(O)-NR'R" and optionally further substituted by one or two substituents selected from a group consisting of halogen, C₁₋₄ alkyl and halogenated C₁₋₄ alkyl; R' and R" each are independently H, C₁₋₄ alkyl optionally substituted by hydroxy, or C₃₋₈ cycloalkyl.

In one or more embodiments of the compound of Formula II, A₁, A₂ and A₃ are each independently CR₁; each R₁ is independently H, C₁₋₃ alkyl or halogen; R₆ is pyridyl substituted with -C(O)-NR'R" and optionally further substituted by one or two substituents selected from a group consisting of halogen and C₁₋₄ alkyl; R' and R" each are independently H, C₁₋₄ alkyl optionally substituted by hydroxy, or C₃₋₈ cycloalkyl.

One group of preferred compounds of Formula I in this disclosure is represented by compounds of Formula III (including Formulae IIIa and IIIb): 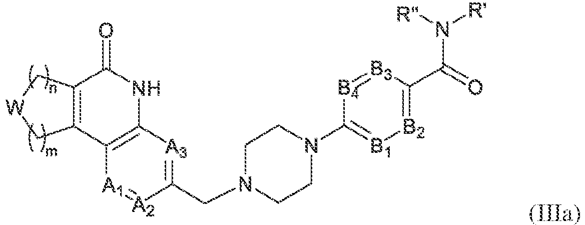 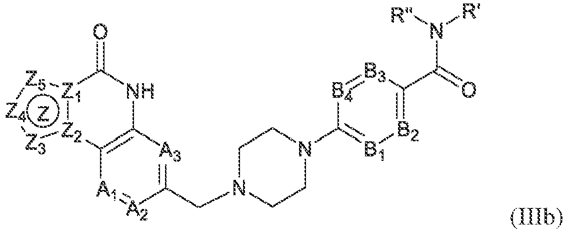 or stereoisomers, tautomers, N-oxides, hydrates, solvates, isotope-substituted derivatives, or pharmaceutically acceptable salts thereof, or mixtures thereof, or prodrugs thereof, wherein:
W, Z₁, Z₂, Z₃, Z₄, Z₅, A₁, A₂, A₃, B₁, B₂, B₃, B₄, n and m are as defined in any of the foregoing embodiments;
R' and R" each are independently H, an optionally substituted C₁₋₁₀ alkyl, an optionally substituted cycloalkyl, an optionally substituted aryl or an optionally substituted heteroaryl; or
B₃ and R" together with the attached aminoacyl group form a 6-membered heterocyclic group.

In one or more embodiments of the compound of Formula IIIa, W is O, CH₂ or N-C₁₋₃ alkyl, preferably O, CH₂ or N-CH₃.

In one or more embodiments of the compound of Formula IIIb, the Z ring is selected from the following groups: 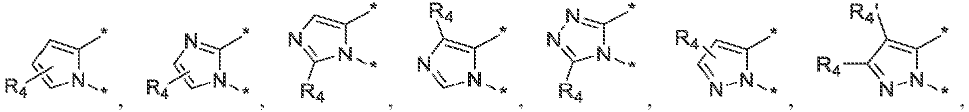 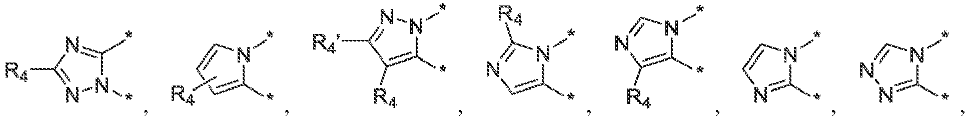 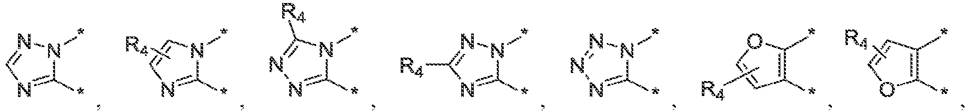 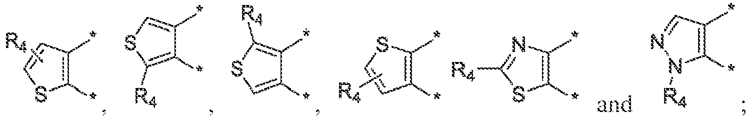 preferably the Z ring is selected from following groups: 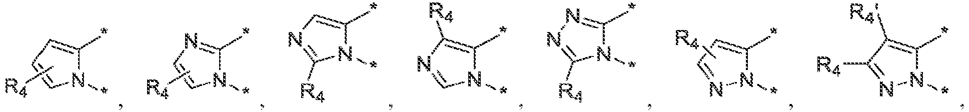 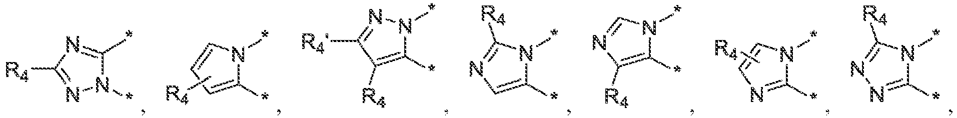 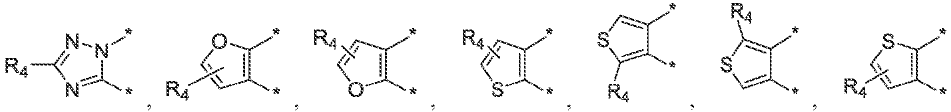 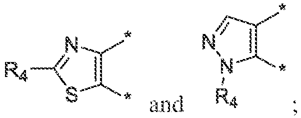 more preferably the Z ring is selected from following groups: 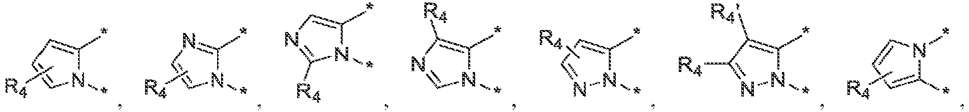 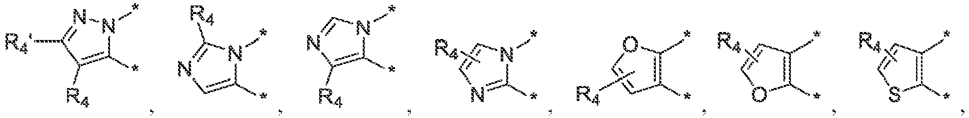 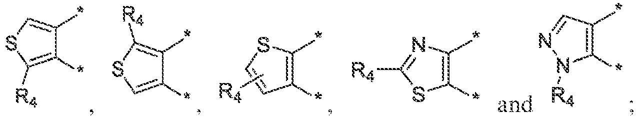 wherein each R₄ is independently selected from a group consisting of hydrogen, halogen and an optionally substituted alkyl, preferably hydrogen, halogen and an optionally substituted C₁₋₃ alkyl; each R₄' is independently selected from a group consisting of hydrogen, halogen and an optionally substituted alkyl, preferably hydrogen, halogen and an optionally substituted C₁₋₃ alkyl. In some embodiments, each R₄ is independently selected from a group consisting of hydrogen and an optionally substituted alkyl, preferably hydrogen and an optionally substituted C₁₋₃ alkyl.

In one or more embodiments of the compound of Formula III (including Formulae IIIa and IIIb), the Z ring is selected from the following groups: 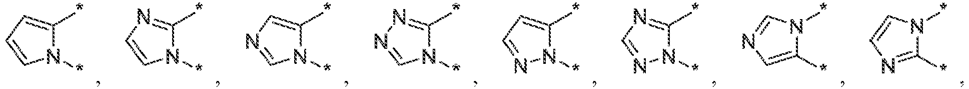 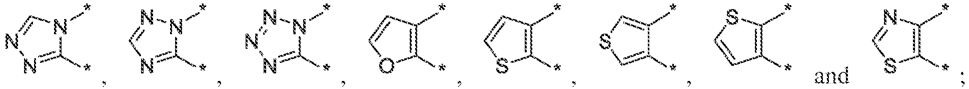 preferably from the following groups:

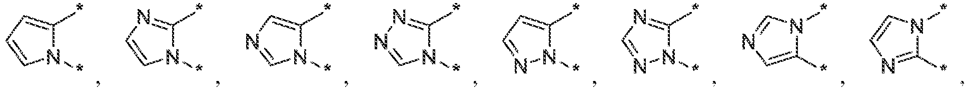

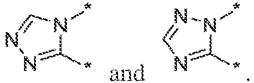

In one or more embodiments of the compound of Formula III (including Formulae IIIa and IIIb), A₁, A₂ and A₃ are each independently selected from N and CR₁, wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. In some embodiments, only one of A₁, A₂ and A₃ is N, and the other two are independently CR₁, preferably, R₁ is independently H, C₁₋₃ alkyl or halogen. In more preferred embodiment, A₃ is CH, one of A₁ and A₂ is N and the other is CR₁, wherein, R₁ is H, C₁₋₃ alkyl or halogen. In some embodiments, A₁ is N, both of A₂ and A₃ are CH. In some embodiments, A₂ is N, both A₁ and A₃ are CH. In some embodiments, all of A₁, A₂ and A₃ are CR₁, each R₁ is independently H, C₁₋₃ alkyl or halogen. Preferably, A₃ is CH, one of A₁ and A₂ is CR₁, wherein R₁ is halogen; more preferably, both of A₂ and A₃ are CH, A₁ is CR₁, wherein R₁ is halogen.

In one or more embodiments of the compound of Formula III (including Formulae IIIa and IIIb), when the Z ring is not 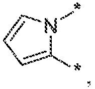 A₁, A₂ and A₃ each are independently selected from N and CR₁, wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. In preferred embodiments, only one of A₁, A₂ and A₃ is N, and the other two are independently CR₁, preferably, R₁ is independently H, C₁₋₃ alkyl or halogen. In more preferred embodiment, one of A₁ and A₂ is N and the other is CR₁, A₃ is CH, wherein, R₁ is H, C₁₋₃ alkyl or halogen. In some embodiments, A₁ is N, and both of A₂ and A₃ are CH. In some embodiments, A₂ is N and both of A₁ and A₃ are CH. In some embodiments, all of A₁, A₂ and A₃ are CR₁, each R₁ is independently H, C₁₋₃ alkyl or halogen. Preferably, A₃ is CH, one of A₁ and A₂ is CR₁, wherein, R₁ is C₁₋₃ alkyl or halogen; more preferably, both of A₂ and A₃ are CH, A₁ is CR₁, wherein, R₁ is C₁₋₃ alkyl or halogen. Preferably, A₂ is CH, one of A₁ and A₃ is CR₁, wherein, R₁ is C₁₋₃ alkyl or halogen; more preferably, both of A₁ and A₂ are CH, A₃ is CR₁, wherein, R₁ is C₁₋₃ alkyl or halogen. In some embodiments, A₁ is CR₁, both of A₂ and A₃ are CH, wherein, R₁ is C₁₋₃ alkyl or halogen. In some embodiments, A₂ is CR₁, both A₁ and A₃ are CH, wherein, R₁ is C₁₋₃ alkyl or halogen. In some embodiments, A₃ is CR₁, both A₁ and A₂ are CH, wherein, R₁ is C₁₋₃ alkyl or halogen. In some embodiments, all of A₁, A₂ and A₃ are CH. Preferably, A₁ is CR₁, both of A₂ and A₃ are CH; or both of A₁ and A₂ are CH, A₃ is CR₁, wherein, R₁ is C₁₋₃ alkyl or halogen.

In one or more embodiments of the compound of Formula IIIb, when the Z ring is 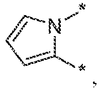 A₁ is CR₁, A₂ and A₃ each are independently N or CR₁, wherein R₁ is preferably hydrogen, halogen, an optionally substituted C₁₋₃ alkyl or an optionally substituted C₁₋₃ alkoxy, more preferably, R₁ is hydrogen, C₁₋₃ alkyl or halogen. Preferably, A₁, A₂ and A₃ each are independently CR₁, R₁ is independently H, halogen or C₁₋₃ alkyl. In some embodiments, A₁ is CR₁, both of A₂ and A₃ are CH, wherein, R₁ is C₁₋₃ alkyl or halogen. In some embodiments, A₂ is CR₁, both A₁ and A₃ are CH, wherein, R₁ is C₁₋₃ alkyl or halogen. In some embodiments, A₃ is CR₁, both A₁ and A₂ are CH, wherein, R₁ is C₁₋₃ alkyl or halogen. In some embodiments, all of A₁, A₂ and A₃ are CH. Preferably, A₁ is CR₁, both of A₂ and A₃ are CH; or both of A₁ and A₂ are CH, A₃ is CR₁, wherein, R₁ is C₁₋₃ alkyl or halogen.

In one or more embodiments of the compound of Formula III (including Formulae IIIa and IIIb), B₁, B₂, B₃ and B₄ are independently selected from a group consisting of N and CR₇; wherein R₇ is preferably selected from a group consisting of hydrogen, halogen, an optionally substituted C₁₋₃ alkyl, an optionally substituted C₁₋₃ alkoxy, more preferably, R₇ is H, C₁₋₃ alkyl, C₁₋₃ alkoxy, halogenated C₁₋₃ alkyl or halogen. In some preferred embodiments, both of B₁ and B₂ are CH, B₃ is N, B₄ is CR₇, wherein R₇ is preferably hydrogen, halogen, an optionally substituted C₁₋₃ alkyl or an optionally substituted C₁₋₃ alkoxy, more preferably, R₇ is hydrogen, C₁₋₃ alkyl, halogenated C₁₋₃ alkyl or halogen.

In one or more embodiments of the compound of Formula III, R' and R" each are independently hydrogen, an optionally substituted C₁₋₄ alkyl or an optionally substituted C₃₋₆ cycloalkyl; Preferably, R' is hydrogen, R" is hydrogen, C₁₋₃ alkyl, halogenated C₁₋₃ alkyl, deuterated C₁₋₃ alkyl or hydroxy C₁₋₃ alkyl.

In one or more embodiments of the compound of Formula IIIa, n is 1 or 2.

In one or more embodiments of the compound of Formula IIIa, m is 1 or 2.

In one or more embodiments of the compound of Formula IIIa, when m is 0, W is O or CH₂.

In some preferred embodiments, in the said -C(O)-NR'R" described herein, R' and R" each are independently H, C₁₋₄ alkyl or C₃₋₆ cycloalkyl. In further preferred embodiments, R' is hydrogen and R" is hydrogen or C₁₋₃ alkyl.

One group of preferred compounds of Formula I in this disclosure is represented by compounds of Formula IV (including Formulae IVa, IVb and IVc): 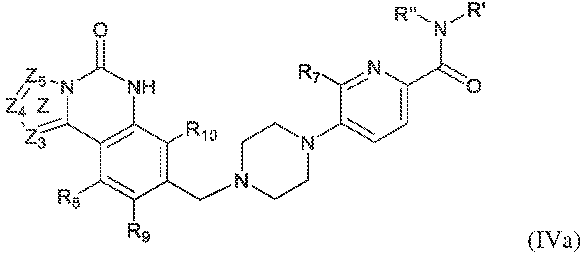 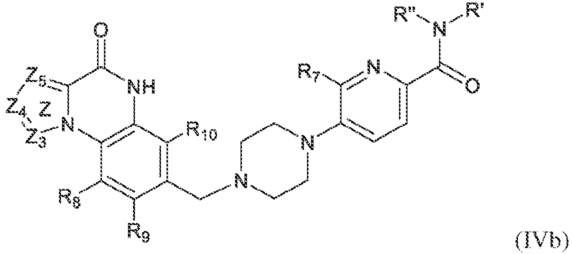 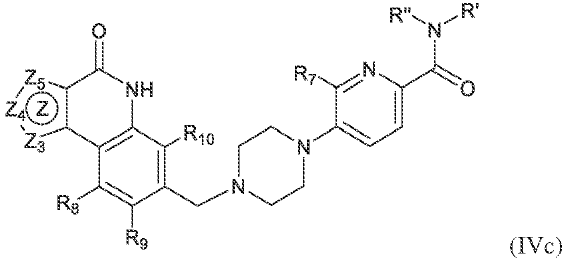 or stereoisomers, tautomers, N-oxides, hydrates, solvates, isotope-substituted derivatives, or pharmaceutically acceptable salts thereof, or mixtures thereof, or prodrugs thereof, wherein:
Z₃, Z₄, Z₅, R₇, R' and R" are as defined in any of the foregoing embodiments;
R₈, R₉ and R₁₀ each are independently selected from a group consisting of hydrogen, halogen, an optionally substituted alkyl, and an optionally substituted alkoxy.

In one or more embodiments of the compound of Formula IV, the 5-membered ring containing Z₃, Z₄ and Z₅ is selected from the following groups: 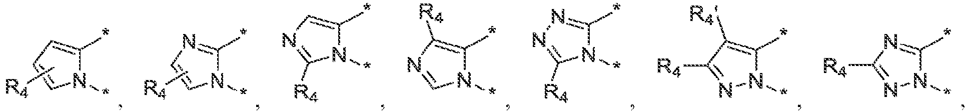 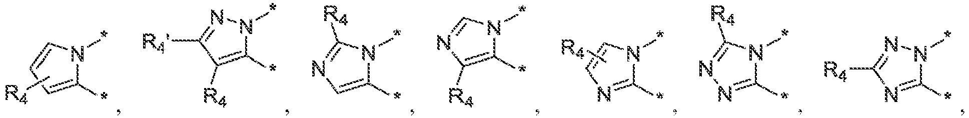 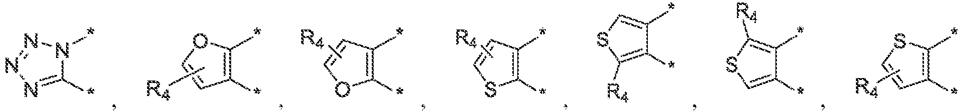 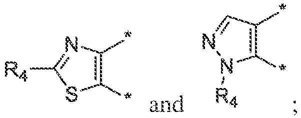 preferably the Z ring is selected from following groups: 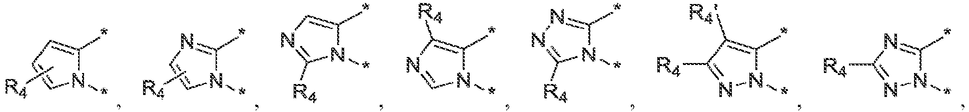 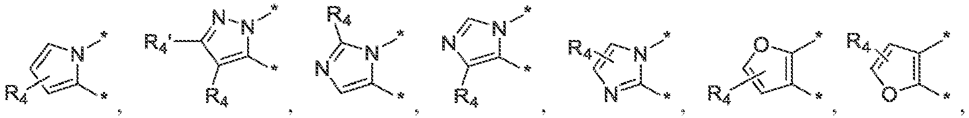 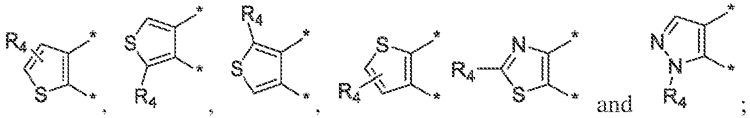 wherein each R₄ is independently selected from a group consisting of hydrogen, halogen and an optionally substituted alkyl, preferably hydrogen, halogen and an optionally substituted C₁₋₃ alkyl; each R₄' is independently selected from a group consisting of hydrogen, halogen and an optionally substituted alkyl, preferably hydrogen, halogen and an optionally substituted C₁₋₃ alkyl. In some embodiments, each R₄ is independently selected from a group consisting of hydrogen and an optionally substituted alkyl, preferably hydrogen and an optionally substituted C₁₋₃ alkyl.

In one or more embodiments of the compound of Formula IV (including Formulae IVa, IVb and IVc), R₈, R₉ and R₁₀ each are preferably hydrogen, halogen, an optionally substituted C₁₋₃ alkyl or an optionally substituted C₁₋₃ alkoxy, more preferably, R₈, R₉ and R₁₀ each are hydrogen, C₁₋₃ alkyl or halogen. In some embodiments, R₈ is C₁₋₃ alkyl or halogen, and both of R₉ and R₁₀ are H. In some embodiments, R₉ is C₁₋₃ alkyl or halogen, and both of R₈ and R₁₀ are H. In some embodiments, both of R₈ and R₉ are H, and R₁₀ is C₁₋₃ alkyl or halogen. In some embodiments, all of R₈, R₉ and R₁₀ are H. Preferably, R₈ is C₁₋₃ alkyl or halogen, both of R₉ and R₁₀ are H; or both of R₈ and R₉ are H, R₁₀ is C₁₋₃ alkyl or halogen.

In one or more embodiments of the compound of Formula IV (including Formulae IVa, IVb and IVc), R₇ is selected from a group consisting of hydrogen, halogen, an optionally substituted C₁₋₃ alkyl, and an optionally substituted C₁₋₃ alkoxy, preferably, R₇ is H, C₁₋₃ alkyl, C₁₃ alkoxy, halogenated C₁₋₃ alkyl or halogen. More preferably, R₇ is H, C₁₋₃ alkyl or halogen.

In one or more embodiments of the compound of Formula IV, R' and R" each are independently hydrogen, an optionally substituted C₁₋₄ alkyl or an optionally substituted C₃₋₆ cycloalkyl; Preferably, R' is hydrogen, R" is hydrogen, C₁₋₃ alkyl, halogenated C₁₋₃ alkyl, hydroxy C₁₋₃ alkyl or C₃₋₆ cycloalkyl. More preferably, R' is hydrogen, R" is hydrogen, C₁₋₃ alkyl, halogenated C₁₋₃ alkyl, or hydroxy C₁₋₃ alkyl.

In one or more embodiments of the compound of Formula IV, the 5-membered ring containing Z₃, Z₄ and Z₅ is selected from the following groups: 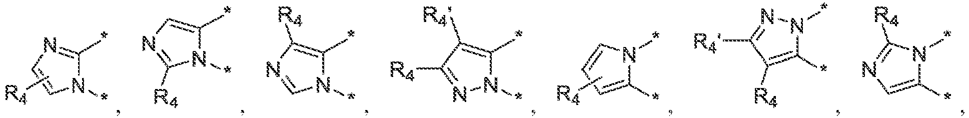 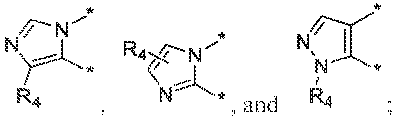 wherein each R₄ is independently selected from a group consisting of hydrogen and C₁₋₃ alkyl; each R₄' is independently selected from a group consisting of hydrogen and C₁₋₃ alkyl; preferably, the 5-membered ring containing Z₃, Z₄ and Z₅ is selected from the following groups: 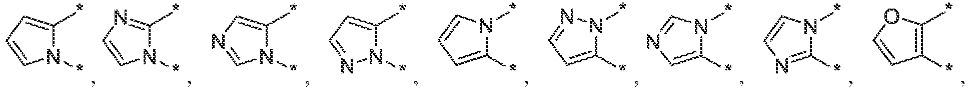 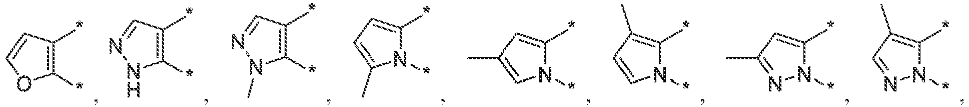 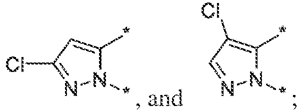
R₇ is H, C₁₋₃ alkyl or halogen;
R₈, R₉ and R₁₀ each are hydrogen, halogen or C₁₋₃ alkyl; preferably, R₈ is C₁₋₃ alkyl or halogen and both of R₉ and R₁₀ are H; or R₉ is C₁₋₃ alkyl or halogen and both of R₈ and R₁₀ are H; or both of R₈ and R₉ are H and R₁₀ is C₁₋₃ alkyl or halogen; or all of R₈, R₉ and R₁₀ are H; and
R' and R" are each independently H or C₁₋₃ alkyl or C₃₋₆ cycloalkyl.

It should be understood that although W, Z₁, Z₂, Z₃, Z₄, Z₅, A₁, A₂, A₃, L, Cy, R₆, B₁, B₂, B₃, B₄, R₇, R₈, R₉, R₁₀, R', R", n and m are described separately above, the described features, especially the preferred features, can be arbitrarily combined to form the scope of different compounds of Formula I (including Formulae II, III and IV) in this disclosure. For example, in some embodiments of compounds of Formula I (including Formulae II, III and IV) of this disclosure.

The preferred compounds of Formula I include, without limitation:
7-((4-(6-(methylcarbamoyl)pyridin-3-yl)piperazin-1-yl)methyl)-3,5-dihydrofuro[3,4-c]quinolin-4(1H)-one (Example 1);
7-((4-(2-fluoro-6-(methylcarbamoyl)pyridin-3-yl)piperazin-1-yl)methyl)-3,5-dihydrofuro[3,4-c]quinolin-4(1H)-one (Example 2);
7-((4-(2-chloro-6-(methylcarbamoyl)pyridin-3-yl)piperazin-1-yl)methyl)-3,5-dihydrofuro[3,4-c]quinolin-4(1H)-one (Example 3);
7-((4-(2-methyl-6-(methylcarbamoyl)pyridin-3-yl)piperazin-1-yl)methyl)-3,5-dihydrofuro[3,4-c]quinolin-4(1H)-one (Example 4);
7-((4-(2-trifluoromethyl-6-(methylcarbamoyl)pyridin-3-yl)piperazin-1-yl)methyl)-3,5-dihydrofuro[3,4-c]quinolin-4(1H)-one (Example 5);
7-((4-(2-chloro-6-(ethylcarbamoyl)pyridin-3-yl)piperazin-1-yl)methyl)-3,5-dihydrofuro[3,4-c]quinolin-4(1H)-one (Example 6);
7-((4-(2-methyl-6-(ethylcarbamoyl)pyridin-3-yl)piperazin-1-yl)methyl)-3,5-dihydrofuro[3,4-c]quinolin-4(1H)-one (Example 7);
7-((4-(2-trifluoromethyl-6-(ethylcarbamoyl)pyridin-3-yl)piperazin-1-yl)methyl)-3,5-dihydrofuro[3,4-c]quinolin-4(1H)-one (Example 8);
7-((4-(2-chloro-6-(methylcarbamoyl)pyridin-3-yl)piperazin-1-yl)methyl)-9-fluoro-3,5-dihydrofuro[3,4-c]quinolin-4(1H)-one (Example 9);
7-((4-(2-methyl-6-(methylcarbamoyl)pyridin-3-yl)piperazin-1-yl)methyl)-9-fluoro-3,5-dihydrofuro[3,4-c]quinolin-4(1H)-one (Example 10);
7-((4-(2-trifluoromethyl-6-(methylcarbamoyl)pyridin-3-yl)piperazin-1-yl)methyl)-9-fluoro-3,5-dihydrofuro[3,4-c]quinolin-4(1H)-one (Example 11);
7-((4-(2-chloro-6-(ethylcarbamoyl)pyridin-3-yl)piperazin-1-yl)methyl)-9-fluoro-3,5-dihydrofuro[3,4-c]quinolin-4(1H)-one (Example 12);
7-((4-(2-methyl-6-(ethylcarbamoyl)pyridin-3-yl)piperazin-1-yl)methyl)-9-fluoro-3,5-dihydrofuro[3,4-c]quinolin-4(1H)-one (Example 13);
7-((4-(2-trifluoromethyl-6-(ethylcarbamoyl)pyridin-3-yl)piperazin-1-yl)methyl)-9-fluoro-3,5-dihydrofuro[3,4-c]quinolin-4(1H)-one (Example 14);
3-((4-(2-methyl-6-(methylcarbamoyl)pyridin-3-yl)piperazin-1-yl)methyl)-7,8,9,10-tetrahydrophenanthridin-6(5H)-one (Example 15);
7-((4-(2-chloro-6-(methylcarbamoyl)pyridin-3-yl)piperazin-1-yl)methyl)-1,2,3,5-tetrahydro-4H-cyclopenta[c]quinolin-4-one (Example 16);
7-((4-(2-methyl-6-(methylcarbamoyl)pyridin-3-yl)piperazin-1-yl)methyl)-1,2,3,5-tetrahydro-4H-cyclopenta[c]quinolin-4-one (Example 17);
7-((4-(2-chloro-6-(methylcarbamoyl)pyridin-3-yl)piperazin-1-yl)methyl)-3,5-dihydrofuro[3,2-c]quinolin-4(2H)-one (Example 18);
7-((4-(2-methyl-6-(methylcarbamoyl)pyridin-3-yl)piperazin-1-yl)methyl)-3,5-dihydrofuro[3,2-c]quinolin-4(2H)-one (Example 19);
3-((4-(2-methyl-6-(methylcarbamoyl)pyridin-3-yl)piperazin-1-yl)methyl)-7,9-dihydrofuro[3,4-c][1,5]naphthyridin-6(5H)-one (Example 20);
7-((4-(2-chloro-6-(methylcarbamoyl)pyridin-3-yl)piperazin-1-yl)methyl)-2-methyl-1,2,3,5-tetrahydro-4H-pyrrolo[3,4-c]quinolin-4-one (Example 21);
8-((4-(2-chloro-6-(methylcarbamoyl)pyridin-3-yl)piperazin-1-yl)methyl)-1,2,4,6-tetrahydro-5H-pyrano[3,4-c]quinolin-5-one (Example 22);
8-((4-(2-methyl-6-(methylcarbamoyl)pyridin-3-yl)piperazin-1-yl)methyl)-1,2,4,6-tetrahydro-5H-pyrano[3,4-c]quinolin-5-one (Example 23);
7-((4-(2-methyl-6-(methylcarbamoyl)pyridin-3-yl)piperazin-1-yl)methyl)pyrrolo[1,2-a]quinoxalin-4(5H)-one (Example 24);
7-((4-(2-methyl-6-(methylcarbamoyl)pyridin-3-yl)piperazin-1-yl)methyl)imidazo[1,2-a]quinoxalin-4(5H)-one (Example 25);
7-((4-(2-methyl-6-(methylcarbamoyl)pyridin-3-yl)piperazin-1-yl)methyl)imidazo[1,5-a]quinoxalin-4(5H)-one (Example 26);
7-((4-(2-methyl-6-(methylcarbamoyl)pyridin-3-yl)piperazin-1-yl)methyl)-[1,2,4]triazolo[4,3-a]quinoxalin-4(5H)-one (Example 27);
7-((4-(2-methyl-6-(methylcarbamoyl)pyridin-3-yl)piperazin-1-yl)methyl)pyrazolo[1,5-a]quinoxalin-4(5H)-one (Example 28);
7-((4-(2-methyl-6-(methylcarbamoyl)pyridin-3-yl)piperazin-1-yl)methyl)-[1,2,4]triazolo[1,5-a]quinoxalin-4(5H)-one (Example 29);
8-((4-(2-methyl-6-(methylcarbamoyl)pyridin-3-yl)piperazin-1-yl)methyl)imidazo[1,5-c]quinazolin-5(6H)-one (Example 30);
8-((4-(2-methyl-6-(methylcarbamoyl)pyridin-3-yl)piperazin-1-yl)methyl)imidazo[1,2-c]quinazolin-5(6H)-one (Example 31);
8-((4-(2-methyl-6-(methylcarbamoyl)pyridin-3-yl)piperazin-1-yl)methyl)-[1,2,4]triazolo[4,3-c]quinazolin-5(6H)-one (Example 32);
8-((4-(2-methyl-6-(methylcarbamoyl)pyridin-3-yl)piperazin-1-yl)methyl)-[1,2,4]triazolo[1,5-c]quinazolin-5(6H)-one (Example 33);
7-((4-(2-fluoro-6-(methylcarbamoyl)pyridin-3-yl)piperazin-1-yl)methyl)pyrrolo[1,2-a]quinoxalin-4(5H)-one (Example 34);
7-((4-(2-chloro-6-(methylcarbamoyl)pyridin-3-yl)piperazin-1-yl)methyl)pyrrolo[1,2-a]quinoxalin-4(5H)-one (Example 35);
7-((4-(2-fluoro-6-(methylcarbamoyl)pyridin-3-yl)piperazin-1-yl)methyl)pyrazolo[1,5-a]quinoxalin-4(5H)-one (Example 36);
7-((4-(2-chloro-6-(methylcarbamoyl)pyridin-3-yl)piperazin-1-yl)methyl)pyrazolo[1,5-a]quinoxalin-4(5H)-one (Example 37);
7-((4-(2-fluoro-6-(methylcarbamoyl)pyridin-3-yl)piperazin-1-yl)methyl)imidazo[1,5-a]quinoxalin-4(5H)-one (Example 38);
7-((4-(2-chloro-6-(methylcarbamoyl)pyridin-3-yl)piperazin-1-yl)methyl)imidazo[1,5-a]quinoxalin-4(5H)-one (Example 39);
7-((4-(2-methyl-6-(methylcarbamoyl)pyridin-3-yl)piperazin-1-yl)methyl)-9-fluoropyrrolo[1,2-a]quinoxalin-4(5H)-one (Example 40);
7-((4-(2-fluoro-6-(methylcarbamoyl)pyridin-3-yl)piperazin-1-yl)methyl)-9-fluoropyrrolo[1,2-a]quinoxalin-4(5H)-one (Example 41);
7-((4-(2-chloro-6-(methylcarbamoyl)pyridin-3-yl)piperazin-1-yl)methyl)-9-fluoropyrrolo[1,2-a]quinoxalin-4(5H)-one (Example 42);
7-((4-(2-methyl-6-(methylcarbamoyl)pyridin-3-yl)piperazin-1-yl)methyl)-9-fluoropyrazolo[1,5-a]quinoxalin-4(5H)-one (Example 43);
7-((4-(2-fluoro-6-(methylcarbamoyl)pyridin-3-yl)piperazin-1-yl)methyl)-9-fluoropyrazolo[1,5-a]quinoxalin-4(5H)-one (Example 44);
7-((4-(2-chloro-6-(methylcarbamoyl)pyridin-3-yl)piperazin-1-yl)methyl)-9-fluoropyrazolo[1,5-a]quinoxalin-4(5H)-one (Example 45);
7-((4-(2-methyl-6-(methylcarbamoyl)pyridin-3-yl)piperazin-1-yl)methyl)-9-chloropyrazolo[1,5-a]quinoxalin-4(5H)-one (Example 46);
7-((4-(2-fluoro-6-(methylcarbamoyl)pyridin-3-yl)piperazin-1-yl)methyl)-9-chloropyrazolo[1,5-a]quinoxalin-4(5H)-one (Example 47);
8-((4-(2-methyl-6-(methylcarbamoyl)pyridin-3-yl)piperazin-1-yl)methyl)pyrmlo[1,2-c]quinazolin-5(6H)-one (Example 48);
7-((4-(2-methyl-6-(ethylcarbamoyl)pyridin-3-yl)piperazin-1-yl)methyl)-1,2,3,5-tetrahydro-4H-cyclopenta[c]quinolin-4-one (Example 49);
7-((4-(2-chloro-6-(ethylcarbamoyl)pyridin-3-yl)piperazin-1-yl)methyl)-1,2,3,5-tetrahydro-4H-cyclopenta[c]quinolin-4-one (Example 50);
8-((4-(2-fluoro-6-(methylcarbamoyl)pyridin-3-yl)piperazin-1-yl)methyl)imidazo[1,2-c]quinazolin-5(6H)-one (Example 51);
8-((4-(2-chloro-6-(methylcarbamoyl)pyridin-3-yl)piperazin-1-yl)methyl)imidazo[1,2-c]quinazolin-5(6H)-one (Example 52);
8-((4-(2-fluoro-6-(methylcarbamoyl)pyridin-3-yl)piperazin-1-yl)methyl)-10-fluoroimidazo[1,2-c]quinazolin-5(6H)-one (Example 53);
7-((4-(2-methyl-6-(methylcarbamoyl)pyridin-3-yl)piperazin-1-yl)methyl)-1-methylpyrrolo[1,2-a]quinoxalin-4(5H)-one (Example 54);
7-((4-(2-methyl-6-(methylcarbamoyl)pyridin-3-yl)piperazin-1-yl)methyl)-2-methylpyrrolo[1,2-a]quinoxalin-4(5H)-one (Example 55);
7-((4-(2-methyl-6-(methylcarbamoyl)pyridin-3-yl)piperazin-1-yl)methyl)-3-methylpyrrolo[1,2-a]quinoxalin-4(5H)-one (Example 56);
7-((4-(2-methyl-6-(methylcarbamoyl)pyridin-3-yl)piperazin-1-yl)methyl)-2-methylpyrazolo[1,5-a]quinoxalin-4(5H)-one (Example 57);
7-((4-(2-methyl-6-(methylcarbamoyl)pyridin-3-yl)piperazin-1-yl)methyl)-3-methylpyrazolo[1,5-a]quinoxalin-4(5H)-one (Example 58);
7-((4-(2-methyl-6-(cyclopropylcarbamoyl)pyridin-3-yl)piperazin-1-yl)methyl)pyrrolo[1,2-a]quinoxalin-4(5H)-one (Example 59);
7-((4-(2-fluoro-6-(cyclopropylcarbamoyl)pyridin-3-yl)piperazin-1-yl)methyl)pyrrolo[1,2-a]quinoxalin-4(5H)-one (Example 60);
7-((4-(2-chloro-6-(cyclopropylcarbamoyl)pyridin-3-yl)piperazin-1-yl)methyl)pyrrolo[1,2-a]quinoxalin-4(5H)-one (Example 61);
7-((4-(2-methyl-6-(cyclopropylcarbamoyl)pyridin-3-yl)piperazin-1-yl)methyl)pyrazolo[1,5-a]quinoxalin-4(5H)-one (Example 62);
7-((4-(2-methyl-6-(cyclopropylcarbamoyl)pyridin-3-yl)piperazin-1-yl)methyl)-3,5-dihydrofuro[3,4-c]quinolin-4(1H)-one (Example 63);
7-((4-(2-fluoro-6-(methylcarbamoyl)pyridin-3-yl)piperazin-1-yl)methyl)-9-fluoro-3,5-dihydrofuro[3,4-c]quinolin-4(1H)-one (Example 64);
7-((4-(6-(methylcarbamoyl)pyridin-3-yl)piperazin-1-yl)methyl)pyrrolo[1,2-a]quinoxalin-4(5H)-one (Example 65);
7-((4-(2-methyl-6-(cyclopropylcarbamoyl)pyridin-3-yl)piperazin-1-yl)methyl)-9-fluoropyrrolo[1,2-a]quinoxalin-4(5H)-one (Example 66);
7-((4-(2-fluoro-6-(cyclopropylcarbamoyl)pyridin-3-yl)piperazin-1-yl)methyl)-9-fluoropyrrolo[1,2-a]quinoxalin-4(5H)-one (Example 67);
7-((4-(2-chloro-6-(cyclopropylcarbamoyl)pyridin-3-yl)piperazin-1-yl)methyl)-9-fluoropyrrolo[1,2-alquinoxalin-4(5H)-one (Example 68);
7-((4-(2-fluoro-6-(methylcarbamoyl)pyridin-3-yl)piperazin-1-yl)methyl)-2-methylpyrazolo[1,5-a]quinoxalin-4(5H)-one (Example 69);
7-((4-(2-chloro-6-(methylcarbamoyl)pyridin-3-yl)piperazin-1-yl)methyl)-2-methylpyrazolo[1,5-a]quinoxalin-4(5H)-one (Example 70);
7-((4-(2-fluoro-6-(methylcarbamoyl)pyridin-3-yl)piperazin-1-yl)methyl)-3-methylpyrazolo[1,5-a]quinoxalin-4(5H)-one (Example 71);
7-((4-(2-chloro-6-(methylcarbamoyl)pyridin-3-yl)piperazin-1-yl)methyl)-3-methylpyrazolo[1,5-a]quinoxalin-4(5H)-one (Example 72);
7-((4-(2-fluoro-6-(methylcarbamoyl)pyridin-3-yl)piperazin-1-yl)methyl)-9-fluoro-2-methylpyrazolo[1,5-a]quinoxalin-4(5H)-one (Example 73);
7-((4-(2-chloro-6-(methylcarbamoyl)pyridin-3-yl)piperazin-1-yl)methyl)-9-fluoro-2-methylpyrazolo[1,5-a]quinoxalin-4(5H)-one (Example 74);
7-((4-(2-methyl-6-(methylcarbamoyl)pyridin-3-yl)piperazin-1-yl)methyl)-9-fluoro-2-methylpyrazolo[1,5-a]quinoxalin-4(5H)-one (Example 75);
7-((4-(2-fluoro-6-(methylcarbamoyl)pyridin-3-yl)piperazin-1-yl)methyl)-9-fluoro-3-methylpyrazolo[1,5-a]quinoxalin-4(5H)-one (Example 76);
7-((4-(2-chloro-6-(methylcarbamoyl)pyridin-3-yl)piperazin-1-yl)methyl)-9-fluoro-3-methylpyrazolo[1,5-a]quinoxalin-4(5H)-one (Example 77);
7-((4-(2-methyl-6-(methylcarbamoyl)pyridin-3-yl)piperazin-1-yl)methyl)-9-fluoro-3-methylpyrazolo[1,5-a]quinoxalin-4(5H)-one (Example 78);
7-((4-(2-methyl-6-(methylcarbamoyl)pyridin-3-yl)piperazin-1-yl)methyl)-9-fluoro-1,2,3,5-tetrahydro-4H-cyclopenta[c]quinolin-4-one (Example 79);
7-((4-(2-fluoro-6-(methylcarbamoyl)pyridin-3-yl)piperazin-1-yl)methyl)-9-fluoro-1,2,3,5-tetrahydro-4H-cyclopenta[c]quinolin-4-one (Example 80);
7-((4-(2-methyl-6-(cyclopropylcarbamoyl)pyridin-3-yl)piperazin-1-yl)methyl)-9-fluoro-1,2,3,5-tetrahydro-4H-cyclopenta[c]quinolin-4-one (Example 81);
7-((4-(2-fluoro-6-(cyclopropylcarbamoyl)pyridin-3-yl)piperazin-1-yl)methyl)-9-fluoro-1,2,3,5-tetrahydro-4H-cyclopenta[c]quinolin-4-one (Example 82);
7-((4-(2-methyl-6-(methylcarbamoyl)pyridin-3-yl)piperazin-1-yl)methyl)thieno[3,4-c]quinolin-4(5H)-one (Example 83);
7-((4-(2-methyl-6-(methylcarbamoyl)pyridin-3-yl)piperazin-1-yl)methyl)thieno[2,3-c]quinolin-4(5H)-one (Example 84);
7-((4-(2-methyl-6-(methylcarbamoyl)pyridin-3-yl)piperazin-1-yl)methyl)thieno[3,2-c]quinolin-4(5H)-one (Example 85);
7-((4-(2-methyl-6-(methylcarbamoyl)pyridin-3-yl)piperazin-1-yl)methyl)-9-fluorothieno[3,4-c]quinolin-4(5H)-one (Example 86);
7-((4-(2-methyl-6-(methylcarbamoyl)pyridin-3-yl)piperazin-1-yl)methyl)-2-chloro-9-fluoropyrazolo[1,5-a]quinoxalin-4(5H)-one (Example 87);
7-((4-(2-fluoro-6-(methylcarbamoyl)pyridin-3-yl)piperazin-1-yl)methyl)-2-chloro-9-fluoropyrazolo[1,5-a]quinoxalin-4(5H)-one (Example 88);
7-((4-(2-methyl-6-(carbamoyl)pyridin-3-yl)piperazin-1-yl)methyl)-9-fluoropyrazolo[1,5-a]quinoxalin-4(5H)-one (Example 89);
7-((4-(2-methyl-6-((methyl-d3)-carbamoyl)pyridin-3-yl)piperazin-1-yl)methyl)-9-fluoropyrazolo[1,5-a]quinoxalin-4(5H)-one (Example 90);
7-((4-(2-methyl-6-(ethylcarbamoyl)pyridin-3-yl)piperazin-1-yl)methyl)-9-fluoropyrazolo[1,5-a]quinoxalin-4(5H)-one (Example 91);
7-((4-(2-methyl-6-(cyclopropylcarbamoyl)pyridin-3-yl)piperazin-1-yl)methyl)-9-fluoropyrazolo[1,5-a]quinoxalin-4(5H)-one (Example 92);
7-((4-(2-methyl-6-(methylcarbamoyl)pyridin-3-yl)piperazin-1-yl)methyl)-8-fluoropyrazolo[1,5-a]quinoxalin-4(5H)-one (Example 93);
7-((4-(2-methyl-6-(methylcarbamoyl)pyridin-3-yl)piperazin-1-yl)methyl)-6-fluoropyrazolo[1,5-a]quinoxalin-4(5H)-one (Example 94);
7-((4-(2-fluoro-6-(methylcarbamoyl)pyridin-3-yl)piperazin-1-yl)methyl)-6-fluoropyrazolo[1,5-a]quinoxalin-4(5H)-one (Example 95);
8-((4-(2-methyl-6-(methylcarbamoyl)pyridin-3-yl)piperazin-1-yl)methyl)-10-fluoroimidazo[1,2-c]quinazolin-5(6H)-one (Example 96);
7-((4-(6-(methylcarbamoyl)pyridin-3-yl)piperazin-1-yl)methyl)-9-fluoropyrazolo[1,5-a]quinoxalin-4(5H)-one (Example 97);
7-((4-(2-methyl-6-(methylcarbamoyl)pyridin-3-yl)piperazin-1-yl)methyl)-3-chloropyrazolo[1,5-a]quinoxalin-4(5H)-one (Example 98);
7-((4-(2-fluoro-6-(methylcarbamoyl)pyridin-3-yl)piperazin-1-yl)methyl)-3-chloropyrazolo[1,5-a]quinoxalin-4(5H)-one (Example 99);
7-((4-(2-methyl-6-(methylcarbamoyl)pyridin-3-yl)piperazin-1-yl)methyl)-2-chloropyrazolo[1,5-a]quinoxalin-4(5H)-one (Example 100);
7-((4-(2-methyl-6-(methylcarbamoyl)pyridin-3-yl)piperazin-1-yl)methyl)-2,3-dimethylpyrazolo[1,5-a]quinoxalin-4(5H)-one (Example 101);
7-((4-(2-methyl-6-(methylcarbamoyl)pyridin-3-yl)piperazin-1-yl)methyl)-9-methylpyrazolo[1,5-a]quinoxalin-4(5H)-one (Example 102);
7-((4-(2-methyl-6-(methylcarbamoyl)pyridin-3-yl)piperazin-1-yl)methyl)-6-fluoro-2-chloropyrazolo[1,5-a]quinoxalin-4(5H)-one (Example 103);
7-((4-(2-methyl-6-(methylcarbamoyl)pyridin-3-yl)piperazin-1-yl)methyl)furo[2,3-c]quinolin-4(5H)-one (Example 104);
7-((4-(2-methyl-6-(methylcarbamoyl)pyridin-3-yl)piperazin-1-yl)methyl)-1-methyl-1,5-dihydro-4H-pyrazolo[4,3-c]quinolin-4-one (Example 105);
7-((4-(2-methyl-6-(methylcarbamoyl)pyridin-3-yl)piperazin-1-yl)methyl)-1,5-dihydro-4H-pyrazolo[4,3-c]quinolin-4-one (Example 106);
7-((4-(2-methyl-6-(methylcarbamoyl)pyridin-3-yl)piperazin-1-yl)methyl)-9-fluoro-furo[2,3-c]quinolin-4(5H)-one (Example 107);
7-((4-(2-fluoro-6-(methylcarbamoyl)pyridin-3-yl)piperazin-1-yl)methyl)-9-fluoro-furo[2,3-c]quinolin-4(5H)-one (Example 108);
7-((4-(2-methyl-6-(methylcarbamoyl)pyridin-3-yl)piperazin-1-yl)methyl)-6-fluoro-2-methylpyrazolo[1,5-a]quinoxalin-4(5H)-one (Example 109);
7-((4-(2-fluoro-6-(ethylcarbamoyl)pyridin-3-yl)piperazin-1-yl)methyl)-6-fluoropyrazolo[1,5-a]quinoxalin-4(5H)-one (Example 110);
7-((4-(2-fluoro-6-(cyclopropylcarbamoyl)pyridin-3-yl)piperazin-1-yl)methyl)-6-fluoropyrazolo[1,5-a]quinoxalin-4(5H)-one (Example 111);
7-((4-(2-fluoro-6-(methylcarbamoyl)pyridin-3-yl)piperazin-1-yl)methyl)-6-fluoro-2-chloropyrazolo[1,5-a]quinoxalin-4(5H)-one (Example 112);
7-((4-(2-methyl-4-(methylcarbamoyl)phenyl)piperazin-1-yl)methyl)-9-fluoropyrazolo[1,5-a]quinoxalin-4(5H)-one (Example 113);
7-((4-(2-methyl-6-(methylcarbamoyl)pyridin-3-yl)piperazin-1-yl)methyl)-9-fluorothieno[2,3-c]quinolin-4(5H)-one (Example 114);
7-((4-(2-fluoro-6-(methylcarbamoyl)pyridin-3-yl)piperazin-1-yl)methyl)-6-fluoro-2-methylpyrazolo[1,5-a]quinoxalin-4(5H)-one (Example 115);
7-((4-(2-methyl-6-carboxypyridin-3-yl)piperazin-1-yl)methyl)-9-fluoropyrazolo[1,5-a]quinoxalin-4(5H)-one (Example 116);
7-((4-(2-methyl-6-(N-(hydroxymethyl)carbamoyl)pyridin-3-yl)piperazin-1-yl)methyl)-9-fluoropyrazolo[1,5-a]quinoxalin-4(5H)-one (Example 117);
8-((4-(2-methyl-6-(methylcarbamoyl)pyridin-3-yl)piperazin-1-yl)methyl)pyrazolo[1,5-c]quinazolin-5(6H)-one (Example 118);
8-((4-(2-chloro-6-(methylcarbamoyl)pyridin-3-yl)piperazin-1-yl)methyl)-10-fluoroimidazo[1,2-c]quinazolin-5(6H)-one (Example 119);
8-((4-(2-fluoro-6-(methylcarbamoyl)pyridin-3-yl)piperazin-1-yl)methyl)-10-fluoroimidazo[1,2-c]quinazolin-5(6H)-one (Example 120);
7-((4-(2-methyl-6-(methylcarbamoyl)pyridin-3-yl)piperazin-1-yl)methyl)furo[3,2-c]quinolin-4(5H)-one (Example 121);
7-((4-(2-methyl-6-(methylcarbamoyl)pyridin-3-yl)piperazin-1-yl)methyl)thiazolo[4,5-c]quinolin-4(5H)-one (Example 122);
7-((4-(2-fluoro-6-(methylcarbamoyl)pyridin-3-yl)piperazin-1-yl)methyl)-2-chloropyrazolo[1,5-a]quinoxalin-4(5H)-one (Example 123);
7-((4-(2-methyl-6-(methylcarbamoyl)pyridin-3-yl)piperazin-1-yl)methyl)-3-fluoropyrazolo[1,5-a]quinoxalin-4(5H)-one (Example 124);
7-((4-(2-fluoro-6-(methylcarbamoyl)pyridin-3-yl)piperazin-1-yl)methyl)-2,3-dimethylpyrazolo[1,5-a]quinoxalin-4(5H)-one (Example 125);
7-((4-(2-fluoro-6-(methylcarbamoyl)pyridin-3-yl)piperazin-1-yl)methyl)-3-chloro-2-methylpyrazolo[1,5-a]quinoxalin-4(5H)-one (Example 126);
7-((4-(2-methyl-6-(methylcarbamoyl)pyridin-3-yl)piperazin-1-yl)methyl)-3-chloro-9-fluoropyrazolo[1,5-a]quinoxalin-4(5H)-one (Example 127);
7-((4-(2-methyl-6-(methylcarbamoyl)pyridin-3-yl)piperazin-1-yl)methyl)-6-fluoropyrrolo[1,2-a]quinoxalin-4(5H)-one (Example 128);
7-((4-(2-methyl-6-(methylcarbamoyl)pyridin-3-yl)piperazin-1-yl)methyl)-6-methylpyrazolo[1,5-a]quinoxalin-4(5H)-one (Example 129);
7-((4-(2-methyl-6-(methylcarbamoyl)pyridin-3-yl)piperazin-1-yl)methyl)-6-chloropyrazolo[1,5-a]quinoxalin-4(5H)-one (Example 130);
8-((4-(2-methyl-6-(methylcarbamoyl)pyridin-3-yl)piperazin-1-yl)methyl)-10-fluoropyrrolo[1,2-c]quinazolin-5(6H)-one (Example 131);
8-((4-(2-fluoro-6-(methylcarbamoyl)pyridin-3-yl)piperazin-1-yl)methyl)-10-fluoropyrrolo[1,2-c]quinazolin-5(6H)-one (Example 132);
7-((4-(2-fluoro-6-(methylcarbamoyl)pyridin-3-yl)piperazin-1-yl)methyl)-6-methylpyrazolo[1,5-a]quinoxalin-4(5H)-one (Example 133);
7-((4-(2-fluoro-6-(methylcarbamoyl)pyridin-3-yl)piperazin-1-yl)methyl)-6-chloropyrazolo[1,5-a]quinoxalin-4(5H)-one (Example 134);
7-((4-(2-methyl-6-(cyclopropylcarbamoyl)pyridin-3-yl)piperazin-1-yl)methyl)-6-fluoropyrazolo[1,5-a]quinoxalin-4(5H)-one (Example 135);
6-fluoro-7-((4-(2-fluoro-6-((methyl-d3)carbamoyl)pyridin-3-yl)piperazin-1-yl)methyl)pyrazolo[1,5-a]quinoxalin-4(5H)-one (Example 136);
6-fluoro-7-((4-(2-methyl-6-((methyl-d3)carbamoyl)pyridin-3-yl)piperazin-1-yl)methyl)pyrazolo[1,5-a]quinoxalin-4(5H)-one (Example 137);
6-fluoro-7-((4-(2-fluoro-6-((methyl-d3)carbamoyl)pyridin-3-yl)piperazin-1-yl)methyl)furo[2,3-c]quinolin-4(5H)-one (Example 138);
6-fluoro-7-((4-(2-methyl-6-((methyl-d3)carbamoyl)pyridin-3-yl)piperazin-1-yl)methyl)furo[2,3-c]quinolin-4(5H)-one (Example 139);
6-fluoro-7-((4-(2-methyl-6-((methyl-d3)carbamoyl)pyridin-3-yl)piperazin-1-yl)methyl)-2-chloropyrazolo[1,5-a]quinoxalin-4(5H)-one (Example 140);
9-fluoro-7-((4-(2-fluoro-6-((methyl-d3)carbamoyl)pyridin-3-yl)piperazin-1-yl)methyl)pyrazolo[1,5-a]quinoxalin-4(5H)-one (Example 141);
6-fluoro-7-((4-(2-fluoro-6-((methyl-d3)carbamoyl)pyridin-3-yl)piperazin-1-yl)methyl)-2-chloropyrazolo[1,5-a]quinoxalin-4(5H)-one (Example 142);
7-((4-(2-methyl-6-(methylcarbamoyl)pyridin-3-yl)piperazin-1-yl)methyl)-6-fluoro-furo[2,3-c]quinolin-4(5H)-one (Example 143);
7-((4-(2-methyl-6-(ethylcarbamoyl)pyridin-3-yl)piperazin-1-yl)methyl)-6-fluoropyrazolo[1,5-a]quinoxalin-4(5H)-one (Example 144);
7-((4-(2-fluoro-6-(methylcarbamoyl)pyridin-3-yl)piperazin-1-yl)methyl)-6-fluoropyrrolo[1,2-a]quinoxalin-4(5H)-one (Example 145);
7-((4-(2-methyl-6-(methylcarbamoyl)pyridin-3-yl)piperazin-1-yl)methyl)-2-methyl-2,5-dihydro-4H-pyrazolo[4,3-c]quinolin-4-one (Example 146);
7-((4-(2-methyl-6-(methylcarbamoyl)pyridin-3-yl)piperazin-1-yl)methyl)-3-chloro-2-methylpyrazolo[1,5-a]quinoxalin-4(5H)-one (Example 147);
7-((4-(2-methyl-6-(methylcarbamoyl)pyridin-3-yl)piperazin-1-yl)methyl)-3,9-difluoropyrazolo[1,5-a]quinoxalin-4(5H)-one (Example 148);
7-((4-(2-fluoro-6-(methylcarbamoyl)pyridin-3-yl)piperazin-1-yl)methyl)-6-fluoro-furo[2,3-c]quinolin-4(5H)-one (Example 149);
8-((4-(2-fluoro-6-(methylcarbamoyl)pyridin-3-yl)piperazin-1-yl)methyl)-7-fluoropyrrolo[1,2-c]quinazolin-5(6H)-one (Example 150);
or stereoisomers, tautomers, N-oxides, hydrates, isotope-substituted derivatives, solvates or pharmaceutically acceptable salts thereof, or mixtures thereof, or prodrugs thereof.

Some of the compounds of the present disclosure may exist as stereoisomers including optical isomers. The disclosure includes all stereoisomers and the racemic mixtures of such stereoisomers as well as the individual enantiomers that may be separated according to methods that are well known to those of ordinary skill in the art.

Examples of pharmaceutically acceptable salts include inorganic and organic acid salts, such as hydrochloride, hydrobromide, phosphate, sulphate, citrate, lactate, tartrate, maleate, fumarate, mandelate and oxalate; and inorganic and organic base salts formed with bases, such as sodium hydroxy, tris(hydroxymethyl)aminomethane (TRIS, tromethamine) and N-methyl-glucamine.

Examples of prodrugs of the compounds of the disclosure include the simple esters of carboxylic acid-containing compounds (e.g., those obtained by condensation with a C₁₋₄ alcohol according to methods known in the art); esters of hydroxy containing compounds (e.g., those obtained by condensation with a C₁₋₄ carboxylic acid, C₃₋₆ diacid or anhydride thereof, such as succinic anhydride and fumaric anhydride according to methods known in the art); imines of amino containing compounds (e.g., those obtained by condensation with a C₁₋₄ aldehyde or ketone according to methods known in the art); carbamate of amino containing compounds, such as those described by Leu, et al., (J. Med. Chem. 42: 3623-3628 (1999)) and Greenwald, et al., (J. Med. Chem. 42: 3657-3667 (1999)); and acetals and ketals of alcohol-containing compounds (e.g., those obtained by condensation with chloromethyl methyl ether or chloromethyl ethyl ether according to methods known in the art).

The compounds of this disclosure may be prepared using methods known to those skilled in the art, or the novel methods of this disclosure. Specifically, the compounds of this disclosure with Formula I (including Formulae II, III and IV) can be prepared as illustrated by the exemplary reaction in Scheme 1. Reaction of methyl 4-oxotetrahydrofuran-3-carboxylate and trifluoromethanesulfonic anhydride (Tf₂O) under the catalysis of N,N-diisopropylethylamine (DIEA) produced methyl 4-(((trifluoromethyl)sulfonyl)oxy)-2,5-dihydrofuran-3-carboxylate. Suzuki reaction of methyl 4-(((trifluoromethyl)sulfonyl)oxy)-2,5-dihydrofuran-3-carboxylate and (4-(methoxycarbonyl)-2-nitrophenyl)boronic acid under the catalysis of Pd₂(dba)₃ produced methyl 4-(4-(methoxycarbonyl)-2-nitrophenyl)-2,5-dihydrofuran-3-carboxylate. Reaction of methyl 4-(4-(methoxycarbonyl)-2-nitrophenyl)-2,5-dihydrofuran-3-carboxylate and Fe/AcOH under the catalysis of AcOH produced methyl 4-oxo-1,3,4,5-tetrahydrofuro[3,4-c]quinoline-7-carboxylate. Reduction reaction of methyl 4-oxo-1,3,4,5-tetrahydrofuro[3,4-c]quinoline-7-carboxylate and lithium aluminum hydride (LiAlH₄) produced 7-(hydroxymethyl)-3,5-dihydrofuro[3,4-c]quinolin-4(1H)-one. Chlorination of 7-(hydroxymethyl)-3,5-dihydrofuro[3,4-c]quinolin-4(1H)-one with SOCl₂ produced 7-(chloromethyl)-3,5-dihydrofuro[3,4-c]quinolin-4(1H)-one. Substitution reaction of 7-(chloromethyl)-3,5-dihydrofuro[3,4-c]quinolin-4(1H)-one and N-methyl-5-(piperazin-1-yl)picolinamide under the catalysis of DIEA and KI produced the target compound 7-((4-(6-(methylcarbamoyl)pyridin-3-yl)piperazin-1-yl)methyl)-3,5-dihydrofuro[3,4-c]quinolin-4(1H)-one.

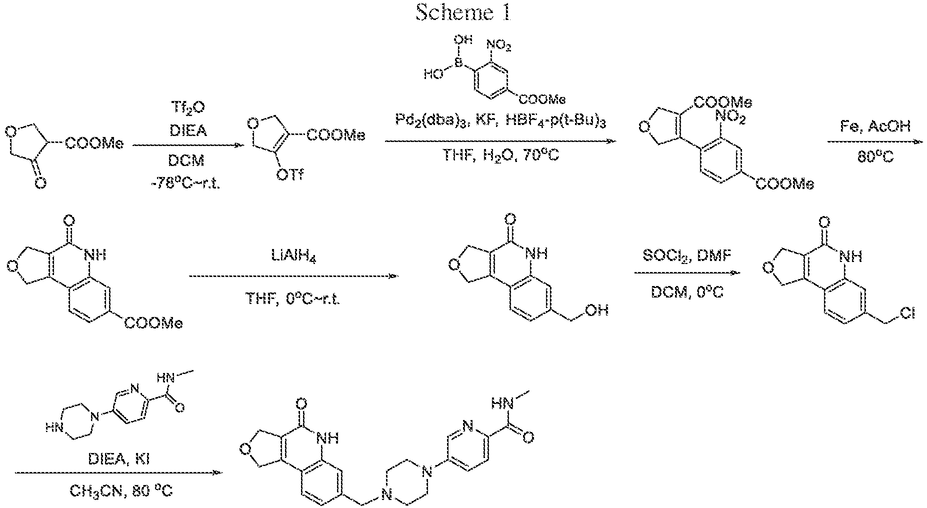

Other related compounds can be prepared using similar methods. For example, replacement of N-methyl-5-(piperazin-1-yl)picolinamide with 6-fluoro-N-methyl-5-(piperazin-1-yl)picolinamide produced the target compound 7-((4-(2-fluoro-6-(methylcarbamoyl)pyridin-3-yl)piperazin-1-yl)methyl)-3,5-dihydrofuro[3,4-c]quinolin-4(1H)-one. Replacement of N-methyl-5-(piperazin-1-yl)picolinamide with 6-chloro-N-methyl-5-(piperazin-1-yl)picolinamide produced the target compound 7-((4-(2-chloro-6-(methylcarbamoyl)pyridin-3-yl)piperazin-1-yl)methyl)-3,5-dihydrofuro[3,4-c]quinolin-4(1H)-one. Replacement of N-methyl-5-(piperazin-1-yl)picolinamide with N,6-dimethyl-5-(piperazin-1-yl)picolinamide produced the target compound 7-((4-(2-methyl-6-(methylcarbamoyl)pyridin-3-yl)piperazin-1-yl)methyl)-3,5-dihydrofuro[3,4-c]quinolin-4(1H)-one. Replacement of N-methyl-5-(piperazin-1-yl)picolinamide with 6-chloro-N-ethyl-5-(piperazin-1-yl)picolinamide produced the target compound 7-((4-(2-chloro-6-(ethylcarbamoyl)pyridin-3-yl)piperazin-1-yl)methyl)-3,5-dihydrofuro[3,4-c]quinolin-4(1H)-one. Replacement of N-methyl-5-(piperazin-1-yl)picolinamide with N-ethyl-6-methyl-5-(piperazin-1-yl)picolinamide produced the target compound 7-((4-(2-methyl-6-(ethylcarbamoyl)pyridin-3-yl)piperazin-1-yl)methyl)-3,5-dihydrofuro[3,4-c]quinolin-4(1H)-one.

The compounds of this disclosure can be prepared as illustrated by the exemplary reaction in Scheme 2. Substitution reaction of methyl 4-fluoro-3-nitrobenzoate and methyl 1H-pyrrole-2-carboxylate under the catalysis of Cs₂CO₃ produced methyl 1-(4-(methoxycarbonyl)-2-nitrophenyl)-1H-pyrrole-2-carboxylate. Reaction of methyl 1-(4-(methoxycarbonyl)-2-nitrophenyl)-1H-pyrrole-2-carboxylate and Fe/AcOH under the catalysis of AcOH produced methyl 4-oxo-4,5-dihydropyrrolo[1,2-a]quinoxaline-7-carboxylate. Reduction reaction of methyl 4-oxo-4,5-dihydropyrrolo[1,2-a]quinoxaline-7-carboxylate and LiAlH₄ produced 7-(hydroxymethyl)pyrrolo[1,2-a]quinoxalin-4(5H)-one. Chlorination of 7-(hydroxymethyl)pyrrolo[1,2-a]quinoxalin-4(5H)-one with SOCl₂ under the catalysis of DMF produced 7-(chloromethyl)pyrrolo[1,2-a]quinoxalin-4(5H)-one. Substitution reaction of 7-(chloromethyl)pyrrolo[1,2-a]quinoxalin-4(5H)-one and N,6-dimethyl-5-(piperazin-1-yl)picolinamide under the catalysis of DIEA and KI produced the target compound 7-((4-(2-methyl-6-(methylcarbamoyl)pyridin-3-yl)piperazin-1-yl)methyl)pyrrolo[1,2-a]quinoxalin-4(5H)-one.

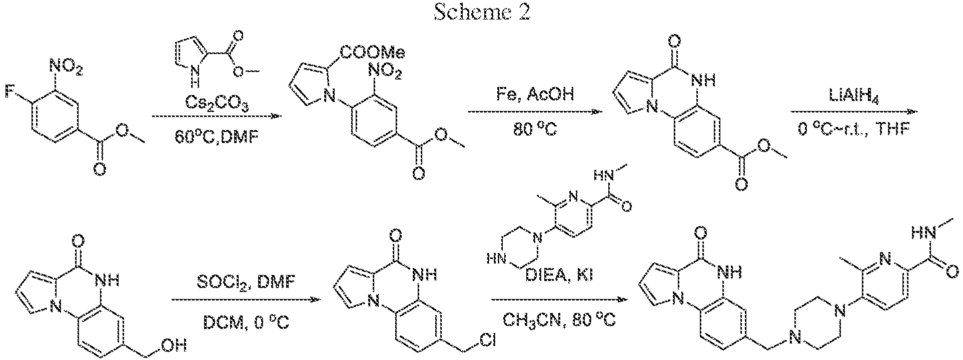

Other related compounds can be prepared using similar methods. For example, replacement of methyl 1H-pyrrole-2-carboxylate with methyl 1H-imidazole-2-carboxylate produced the target compound 7-((4-(2-methyl-6-(methylcarbamoyl)pyridin-3-yl)piperazin-1-yl)methyl)imidazo[1,2-a]quinoxalin-4(5H)-one. Replacement of methyl 1H-pyrrole-2-carboxylate with methyl 1H-imidazole-5-carboxylate produced the target compound 7-((4-(2-methyl-6-(methylcarbamoyl)pyridin-3-yl)piperazin-1-yl)methyl)imidazo[1,5-a]quinoxalin-4(5H)-one. Replacement of methyl 1H-pyrrole-2-carboxylate with methyl 1H-pyrazole-5-carboxylate produced the target compound 7-((4-(2-methyl-6-(methylcarbamoyl)pyridin-3-yl)piperazin-1-yl)methyl)pyrazolo[1,5-a]quinoxalin-4(5H)-one. Replacement of methyl 1H-pyrrole-2-carboxylate with methyl 5-methyl-1H-pyrrole-2-carboxylate produced the target compound 7-((4-(2-methyl-6-(methylcarbamoyl)pyridin-3-yl)piperazin-1-yl)methyl)-1-methylpyrrolo[1,2-a]quinoxalin-4(5H)-one.

The compounds of this disclosure can be prepared as illustrated by the exemplary reaction in Scheme 3. Reduction reaction of methyl 4-fluoro-3-nitrobenzoate and H₂ under the catalysis of Pd/C produced methyl 3-amino-4-fluorobenzoate. Reaction of methyl 3-amino-4-fluorobenzoate and 1H-pyrazole-5-carboxylic acid under the catalysis of DIEA and O-(7-Azabenzotriazol-1-yl)-N,N,N',N'-tetramethyluronium hexafluorophosphate (HATU) produced methyl 4-fluoro-3-(1H-pyrazole-5-carboxamido)benzoate. The intramolecular ring closure reaction of methyl 4-fluoro-3-(1H-pyrazole-5-carboxamido)benzoate under the catalysis of K₂CO₃ produced methyl 4-oxo-4,5-dihydropyrazolo[1,5-a]quinoxaline-7-carboxylate. Reduction reaction of methyl 4-oxo-4,5-dihydropyrazolo[1,5-a]quinoxaline-7-carboxylate and LiAlH₄ produced 7-(hydroxymethyl)pyrazolo[1,5-a]quinoxalin-4(5H)-one. Chlorination of 7-(hydroxymethyl)pyrrolo[1,2-a]quinoxalin-4(5H)-one with SOCl₂ produced 7-(chloromethyl)pyrazolo[1,5-a]quinoxalin-4(5H)-one. Substitution reaction of 7-(chloromethyl)pyrazolo[1,5-a]quinoxalin-4(5H)-one and N,6-dimethyl-5-(piperazin-1-yl)picolinamide under the catalysis of DIEA and KI produced the target compound 7-((4-(2-methyl-6-(methylcarbamoyl)pyridin-3-yl)piperazin-1-yl)methyl)pyrazolo[1,5-a]quinoxalin-4(5H)-one.

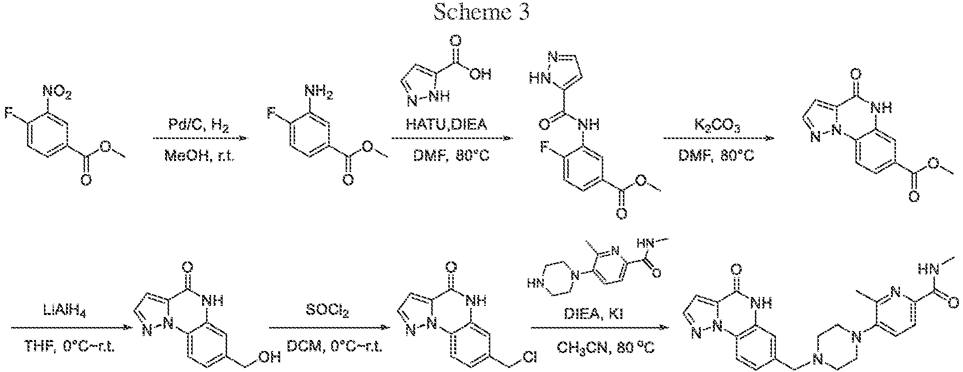

Other related compounds can be prepared using similar methods. For example, replacement of N,6-dimethyl-5-(piperazin-1-yl)picolinamide with 6-fluoro-N-methyl-5-(piperazin-1-yl)picolinamide produced the target compound 7-((4-(2-fluoro-6-(methylcarbamoyl)pyridin-3-yl)piperazin-1-yl)methyl)pyrazolo[1,5-a]quinoxalin-4(5H)-one. Replacement of N,6-dimethyl-5-(piperazin-1-yl)picolinamide with 6-chloro-N-methyl-5-(piperazin-1-yl)picolinamide produced the target compound 7-((4-(2-chloro-6-(methylcarbamoyl)pyridin-3-yl)piperazin-1-yl)methyl)pyrazolo[1,5-a]quinoxalin-4(5H)-one. Replacement of methyl 3-amino-4-fluorobenzoate with methyl 3-amino-4,5-difluorobenzoate produced the target compound 7-((4-(2-methyl-6-(methylcarbamoyl)pyridin-3-yl)piperazin-1-yl)methyl)-9-fluoropyrazolo[1,5-a]quinoxalin-4(5H)-one. Replacement of methyl 3-amino-4-fluorobenzoate with methyl 3-amino-5-chloro-4-fluorobenzoate produced the target compound 7-((4-(2-methyl-6-(methylcarbamoyl)pyridin-3-yl)piperazin-1-yl)methyl)-9-chloropyrazolo[1,5-a]quinoxalin-4(5H)-one. Replacement of 1H-pyrazole-5-carboxylic acid with 3-methyl-1H-pyrazole-5-carboxylic acid produced the target compound 7-((4-(2-methyl-6-(methylcarbamoyl)pyridin-3-yl)piperazin-1-yl)methyl)-2-methylpyrazolo[1,5-a]quinoxalin-4(5H)-one.

The compounds of this disclosure can be prepared as illustrated by the exemplary reaction in Scheme 4. Reaction of methyl 4-formyl-3-nitrobenzoate, glyoxal and NH₃-MeOH produced methyl 4-(1H-imidazol-2-yl)-3-nitrobenzoate. Reduction of methyl 4-(1H-imidazol-2-yl)-3-nitrobenzoate with SnCl₂ · 2H₂O produced methyl 3-amino-4-(1H-imidazol-2-yl)benzoate. Reaction of methyl 3-amino-4-(1H-imidazol-2-yl)benzoate and triphosgene (BTC) produced methyl 5-oxo-5,6-dihydroimidazo[1,2-c]quinazoline-8-carboxylate. Reduction of methyl 5-oxo-5,6-dihydroimidazo[1,2-c]quinazoline-8-carboxylate with LiAlH₄ produced 8-(hydroxymethyl)imidazo[1,2-c]quinazolin-5(6H)-one. Reaction of 8-(hydroxymethyl)imidazo[1,2-c]quinazolin-5(6H)-one and SOCl₂ produced 8-(chloromethyl)imidazo[1,2-c]quinazolin-5(6H)-one. Reaction of 8-(chloromethyl)imidazo[1,2-c]quinazolin-5(6H)-one and N,6-dimethyl-5-(piperazin-1-yl)picolinamide produced the target compound 8-((4-(2-methyl-6-(methylcarbamoyl)pyridin-3-yl)piperazin-1-yl)methyl)imidazo[1,2-c]quinazolin-5(6H)-one.

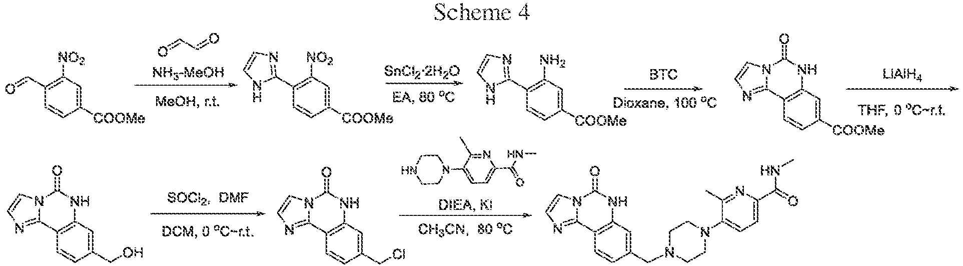

Other related compounds can be prepared using similar methods. For example, replacement of N,6-dimethyl-5-(piperazin-1-yl)picolinamide with 6-fluoro-N-methyl-5-(piperazin-1-yl)picolinamide produced the target compound 8-((4-(2-fluoro-6-(methylcarbamoyl)pyridine-3-yl)piperazin-1-yl)methyl)imidazo[1,2-c]quinazolin-5(6H)-one. Replacement of N,6-dimethyl-5-(piperazin-1-yl)picolinamide with 6-chloro-N-methyl-5-(piperazin-1-yl)picolinamide produced the target compound 8-((4-(2-chloro-6-(methylcarbamoyl)pyridin-3-yl)piperazin-1-yl)methyl)imidazo[1,2-c]quinazolin-5(6H)-one.

The compounds of this disclosure can be prepared as illustrated by the exemplary reaction in Scheme 5. Reaction of methyl 3-amino-4-bromobenzoate and (1-(tert-butoxycarbonyl)-1H-pyrrol-2-yl)boronic acid under the catalysis of Pd(PPh₃)₂Cl₂ and Na₂CO₃ produced methyl 5-oxo-5,6-dihydropyrrolo[1,2-c]quinazoline-8-carboxylate. Reduction of methyl 5-oxo-5,6-dihydropyrrolo[1,2-c]quinazoline-8-carboxylate with LiAlH₄ produced 8-(hydroxymethyl)pyrrolo[1,2-c]quinazolin-5(6H)-one. Reaction of 8-(hydroxymethyl)pyrrolo[1,2-c]quinazolin-5(6H)-one and methanesulfonyl chloride under the catalysis of triethylamine produced (5-oxo-5,6-dihydropyrrolo[1,2-c]quinazolin-8-yl)methyl methanesulfonate. Reaction of (5-oxo-5,6-dihydropyrrolo[1,2-c]quinazolin-8-yl)methyl methanesulfonate and N,6-dimethyl-5-(piperazin-1-yl)picolinamide under the catalysis of DIEA and KI produced the target compound 8-((4-(2-methyl-6-(methylcarbamoyl)pyridin-3-yl)piperazin-1-yl)methyl)pyrrolo[1,2-c]quinazolin-5(6H)-one.

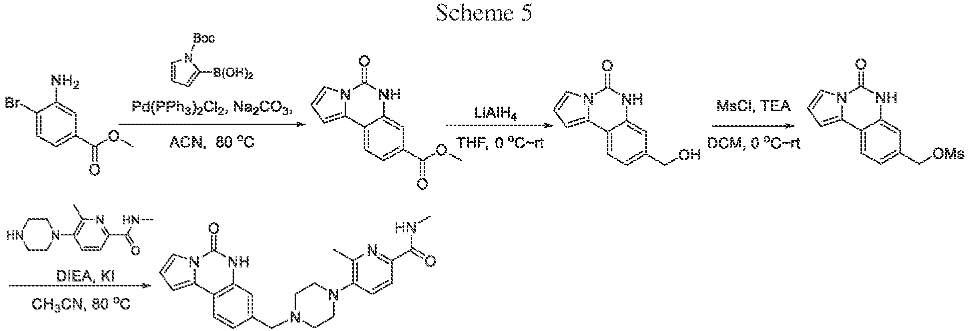

Other related compounds can be prepared using similar methods. For example, replacement of (1-(tert-butoxycarbonyl)-1H-pyrrol-2-yl)boronic acid with (1-(tert-butoxycarbonyl)-1H-pyrazol-5-yl)boronic acid produced the target compound 8-((4-(2-methyl-6-(methylcarbamoyl)pyridin-3-yl)piperazin-1-yl)methyl)pyrazolo[1,5-c]quinazolin-5(6H)-one.

The compounds of this disclosure can be prepared as illustrated by the exemplary reaction in Scheme 6. Esterification of methyl 2-oxocyclopentane-1-carboxylate and trifluoromethanesulfonic anhydride under the catalysis of DIEA produced methyl 2-(((trifluoromethyl)sulfonyl)oxy)cyclopent-1-ene-1-carboxylate. Borylation of methyl 2-(((trifluoromethyl)sulfonyl)oxy)cyclopent-1-ene-1-carboxylate and bis(pinacolato)diboron under the catalysis of Pd(dppf)Cl₂ · CH₂Cl₂ produced methyl 2-(4,4,5,5-tetramethyl-1,3,2-dioxaborolan-2-yl)cyclopent-1-ene-1-carboxylate. Suzuki reaction of methyl 2-(4,4,5,5-tetramethyl-1,3,2-dioxaborolan-2-yl)cyclopent-1-ene-1-carboxylate and methyl 4-bromo-3-fluoro-5-nitrobenzoate under the catalysis of Pd(PPh₃)₂Cl₂ produced methyl 3-fluoro-4-(2-(methoxycarbonyl)cyclopent-1-en-1-yl)-5-nitrobenzoate. Reaction of methyl 3-fluoro-4-(2-(methoxycarbonyl)cyclopent-1-en-1-yl)-5-nitrobenzoate and Fe/AcOH produced methyl 9-fluoro-4-oxo-2,3,4,5-tetrahydro-1H-cyclopenta[c]quinoline-7-carboxylate. Reduction of methyl 9-fluoro-4-oxo-2,3,4,5-tetrahydro- 1H-cyclopenta[c]quinoline-7-carboxylate with LiAlH₄ produced 9-fluoro-7-(hydroxymethyl)-1,2,3,5-tetrahydro-4H-cyclopenta[c]quinolin-4-one. Chlorination of 9-fluoro-7-(hydmxymethyl)-1,2,3,5-tetrahydro-4H-cyclopenta[c]quinolin-4-one with SOCl₂ under the catalysis of DMF produced 9-fluoro-7-(chloromethyl)-1,2,3,5-tetrahydro-4H-cyclopenta[c]quinolin-4-one. Reaction of 9-fluoro-7-(chloromethyl)-1,2,3,5-tetrahydro-4H-cyclopenta[c]quinolin-4-one and N,6-dimethyl-5-(piperazin-1-yl)picolinamide under the catalysis of DIEA and KI produced the target compound 7-((4-(2-methyl-6-(methylcarbamoyl)pyridin-3-yl)piperazin-1-yl)methyl)-9-fluoro-1,2,3,5-tetrahydro-4H-cyclopenta[c]quinolin-4-one.

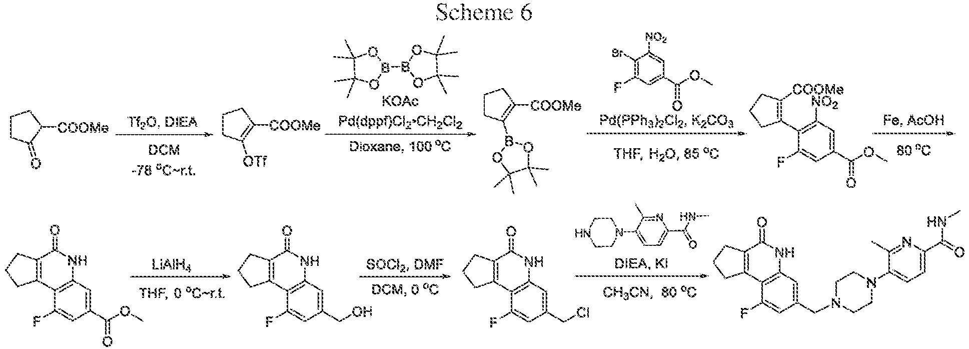

Other related compounds can be prepared using similar methods. For example, replacement of N,6-dimethyl-5-(piperazin-1-yl)picolinamide with 6-fluoro-N-methyl-5-(piperazin-1-yl)picolinamide produced the target compound 7-((4-(2-fluoro-6-(methylcarbamoyl)pyridin-3-yl)piperazin-1-yl)methyl)-9-fluoro-1,2,3,5-tetrahydro-4H-cyclopenta[c]quinolin-4-one. Replacement of N,6-dimethyl-5-(piperazin-1-yl)picolinamide with N-cyclopropyl-6-methyl-5-(piperazin-1-yl)picolinamide produced the target compound 7-((4-(2-methyl-6-(cyclopropylcarbamoyl)pyridin-3-yl)piperazin-1-yl)methyl)-9-fluoro-1,2,3,5-tetrahydro-4H-cyclopenta[c]quinolin-4-one. Replacement of N,6-dimethyl-5-(piperazin-1-yl)picolinamide with N-cyclopropyl-6-fluoro-5-(piperazin-1-yl)picolinamide produced the target compound 7-((4-(2-fluoro-6-(cyclopropylcarbamoyl)pyridin-3-yl)piperazin-1-yl)methyl)-9-fluoro-1,2,3,5-tetrahydro-4H-cyclopenta[c]quinolin-4-one.

The compounds of this disclosure can be prepared as illustrated by the exemplary reaction in Scheme 7. Reaction of 3-methyl-1H-pyrazole-5-carbonyl chloride and 5-bromo-2,3-difluoroaniline under the catalysis of NaH produced N-(5-bromo-2,3-difluorophenyl)-5-methyl-1H-pyrazole-3-carboxamide. Reaction of N-(5-bromo-2,3-difluorophenyl)-5-methyl-1H-pyrazole-3-carboxamide and K₂CO₃ produced 7-bromo-9-fluoro-2-methylpyrazolo[1,5-a]quinoxalin-4(5H)-one. Reaction of 7-bromo-9-fluoro-2-methylpyrazolo[1,5-a]quinoxalin-4(5H)-one and (tributylstannyl)methanol under the catalysis of XphosPdG₂ produced 9-fluoro-7-(hydroxymethyl)-2-methylpyrazolo[1,5-a]quinoxalin-4(5H)-one. Reaction of 9-fluoro-7-(hydroxymethyl)-2-methylpyrazolo[1,5-a]quinoxalin-4(5H)-one and SOCl₂ under the catalysis of DMF produced 7-(chloromethyl)-9-fluoro-2-methylpyrazolo[1,5-a]quinoxalin-4(5H)-one. Reaction of 7-(chloromethyl)-9-fluoro-2-methylpyrazolo[1,5-a]quinoxalin-4(5H)-one and 6-fluoro-N-methyl-5-(piperazin-1-yl)picolinamide under the catalysis of KI and DIEA produced the target compound 7-((4-(2-fluoro-6-(methylcarbamoyl)quinoxal-3-yl)piperazin-1-yl)methyl)-9-fluoro-2-methylpyrazolo[1,5-a]quinoxaline-4(5H)-one.

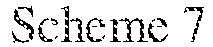

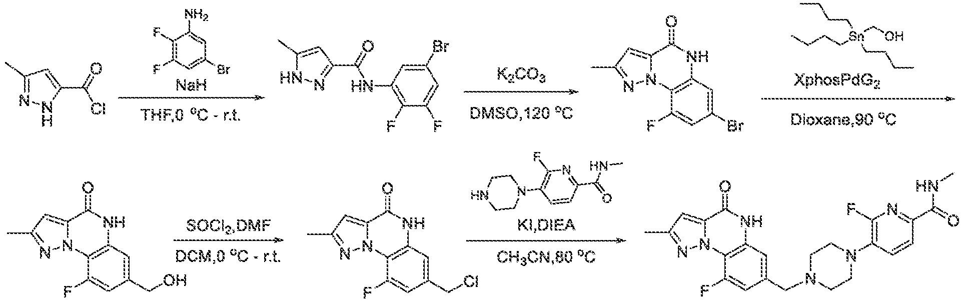

Other related compounds can be prepared using similar methods. For example, replacement of 6-fluoro-N-methyl-5-(piperazin-1-yl)picolinamide with N,6-dimethyl-5-(piperazin-1-yl)picolinamide produced the target compound 7-((4-(2-methyl-6-(methylcarbamoyl)quinoxal-3-yl)piperazin-1-yl)methyl)-9-fluoro-2-methylpyrazolo[1,5-a]quinoxaline-4(5H)-one. Replacement of 3-methyl-1H-pyrazole-5-carbonyl chloride with 4-methyl-1H-pyrazole-5-carbonyl chloride produced the target compound 7-((4-(2-methyl-6-(methylcarbamoyl)quinoxal-3-yl)piperazin-1-yl)methyl)-9-fluoro-3-methylpyrazolo[1,5-a]quinoxaline-4(5H)-one. Replacement of 3-methyl-1H-pyrazole-5-carbonyl chloride with 3-chloro-1H-pyrazole-5-carbonyl chloride produced the target compound 7-((4-(2-methyl-6-(methylcarbamoyl)quinoxal-3-yl)piperazin-1-yl)methyl)-2-chloro-9-fluoropyrazolo[1,5-a]quinoxaline-4(5H)-one. Replacement of 3-methyl-1H-pyrazole-5-carbonyl chloride with 1H-pyrazole-5-carbonyl chloride, replacement of 5-bromo-2,3-difluoroaniline with 3-bromo-2,6-difluoroaniline, and replacement of 6-fluoro-N-methyl-5-(piperazin-1-yl)picolinamide with N,6-dimethyl-5-(piperazin-1-yl)picolinamide, produced the target compound 7-((4-(2-methyl-6-(methylcarbamoyl)pyridin-3-yl)piperazin-1-yl)methyl)-6-fluoropyrazolo[1,5-a]quinoxalin-4(5H)-one. Replacement of 5-bromo-2,3-difluoroaniline with 3-bromo-2,6-difluoroaniline, and replacement of 6-fluoro-N-methyl-5-(piperazin-1-yl)picolinamide with N,6-dimethyl-5-(piperazin-1-yl)picolinamide, produced the target compound 7-((4-(2-methyl-6-(methylcarbamoyl)quinoxal-3-yl)piperazin-1-yl)methyl)-6-fluoro-2-methylpyrazolo[1,5-a]quinoxaline-4(5H)-one. Replacement of 3-chloro-1H-pyrazole-5-carbonyl chloride with 1H-pyrazole-5-carbonyl chloride, replacement of 5-bromo-2,3-difluoroaniline with 3-bromo-2,6-difluoroaniline, and replacement of 6-fluoro-N-methyl-5-(piperazin-1-yl)picolinamide with N,6-dimethyl-5-(piperazin-1-yl)picolinamide, produced the target compound 7-((4-(2-methyl-6-(methylcarbamoyl)pyridin-3-yl)piperazin-1-yl)methyl)-6-fluoro-2-chloropyrazolo[1,5-a]quinoxalin-4(5H)-one.

The compounds of this disclosure can be prepared as illustrated by the exemplary reaction in Scheme 8. Reaction of methyl 4-bromothiophene-3-carboxylate and (2-amino-4-(methoxycarbonyl)phenyl)boronic acid under the catalysis of NaOAc and Pd(dppf)Cl₂ produced methyl 4-oxo-4,5-dihydrothieno[3,4-c]quinoline-7-carboxylate. Reaction of methyl 4-oxo-4,5-dihydrothieno[3,4-c]quinoline-7-carboxylate and LiAlH₄ produced 7-(hydroxymethyl)thieno[3,4-c]quinolin-4(5H)-one. Reaction of 7-(hydroxymethyl)thieno[3,4-c]quinolin-4(5H)-one and SOCl₂ under the catalysis of DMF produced 7-(chloromethyl)thieno[3,4-c]quinolin-4(5H)-one. Reaction of 7-(chloromethyl)thieno[3,4-c]quinolin-4(5H)-one and N,6-dimethyl-5-(piperazin-1-yl)picolinamide under the catalysis of KI and DIEA produced the target compound 7-((4-(2-methyl-6-(methylcarbamoyl)quinolin-3-yl)piperazin-1-yl)methyl)thieno[3,4-c]quinoline-4(5H)-one.

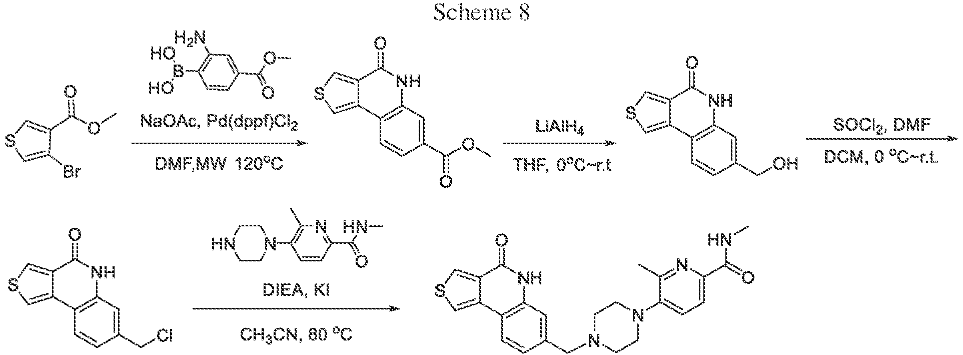

Other related compounds can be prepared using similar methods. For example, replacement of methyl 4-bromothiophene-3-carboxylate with methyl 2-bromothiophene-3-carboxylate produced the target compound 7-((4-(2-methyl-6-(methylcarbamoyl)quinolin-3-yl)piperazin-1-yl)methyl)thieno[3,2-c]quinoline-4(5H)-one. Replacement of methyl 4-bromothiophene-3-carboxylate with methyl 3-bromothiophene-2-carboxylate produced the target compound 7-((4-(2-methyl-6-(methylcarbamoyl)pyridin-3-yl)piperazin-1-yl)methyl)thieno[2,3-c]quinolin-4(5H)-one. Replacement of methyl 4-bromothiophene-3-carboxylate with methyl 5-bromothiazole-4-carboxylate produced the target compound 7-((4-(2-methyl-6-(methylcarbamoyl)pyridin-3-yl)piperazin-1-yl)methyl)thiazolo[4,5-c]quinolin-4(5H)-one.

An important aspect of the present disclosure is the discovery that compounds of Formula I (including Formulae II, III and IV) are PARP inhibitors, especially selective PARP1 inhibitors. Therefore, the compounds of Formula I (including Formulae II, III and IV) or stereoisomers, tautomers, N-oxides, hydrates, isotope-substituted derivatives, solvates or pharmaceutically acceptable salts thereof, or mixtures thereof, or prodrugs thereof can be used to treat a variety of diseases or conditions responsive to the inhibition of PARP activity (especially PARP1 activity), or used to prepare a medicament for treating or preventing diseases or conditions responsive to the inhibition of PARP activity (especially PARP1 activity).

In the disclosure, the diseases or conditions responsive to the inhibition of PARP activity (especially PARP1 activity), include cancer. Cancer can be a solid tumor or hematological tumor, including but is not limited to liver cancer, melanoma, Hodgkin's disease, non-Hodgkin's lymphoma, acute lymphocytic leukemia, chronic lymphocytic leukemia, multiple myeloma, neuroblastoma, breast cancer, ovarian cancer, lung cancer (such as small cell lung cancer), Wilms tumor, cervical cancer, testicular cancer, soft tissue sarcoma, primary macroglobulinemia, bladder cancer, chronic myeloid leukemia, primary brain cancer, malignant melanoma, gastric cancer, colon cancer, malignant pancreatic islet tumor, malignant carcinoid cancer, choriocarcinoma, mycosis fungoides, head and neck cancer, osteogenic sarcoma, pancreatic cancer, acute myeloid leukemia, hairy cell leukemia, rhabdomyosarcoma, Kaposi's sarcoma, urogenital tumors, thyroid cancer, esophageal cancer, malignant hypercalcemia, cervical hyperplasia, renal cell carcinoma, endometrial cancer, polycythemia vera, idiopathic thrombocythemia, adrenocortical carcinoma, skin cancer, and prostate cancer. Preferably, the cancer is responsive to the inhibition of PARP activity, especially PARP1 activity.

Therefore, the present disclosure includes methods for the treatment or prevention of diseases or conditions responsive to the inhibition of PARP activity (especially PARP1 activity), comprising administering to a subject (especially mammal, more specifically human) in need thereof an effective amount of the compound of Formula I (including Formulae II, III and IV) or stereoisomers, tautomers, N-oxides, hydrates, isotope-substituted derivatives, solvates or pharmaceutically acceptable salts thereof, or mixtures thereof, or prodrugs thereof, or a pharmaceutical composition comprising an effective amount of the compound of Formula I (including Formulae II, III and IV) or stereoisomers, tautomers, N-oxides, hydrates, isotope-substituted derivatives, solvates or pharmaceutically acceptable salts thereof, or mixtures thereof, or prodrugs thereof.

In practicing the therapeutic methods, effective amounts of pharmaceutical preparations are administered to an individual exhibiting the symptoms of one or more of these disorders. The pharmaceutic preparations comprise a therapeutically effective amount of the compound of Formula I (including Formulae II, III and IV), formulated for oral, intravenous, local or topical application, for the treatment of cancer and other diseases. The amount is effective to ameliorate or eliminate one or more symptoms of the disorders. An effective amount of a compound for treating a particular disease is an amount that is sufficient to ameliorate or in some manner reduce the symptoms associated with the disease. Such amount may be administered as a single dosage or may be administered according to an effective regimen. The amount may cure the disease but, typically, is administered in order to ameliorate the symptoms of the disease. Typically, repeated administration is required to achieve the desired amelioration of symptom.

In another embodiment, there is provided a pharmaceutical composition comprising a compound of Formula I (including Formulae II, III and IV) as a PARP inhibitor, or stereoisomers, tautomers, N-oxides, hydrates, solvates, isotope-substituted derivatives, or pharmaceutically acceptable salts thereof, or mixtures thereof, or prodrugs thereof and a pharmaceutically acceptable carrier.

Another embodiment of the present disclosure is directed to a pharmaceutical composition effective to treat or prevent cancer comprising a compound of Formula I (including Formulae II, III and IV) as a PARP inhibitor, or stereoisomers, tautomers, N-oxides, hydrates, solvates, isotope-substituted derivatives, or pharmaceutically acceptable salts thereof and prodrugs thereof, in combination with at least one known anticancer agent or a pharmaceutically acceptable salt thereof. In particular, the compound herein can be combined with other anticancer drugs related to the mechanism of DNA damage and repair, such as ATM inhibitors, ATR inhibitors, Wee1 inhibitors, DNA-PK inibitors; HDAC inhibitors such as Volinota, Romididesin, Papiseta and Bailesta;other anticancer drugs related to cell division, including Chk1/2 inhibitors, CDK4/6 inhibitors such as Paposinib; other targeted anticancer agents, including USP1 inhibitors, PRMT5 inhibitors, Polθ inhibitors, RAD51 inhibitors, and so on. Other known anticancer agents which may be used for anticancer combination therapy include, but are not limited to alkylating agents, such as busulfan, melphalan, chlorambucil, cyclophosphamide, ifosfamide, temozolomide, bendamustine, cis-platin, mitomycin C, bleomycin and carboplatin; topoisomerase I inhibitors, such as camptothecin, irinotecan and topotecan; topoisomerase II inhibitors, such as doxorubicin, epirubicin, aclacinomycin, mitoxantrone, elliptinium and etoposide; RNA/DNA antimetabolites, such as 5-azacytidine, gemcitabine, 5-fluorouracil, capecitabine and methotrexate; DNA antimetabolites, such as 5-fluoro-2'-deoxy-uridine, fludarabine, nelarabine, ara-C, pralatrexate, pemetrexed, hydroxyurea and thioguanine; antimitotic agent such as colchicine, vinblastine, vincristine, vinorelbine, paclitaxel, ixabepilone, cabazitaxel and docetaxel; antibodies such as mAb, panitumumab, necitumumab, nivolumab, pembrolizumab, ramucirumab, bevacizumab, pertuzumab, trastuzumab, cetuximab, obinutuzumab, ofatumumab, rituximab, alemtuzumab, ibritumomab, tositumomab, brentuximab, daratumumab, elotuzumab, Ofatumumab, Dinutuximab, Blinatumomab, ipilimumab, avastin, herceptin and mabthera; Antibody-Drug Conjugates (ADC) such as T-DM1, Trastuzumab Deruxtecan, Trastuzumab Emtansine, Datopotamab Deruxtecan, Gemtuzumab Ozogamicin, Brentuximab Vedotin, Inotuzumab Ozogamicin, Sacituzumab govitecan, Enfortumab Vedotin, Belantamab Mafodotin; kinase inhibitors such as imatinib, gefitinib, erlotinib, osimertinib, afatinib, ceritinib, alectinib, crizotinib, erlotinib, lapatinib, sorafenib, regorafenib, vemurafenib, dabrafenib, aflibercept, sunitinib, nilotinib, dasatinib, bosutinib, ponatinib, ibrutinib, cabozantinib, lenvatinib, vandetanib, trametinib, cobimetinib, axitinib, temsirolimus, Idelalisib, pazopanib, Torisel and everolimus. Other known anticancer agents which may be used for anticancer combination therapy include tamoxifen, letrozole, fulvestrant, mitoguazone, octreotide, retinoic acid, arsenic, zoledronic acid, bortezomib, carfilzomib, Ixazomib, vismodegib, sonidegib, denosumab, thalidomide, lenalidomide, Venetoclax, Aldesleukin (recombinant human interleukin-2) and Sipueucel-T (prostate cancer treatment vaccine).

In practicing the methods of the present disclosure, the compound of the disclosure may be administered together with at least one known anticancer agent in a unitary pharmaceutical composition. Alternatively, the compound of the disclosure may be administered separately from at least one known anticancer agent. In one embodiment, the compound of the disclosure and at least one known anticancer agent are administered substantially simultaneously, i.e. all agents are administered at the same time or one after another, provided that compounds reach therapeutic levels in the blood at the same time. In another embodiment, the compound of the disclosure and at least one known anticancer agent are administered according to individual dose schedule, provided that the compounds reach therapeutic levels in the blood.

Another embodiment of the present disclosure is directed to a bioconjugate to inhibit tumor. The bioconjugate is consisted of the compound described herein and at least one known therapeutically useful antibody, such as trastuzumab or rituximab, or growth factor, such as EGF or FGF, or cytokine, such as IL-2 or IL-4, or any molecule that can bind to cell surface. The antibodies and other molecules could deliver the compound described herein to its targets, making it an effective anticancer agent. The bioconjugates could also enhance the anticancer effect of the therapeutically useful antibodies, such as trastuzumab or rituximab.

Another embodiment of the present disclosure is directed to a pharmaceutical composition effective to inhibit tumor comprising the PARP inhibitor of Formula I (including Formulae II, III and IV), or pharmaceutically acceptable salts thereof, or prodrugs thereof, in combination with radiation therapy. In this embodiment, the compound of the disclosure may be administered at the same time as the radiation therapy or at a different time.

Yet another embodiment of the present disclosure is directed to a pharmaceutical composition effective for post-surgical treatment of cancer, comprising the PARP inhibitor of Formula I (including Formulae II, III and IV), or pharmaceutically acceptable salts thereof, or prodrug thereof. The disclosure also relates to a method of treating cancer by surgically removing tumor and then treating the mammal with the pharmaceutical composition described herein.

Pharmaceutical compositions of this disclosure include all pharmaceutical preparations which contain the compounds of the present disclosure in an amount that is effective to achieve its intended purpose. While individual needs vary, determination of optimal amounts of each component in the pharmaceutical preparations is within the skill of the art. Typically, the compounds or the pharmaceutically acceptable salt thereof may be administered to mammals, orally at a dose of about 0.0025 to 50 mg per kg body weight per day. Preferably, from approximately 0.01 mg/kg to approximately 10 mg/kg body weight is orally administered. If a known anticancer agent is also administered, it is administered in an amount that is effective to achieve its intended purpose. The optimal amounts of such known anticancer agents are well known to those skilled in the art.

The unit oral dose may comprise from approximately 0.01 to approximately 50 mg, preferably approximately 0.1 to approximately 10 mg of the compound of the disclosure. The unit dose may be administered one or more times, with one or more tablets daily, each containing from approximately 0.1 to approximately 50 mg, conveniently approximately 0.25 to 10 mg of the compound of the disclosure or its solvates.

In a topical formulation, the compound of the disclosure may be present at a concentration of approximately 0.01 to 100 mg per gram of carrier.

The compound of the disclosure may be administered as a raw chemical. The compounds of the disclosure may also be administered as part of a suitable pharmaceutical preparation containing pharmaceutically acceptable carriers (comprising excipients and auxiliaries), which facilitate the processing of the compounds into pharmaceutically acceptable preparations. Preferably, the pharmaceutical preparations, particularly oral preparations and those used for the preferred administration, such as tablets, draggers, and capsules, as well as solutions suitable for injection or oral administration, contain from approximately 0.01% to 99%, preferably from approximately 0.25% to 75% of active compound(s), together with excipient(s).

Also included within the scope of the present disclosure are the non-toxic pharmaceutically acceptable salts of the compounds of the present disclosure. Acid addition salts are formed by mixing a solution of the compounds of the present disclosure with a solution of a pharmaceutically acceptable non-toxic acid, such as hydrochloric acid, fumaric acid, maleic acid, succinic acid, acetic acid, citric acid, tartaric acid, carbonic acid, phosphoric acid, oxalic acid, and the like. Base addition salts are formed by mixing a solution of the compounds of the present disclosure with a solution of a pharmaceutically acceptable non-toxic base, such as sodium hydroxide, potassium hydroxide, choline hydroxide, sodium carbonate, tris(hydroxymethyl)aminomethane, N-methyl-glucamine and the like.

The pharmaceutical preparations of the disclosure may be administered to any mammal, so long as they may experience the therapeutic effects of the compounds of the disclosure. Foremost among such mammals are humans and veterinary animals, although the disclosure is not intended to be so limited.

The pharmaceutical preparations of the present disclosure may be administered by any means that achieve their intended purpose. For example, administration may be by parenteral, subcutaneous, intravenous, intramuscular, intraperitoneal, transdermal, buccal, intrathecal, intracranial, intranasal or topical routes. Alternatively or concurrently, administration may be by oral route. The dosage administered will be dependent upon the age, health, and weight of the recipient, type of concurrent treatment, frequency of treatment, and the nature of the effect desired.

The pharmaceutical preparations of the present disclosure are manufactured in a known manner, e.g., by means of conventional mixing, granulating, dragee-making, dissolving, or lyophilizing processes. Pharmaceutical preparations for oral use may be obtained by combining the active compounds with solid excipients, optionally grinding the resulting mixture, processing the mixture of granules after adding suitable auxiliaries if desired or necessary, thereby obtaining tablets or dragee cores.

Suitable excipients are, in particular, fillers, such as saccharides, e.g. lactose or sucrose, mannitol or sorbitol; cellulose preparations and/or calcium phosphates, e.g. tricalcium phosphate or calcium hydrogen phosphate; as well as binders, such as starch paste, including, e.g., maize starch, wheat starch, rice starch, potato starch, gelatin, tragacanth, methylcellulose, hydroxypropyl methylcellulose, sodium carboxymethylcellulose, and/or polyvinyl pyrrolidone. If desired, disintegrating agents may be added, such as the above-mentioned starches and also carboxymethyl-starch, cross-linked polyvinyl pyrrolidone, agar, or alginic acid or a salt thereof, such as sodium alginate. Auxiliaries are, in particular, flow-regulating agents and lubricants, e.g., silica, talc, stearic acid or salts thereof, such as magnesium stearate or calcium stearate, and/or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations, such as acetylcellulose phthalate or hydroxypropyl methylcellulose phthalate, are used. Dyes or pigments may be added to the tablets or dragee coatings, e.g., for identification or in order to characterize combinations of active compound doses.

Other pharmaceutical preparations, which may be used orally, include push-fit capsules made of gelatin, as well as soft, sealed capsules made of gelatin and a plasticizer, such as glycerol or sorbitol. The push-fit capsules may contain the active compounds in the form of granules, which may be mixed with fillers, such as lactose; binders, such as starches; and/or lubricants, such as talc or magnesium stearate and stabilizers. In soft capsules, the active compounds are preferably dissolved or suspended in suitable liquids, such as fatty oils, or liquid paraffin. In addition, stabilizers may be added.

Suitable formulations for parenteral administration include aqueous solutions of the active compounds, e.g., aqueous solutions and alkaline solutions of water-soluble salts. In addition, suspensions of the active compounds as appropriate oily injection suspensions may be administered. Suitable lipophilic solvents or vehicles include fatty oils, e.g., sesame oil, or synthetic fatty acid esters, e.g., ethyl oleate or triglycerides or polyethylene glycol-400, or cremophor, or cyclodextrins. Aqueous injection suspensions may contain substances which increase the viscosity of the suspension, e.g., sodium carboxymethyl cellulose, sorbitol, and/or dextran. Optionally, suspension stabilizers may also be contained.

In accordance with one aspect of the present disclosure, compounds of the disclosure are employed in topical and parenteral formulations and are used for the treatment of skin cancer.

The topical formulations of this disclosure are formulated preferably as oils, creams, lotions, ointments and the like by choice of appropriate carriers. Suitable carriers include vegetable or mineral oils, white petrolatum (white soft paraffin), branched chain fats or oils, animal fats and high molecular weight alcohol (greater than C₁₂). The preferred carriers are those in which the active ingredient is soluble. Emulsifiers, stabilizers, humectants and antioxidants may also be included, as well as agents imparting color or fragrance, if desired. Additionally, transdermal penetration enhancers may be employed in these topical formulations. Examples of such enhancers are found in U.S. Patent Nos. 3,989,816 and 4,444,762.

Creams are preferably formulated from a mixture of mineral oil, self-emulsifying beeswax and water in which the active ingredient, dissolved in a small amount of an oil, such as almond oil, is admixed. A typical example of such a cream is one which includes approximately 40 parts water, approximately 20 parts beeswax, approximately 40 parts mineral oil and approximately 1 part almond oil.

Ointments may be formulated by mixing a solution of the active ingredient in a vegetable oil, such as almond oil, with warm soft paraffin and allowing the mixture to cool. A typical example of such an ointment is one which includes approximately 30% almond oil and approximately 70% white soft paraffin by weight.

The present disclosure also involves use of the compounds of the disclosure for the manufacture of a medicament for the treatment of clinical symptoms in response to inhibition of the activity of PARP. The medicament may include the above-mentioned pharmaceutical compositions.

The following examples are illustrative, but not limiting, of the method and compositions of the present disclosure. Other suitable modifications and adaptations of the variety of conditions and parameters normally encountered in clinical therapy and which are obvious to those skilled in the art are within the spirit and scope of the disclosure.

### EXAMPLES

### General remarks

All reagents were of commercial quality. Solvents were dried and purified by standard methods. Mass spectrum analyses were recorded on a Platform II (Agilent 6110) quadrupole mass spectrometer fitted with an electrospray interface. ¹H NMR spectra was recorded at 400 MHz, on a Brücker Ascend 400 apparatus. Chemical shifts were recorded in parts per million (ppm) downfield from TMS (0.00 ppm), and J coupling constants were reported in hertz (Hz).

### Example 1

### 7-((4-(6-(methylcarbamoyl)pyridin-3-yl)piperazin-1-yl)methyl)-3,5-dihydrofuro[3,4-c]quinolin-4(1H)-one

a) Preparation of methyl 4-(((trifluoromethyl)sulfonyl)oxy)-2,5-dihydrofuran-3-carboxylate: To a solution of methyl 4-oxotetrahydrofuran-3-carboxylate (1.00 g, 0.69 mmol) in dry DCM (40 mL) was added DIEA (2.68 g, 2.07 mmol) at -78 °C under N₂ atmosphere and the solution was stirred at -78 °C for 30 min. Then Tf₂O (5.87 g, 2.07 mmol, 3.0 eq) was added slowly. The mixture was stirred at -78 °C for 30 min and room temperature for 1.5 hours. After completion, the mixture was quenched with water (5 mL) and the mixture was extracted with DCM (20 mL × 2). The combined organic phase was washed with brine, dried over anhydrous sodium sulfate and concentrated under reduced pressure. The residue was purified by chromatography over silica gel (Petroleum ether: Ethyl acetate = 20/1 to 10/1) to afford the title compound (1.60 g, yellow oil, yield: 83 %).
b) Preparation of methyl 4-(4-(methoxycarbonyl)-2-nitrophenyl)-2,5-dihydrofuran-3-carboxylate: To a mixture of methyl 4-(((trifluoromethyl)sulfonyl)oxy)-2,5-dihydrofuran-3-carboxylate (2.00 g, 7.24 mmol), (4-(methoxycarbonyl)-2-nitrophenyl)boronic acid (1.95 g, 8.69 mmol), KF (1.38 g, 23.89 mmol) in THF (40 mL) and H₂O (2 mL) was added Pd₂(dba)₃ (0.66 g, 0.72 mmol) and tri-tert-butylphosphine tetrafluoroborate (0.50 g, 1.73 mmol). The mixture was degassed and purged with N₂ 3 times and stirred at 70 °C for 16 hours under N₂ atmosphere. After completion, the mixture was quenched with water (20 mL) and the mixture was extracted with DCM (30 mL × 2). The combined organic phase was washed with brine, dried over anhydrous sodium sulfate and concentrated under reduced pressure. The residue was purified by chromatography over silica gel (Petroleum ether: Ethyl acetate = 10/1 to 3/1) to afford the title compound (1.76 g, yellow solid, yield: 76 %).
c) Preparation of methyl 4-oxo-1,3,4,5-tetrahydrofuro[3,4-c]quinoline-7-carboxylate: To a solution of methyl 4-(4-(methoxycarbonyl)-2-nitrophenyl)-2,5-dihydrofuran-3-carboxylate (1.76 g, 5.71 mmol) in AcOH (17 mL) was added Fe powder (1.60 g, 28.57 mmol). The resulting mixture was stirred at 80 °C for 2 hours. After completion, the reaction mixture was filtered and the filtrate was concentrated under reduced pressure. The residue was diluted with water (10 mL) and extracted with DCM (20 mL x 3). The combined organic phase was washed with brine, dried over anhydrous sodium sulfate and concentrated under reduced pressure to afford the title compound (1.40 g, red-brown solid, crude). MS(ESI, m/z): 246.05 [M+H]⁺.
d) Preparation of 7-(hydroxymethyl)-3,5-dihydrofuro[3,4-c]quinolin-4(1H)-one: To a suspension of methyl 4-oxo-1,3,4,5-tetrahydrofurol[3,4-c]quinoline-7-carboxylate (1.00 g, crude, 4.10 mmol) in THF (10 mL) was added LiAlH₄ (0.78 g, 20.40 mmol) at 0 °C under N₂. The resulting mixture was stirred at 0 °C for 20 minutes and then warmed to room temperature. After completion, the mixture was quenched with ice-water (10 mL) and 1 M aq. HCl to adjust pH to 3. The resulting mixture was extracted with EtOAc (30 mL). The combined organic phase was washed with brine, dried over anhydrous sodium sulfate and concentrated under reduced pressure to afford the title compound (0.13 g, light yellow solid, yield: 15%). MS(ESI, m/z): 218.21 [M+H]⁺.
e) Preparation of 7-(chloromethyl)-3,5-dihydrofuro[3,4-c]quinolin-4(1H)-one: To a suspension of 7-(hydroxymethyl)-3,5-dihydrofuro[3,4-c]quinolin-4(1H)-one (0.13 g, 0.60 mmol) in DCM (3 mL) was added DMF (4.00 mg, 0.06 mmol) and SOCl₂ (0.43 g, 3.60 mmol) at 0 °C. The resulting mixture was stirred at room temperature for 2 hours. After completion, the mixture was concentrated to afford the title compound (0.14 g, grey solid, crude). MS(ESI, m/z): 236.03 [M+H]⁺.
f) Preparation of 7-((4-(6-(methylcarbamoyl)pyridin-3-yl)piperazin-1-yl)methyl)-3,5-dihydrofuro[3,4-c]quinolin-4(1H)-one: To a suspension of 7-(chloromethyl)-3,5-dihydrofuro[3,4-c]quinolin-4(1H)-one (90.00 mg, crude), KI (13.92 mg, 0.80 mmol) and N-methyl-5-(piperazin-1-yl)picolinamide hydrochloride (115.14 mg, 0.57 mmol) in CH₃CN (4 mL) was added DIEA (245.00 mg, 1.90 mmol) at room temperature. The resulting suspension was stirred at 80 °C for 2 hours. After completion, the solvent was removed under vacuum. The residue was purified by Prep-HPLC (C18, CH₃CN/H₂O, 10-30%, 0.1% HCOOH added) to afford the target compound (40.00 mg, white solid, yield: 25%).

The following compounds of Examples 2-23 were prepared using a synthesis method similar to that described in Example 1.

| Example | Compound | MW | LC-MS (ESI) | ¹H NMR (400 MHz) |
|---|---|---|---|---|
| 1 | 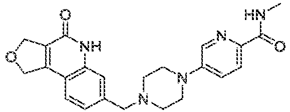 | 419.49 | 420.15 [M+H]⁺ | DMSO-d₆: δ 11.78 (s, 1H), 8.36 (d, J = 5.0 Hz, 1H). 8.23 (d, J = 2.9 Hz, 1H), 7.79 (d, J = 8.8 Hz, 1H), 7.55 - 7.27 (m, 3H), 7.17 (d, J = 8.0 Hz, 1H), 5.26 (t, J = 4.0 Hz, 2H), 4.92 (t, J = 3.9 Hz, 2H), 3.58 (s, 2H), 2.74 (d, J = 4.8 Hz, 3H), 2.56 - 2.48 (m, 8H). |
| 2 | 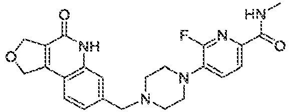 | 437.48 | 438.15 [M+H]⁺ | DMSO-d₆: δ 11.82 (s, 1H), 8.40 (d, J = 5.0 Hz, 1H), 7.84 (d, J = 8.1 Hz, 1H), 7.62 - 7.51 (m, 1H), 7.44 (d, J = 8.1 Hz, 1H), 7.39 (s, 1H), 7.21 (d, J = 7.6 Hz, 1H), 5.39 - 5.19 (m, 2H), 5.07 - 4.70 (m, 2H), 3.62 (s, 2H), 3.21 - 3.09 (m. 4H), 2.76 (d, J = 4.7 Hz, 3H), 2.60 - 2.53 (m, 4H). |
| 3 | 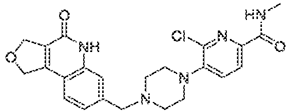 | 453.93 | 454.25 [M+H]⁺ | CDCl₃: δ 10.68 (s, 1H), 8.07 (d, J = 8.2 Hz, 1H), 7.67 (s, 1H), 7.39 (d, J = 10.7 Hz, 2H), 7.34 (d, J = 8.0 Hz. 1H), 7.29 (d, J = 8.8 Hz. 1H), 5.38 (s, 2H), 5.23 (s. 2H). 3.70 (s, 2H), 3.25 - 3.12 (m, 4H), 3.01 (d, J = 4.4 Hz, 3H), 2.76 - 2.63 (m, 4H). |
| 4 | 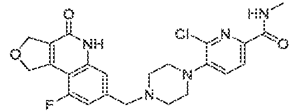 | 433.51 | 434.20 [M+H]⁺ | DMSO-d₆: δ 11.78 (s, 1H), 8.38 (d, J = 4.5 Hz, 1H), 7.76 (d, J = 8.4 Hz, 1H), 7.43 (dd, J = 14.5, 8.0 Hz, 2H), 7.37 (s, 1H), 7.18 (d, J = 7.5 Hz, 1H). 5.26 (s, 2H), 4.93 (s, 2H), 3.60 (s, 2H). 2.99 - 2.88 (m, 4H), 2.77 (d, J = 4.8 Hz, 3H), 2.57 - 2.52 (m, 4H), 2.46 (d, J = 6.4 Hz, 3H). |
| 5 | 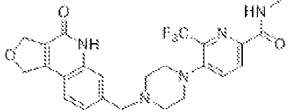 | 487.48 | / | / |
| 6 | 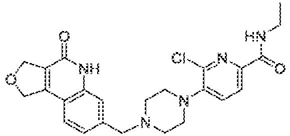 | 467.95 | 468.05 [M+H]⁺ | CDCl₃: δ 10.30 - 10.20 (m, 1H), 8.08 (d, J = 8.2 Hz, 1H), 7.72 - 7.63 (m. 1H), 7.40 (d, J = 8.5 Hz, 1H), 7.34 (d, J = 8.0 Hz, 2H). 7.29 (d, J = 8.5 Hz, 1H). 5.42 - 5.33 (m, 2H), 5.27 5.17 (m, 2H), 3.70 (s, 2H), 3.53 - 3.44 (m, 2H), 3.25 - 3.10 (m, 4H), 2.75 - 2.60 (m, 4H), 1.26 (t, J = 7.3 Hz, 3H). |
| 7 | 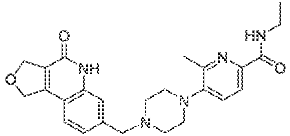 | 447.54 | 448.15 [M+H]⁺ | CDCl₃: δ 10.33 - 10.28 (m, 1H), 8.01 - 7.91 (m, 2H), 7.40 - 7.27 (m, 4H), 5.40 - 5.35 (m. 2H), 5.22 (t, J =4.0 Hz, 2H), 3.70 (s, 2H), 3.53 - 3.45 (m, 2H), 3.05 - 2.95 (m, 4H), 2.72 - 2.60 (m, 4H), 2.52 (s, 3H), 1.27 (t, J = 7.3 Hz, 3H). |
| 8 | 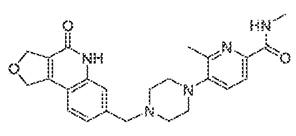 | 501.51 | / | / |
| 9 | 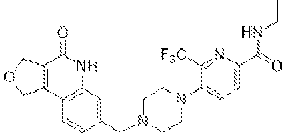 | 471.92 | / | / |
| 10 | 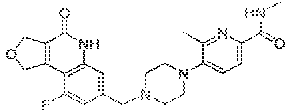 | 451.50 | / | / |
| 11 | 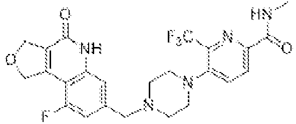 | 505.47 | / | / |
| 12 | 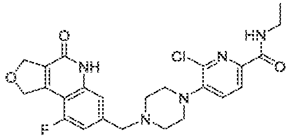 | 485.94 | / | / |
| 13 | 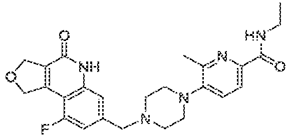 | 465.53 | / | / |
| 14 | 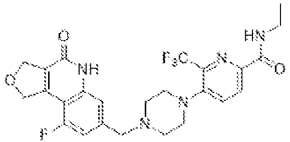 | 519.50 | / | / |
| 15 | 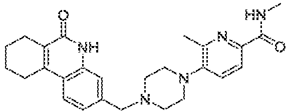 | 445.57 | / | / |
| 16 | 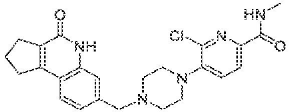 | 451.96 | 452.40 [M+H]⁺ | CDCl₃: δ 10 71 (s, 1H), 8.07 (d, J = 8.2 Hz, 1H), 7.73 - 7.61 (m, 1H), 7.50 (d, J = 8.1 Hz. 2H), 7.42 (d, J = 8.2 Hz, 1H), 7.31 (d, J = 7.8 Hz, 1H), 3.85 (s, 2H), 3.37 - 3.24 (m, 4H), 3.13 (t, J = 7.5 Hz, 2H), 3.00 (d, J = 5.1 Hz. 5H), 2.91 - 2.80 (m. 4H), 2.32 --- 2.09 (m, 2H). |
| 17 | 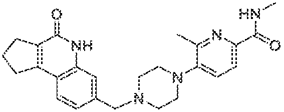 | 431.54 | 432.15 [M+H]⁺ | CDCl₃: δ 10.85 (s. 1H), 8.02 - 7.89 (m, 2H), 7.49 (d, J = 8.1 Hz, 1H), 7.40 - 7.31 (m, 2H), 7.28 - 7.27 (m, 1H). 3.71 (s, 2H), 3.15 (1, J = 7.5 Hz, 2H), 3.05 - 2.95 (m, 9H), 2.74 - 2.64 (m, 4H), 2.51 (s, 3H), 2.34 - 2.11 (m, 2H). |
| 18 | 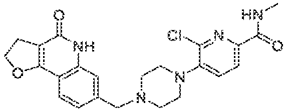 | 453.93 | / | / |
| 19 | 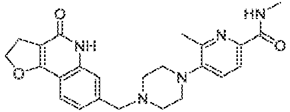 | 433.51 | / | / |
| 20 | 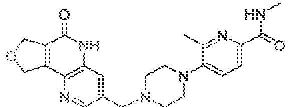 | 434.50 | / | / |
| 21 | 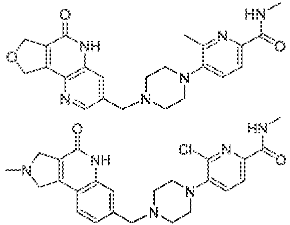 | 466.97 | / | / |
| 22 | 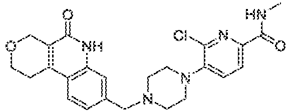 | 467.95 | / | / |
| 23 | 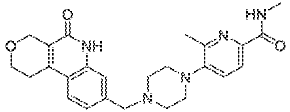 | 447.54 | / | / |

### Example 24

### 7-((4-(2-methyl-6-(methylcarbamoyl)pyridin-3-yl)piperazin-1-yl)methyl)pyrrolo[1,2-a]quinoxalin-4(5H)-one

a) Preparation of methyl 1-(4-(methoxycarbonyl)-2-nitrophenyl)-1H-pyrrole-2-carboxylate: To a solution of methyl 4-fluoro-3-nitrobenzoate (1.00 g, 5.03 mmol) in DMF (20 mL) was added Cs₂CO₃ (1.97 g, 6.04 mmol) and methyl 1H-pyrrole-2-carboxylate (0.63 g, 5.03 mmol). The mixture was stirred at 60°C for 4 hours. After completion, the mixture was added water (50 mL) and extracted with DCM (40 mL × 2). The combined organic phase was washed with brine, dried over anhydrous sodium sulfate and concentrated under reduced pressure. The residue was purified by chromatography over silica gel (Petroleum ether: Ethyl acetate = 100/1 to 50/1) to give the title compound (0.8 g, yellow solid, yield: 52.3%). MS(ESI, m/z): 305.05 [M+H]⁺.
b) Preparation of methyl 4-oxo-4,5-dihydropyrrolo[1,2-a]quinoxaline-7-carboxylate: To a solution of methyl 1-(4-(methoxycarbonyl)-2-nitrophenyl)-1H-pyrrole-2-carboxylate (800.0 mg, 2.6 mmol) in AcOH (16 mL) was added Fe powder (736.8 mg, 13.2 mmol). The resulting mixture was stirred at 80 °C for 2 hours. After completion, the reaction mixture was filtered and the filtrate was concentrated under reduced pressure. The residue was diluted with water (20 mL) and extracted with DCM (20 mL × 3). The combined organic phase was washed with brine, dried over anhydrous sodium sulfate and concentrated under reduced pressure to give the title compound (200 mg, yellow solid, yield: 31.4%). MS(ESI, m/z): 243.05 [M+H]⁺.
c) Preparation of 7-(hydroxymethyl)pyrrolo[1,2-a]quinoxalin-4(5H)-one: To a solution of methyl 4-oxo-4,5-dihydropyrrolo[1,2-a]quinoxaline-7-carboxylate (100.0 mg, 0.4 mmol) in THF (5 mL) was added LiAlH₄ (0.06 g, 1.6 mmol) at 0 °C under N₂. The resulting mixture was stirred at 0 °C for 20 minutes and then warmed to room temperature. After stirred for 4 hours, the mixture was added DCM (30 mL) and quenched with water (0.06 mL), then added 15% sodium hydroxide aqueous solution (0.06mL) and water (0.18 mL). The mixture was stirred at room temperature for 15 min. The mixture was dried over anhydrous sodium sulfate and filtered, and filter cake was washed with MeOH (60 mL). The filtrate was concentrated under reduced pressure to give the title compound (120 mg crude, white solid). MS(ESI, m/z): 215.25 [M+H]⁺.
d) Preparation of 7-(chloromethyl)pyrrolo[1,2-a]quinoxalin-4(5H)-one: To a suspension of 7-(hydroxymethyl)pyrrolo[1,2-a]quinoxalin-4(5H)-one (100 mg, 0.46 mmol) in DCM (10 mL) was added DMF (2 drops) and SOCl₂ (220.3 mg, 1.85 mmol) at 0 °C. The resulting mixture was stirred at room temperature for 2 hours. After completion, the mixture was concentrated to give the title compound (140 mg crude, grey solid). MS(ESI, m/z): 233.15 [M+H]⁺.
e) Preparation of 7-((4-(2-methyl-6-(methylcarbamoyl)pyridin-3-yl)piperazin-1-yl)methyl)pyrrolo[1,2-a]quinoxalin-4(5H)-one: To a suspension of 7-(chloromethyl)pyrrolo[1,2-a]quinoxalin-4(5H)-one (30.0 mg crude, 0.13 mmol), KI (2.1 mg, 0.01 mmol) and N,6-dimethyl-5-(piperazin-1-yl)picolinamide (30.3 mg, 0.13 mmol) in CH₃CN (5 mL) was added DIEA (83.9 mg, 0.65 mmol) at room temperature. The resulting suspension was stirred at 80 °C overnight. After completion, the solvent was removed under vacuum. The residue was purified by Prep-TLC (DCM: MeOH=10:1) to give the target compound (23.8 mg, white solid, yield: 42.9%).

The following compounds of Examples 25-27 were prepared using a synthesis method similar to that described in Example 1 or Example 24.

| Example | Compound | MW | LC-MS (ESI) | ¹H NMR (400 MHz) |
|---|---|---|---|---|
| 24 | 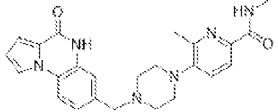 | 430.51 | 431.35 [M+H]⁺ | CD₃₀D: δ 8.43 (s, 1H), 7.91 - 7.81 (m, 2H), 7.77 (d, J = 8.4 Hz, 1H), 7.39 (d, J = 8.3 Hz, 1H), 7.30 (s, 1H), 7.25 (d, J = 8.6 Hz, 1H), 7.18 (d, J = 3.9 Hz, 1H), 6.69 (t, J = 3.6 Hz, 1H), 3.67 (s, 2H), 3.08 - 2.98 (m, 4H), 2.96 (d, J = 4.4 Hz, 3H). 2.76 - 2.64 (m, 4H), 2.51 (s, 3H). |
| 25 | 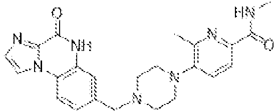 | 431.5 | 432.45 [M+H]⁺ | CDCl₃: δ 12.25 (s, 1H), 7.98 - 7.94 (m, 3H), 771 - 7.64 (m, 3H),7.38 (d, J = 8.1 Hz, 1H), 7.35 (d, J = 8.5 Hz, 1H). 3.68 (s, 2H), 3.01 (s, 3H), 3.01 - 2.98 (m, 4H), 2.72 - 2.62 (m, 4H), 2.51 (s, 3H). |
| 26 | 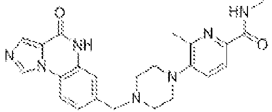 | 431.5 | 432.15 [M+H]⁺ | DMSO-d₆: δ 11.38 (s, 1H), 9.03 (s, 1H), 8.42 (s, 1H), 8.13 (s, 1H), 7.90-7.68 (m, 2H), 7.47 (s, 1H), 7.32 (s, 1H), 7.20 (s, 1H), 3.57 (s, 2H), 3.04 - 2.85 (m, 4H), 2.77 (d, J = 4.7 Hz. 3H), 2.63 - 2.52 (m, 4H), 2.51 (s, 3H). |
| 27 | 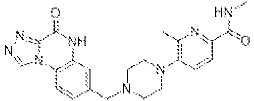 | 432.49 | / | / |

### Example 28

### 7-((4-(2-methyl-6-(methylcarbamoyl)pyridin-3-yl)piperazin-1-yl)methyl)pyrazolo[1,5-a]quinoxalin-4(5H)-one

a) Preparation of methyl 3-amino-4-fluorobenzoate: To a solution of methyl 4-fluoro-3-nitrobenzoate (500 mg, 2.5 mmol) in MeOH (6 mL) was added Pd/C (50 mg). The resulting mixture was stirred at room temperature overnight under H₂. After completion, the reaction mixture was filtered, the filtrate was concentrated to give the title compound (400 mg, white solid, yield: 94%). MS(ESI, m/z): 170.15 [M+H]⁺.
b) Preparation of methyl 4-fluoro-3-(1H-pyrazole-5-carboxamido)benzoate: To a solution of methyl 3-amino-4-fluorobenzoate (400 g, 2.4 mmol) in DMF (20 mL) was added 1H-pyrazole-5-carboxylic acid (398 mg, 3.6 mmol) and HATU (1.35 g, 3.6 mmol) and DIEA (1.2 g, 9.5 mmol). The mixture was stirred at 80°C for 16 hours. After completion, the mixture was added water (50 mL) and extracted with DCM (40 mL × 2). The combined organic phase was washed with brine, dried over anhydrous sodium sulfate and concentrated under reduced pressure. The residue was purified by chromatography over silica gel (Petroleum ether: Ethyl acetate = 100/1 to 50/1) to give the title compound (340 mg, yellow solid, yield: 54%). MS(ESI, m/z): 264.05 [M+H]⁺.
c) Preparation of methyl 4-oxo-4,5-dihydropyrazolo[1,5-a]quinoxaline-7-carboxylate: To a solution of methyl 4-fluoro-3-(1H-pyrazole-5-carboxamido)benzoate (120.0 mg, 0.46 mmol) in DMF (5 mL) was added K₂CO₃(126 mg, 0.9 mmol). The mixture was stirred at 120 °C for 16 hours. After completion, the reaction mixture was filtered and the filtrate was concentrated under reduced pressure. The residue was diluted with water (20 mL) and extracted with DCM (20 mL × 3). The combined organic phase was washed with brine, dried over anhydrous sodium sulfate and concentrated under reduced pressure to give the title compound (100 mg, yellow solid, yield: 90%). MS(ESI, m/z): 244.05 [M+H]⁺.
d) Preparation of 7-(hydroxymethyl)pyrazolo[1,5-a]quinoxalin-4(5H)-one: To a solution of methyl 4-oxo-4,5-dihydropyrazolo[1,5-a]quinoxaline-7-carboxylate (100.0 mg, 0.4 mmol) in THF (5 mL) was added LiAlH₄ (1.6 mL, 1.6mmol) at 0 °C under N₂. The resulting mixture was stirred at 0 °C for 20 minutes and then warmed to room temperature. After stirred for 4 hours, the mixture was added DCM (30 mL) and quenched with water (0.06 mL), then added 15% sodium hydroxide aqueous solution (0.06mL) and water (0.18 mL). The mixture was stirred at room temperature for 15 min. The mixture was dried over anhydrous sodium sulfate and filtered, and the filter cake was washed with MeOH (60 mL). The filtrate was concentrated under reduced pressure to give the title compound (120 mg crude, white solid). MS(ESI, m/z): 216.25 [M+H]⁺.
e) Preparation of 7-(chloromethyl)pyrazolo[1,5-a]quinoxalin-4(5H)-one: To a suspension of 7-(hydroxymethyl)pyrazolo[1,5-a]quinoxalin-4(5H)-one (120 mg, 0.56 mmol) in DCM (10 mL) was added DMF (2 drops) and SOCl₂ (398.0mg, 3.35 mmol) at 0 °C. The resulting mixture was stirred at room temperature for 2 hours. After completion, the mixture was concentrated to give the title compound (130 mg crude, grey solid). MS(ESI, m/z): 234.12 [M+H]⁺.
f) Preparation of 7-((4-(2-methyl-6-(methylcarbamoyl)pyridin-3-yl)piperazin-1-yl)methyl)pyrazolo[1,5-a]quinoxalin-4(5H)-one: To a 7-(chloromethyl)pyrazolo[1,5-a]quinoxalin-4(5H)-one (130.0 mg crude, 0.56 mmol), KI (18 mg, 0.01 mmol) and N,6-dimethyl-5-(piperazin-1-yl)picolinamide (130.5 mg, 0.56 mmol) in CH₃CN (5 mL) was added DIEA (360.0 mg, 2.79 mmol) at room temperature. The resulting suspension was stirred at 80 °C overnight. After completion, the solvent was removed under vacuum. The residue was purified by Prep-TLC (DCM: MeOH=10:1) to give the target compound (17.3 mg, white solid, three-step yield:10%).

The compound of Example 29 was prepared using a synthesis method similar to that described in Example 28.

| Example | Compound | MW | LC-MS | ¹H NMR (400 MHz) |
|---|---|---|---|---|
| 28 | 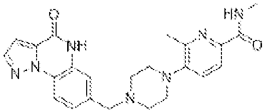 | 431.5 | 432.15 [M+H]⁺ | DMSO-*d*₆: δ 11.87 (s, 1H), 8.42 (d, *J* = 5.5 Hz, 1H), 8.14 - 8.04 (m, 2H), 7.79 (d, *J* = 8.4 Hz, 1H), 7.48 (d, *J* = 8.3 Hz, 1H), 7.41 (s, 1H), 7.30 (d, *J* = 8.4 Hz, 1H), 7.14 (s, 1H), 3.63 (s, 2H), 2.99 - 2.92 (m, 4H), 2.80 (d, *J* = 4.5 Hz, 3H), 2.63 - 2.56 (m, 4H), 2.52 (s, 3H). |
| 29 | 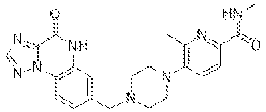 | 432.49 | 433.15 [M+H]⁺ | DMSO-*d*₆: δ 12.33 (s, 1H), 8.58 (s, 1H), 8.39 (d, J = 6.0 Hz, 1H), 8.04 (d, J = 7.9 Hz, 1H), 7.76 (d, J = 8.4 Hz, 1H), 7.45 (d, J = 7.8 Hz, 2H), 7.34 (d, J = 8.2 Hz, 1H), 3.63 (s, 2H), 3.00 - 2.85 (m, 4H), 2.76 (d, J = 4.5 Hz, 3H). 2.63 - 2.54 (m, 4H), 2.49 (s, 3H). |

The compound of Example 30 was prepared using a synthesis method similar to that described in Example 31.

### Example 31

### 8-((4-(2-methyl-6-(methylcarbamoyl)pyridin-3-yl)piperazin-1-yl)methyl)imidazo[1,2-c]quinazolin-5(6H)-one

a) Preparation of methyl 4-(1H-imidazol-2-yl)-3-nitrobenzoate: To a suspension of methyl 4-formyl-3-nitrobenzoate (4.5 g, 21.5 mmol) in MeOH (100.0 mL) was added NH₃-MeOH (7 mol/L, 45.0 mL) and glyoxal (40% wt. in H₂O, 22.0 mL) under N₂ at room temperature for 16 hours. After completion, the mixture was concentrated under reduced pressure. The residue was purified by column chromatography over silica gel (Petroleum ether: Ethyl acetate = 100/ 1 to 20/ 1) to afford the title compound (540.0 mg, yellow solid, 10 % yield). MS(ESI, m/z): 248.05 [M+H]⁺.
b) Preparation of methyl 3-amino-4-(1H-imidazol-2-yl)benzoate: A solution of methyl 4-(1H-imidazol-2-yl)-3-nitrobenzoate (540.0 mg, 2.2 mmol), SnCl₂-H₂O (2.1 g, 10.9 mmol) in EA (20.0 mL) was stirred at 80 °C for 2 hours. After completion, the mixture was adjusted to pH = 8 with NaHCO₃ aqueous solution and extracted with EA (40.0 mL × 3). The combined organic phase was dried over sodium sulfate and concentrated under reduced pressure. The residue was purified by column chromatography over silica gel (Petroleum ether: Ethyl acetate = 50/ 1 to 5/ 1) to afford the title compound (230.0 mg, yellow solid, 48 % yield). MS(ESI, m/z): 218.25 [M+H]⁺.
c) Preparation of methyl 5-oxo-5,6-dihydroimidazo[1,2-c]quinazoline-8-carboxylate: To a suspension of methyl 3-amino-4-(1H-imidazol-2-yl)benzoate (230.0 mg, 1.1 mmol) in dioxane (10 mL) was added BTC (330.0 mg, 1.1 mmol) under N₂. The resulting mixture was stirred at 80 °C for 16 hours . After completion, the mixture was concentrated. The residue was washed by water to give the title compound (crude, 380.0 mg, yellow solid). MS(ESI, m/z): 244.05 [M+H]⁺.
d) Preparation of 8-(hydroxymethyl)imidazo[1,2-c]quinazolin-5(6H)-one: To a suspension of methyl 5-oxo-5,6-dihydroimidazo[1,2-c]quinazoline-8-carboxylate (380.0 mg, 1.6 mmol) in THF (5 mL) was added LiAlH₄-THF (1 mol/L, 3.2 ml) under N₂ at 0 °C. The mixture was warmed to room temperature and stirred for 2 hours. After completion, the mixture was quenched with 1M hydrochloric acid aqueous solution (1.0 mL) and concentrated. The residue was diluted with water (10 mL) to give a yellow suspension. The solid was collected by filtration, washed with water (10.0 mL), diethyl ether (10.0 mL) and dried to give the title compound (crude, 270.0 mg, yellow solid).
e) Preparation of 8-(chloromethyl)imidazo[1,2-c]quinazolin-5(6H)-one: To a suspension of 8-(hydroxymethyl)imidazo[1,2-c]quinazolin-5(6H)-one (270.0 mg, crude, 1.3 mmol) in DCM (10 mL) was added DMF (19.0 mg, 0.3 mmol) and thionyl chloride (773.5 mg, 6.5 mmol) dropwise at 0 °C. The resulting mixture was stirred at room temperature for 1 hour. After completion, the mixture was concentrated to give the title product (crude, 270.0 mg, grey solid) used directly in the next step without further purification. MS(ESI, m/z): 234.00 [M+H]⁺.
f) Preparation of 8-((4-(2-methyl-6-(methylcarbamoyl)pyridin-3-yl)piperazin-1-yl)methyl)imidazo[1,2-c]quinazolin-5(6H)-one: To a solution of 8-(chloromethyl)imidazo[1,2-c]quinazolin-5(6H)-one (270.0 mg, 1.15 mmol), KI (19.1. mg, 0.1mmol) and N,6-dimethyl-5-(piperazin-1-yl)picolinamide (322.9 mg, 1.4 mmol) in CH₃CN (10.0 mL) was added DIEA (741.8 mg, 5.8 mmol) at room temperature. The resulting solution was stirred at 80 °C for 3 hours. After completion, the solvent was removed under vacuum. The residue was purified by Prep-TLC to give the target compound(27.0 mg, white solid, 4 steps yield: 6%,).

The following compounds of Examples 32-33 were prepared using a synthesis method similar to that described in Example 31.

The following compounds of Examples 34-35 were prepared using a synthesis method similar to that described in Example 24.

The following compounds of Examples 36-47 were prepared using a synthesis method similar to that described in Example 28.

| Example | Compound | MW | LC-MS (ESI) | ¹H NMR (400 MHz) |
|---|---|---|---|---|
| 30 | 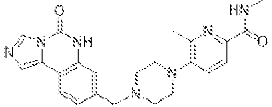 | 431.5 | 432.35 [M+H]+ | DMSO-d6: δ 11.64 (s, 1H), 8.45 (s, 1H), 8.39 (d, J = 5.2 Hz, 1H), 7.91 (d, J = 8.1 Hz, 1H), 7.77 (s, 1H), 7.75 (s, 1H), 7.45 (d, J = 8.3 Hz, 1H), 7.26 (s, 1H), 7.21 (d, J = 8.0 Hz, 1H), 3.59 (s, 2H), 2.97-2.87 (m, 4H). 2.76 (d, J = 4.8 Hz, 3H), 2.60-2.51 (s, 4H), 2.45 (s, 3H). |
| 31 | 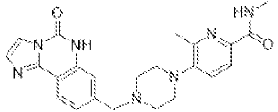 | 431.5 | 432.15 [M+H]+ | DMSO-d6: δ 11.94 (s, 1H), 8.40 (s, 1H), 8.07 (d, J = 8.1 Hz, 1H), 7.84 (s, 1H), 7.76 (d, J = 8.2 Hz, 1H), 7.45 (d, J = 8.4 Hz, 1H), 7.41 (s, 1H), 7.35 (s, 1H), 7.28 (d, J = 8.1 Hz, 1H), 3.62 (s, 2H), 3.01 - 2.89 (m, 4H), 2.76 (d, J = 4.5 Hz, 3H), 2.64 - 2.52 (m, 4H), 2.47 (s, 3H). |
| 32 | 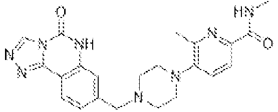 | 432.49 | / | / |
| 33 | 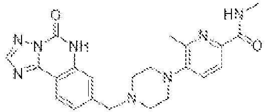 | 432.49 | / | / |
| 34 | 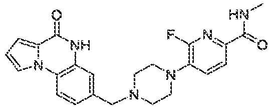 | 434.48 | 435.10 [M+H]+ | CDCl3: δ 9.68 (s, 1H), 7.99 (d, J = 8.3 Hz, 1H), 7.70 (s, 1H), 7.63 (d, J = 8.3 Hz, 1H), 7.51 (s, 1H), 7.37 - 7.26 (m, 3H), 7.23 (d, J = 8.1 Hz, 1H), 6.70 (s, 1H), 3.64 (s, 2H), 3.32 - 3.19 (m, 4H), 2.99 (d, J = 5.0 Hz, 3H), 2.72 - 2.60 (m, 4H). |
| 35 | 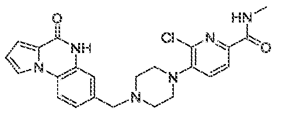 | 450.93 | 451.35 [M+H]+ | CDC13: δ 9.58 (s, 1H), 8.07 (d, J = 7.6 Hz, 1H), 7.80 - 7.56 (m, 3H), 7.40 (d. J = 7.5 Hz, 1H), 7.31 - 7.29 (m, 2H), 7.23 (s, 1H), 6.70 (s, 1H), 3.66 (s, 2H), 3.28 - 3.12 (m, 4H), 3.01 (d, J = 4.5 Hz, 3H), 2.78 - 2.65 (m, 4H). |
| 36 | 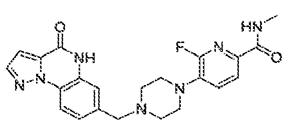 | 435.46 | 436.40 [M+H]+ | DMSO-d6: δ 11.84 (s, 1H), 8.37 (d, J = 4.7 Hz, 1H), 8.16 - 7.96 (m, 2H), 7.81 (d, J = 7.9 Hz, 1H), 7.54 (t, J = 9.3 Hz, 1H), 7.37 (s, 1H). 7.26 (d, J = 8.0 Hz, 1H), 7.11 (s, 1H), 3.58 (s, 2H), 3.21 - 3.05 (m, 4H), 2.73 (d, J = 4.3 Hz, 3H),2.60 - 2.51 (m, 4H). |
| 37 | 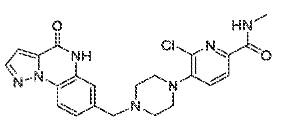 | 451.92 | 452.35 [M+H]+ | DMSO-d6: δ 11.85 (s, 1H), 8.41 (d, J = 4.6 Hz, 1H), 8.14 - 7.98 (m, 2H), 7.90 (d, J = 7.9 Hz, 1H), 7.63 (d, J = 8.3 Hz, 1H), 7.36 (s, 1H), 7.26 (d, J = 7.7 Hz, 1H), 7.11 (s, 1H), 3.59 (s, 2H), 3.15 - 3.03 (m, 4H), 2.75 (d, J = 4.3 Hz, 3H), 2.63 - 2.53 (m, 4H). |
| 38 | 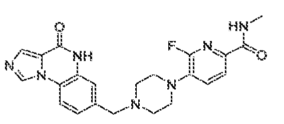 | 435.46 | 436.20 [M+H]+ | DMSO-d6: δ 11.36 (s, 1H), 9.00 (s, 1H), 8.37 (d, J = 4.2 Hz, 1H), 8.11 (d, J = 8.2 Hz, 1H), 7.81 (d, J = 7.7 Hz, 2H), 7.56-7.51 (m, 1H), 7.29 (s, 1H), 7.18 (d, J = 8.2 Hz. 1H), 3.55 (s, 2H), 3.23 - 3.08 (m, 4H), 2.73 (d, J = 4.2 Hz, 3H), 2.62 - 2.50 (m, 4H). |
| 39 | 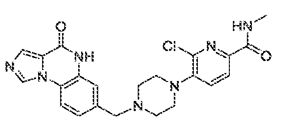 | 451.92 | 452.35 [M+H]+ | DMSO-d6: δ 9.01 (s, 1H), 8.42 (d, J = 4.8 Hz, 1H), 8.12 (d, J = 8.3 Hz, 1H), 7.90 (d, J = 8.2 Hz, 1H), 7.82 (s, 1H), 7.63 (d, J = 8.2 Hz, 1H), 7.29 (s. 1H), 7.19 (d, J = 8.1 Hz, 1H), 3.54 (s. 2H), 3.17 - 2.99 (m, 4H), 2.75 (d. J = 4.4 Hz, 3H),2.62 - 2.52 (m, 4H). |
| 40 | 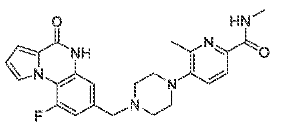 | 448.50 | 449.20 [M+H]+ | DMSO-d6: δ 11.45 (s, 1H), 8.41 (d, J = 4.8 Hz, 1H), 7.96 (s, 1H), 7.77 (d, J = 8.2 Hz, 1H), 7.46 (d, J = 8.5 Hz, 1H), 7.24 - 7.01 (m, 3H), 6.70 (t, J = 3.4 Hz, 1H), 3.56 (s, 2H), 3.00 - 2.90 (m, 4H), 2.77 (d, J = 4.7 Hz, 3H), 2.64 - 2.51 (m, 4H), 2.47 (s, 3H). |
| 41 | 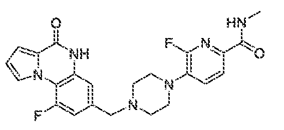 | 452.47 | 453.15 [M+H]+ | DMSO-d6: δ 11.45 (s, 1H), 8.58 - 8.19 (m, 1H), 7.96 (s, 1H), 7.82 (d, J = 8.1 Hz, 1H), 7.55 (t, J = 9.4 Hz, 1H), 7.17 - 7.02 (m, 3H), 6.70 (t, J = 3.4 Hz, 1H), 3.55 (s, 2H), 3.21 - 3.03 (m, 4H), 2.73 (d, J = 4.7 Hz, 3H), 2.59 - 2.52 (m, 4H). |
| 42 | 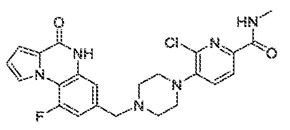 | 468.92 | 469.20 [M+H]+ | DMSO-d6: δ 11.44 (s, 1H), 8.42 (q, J = 5.5 Hz, 1H), 8.02 - 7.83 (m, 2H), 7.65 (d, J = 8.2 Hz, 1H), 7.18 - 6.99 (m, 3H), 6.70 (dd, J = 3.9, 2.8 Hz, 1H), 3.56 (s, 2H), 3.14 - 3.01 (m, 4H), 2.76 (d, J = 4.8 Hz, 3H), 2.64 - 2.53 (m, 4H). |
| 43 | 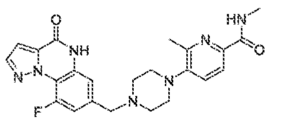 | 449.49 | 450.15 [M+H]+ | DMSO-d6: δ 8.50 - 8.40 (m, 1H), 8.38 (s. 2H), 7.79 (d, J = 8.2 Hz, 1H), 7.49 (d, J = 8.3 Hz, 1H), 7.24 (s, 1H), 7.22 - 7.14 (m, 2H), 3.62 (s, 2H), 3.05 - 2.88 (m, 4H), 2.79 (d, J = 4.8 Hz, 3H), 2.65 - 2.56 (m, 4H), 2.49 (s, 3H). |
| 44 | 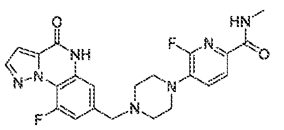 | 453.45 | 454.10 [M+H]+ | DMSO-d6: δ 12.07 (s, 1H), 8.49 - 8.40 (m, 1H), 8.36 (s, 1H), 7.84 (d, J = 8.4 Hz, 1H), 7.58 (dd, J = 10.6. 8.0 Hz, 1H), 7.23 (s, 1H), 7.23 - 7.11 (m, 2H), 3.60 (s, 2H), 3.23 - 3.09 (m, 4H), 2.76 (d, J = 4.7 Hz, 3H), 2.62 - 2.55 (m, 4H). |
| 45 | 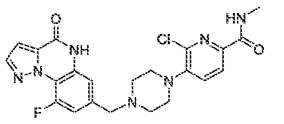 | 469.91 | 470.10 [M+H]+ | DMSO-d6: δ 12.00 (s, 1H), 8.65 - 8.32 (m, 1H), 8.07 (d, J = 2.1 Hz, 1H), 7.90 (d, J = 8.3 Hz, 1H), 7.64 (d, J = 8.2 Hz, 1H), 7.34 - 6.85 (m, 3H), 3.58 (s, 2H), 3.14 - 3.03 (m, 4H), 2.74 (d, J = 4.7 Hz, 3H), 2.61 - 2.52 (m, 4H). |
| 46 | 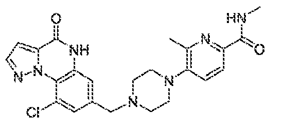 | 465.94 | 466.10 [M+H]+ | DMSO-d6: δ 12.02 (s, 1H), 8.44 (q, J = 5.5 Hz, 1H), 8.13 (d, J = 2.2 Hz, 1H), 7.80 (d, J = 8.2 Hz, 1H), 7.50 (d, J = 8.3 Hz. 1H), 7.38 (d. J = 9.0 Hz, 2H), 7.22 (d, J = 1.9 Hz, 1H), 3.62 (s, 2H), 3.00 - 2.93 (m, 4H), 2.80 (d, J = 4.8 Hz, 3H), 2.65 - 2.57 (m, 4H), 2.51 (s, 3H). |
| 47 | 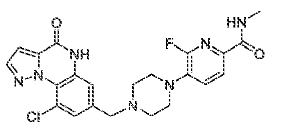 | 469.91 | 470.10 [M+H]+ | DMSO-d6: δ 12.01 (s, 1H), 8.41 (q, J = 5.1, 4.7 Hz, 1H), 8.13 (d, J = 2.1 Hz, 1H), 7.84 (d, J = 8.3 Hz, 1H), 7.62 - 7.53 (m, 1H), 7.37 (d, J = 9.3 Hz, 2H), 7.21 (d, J = 2.0 Hz, 1H), 3.60 (s, 2H), 3.23 - 3.16 (m, 4H), 2.76 (d, J = 4.7 Hz, 3H), 2.62 - 2.55 (m, 4H). |

### Example 48

### 8-((4-(2-methyl-6-(methylcarbamoyl)pyridin-3-yl)piperazin-1-yl)methyl)pyrrolo[1,2-c]quinazolin-5(6H)-one

a) Preparation of methyl 5-oxo-5,6-dihydropyrrolo[1,2-c]quinazoline-8-carboxylate: To a mixture of methyl 3-amino-4-bromobenzoate (1.0 g, 7.2 mmol), (1-(tert-butoxycarbonyl)-1H-pyrrol-2-yl)boronic acid (2.0 g, 8.7 mmol), Na₂CO₃ (1.4 g, 23.9 mmol) in ACN (20.0 mL) was added Pd(PPh₃)₂Cl₂ (660.0 mg, 0.7 mmol). The mixture was degassed and purged with N₂ 3 times. The mixture was stirred at 80 °C for 35 minutes under N₂ atmosphere. The mixture was diluted with water (20.0 mL) and extracted with DCM (30.0 mL × 2). The combined organic phase was washed with brine, dried over anhydrous sodium sulfate and concentrated under reduced pressure. The residue was washed MTBE to give the title compound (0.4 g, yellow solid, yield: 39%).
b) Preparation of 8-(hydroxymethyl)pyrrolo[1,2-c]quinazolin-5(6H)-one: To a suspension of methyl 5-oxo-5,6-dihydropyrrolo[1,2-c]quinazoline-8-carboxylate (0.3 g, 1.2 mmol) in THF (10 mL) was added LiAlH₄ (1 M in THF, 5.0 mL, 4.9 mmol) under N₂ at 0 °C. The mixture was warmed to room temperature and stirred for 2 hours. The mixture was quenched with ice-water (10.0 mL) and was adjusted to pH=3 with 1M hydrochloric acid aqueous solution. The resulting mixture was extracted with EA (20.0 mL×3). The combined organic phase was washed with brine, dried over anhydrous sodium sulfate and concentrated under reduced pressure to give the title compound (200.0 mg, white solid, yield: 75%).
c) Preparation of (5-oxo-5,6-dihydropyrrolo[1,2-c]quinazolin-8-yl)methyl methanesulfonate: To a suspension of 8-(hydroxymethyl)pyrrolo[1,2-c]quinazolin-5(6H)-one (60.0 mg, 0.3 mmol) in DCM (5 mL) was added TEA (84.9 mg, 0.8 mmol) and methanesulfonyl chloride (48.3 mg, 0.4 mmol) dropwise at 0 °C. The resulting mixture was stirred at room temperature for 2 hours. The mixture was concentrated under reduced pressure to give the title compound (crude, 90.0 mg, yellow solid).
d) Preparation of 8-((4-(2-methyl-6-(methylcarbamoyl)pyridin-3-yl)piperazin-1-yl)methyl)pyrrolo[1,2-c]quinazolin-5(6H)-one: To a solution of (5-oxo-5,6-dihydropyrrolo[1,2-c]quinazolin-8-yl)methyl methanesulfonate (90.0 mg, 0.3 mmol), KI (10.0 mg, 0.1 mmol) and N,6-dimethyl-5-(piperazin-1-yl)picolinamide (90.0 mg, 0.4 mmol) in CH₃CN (10.0 mL) was added DIEA (193.5 mg, 1.5 mmol) at room temperature. The resulting solution was stirred at 80 °C for 7 hours. The mixture was cooled to room temperature and filtered. The filter cake was washed with methanol (3.0 mL). The solid was purified by Prep-TLC (MeOH/DCM=25/1) to give the target compound(7.0 mg, white solid, 2 steps yield: 5%).

The following compounds of Examples 49-50 and 63-64 were prepared using a synthesis method similar to that described in Example 1.

The following compounds of Examples 51-53 were prepared using a synthesis method similar to that described in Example 31.

The following compounds of Examples 54-58, 62, and 66-72 were prepared using a synthesis method similar to that described in Example 28.

The following compounds of Examples 59-61 and 65 were prepared using a synthesis method similar to that described in Example 24.

| Example | Compound | MW | LC-MS (ESI) | ¹H NMR (400 MHz) |
|---|---|---|---|---|
| 48 | 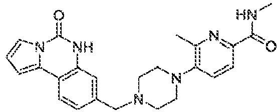 | 430.51 | 431.15 [M+H]+ | DMSO-d6: δ 11.49 (s, 1H), 8.44 (q, J = 4.8 Hz, 1H),7.90 (d, J = 7.9 Hz, 1H), 7.81 (d, J = 8.3 Hz, 1H), 7.59 (dd, J = 3.0, 1.4 Hz, 1H), 7.49 (d, J = 8.3 Hz, 1H), 7.26 (s, 1H), 7.19 (dd, J = 8.1, 1.5 Hz, 1H), 6.99 (dd, J = 3.5, 1.5 Hz, 1H), 6.68 (t, J = 3.3 Hz, 1H), 3.60 (s. 2H), 3.08 - 2.86 (m, 4H), 2.81 (d, J = 4.8 Hz, 3H), 2.67 - 2.56 (m, 4H), 2.50 (s, 3H). |
| 49 | 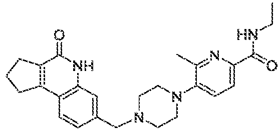 | 445.57 | 446.20 [M+H]+ | CDC13: δ 10.32 (s, 1H), 8.07 - 7.86 (m, 2H), 7.50 (d, J = 8.0 Hz, 1H), 7.35 (d, J = 7.9 Hz, 2H), 7.29 (s, 1H), 3.75 (s, 2H), 3.49 (t, J = 7.0 Hz, 2H), 3.14 (t, J = 7.6 Hz, 2H), 3.09 - 2.95 (m, 6H), 2.72 (s, 4H), 2.51 (s, 3H), 2.23 (t, 2H), 1.26 (t, J = 7.3 Hz, 3H). |
| 50 | 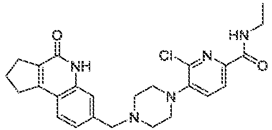 | 465.98 | 466.20 [M+H]+ | CDC13: δ 9.74 (s, 1H), 8.06 (d, J = 8.2 Hz, 1H), 7.64 (1, J = 6.3 Hz, 1H), 7.48 (d, J = 7.9 Hz, 1H), 7.39 (d, J = 8.2 Hz, 1H), 7.29 (d, J = 14.4 Hz, 1H), 3.75 (s, 2H), 3.56 - 3.37 (m, 2H), 3.30 - 3.18 (m, 4H), 3.12 (t, J = 7.4 Hz. 2H), 2.97 (t, J = 7.6 Hz, 2H), 2.86 - 2.66 (m, 4H), 2.34 - 2.02 (m, 2H), 1.24 (t, J = 7.3 Hz, 3H). |
| 51 | 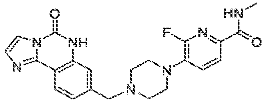 | 435.46 | / | / |
| 52 | 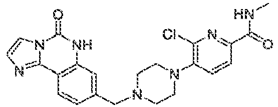 | 451.92 | / | / |
| 53 | 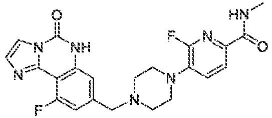 | 453.45 | 454.10 [M+H]+ | DMSO-d6: δ 8.41-8.39 (m, 1H), 7.86 (s, 1H), 7.81 (d, J = 7.6 Hz, 1H), 7.57-7.52 (m, 1H). 7.43 (s, 1 II), 7.17 (s, 1H), 7.08 (d, J = 10.0 Hz, 1H), 3.59 (s, 2H), 3.18-3.12 (m, 4H), 2.72 (d, J= 4.4 Hz, 3H), 2.58-2.52 (m, 4H). |
| 54 | 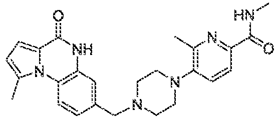 | 444.54 | 445.25 [M+H]+ | DMSO-d6: δ 11.14 (s, 1H), 8.41 (q, J = 4.6 Hz, 1H), 8.04 (d, J = 8.6 Hz, 1H), 7.77 (d, J = 8.2 Hz, 1H), 7.45 (d, J = 8.3 Hz, 1H), 7.27 (s, 1H), 7.12 (d, J = 8.5 Hz, 1H), 6.94 (d, J = 3.8 Hz, 1H), 6.41 (d, J = 3.8 Hz, 1H), 3.55 (s, 2H), 3.01 - 2.85 (m, 4H), 2.81 - 2.71 (m, 6H), 2.61 - 2.52 (m, 4H), 2.46 (s, 3H). |
| 55 | 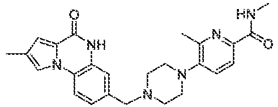 | 444.54 | 445.25 [M+H]+ | DMSO-d6: δ 11.21 (s, 1H), 8.45 (q, J = 5.9 Hz, 1H), 7.95 (s, 1H), 7.91 (d, J = 8.4 Hz, 1H), 7.79 (d, J = 8.3 Hz, 1H), 7.48 (d, J = 8.3 Hz, 1H), 7.28 (s, 1H), 7.15 (d, J = 8.5 Hz, 1H), 6.83 (s, 1H), 3.57 (s, 2H), 3.00 - 2.90 (m, 4H), 2.79 (d, J = 4.9 Hz, 3H), 2.64 - 2.53 (m, 4H), 2.49 (s, 3H), 2.22 (s, 3H). |
| 56 | 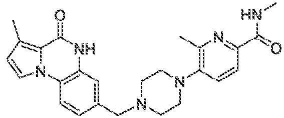 | 444.54 | 445.50 [M+H]+ | DMSO-d6: δ 10.97 (s, 1H), 8.41 (d, J = 5.1 Hz, 1H), 7.98 (d, J = 2.8 Hz, 1H), 7.89 (d, J = 8.4 Hz, 1H), 7.76 (d, J = 8.3 Hz, 1H), 7.45 (d, J = 8.4 Hz, 1H), 7.20 (s, 1H), 7.08 (d, J = 8.4 Hz, 1H), 6.46 (d, J = 2.7 Hz, 1H), 3.53 (s, 2H), 2.98 - 2.87 (m, 4H), 2.77 (d, J = 4.8 Hz, 3H), 2.61 - 2.50 (m, 4H), 2.46 (d, J = 2.1 Hz, 6H). |
| 57 | 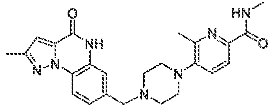 | 445.53 | 446.20 [M+H]+ | DMSO-d6: δ 11.83 (s, 1H), 8.44 (q, J = 5.1 Hz, 1H), 8.02 (d, J = 8.3 Hz, 1H), 7.79 (d, J = 8.2 Hz, 1H), 7.47 (d, J = 8.3 Hz, 1H), 7.37 (s, 1H), 7.26 (dd, J = 8.3, 1.7 Hz, 1H), 6.92 (s, 1H), 3.61 (s, 2H), 2.98 - 2.91 (m, 4H), 2.79 (d, J = 4.8 Hz, 3H), 2.61 - 2.55 (m, 4H), 2.48 (s, 3H). 2.42 (s, 3H). |
| 58 | 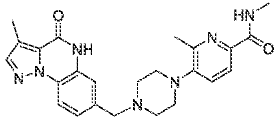 | 445.53 | 446.25 [M+H]+ | CDCl3: δ 9.50 (s, 1H), 8.13 (d, J = 8.1 Hz, 1H), 8.04 - 7.86 (m, 2H), 7.73 (s, 1H), 7.33 (d, J = 8.1 Hz, 1H), 7.27 (s, 1H), 3.67 (s, 2H), 3.04 - 2.96 (m, 7H), 2.77 - 2.61 (m, 4H), 2.55 (s, 3H), 2.50 (s, 3H). |
| 59 | 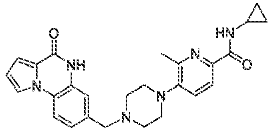 | 456.55 | 457.25 [M+H]+ | DMSO-d6: δ 11.25 (s, 1H), 8.34 (d, J = 4.8 Hz, 1H), 8.17 (s, 1H), 8.01 (d, J = 8.4 Hz, 1H), 7.78 (d, J = 8.3 Hz, 1H), 7.47 (d, J = 8.3 Hz, 1H), 7.29 (s, 1H), 7.17 (d, J = 8.3 Hz, 1H), 7.01 (dd, J = 3.8, 1.5 Hz, 1H), 6.68 (t, J = 3.4 Hz, 1H), 3.58 (s, 2H), 3.05 - 2.89 (m, 4H), 2.89 - 2.79 (m, 1H), 2.61 - 2.54 (m, 4H), 2.47 (s, 3H), 0.79 - 0.47 (m, 4H). |
| 60 | 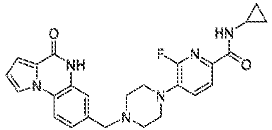 | 460.51 | 461.15 [M+H]+ | CD3OD: δ 8.00 (dd, J = 2.9, 1.4 Hz, 1H), 7.89 (d, J = 8.4 Hz, 2H), 7.58 - 7.42 (m, 1H), 7.34 (s, 1H), 7.28 (d, J = 8.2 Hz, 1H), 7.17 (dd, J = 4.1, 1.4 Hz, 1H), 6.71 (t, J = 3.4 Hz, 1H), 3.65 (s, 2H), 3.30 - 3.22 (m, 4H), 2.88 - 2.76 (m, 1H), 2.71 - 2.54 (m, 4H), 0.84 - 0.74 (m, 2H), 0.68 - 0.58 (m, 2H). |
| 61 | 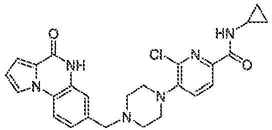 | 476.97 | 477.35 [M+H]+ | DMSO-d6: δ 11.21 (s, 1H), 8.37 (d, J = 4.9 Hz, 1H), 8.14 (s, 1H), 7.98 (d, J = 8.4 Hz, 1H), 7.89 (d, J = 8.2 Hz, 1H), 7.63 (d, J = 8.2 Hz, 1H), 7.25 (s, 1H), 7.13 (d, J = 8.2 Hz, 1H), 6.98 (d, J = 3.7 Hz, 1H), 6.64 (1, J = 3.3 Hz, 1H), 3.55 (s, 2H), 3.14 - 2.90 (m, 4H), 2.89 - 2.71 (m, 1H), 2.59 - 2.51 (m, 4H), 0.67 - 0.54 (m, 4H). |
| 62 | 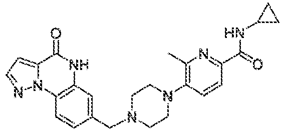 | 457.34 | 458.35 [M+H]+ | DMSO-d6: δ 11.92 (s, 1H), 8.36 (d, J = 4.8 Hz, 1H), 8.13 (d, J = 8.3 Hz, 1H), 8.09 (d, J = 2.0 Hz, 1H), 7.81 (d, J = 8.2 Hz, 1H), 7.50 (d, J = 8.3 Hz, 1H), 7.42 (s, 1H), 7.32 (d, J = 9.7 Hz, 1H), 7.17 (d, J = 2.0 Hz, 1H), 3.65 (s, 2H), 3.02 - 2.93 (m, 4H). 2.92 - 2.80 (m, 1H), 2.68 - 2.57 (m, 4H), 2.50 (s, 3H), 0.89 - 0.05 (m. 4H). |
| 63 | 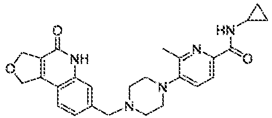 | 459.55 | 460.25 [M+H]+ | CDCl3:δ 11.16 (s, 1H), 8.21 - 7.82 (m, 2H), 7.41 (s, 1H), 7.37 - 7.28 (m, 2H), 5.39 (t, J = 3.8 Hz, 2H), 5.24 (t, J = 3.8 Hz, 2H), 3.70 (s, 2H), 3.06 - 2.96 (m, 4H), 2.96 - 2.81 (m, 1H), 2.74 - 2.62 (m, 4H), 2.49 (s, 3H), 0.99 - 0.77 (m, 2H), 0.73 - 0.56 (m, 2H). |
| 64 | 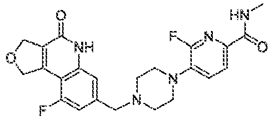 | 455.47 | / | / |
| 65 | 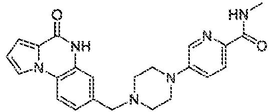 | 416.49 | 417.35 [M+H]+ | CDCl3: δ 8.02 (s, 1H), 7.81 (d, J = 8.8 Hz, 1H), 7.61 (s, 1H), 7.54 (d, J = 8.7 Hz, 1H), 7.15 - 6.98 (m, 4H), 6.55 (s, 1H), 3.48 (s, 2H), 3.27 - 3.20 (m, 4H), 2.83 (d, J = 4.4 Hz, 3H), 2.62 - 2.36 (m, 4H). |
| 66 | 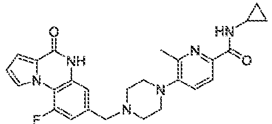 | 474.54 | 475.45 [M+H]+ | DMSO-d6: δ 11.42 (s, 1H), 8.31 (d, J = 4.9 Hz, 1H), 7.96 (s, 1H), 7.76 (d, J = 8.1 Hz, 1H), 7.46 (d, J = 8.3 Hz, 1H), 7.26 - 7.00 (m, 3H), 6.70 (t, J = 3.4 Hz, 1H), 3.56 (s, 2H), 2.99 - 2.88 (m, 4H), 2.87 -2.75 (m, 1H), 2.62 - 2.51 (m, 4H), 2.45 (s, 3H), 0.76 - 0.52 (m, 4H). |
| 67 | 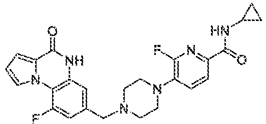 | 478.50 | 479.4 [M+H]+ | DMSO-d6: δ 11.42 (s, 1H), 8.34 (d, J = 5.1 Hz, 1H), 7.96 (s, 1H), 7.81 (d, J = 8.1 Hz, 1H), 7.59 - 7.51 (m, 1H), 7.23 - 6.98 (m, 3H), 6.71 - 6.68 (m, 1H), 3.54 (s, 2H), 3.23 - 3.05 (m, 4H), 2.94 - 2.75 (m, 1H), 2.62 - 2.49 (m, 4H), 0.90 - 0.23 (m, 4H). |
| 68 | 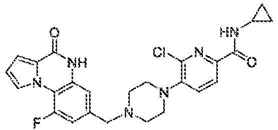 | 494.96 | 495.4 [M+H]+ | DMSO-d6: δ 11.44 (s, 1H), 8.37 (d, J = 4.7 Hz, 1H), 7.96 (s, 1H), 7.90 (d, J = 8.2 Hz, 1H), 7.64 (d, J = 8.2 Hz, 1H), 7.26 - 7.02 (m, 3H), 6.70 (t, J = 3.3 Hz, 1H), 3.56 (s, 2H), 3.16 - 3.01 (m, 4H), 2.94 - 2.75 (m, 1H), 2.62 - 2.51 (m, 4H), 0.77 - 0.40 (m, 4H). |
| 69 | 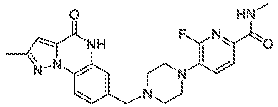 | 449.49 | 450.2 [M+H]+ | DMSO-d6: δ 11.81 (s, 1H), 8.44-8.39 (m, 1H), 8.02 (d, J = 8.3 Hz, 1H), 7.84 (d, J = 8.0 Hz, 1H), 7.60-7.54 (m, 1H), 7.37 (s. 1H), 7.26 (d, J = 8.4 Hz, 1H), 6.93 (s, 1H), 3.60 (s, 2H), 3.21 - 3.10 (m, 4H), 2.76 (d, J = 4.5 Hz, 3H), 2.63 - 2.53 (m, 4H), 2.42 (s, 3H). |
| 70 | 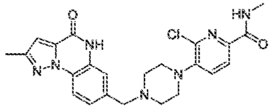 | 465.94 | 466.2 [M+H]+ | CDC13: δ 10.70 (s, 1H), 8.15 (d, J = 8.3 Hz, 1H), 8.07 (d, J = 8.2 Hz, 1H), 7.67 (d, J = 5.9 Hz, 1H), 7.39 (d, J = 7.5 Hz, 2H), 7.31 (d, J = 8.4 Hz, 1H), 6.98 (s, 1H), 3.68 (s, 2H), 3.27 - 3.12 (m, 4H), 3.00 (d, J = 5.1 Hz, 3H), 2.83 - 2.64 (m, 4H), 2.51 (s, 3H). |
| 71 | 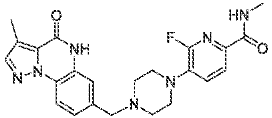 | 449.49 | 450.2 [M+H]+ | CDCl3+CD3OD: δ 8.07 (d, J = 8.0 Hz, 1H), 8.0 (s,1H), 7.87 (d, J = 8.0 Hz, 1H), 7.70 (s, 1H), 7.34-7.33 (m, 1H), 7.27-7.24 (m, 1H), 3.62 (br, 2H), 3.25-3.19 (m, 4H), 2.91 (s, 3H), 2.67-2.61 (m, 4H), 2.48 (s, 3H). |
| 72 | 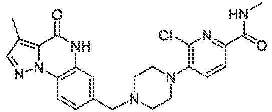 | 465.94 | 466.15 [M+H]+ | CDCl3+CD3OD: δ 8.08 (d, J = 8.0 Hz, 2H), 7.97 (d, J = 8.0 Hz. 1H). 7.71 (s, 1H), 7.44-7.43 (m, 1H), 7.27-7.25 (m, 2H), 3.64 (br, 2H), 3.20-3.14 (m, 4H),2.95 (s, 3H), 2.70-2.64 (m, 4H), 2.49 (s, 3H). |

### Example 73

### 7-((4-(2-fluoro-6-(methylcarbamoyl)pyridin-3-yl)piperazin-1-yl)methyl)-9-fluoro-2-methylpyrazolo[1,5-a]quinoxalin-4(5H)-one

a) Preparation of N-(5-bromo-2,3-difluorophenyl)-5-methyl-1H-pyrazole-3-carboxamide: To a solution of 5-bromo-2,3-difluoroaniline (1.2 g, 5.7 mmol) in anhydrous THF (50 mL) was added NaH (912.0 mg, 22.8 mmol) at 0 °C. The mixture was stirred at room temperature for 15 mins. Then a solution of 3-methyl-1H-pyrazole-5-carbonyl chloride (826.0 mg, 5.7 mmol) in anhydrous THF was added dropwised at 0 °C. The resulting solution was stirred at room temperature for 2 hours. After completion, the reaction was quenched with saturated NH₄Cl aqueous solution (5 mL), the residue was diluted with water (50 mL) and extracted with EA (50 mL × 3). The combined organic phase was washed with brine, dried over Na₂SO₄ and concentrated under reduced pressure to afford the title compound (1.1 g, crude, off-white solid, yield: 61%).
b) Preparation of 7-bromo-9-fluoro-2-methylpyrazolo[1,5-a]quinoxalin-4(5H)-one: A mixture of N-(5-bromo-2,3-difluorophenyl)-5-methyl-1H-pyrazole-3-carboxamide (1.1 g, 3.48 mmol) and K₂CO₃ (1.4 g, 10.44 mmol) in DMSO (25 mL) was stirred at 120 °C overnight. After reaction completed, the residue was diluted with water (50 mL) and extracted with EA (50 mL × 3). The combined organic phase was washed with brine, dried over Na₂SO₄ and concentrated under reduced pressure. The residue was triturated with DCM (5 mL) and the solid was collected by filtration to afford the title compound (440.1 mg, while solid, yield: 43%). MS(ESI, m/z): 295.95 [M+H]⁺.
c) Preparation of 9-fluoro-7-(hydroxymethyl)-2-methylpyrazolo[1,5-a]quinoxalin-4(5H)-one: To a solution of 7-bromo-9-fluoro-2-methylpyrazolo[1,5-a]quinoxalin-4(5H)-one (400.0mg, 1.35 mmol) in dioxane (25 mL) was added (tributylstannyl)methanol (877.0 mg, 2.70 mmol) and XphosPdG₂ (106.0 mg, 0.135 mmol) at room temperature. The mixture was stirred at 90 °C overnight under N₂ atmosphere. After completion, a solution of KF (1 M, 10 mL) was added at room temperature, the mixture was stirred at the same temperature for 10 min and filtered, the filtrate was extracted with EA (50 mL × 3). The combined organic phase was washed with brine, dried over Na₂SO₄ and concentrated under reduced pressure. The residue was triturated with the mix solvent of PE and EA (PE:EA=1:1, 50 mL) and the solid was collected by filtration to afford the title compound (280 mg, white solid, yield: 84%). MS(ESI, m/z): 248.10 [M+H]⁺.
d) Preparation of 7-(chloromethyl)-9-fluoro-2-methylpyrazolo[1,5-a]quinoxalin-4(5H)-one: To a solution of methyl 9-fluoro-7-(hydroxymethyl)-2-methylpyrazolo[1,5-a]quinoxalin-4(5H)-one (230.0 mg, 0.93 mmol) in DCM (5 mL) was added SOCl₂ (554.0 mg, 4.95 mmol) and DMF (1 drop) under N₂ at 0 °C. The mixture was warmed to room temperature and stirred for 2 hours. Then the solvent was concentrated under reduced pressure and the residue was triturated with DCM (5 mL) and the solid was collected by filtration to afford the title compound (160.0 mg, white solid, yield: 65%).
e) Preparation of 7-((4-(2-fluoro-6-(methylcarbamoyl)pyridin-3-yl)piperazin-1-yl)methyl)-9-fluoro-2-methylpyrazolo[1,5-a]quinoxalin-4(5H)-one: To a solution of 7-(chloromethyl)-9-fluoro-2-methylpyrazolo[1,5-a]quinoxalin-4(5H)-one (40.0 mg, 0.15 mmol), KI (3.0 mg, 0.015 mmol) and 6-fluoro-N-methyl-5-(piperazin-1-yl)picolinamide (43.0 mg, 0.18 mmol) in CH₃CN (10 mL) was added DIEA (97.1 mg, 0.75 mmol) at room temperature. The resulting solution was stirred at 80 °C for 2 hours. After reaction completed, the solvent was removed under vacuum. The residue was purified by Prep-TLC (DCM:MeOH=10:1) to give the target compound (21.9 mg, white solid, yield: 31%).

The following compounds of Examples 74-78 were prepared using a synthesis method similar to that described in Example 73.

| Example | Compound | MW | LC-MS (ESI) | ¹H NMR (400 MHz) |
|---|---|---|---|---|
| 73 | 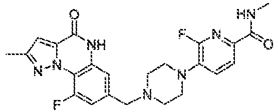 | 467.48 | 468.20 [M+H]⁺ | DMSO-d₆: δ 11.94 (br, 1H), 8.37 (d, J =5.6 Hz, 1H), 7.81 (d, J = 8.0 Hz, 1H), 7.56-7.52 (m, 1H), 7.17 (s,1H), 7.12 (d, J = 13.6 Hz, 1H), 6.94 (s, 1H), 3.56 (s, 2H), 3.18-3.12 (m, 4H), 2.72 (d, J=4.8 Hz, 3H), 2.58-2.52 (m, 4H), 2.38 (s, 3H). |
| 74 | 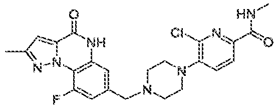 | 483.93 | 484.25 [M+H]⁺ | DMSO-d₆: δ 11.94 (br, 1H), 8.39 (d, J =5.2 Hz, 1H), 7.89 (d, J = 8.0 Hz, 1H), 7.63 (d, J = 8.0 Hz, 1H), 7.16 (s,1H), 7.12 (d, J = 12.8 Hz, 1H), 6.93 (s, 1H), 3.56 (s, 2H), 3.12-3.06 (m, 4H), 2.74 (d, J=4.8 Hz, 3H), 2.58-2.52 (m, 4H), 2.37 (s, 3H). |
| 75 | 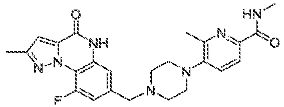 | 463.52 | 464.20 [M+H]⁺ | DMSO-d₆: δ 11.94 (br, 1H), 8.38-8.37 (m, 1H), 7.76 (d, J = 8.0 Hz, 1H), 7.45 (d, J = 8.0 Hz, 1H), 7.18 (s, 1H), 7.12 (d, J = 13.2 Hz, 1H), 6.93 (s,1H), 3.57 (s, 2H), 2.96-2.90 (m,4H). 2.76 (d, J=4.4 Hz, 3H), 2.58-2.52(m. 4H), 2.45 (s, 3H), 2.38 (s. 3H). |
| 76 | 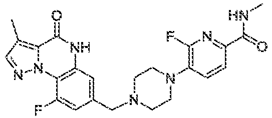 | 467.48 | 468.15 [M+H]⁺ | DMSO-d₆: δ 11.80 (br, 1H), 8.37 (br, 1H), 7.89-7.87 (m, 1H), 7.80-7.78 (m, 1H), 7.56-7.50 (m,1H), 7.13-7.06 (m, 2H), 3.54 (s, 2H), 3.17-3.12 (m, 4H), 2.72 (d, J=4.8 Hz, 3H). 2.55-2.50 (m, 4H), 2.40 (s, 3H). |
| 77 | 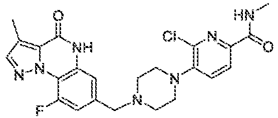 | 483.93 | 484.15 [M+H]⁺ | DMSO-d₆: δ 11.79 (br, 1H), 8.40 (d, J =4.8 Hz, 1H), 7.90 (d, J = 7.6 Hz, 1H), 7.63 (d, J = 8.0 Hz, 1H), 7.13 (s,1H), 7.09 (d, J = 13.6 Hz, 1H), 6.94 (s, 1H), 3.56 (s, 2H). 2.12-3.06 (br. 4H), 2.74 (d, J=4.8 Hz, 3H), 2.58-2.52 (m, 4H), 2.40 (s, 3H). |
| 78 | 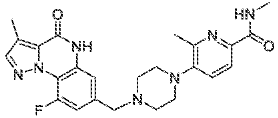 | 463.52 | 464.20 [M+H]⁺ | DMSO-d₆: δ 11.94 (br, 1H), 8.40-8.38 (m, 1H), 7.89 (s,1H), 7.75 (d, J = 8.0 Hz, 1H), 7.44 (d, J = 8.0 Hz, 1H), 7.14 (s, 1H), 7.12 (d, J = 13.2 Hz, 1H), 3.55 (s, 2H), 2.96-2.90 (m, 4H), 2.75 (d, J= 4.8 Hz, 3H), 2.59-2.52 (m, 4H), 2.45 (s, 3H). 2.39 (s,3H). |

### Example 79

### 7-((4-(2-methyl-6-(methylcarbamoyl)pyridin-3-yl)piperazin-1-yl)methyl)-9-fluoro-1,2,3,5-tetrahydro-4H-cyclopenta[c]quinolin-4-one

a) Preparation of methyl 2-(((trifluoromethyl)sulfonyl)oxy)cyclopent-1-ene-1-carboxylate: To a solution of methyl 2-oxocyclopentane-1-carboxylate (2.0 g, 14.1 mmol) in dry DCM (40.0 mL) was added DIEA (5.5 g, 42.2 mmol) at -78 °C under N₂ and stirred for 30 min. Then Tf₂O (11.9 g, 42.2 mmol) was added slowly. The mixture was stirred at -78 °C for 30 min and at rt for 12h. The mixture was quenched with water (5.0 mL) and extracted with DCM (20.0 mL × 2). The combined organic phase was washed with brine, dried over anhydrous sodium sulfate and concentrated under reduced pressure. The residue was purified by column chromatography (SiO₂, Petroleum ether: Ethyl acetate = 100/ 1 to 50/ 1) to give the title compound (2.8 g, 72 % yield) as a yellow oil.
b) Preparation of methyl 2-(4,4,5,5-tetramethyl-1,3,2-dioxaborolan-2-yl)cyclopent-1-ene-1-carboxylate: A mixture of methyl methyl 2-(((trifluoromethyl)sulfonyl)oxy)cyclopent-1-ene-1-carboxylate (1.2 g, 4.4 mmol), bis(pinacolato)diboron (1.3 g, 5.3 mmol), KOAc (0.9 g, 8.8 mmol, 2.0 eq) in THF (40 mL) was added Pd(dppf)Cl₂-CH₂Cl₂ (0.4 g, 0.4 mmol, 0.1 eq). The mixture was degassed and purged with N₂ for 3 times, and stirred at 100 °C under N₂ for 4 hours. After the reaction is completed. The mixture was filitered and the filtrate was concentrated under reduced pressure to give the title compound (1.2 g, crude) as a black solid.
c) Preparation of methyl 3-fluoro-4-(2-(methoxycarbonyl)cyclopent-1-en-1-yl)-5-nitrobenzoate: In a sealed tube, a solution of methyl 2-(4,4,5,5-tetramethyl-1,3,2-dioxaborolan-2-yl)cyclopent-1-ene-1-carboxylate (1.2 g, 4.8 mmol), methyl 4-bromo-3-fluoro-5-nitrobenzoate (1.3 g, 4.8mmol) and K₂CO₃ (1.6 g, 23.7 mmol) in THF (10.0 mL) and H₂O (2.0 mL) was added Pd(PPh₃)Cl₂ (0.3 g, 0.5 mmol). The mixture was degassed and purged with N₂ for 2 minutes, and stirred at 85 °C for 16 hours under N₂. After the reaction is completed. The mixture was diluted with water (20.0 mL) and extracted with DCM (30.0 mL × 2). The combined organic phase was washed with brine, dried over anhydrous sodium sulfate and concentrated under reduced pressure. The residue was purified by column chromatography (SiO₂, Petroleum ether: Ethyl acetate = 10/ 1 to 3/ 1) to give the title compound (0.5 g, impure) as a yellow solid.
d) Preparation of methyl 9-fluoro-4-oxo-2,3,4,5-tetrahydro-1H-cyclopenta[c]quinoline-7-carboxylate: To a solution of methyl 3-fluoro-4-(2-(methoxycarbonyl)cyclopent-1-en-1-yl)-5-nitrobenzoate (0.5 g, crude) in AcOH (20 mL) was added Fe powder (0.4 g, 28.6 mmol). The resulting mixture was stirred at 80 °C for 2 hours. After the reaction is completed, the reaction mixture was concentrated under reduced pressure. The residure was diluted with water (10.0 mL) and extacted with DCM (50.0 mL x 3). The combined organic phase was washed with brine, dried over anhydrous sodium sulfate and concentrated under reduced pressure to give the title compound (0.8 g, impure) as a brown solid. LC-MS: 262.05 [M+1]⁺.
e) Preparation of 9-fluoro-7-(hydroxymethyl)-1,2,3,5-tetrahydro-4H-cyclopenta[c]quinolin-4-one: To a suspention of methyl 9-fluoro-4-oxo-2,3,4,5-tetrahydro-1H-cyclopenta[c]quinoline-7-carboxylate (0.8 g, impure) in THF (10.0 mL) was added LiAlH₄ (1 M in THF, 1.5 mL, 1.5 mmol) at 0 °C under N₂. The resulting mixture was stirred at 0 °C for 20 minutes and at rt for 4 hours. The mixture was quenched with ice-water (10.0 mL) and adjusted to pH = 3 with aq. HCl (1 M). The resulting mixture was extracted with EA (50.0 mL x 3). The combined organic phase was washed with brine, dried over anhydrous sodium sulfate and concentrated under reduced pressure to give the title compound as a white solid (170.0 mg, yield:17%, 4 steps).
f) Preparation of 7-(chloromethyl)-9-fluoro-1,2,3,5-tetrahydro-4H-cyclopenta[c]quinolin-4-one: To a suspension of 9-fluoro-7-(hydroxymethyl)-1,2,3,5-tetrahydro-4H-cyclopenta[c]quinolin-4-one (40.0 mg, 0.2 mmol) in DCM (3 mL) was added DMF (5.0 mg, 0.1 mmol) and SOCl₂ (142.8 mg, 1.2 mmol) at 0 °C. The resulting mixture was stirred at room temperature for 2 hours. After the reaction was completed, the mixture was concentrated to give the compound as a grey solid (50.0 mg, impure). LC-MS: 252.05 [M+1]⁺.
g) Preparation of 5-(4-((9-fluoro-4-oxo-2,3,4,5-tetrahydro-1H-cyclopenta[c]quinolin-7-yl)methyl)piperazin-1-yl)-N,6-dimethylpicolinamide: To a suspension of 7-(chloromethyl)-9-fluoro-1,2,3,5-tetrahydro-4H-cyclopenta[c]quinolin-4-one (50.0 mg, impure, 1.0 eq), KI (7.0 mg, 0.1 mmol) and N,6-dimethyl-5-(piperazin-1-yl)picolinamide (56.0 mg, 0.2 mmol) in CH₃CN (4.0 mL) was added DIEA (129.0 mg, 1.9 mmol) at room temperature. The resulting suspension was stirred at 80 °C for 2 hours. The mixture was cooled to room temperature and filtered. The filter cake was washed with methanol (2.0 mL). The solid was purified by Prep-TLC (MeOH:DCM=20/1) t to give the targeted compound as a white solid (21.0 mg, yield: 27%).

The following compounds of Examples 80-82 were prepared using a synthesis method similar to that described in Example 79.

| Example | Compound | MW | LC-MS (ESI) | ¹H NMR (400 MHz) |
|---|---|---|---|---|
| 79 | 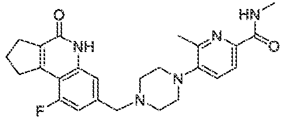 | 449.53 | 450.25 [M+H]⁺ | CDCl₃: δ 10.44 (s, 1H), 8.24 - 7.80 (m, 2H), 7.34 (d, J = 8.4 Hz, 1H), 7.08 (s, 1H), 6.96 (d, J = 13.4 Hz, 1H), 3.64 (s, 2H), 3.33 (t, J = 9.0 Hz, 2H), 3.04 - 2.95 (m, 7H), 2.92 (t, J = 7.9 Hz, 2H), 2.75 - 2.61 (m, 4H), 2.49 (s, 3H), 2.31 - 1.98 (m, 2H). |
| 80 | 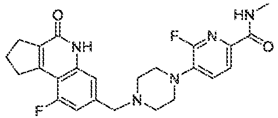 | 453.49 | 454.20 [M+H]⁺ | CDCl₃ + CD₃OD: δ 7.81 (d, J = 7.7 Hz, 1H), 7.69 (q, J = 4.9 Hz, 1H), 7.21 (d, J = 7.6 Hz, 1H), 6.97 (s, 1H), 6.85 (d, J = 11.0 Hz, 1H), 3.51 (s, 2H), 3.21 (t, J = 7.4 Hz, 2H), 3.16 - 3.05 (m, 4H), 2.85 (d, J = 5.0 Hz, 3H), 2.76 (t, J = 7.4 Hz, 2H), 2.66 - 2.49 (m, 4H), 2.14 - 1.98 (m, 2H). |
| 81 | 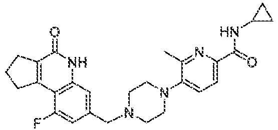 | 475.57 | 476.20 [M+H]⁺ | CDCl₃: δ 10.85 (s, 1H), 8.31 - 7.72 (m, 2H), 7.32 (d, J = 8.2 Hz, 1H), 7.10 (s, 1H), 6.96 (d, J = 11.2 Hz, 1H), 3.63 (s, 2H), 3.33 (t, J = 8.6 Hz, 2H), 3.08 - 2.79 (m, 7H), 2.72 - 2.60 (m, 4H), 2.48 (s, 3H), 2.31 - 1.91 (m, 2H), 0.92 - 0.70 (m, 2H), 0.70 - 0.49 (m, 2H). |
| 82 | 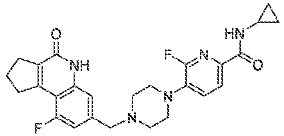 | 479.53 | 480.20 [M+H]⁺ | CDCl₃ + CD₃OD: δ 7.83 (d, J = 7.9 Hz, 1H), 7.62 (d, J = 3.1 Hz, 1H), 7.21 (d, J = 9.8 Hz, 1H), 6.97 (s, 1H), 6.85 (d, J = 11.3 Hz, 1H), 3.52 (s, 2H), 3.34 - 3.18 (m, 2H), 3.18 - 3.03 (m, 4H), 2.89 - 2.61 (m, 3H), 2.60 - 2.46 (m, 4H), 2.14-2.01 (m, 2H), 0.85 - 0.67 (m, 2H), 0.61 - 0.38 (m, 2H). |

### Example 83

### 7-((4-(2-methyl-6-(methylcarbamoyl)pyridin-3-yl)piperazin-1-yl)methyl)thieno[3,4-c]quinolin-4(5H)-one

a) Preparation of methyl 4-oxo-4,5-dihydrothieno[3,4-c]quinoline-7-carboxylate: To a solution of methyl 4-bromothiophene-3-carboxylate (350.0 mg, 1.6 mmol) in DMF (5 mL) was added 2-amino-4-(methoxycarbonyl)phenylboronic acid (549.8 mg, 2.4 mmol), NaOAc (196.8 mg, 2.4 mmol), Pd(dppf)Cl₂ (347.8 mg, 0.47 mmol). The reaction mixture was stirred at 130°C for 15 min under microwave under N₂. After completion, the reaction mixture was filtered and the filtrate was concentrated. The residue was diluted with water (20 mL) and extracted with EA (20 mL×3). The combined organic phase was washed with brine, dried over anhydrous sodium sulfate and concentrated under reduced pressure. The residue was purified by Prep-TLC (DCM/MeOH = 20/1) to give the title compound (108.0 mg, yellow solid, yield: 26%). MS(ESI, m/z): 259.95 [M+H]⁺.
b) Preparation of 7-(hydroxymethyl)thieno[3,4-c]quinolin-4(5H)-one: To a suspension of methyl 4-oxo-4,5-dihydrothieno[3,4-c]quinoline-7-carboxylate (108.0 mg, 0.4 mmol) in THF (3 mL) was added LiAlH₄ (1 M in THF, 1.7 mL, 1.7 mmol) under N₂ at 0 °C. The mixture was warmed to room temperature and stirred for 2 hours. Then the mixture was quenched with water (2 mL) and extracted with EA (10 mL×3). The combined organic phase was washed with brine, dried over anhydrous sodium sulfate and concentrated under reduced pressure to give the title compound (72.0 mg crude, white solid). MS(ESI, m/z): 232.00 [M+H]⁺.
c) Preparation of 7-(chloromethyl)thieno[3,4-c]quinolin-4(5H)-one: To a suspension of 7-(hydroxymethyl)thieno[3,4-c]quinolin-4(5H)-one (72.0 mg, 0.3 mmol) in DCM (3 mL) was added DMF (2 drops) and thionyl chloride (222.3 mg, 1.9 mmol, 6.0 eq.) dropwise at 0 °C. The resulting mixture was stirred at room temperature for 10 min. After completion, the mixture was concentrated to give the title compound (40.0 mg crude, white solid). MS(ESI, m/z): 250.20 [M+H]⁺.
d) Preparation of 7-((4-(2-methyl-6-(methylcarbamoyl)pyridin-3-yl)piperazin-1-yl)methyl)thieno[3,4-c]quinolin-4(5H)-one: To a solution of 7-(chloromethyl)thieno[3,4-c]quinolin-4(5H)-one (40.0 mg, 0.2 mmol) in CH₃CN was added N,6-dimethyl-5-(piperazin-1-yl)picolinamide (37.4 mg, 0.1 mmol), DIEA (103.2 mg, 0.8 mmol), KI (5.0 mg, 0.03 mmol) under N₂. The resulting solution was stirred at 80 °C for 2 hours. After completion, the solvent was removed in vacuum. The residue was purified by Prep-TLC (DCM/MeOH = 20/1, 0.1% TEA) to give the target compound (9.2 mg, white powder, 3 steps yield: 5%).

The following compounds of Examples 84-85 and 104 were prepared using a synthesis method similar to that described in Example 83.

The following compounds of Examples 86-103 were prepared using a synthesis method similar to that described in Example 73.

| Example | Compound | MW | LC-MS (ESI) | ¹H NMR (400MHz) |
|---|---|---|---|---|
| 83 | 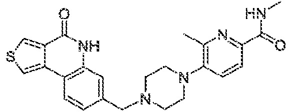 | 447.56 | 448.15 [M+H]⁺ | CDCl₃: δ 9.61 (s, 1H), 8.47 (d, J=4.0Hz, 1H), 7.83 (d, J = 5.2 Hz, 1H), 7.74 (d, J = 5.2 Hz, 1H), 7.44 (s, 1H), 7.39 - 7.30 (m, 2H), 3.74 (s, 2H), 3.11 - 2.90 (m, 7H), 2.79 - 2.64 (m, 4H), 2.50 (s, 3H). |
| 84 | 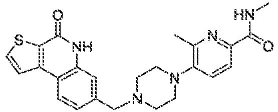 | 447.56 | 448.15 [M+H]⁺ | CDCl₃: δ 10.31 (s, 1H), 8.00 - 7.88 (m, 3H), 7.83 (d, J = 5.2 Hz, 1H), 7.74 (d, J = 5.2 Hz, 1H), 7.44 (s, 1H), 7.39 - 7.30 (m, 2H), 3.74 (s, 2H), 3.11 - 2.90 (m, 7H), 2.79 - 2.64 (m, 4H), 2.50 (s, 3H). |
| 85 | 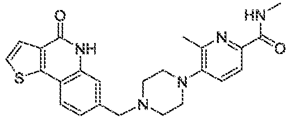 | 447.56 | 448.25 [M+H]⁺ | DMSO-d₆: δ 11.95 (s, 1H), 8.39 (d, J = 5.3 Hz, 1H). 7.91 (d, J = 8.0 Hz, 1H), 7.81 - 7.72 (m, 2H), 7.54 (d, J = 5.1 Hz, 1H), 7.51 - 7.43 (m, 2H), 7.33 (d, J = 8.0 Hz, 1H), 4.43 (s, 2H), 3.47 - 3.39 (m, 4H), 3.03 - 2.87 (m, 4H), 2.72 (d, J = 4.7 Hz, 3H). 2.44 (s, 3H). |
| 86 | 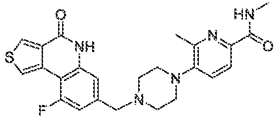 | 465.55 | 466.15 [M+H]⁺ | DMSO-d₆: δ 11.29 (s, 1H), 8.47 (s, 1H), 8.33 (s, 1H), 8.13 (s, 1H), 7.69 (d, J = 8.2 Hz, 1H), 7.38 (d, J = 8.3 Hz, 1H), 7.06 (s, 1H), 6.95 (d, J = 11.7 Hz, 1H), 3.50 (s, 2H), 2.91-2.83 (m, 4H), 2.70 (s, 3H), 2.51-2.45 (m, 4H), 2.42 (s, 3H). |
| 87 | 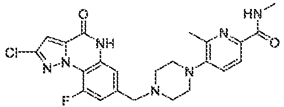 | 483.93 | 484.35 [M+H]⁺ | DMSO-d₆: δ 11.94 (br, 1H), 8.41-8.39 (m, 1H), 7.76 (d, J = 8.0 Hz, 1H), 7.45 (d, J = 8.8 Hz, 1H), 7.26 (s,1H), 7.19-7.16 (m, 2H), 3.59 (s, 2H), 2.96-2.90 (m, 4H), 2.75 (d, J= 4.4 Hz, 3H), 2.58-2.52 (m, 4H), 2.45 (s, 3H). |
| 88 | 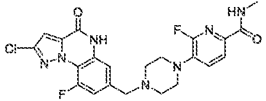 | 487.90 | 488.05 [M+H]⁺ | DMSO-d₆: δ 12.17 (s, 1H), 8.40 (d, J = 5.1 Hz, 1H), 7.84 (d, J = 8.0 Hz, 1H), 7.64 - 7.52 (m, 1H), 7.29 (s, 1H), 7.25 - 7.12 (m, 2H), 3.60 (s, 2H), 3.22 - 3.12 (m, 4H), 2.76 (d, J = 4.7 Hz, 3H), 2.63 - 2.54 (m, 4H). |
| 89 | 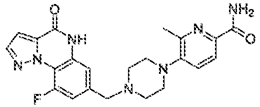 | 435.46 | 436.15 [M+H]⁺ | DMSO-d₆: δ 11.94 (br, 1H), 8.07 (d, J = 2.0 Hz, 1H), 7.80-7.77 (m, 2H), 7.46-7.41 (m, 2H), 7.21 (s,1H), 7.17-7.15 (d, m, 2H), 3.59 (s, 2H), 2.96-2.90 (m, 4H), 2.60-2.53 (m, 4H), 2.45 (s, 3H). |
| 90 | 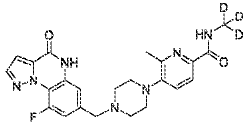 | 452.51 | 453.45 [M+H]⁺ | DMSO-d₆: δ 11.94 (br, 1H), 8.36 (s, 1H), 8.07 (d, J = 2.0 Hz, 1H), 7.76 (d, J = 8.4 Hz, 1H), 7.45 (d, J = 8.4 Hz, 1H), 7.18 (d, J = 14 Hz, 1H), 7.15 - 7.13 (m, 2H), 3.58 (s, 2H), 2.93-2.91 (m, 4H), 2.52-2.51 (m, 4H), 2.47 (s, 3H). |
| 91 | 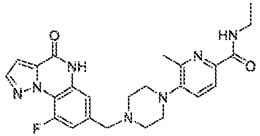 | 463.52 | 464.20 [M+H]⁺ | DMSO-d₆: δ 11.94 (br, 1H), 8.42-8.41 (m, 1H), 8.07 (d, J = 2.4 Hz, 1H), 7.76 (d, J = 8.0 Hz, 1H), 7.45 (d, J = 8.4 Hz, 1H). 7.18 (d, J = 15.6 Hz, 1H), 7.15 - 7.14 (m, 2H), 3.58 (s, 2H), 3.27-3.25 (m, 2H), 2.93-2.91 (m, 4H), 2.57-2.53 (m, 4H), 2.47 (s, 3H), 1.07 (t, J = 7.6 Hz, 3H). |
| 92 | 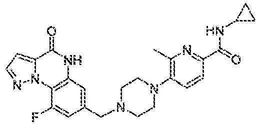 | 475.53 | 476.20 [M+H]⁺ | DMSO-d₆: δ 8.28 (d, J = 4.8 Hz, 1H), 8.07 (d, J = 2.0 Hz, 1H), 7.75 (d, J = 8.8 Hz, 1H), 7.45 (d, J = 2.0 Hz. 1H), 7.21 (s, 1H), 7.15 - 7.14 (m, 2H), 3.58 (s, 2H), 2.93-2.92 (m, 4H), 2.85-2.83 (m, 1H), 2.57-2.51 (m, 4H), 2.45 (s, 3H), 0.66-0.65 (m, 2H), 0.64-0.60 (m, 2H). |
| 93 | 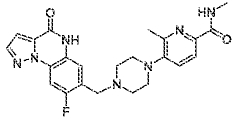 | 449.49 | 450.20 [M+H]⁺ | DMSO-d₆: δ 11.94 (br, 1H), 8.38-8.37 (m, 1H), 8.07 (d, J = 2.0 Hz, 1H), 7.85 (d, J = 9.6 Hz. 1H), 7.76 (d, J = 8.4 Hz, 1H), 7.48-7.43 (m, 2H), 7.13 (d, J = 2.0 Hz, 1H), 3.65 (s, 2H), 2.96-2.90 (m, 4H), 2.77-2.75 (m, 3H), 2.62-2.56 (m, 4H), 2.45 (s, 3H). |
| 94 | 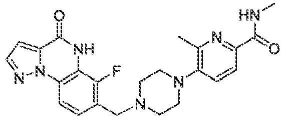 | 449.49 | 450.15 [M+H]⁺ | DMSO-d₆: δ 8.39-8.37 (m,1H), 8.06 (d, J=2.0 Hz, 1H), 7.91 (d, J = 8.0 Hz, 1H), 7.74 (d, J = 8.8 Hz, 1H), 7.43 (d, J = 8.8 Hz, 1H), 7.33-7.30 (m, 1H), 7.15(d. J = 2.0 Hz. 1H), 3.67 (s, 2H), 2.94-2.88 (m, 4H), 2.75 (d, J=5.2 Hz, 3H), 2.60-2.54 (m, 4H), 2.44 (s, 3H). |
| 95 | 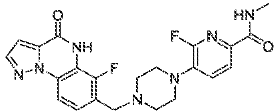 | 453.45 | 454.20 [M+H]⁺ | DMSO-d₆: δ 8.37-8.35 (m,1H), 8.06 (d, J=2.0 Hz, 1H), 7.91 (d, J = 8.4 Hz, 1H), 7.79 (d, J= 8.0 Hz, 1H), 7.52 (d, J = 8.4 Hz, 1H), 7.31 (t, J = 8.4 Hz, 1H), 7.15 (d, J = 2.0 Hz, 1H), 3.66 (s, 2H), 3.16-3.11 (m, 4H), 2.72 (d, J = 4.4 Hz, 3H), 2.59-2.53 (m, 4H). |
| 96 | 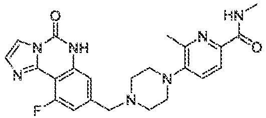 | 449.49 | 450.15 [M+H]⁺ | DMSO-d₆: δ 8.41-8.39 (m,1H), 7.88 (s, 1H), 7.76 (d, J = 7.6 Hz, 1H), 7.47-7.44 (m, 2H), 7.19 (s, 1 H), 7.11 (d, J = 10.8 Hz, 1H), 3.62 (s, 2H), 2.96-2.90 (m, 4H), 2.76 (d, J= 4.8 Hz, 3H), 2.60-2.54 (m, 4H), 2.46 (s, 3H). |
| 97 | 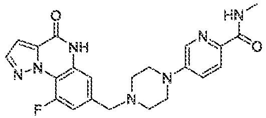 | 435.46 | 436.20 [M+H]⁺ | DMSO-d₆: δ 11.98 (br, 1H), 8.53 (d, J = 2.0 Hz, 1H), 8.17 (d, J= 4.8 Hz, 1H), 8.07 (d, J = 2.0 Hz, 1H), 7.89 (d, J = 8.8 Hz, 1H), 7.20 (s,1H), 7.16-7.13 (m, 2H), 6.81 (d, J = 9.2 Hz, 1H), 3.60-3.57 (m,4H), 3.55 (s, 2H), 3.38-3.32 (m, 4H), 2.71 (d, J = 4.0 Hz, 3H). |
| 98 | 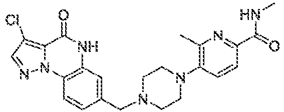 | 465.94 | 466.05 [M+H]⁺ | DMSO-d₆: δ 8.43 (d, *J =* 5.4 Hz, 1H), 8.28 (s, 1H), 8.21 (s, 1H), 8.07 (d, *J* = 7.9 Hz, 1H), 7.79 (d, *J =* 8.5 Hz, 1H), 7.48 (d, *J =* 8.2 Hz, 1H), 7.37 (s, 1H), 7.30 (d, *J =* 8.5 Hz, 1H), 3.62 (s, 2H), 3.00 - 2.90 (m, 4H), 2.79 (d, *J* = 4.9 Hz, 3H), 2.63 - 2.55 (m, 4H), 2.49 (s, 3H). |
| 99 | 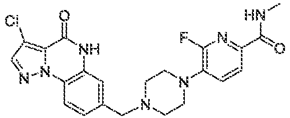 | 469.91 | 470.10 [M+H]⁺ | DMSO-d₆: δ 11.93 (s, 1H), 8.41 (q, J = 4.2, 3.7 Hz, 1H), 8.20 (s, 1H), 8.06 (d, J = 8.3 Hz, 1H), 7.84 (d, J = 8.0 Hz, 1H), 7.57 (dd, J = 10.5, 8.1 Hz, 1H), 7.36 (s, 1H), 7.28 (d, J = 8.4 Hz, 1H), 3.60 (s, 2H), 3.22 - 3.14 (m, 4H), 2.76 (d, J = 4.7 Hz, 3H), 2.61 - 2.53 (m, 4H). |
| 100 | 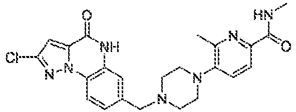 | 465.94 | 466.15 [M+H]⁺ | DMSO-d₆: δ 12.05 (s, 1H), 8.43 (d, J = 5.1 Hz, 1H), 8.03 (d, J = 8.4 Hz, 1H), 7.79 (d, J = 8.3 Hz, 1H), 7.48 (d, J = 8.3 Hz, 1H), 7.40 (s, 1H), 7.31 (dd, J = 8.1, 1.3 Hz, 1H), 7.25 (s, 1H), 3.63 (s, 2H), 3.01 - 2.89 (m, 4H), 2.79 (d, J = 4.8 Hz, 3H), 2.63 - 2.55 (m, 4H), 2.49 (s, 3H). |
| 101 | 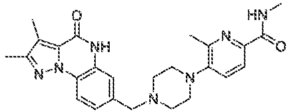 | 459.55 | 460.20 [M+H]⁺ | DMSO-d₆: δ 11.63 (s, 1H), 8.42 (s, 1H), 7.97 (d, J = 8.2 Hz, 1H), 7.79 (d, J = 8.4 Hz, 1H), 7.48 (d, J = 8.4 Hz, 1H), 7.32 (s, 1H), 7.22 (d, J = 8.4 Hz, 1H), 3.59 (s, 2H), 3.00 - 2.90 (m, 4H), 2.79 (d, J = 4.7 Hz, 3H), 2.62 - 2.54 (m, 4H), 2.49 (s, 3H), 2.34 (d, J = 12.8 Hz, 6H). |
| 102 | 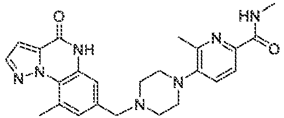 | 445.53 | 446.20 [M+H]⁺ | DMSO-d₆: δ 11.78 (br, 1H), 8.40-8.37 (m,1H), 8.04 (d, J=1.2 Hz, 1H), 7.75 (d, J = 8.4 Hz, 1H), 7.44 (d, J = 8.4 Hz, 1H), 7.25 (s, 1H), 7.11 (d, J = 2.0 Hz, 1H), 7.08 (s, 1H), 3.54 (s, 2H), 2.95-2.90 (m, 4H), 2.87 (s, 3H), 2.75 (d, J=4.8 Hz, 3H), 2.53-2.51 (m, 4H), 2.44 (s, 3H). |
| 103 | 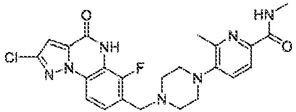 | 483.93 | 484.10 [M+H]⁺ | DMSO-d₆: δ 12.10 (br, 1H), 8.41-8.39 (m, 1H), 7.83 (d, J = 8.4 Hz, 1H), 7.74 (d, J = 8.4 Hz, 1H), 7.43 (d, J = 8.0 Hz, 1H), 7.35-7.31 (m, 1H), 7.27 (s,1H), 3.67 (s, 2H), 2.94-2.88 (m, 4H), 2.75 (d, J= 4.8 Hz, 3H), 2.60-2.54 (m, 4H), 2.45 (s, 3H). |
| 104 | 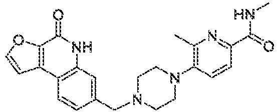 | 431.49 | 432.20 [M+H]⁺ | DMSO-d₆: δ 11.81 (s, 1H), 8.38 (q, J = 5.8, 5.4 Hz, 1H), 8.20 (d, J = 1.9 Hz, 1H), 7.94 (d, J = 8.1 Hz, 1H), 7.76 (d, J = 8.2 Hz, 1H), 7.48 - 7.38 (m, 3H), 7.23 (dd, J = 8.0, 1.5 Hz, 1H), 3.61 (s, 2H), 2.98 - 2.87 (m, 4H), 2.76 (d, J = 4.9 Hz, 3H), 2.62 - 2.51 (m, 4H), 2.48 (s, 3H). |

### Example 105

### 7-((4-(2-methyl-6-(methylcarbamoyl)pyridin-3-yl)piperazin-1-yl)methyl)-1-methyl-1,5-dihydro-4H-pyrazolo[4,3-c]quinolin-4-one

a) Preparation of ethyl-2-(4-bromo-2-nitrobenzoyl)-3-(dimethylamino)acrylate: A solution of 4-bromo-2-nitrobenzoic acid (3.0 g, 12.2 mmol) in SOCl₂ (10 ml) was stirred at 50°C for 3 hours under nitrogen. After completed, the solution was concentrated under reduced pressure. To the residue was added toluene (3 mL) and concentrated again under reduced pressure. A solution of the resulting acid chloride in acetonitrile (8 mL) was added dropwise to a solution of ethyl 3-dimethylaminoacrylate (1.7 g, 12.2 mmol) and TEA (4.0 g, 36.6 mmol) in acetonitrile (30 mL) at room temperature and stirred overnight. To the resulting reaction mixture was added water (20 mL) and extracted with EA (20 mL × 3). The combined organic phase was washed with brine, dried over anhydrous sodium sulfate and filtered. The filtrate was concentrated under reduced pressure. The residue was purified by silica gel chromatography (PE/EA=1/1) to give the title compound (2.0 g, yellow oil, yield: 44%). MS(ESI, m/z): 371.15 [M+H]⁺.
b) Preparation of ethyl 5-(4-bromo-2-nitrophenyl)-1-methyl-1H-pyrazole-4-carboxylate: To a solution of ethyl-2-(4-bromo-2-nitrobenzoyl)-3-(dimethylamino)acrylate (0.8 g, 2.2 mmol) in acetonitrile (8 mL) was added methylhydrazine (40% in H₂O, 0.4 g, 2.6 mmol). The mixture was stirred at 50°C for 1 hour. After the reaction was completed, the mixture was concentrated under reduced pressure. The residue was diluted with water (20 mL) and extracted with EA (20 mL × 3). The combined organic phase was washed with brine, dried over anhydrous sodium sulfate and filtered. The filtrate was concentrated under reduced pressure, and the residue was purified by silica gel chromatography (PE/EA=10/1) to give the title compound (0.6 g, yellow solid, yield: 78 %). MS(ESI, m/z): 354.15 [M+H]⁺.
c) Preparation of 7-bromo-1-methyl-1,5-dihydro-4H-pyrazolo[4,3-c]quinolin-4-one: To a solution of ethyl 5-(4-bromo-2-nitrophenyl)-1-methyl-1H-pyrazole-4-carboxylate (0.3 g, 0.8 mmol) in acetic acid (6 mL) was added iron powder (0.3 g, 5.4 mmol) under nitrogen. The resulting mixture was stirred at 80°C for 2 hours. After the reaction was completed, the mixture was cooled to room temperature. The mixture was filtered and filtrate was poured into ice water, the suspension was filtered. The solid was dried under reduced pressure to give the title compound (0.1 g, white solid, 48 %). MS(ESI, m/z): 277.95 [M+H]⁺.
d) Preparation of 7-(hydroxymethyl)-1-methyl-1,5-dihydro-4H-pyrazolo[4,3-c]quinolin-4-one: To a solution of 7-bromo-1-methyl-1,5-dihydro-4H-pyrazolo[4,3-c]quinolin-4-one (0.7 g, 2.5 mmol) in dioxane (20 mL) was added (tributylstannyl)methanol (1.5 g, 5.0 mmol) and Xphos Pd G2 (165.0 mg, 0.2 mmol) at room temperature. The mixture was stirred at 90 °C overnight under nitrogen atmosphere. The reaction was cooled to room temperature, a solution of KF (1 M, 10 mL) was added, the mixture was stirred for 10 minutes and filtered. The filtrate was extracted with EA (50 mL × 3). The combined organic phase was washed with brine, dried over Na₂SO₄ and concentrated under reduced pressure. The residue was triturated with PE:EA=1:1 (10 mL) and the solid was collected by filtration to afford the title compound (0.5 g, white solid, yield: 87 %). MS(ESI, m/z): 230.30 [M+H]⁺.
e) Preparation of 7-(bromomethyl)-1-methyl-1,5-dihydro-4H-pyrazolo[4,3-c]quinolin-4-one: A solution of 7-(hydroxymethyl)-1-methyl-1,5-dihydro-4H-pyrazolo[4,3-c]quinolin-4-one (200 mg, 0.87mmol) in HBr (48% in water, 10 mL) was stirred at 80°C for 3 hours. the reaction mixture was concentrated under reduced pressure. Acetonitrile (3 mL) was added and the reaction mixture was concentrated under reduced pressure to give the title compound (crude, 254 mg, off-white solid). MS(ESI, m/z): 292.55 [M+H]⁺.
f) Preparation of 7-((4-(2-methyl-6-(methylcarbamoyl)quinolin-3-yl)piperazin-1-yl)methyl)-1-methyl-1,5-dihydro-4H-pyrazolo[4,3-c]quinoline-4-one: To a solution of 7-(bromomethyl)-1-methyl-1,5-dihydro-4H-pyrazolo[4,3-c]quinolin-4-one (60.0 mg, 0.2 mmol), KI (7.0 mg, 0.1 mmol) and N,6-dimethyl-5-(piperazin-1-yl)picolinamide (45.3 mg, 0.2 mmol) in CH₃CN (3 mL) was added DIEA (123.0 mg, 1.0 mmol) at room temperature under N₂. The resulting suspension was stirred at 80 °C for 30 min. After completion, the solvent was removed under vacuum. The residue was purified by Prep-HPLC to give the target compound (11.0 mg, white solid, yield: 12 %).

### Example 106

### 7-((4-(2-methyl-6-(methylcarbamoyl)pyridin-3-yl)piperazin-1-yl)methyl)- 1,5-dihydro-4H-pyrazolo[4,3-c]quinolin-4-one

a) Preparation of ethyl 5-(4-bromo-2-nitrophenyl)-1H-pyrazole-4-carboxylate: To a solution of ethyl 2-(4-bromo-2-nitrobenzoyl)-3-(dimethylamino)acrylate (1.0g, 2.69 mmol) in acetonitrile (8 mL) was added hydrazine hydrate (50 % in water, 323 mg, 2.69 mmol). The resulting mixture was stirred at 50°C for 1 hours. The reaction mixture was concentrated under reduced pressure. The residue was diluted with water (20 mL) and extracted with EA (20 mL × 3). The combined organic phase was washed with brine, dried over anhydrous sodium sulfate and concentrated under reduced pressure. The residue was purified chromatography over silica gel (PE/EA=4/1) to give the title compound (800 mg, yellow solid, yield: 88%). MS(ESI, m/z): 339.90 [M+H]⁺.
b) Preparation of 7-bromo-1,5-dihydro-4H-pyrazolo[4,3-c]quinolin-4-one: To a solution of ethyl 5-(4-bromo-2-nitrophenyl)-1H-pyrazole-4-carboxylate (800 mg, 2.36 mmol) in acetic acid (6 mL) was added iron powder (662 mg, 11.83 mmol) under nitrogen. The resulting mixture was stirred at 80°C for 2 hours. After the reaction was completed, the mixture was cooled to room temperature and filtered. The solid was dried under reduced pressure to give the title compound (400 mg, white solid, 67.6%). MS(ESI, m/z): 261.95 [M+H]⁺.
c) Preparation of tert-butyl 7-bromo-4-oxo-4,5-dihydro-1H-pyrazolo[4,3-c]quinoline-1-carboxylate: To a solution of 7-bromo-1,5-dihydro-4H-pyrazolo[4,3-c]quinolin-4-one (400 mg, 1.4 mmol) in DMF (4 mL) was added di-tert-butyl dicarbonate (325 mg, 1.4 mmol) and DMAP (4-dimethylaminopyridine, 20 mg, 0.14 mmol) at room temperature. The mixture was stirred at room temperature for 2 hours. To the mixture was added water (20 mL) and extracted with EA (2 × 40 mL). The combined organic phase was washed with brine, dried over Na₂SO₄ and concentrated under reduced pressure. The residue was purified by silica gel (DCM/MeOH=10:1) to afford the title compound (500 mg, yellow solid, 73%). MS(ESI, m/z): 364.00 [M+H]⁺.
d) Preparation of tert-butyl 7-(hydroxymethyl)-4-oxo-4,5-dihydro-1H-pyrazolo[4,3-c]quinoline-1-carboxylate: To a solution of tert-butyl 7-bromo-4-oxo-4,5-dihydro-1H-pyrazolo[4,3-c]quinoline-1-carboxylate (500 mg, 1.37 mmol) in dioxane (20 mL) was added (tributylstannyl)methanol (881 mg, 2.7 mmol) and Xphos Pd G2 (80 mg, 0.13 mmol) at room temperature. The mixture was stirred at 90°C overnight under nitrogen atmosphere. After completion, a solution of KF (1 M, 10 mL) was added at room temperature. The mixture was stirred at the same temperature for 10 minutes and filtered, and the filtrate was extracted with EA (50 mL × 3). The combined organic phase was washed with brine, dried over Na₂SO₄ and concentrated under reduced pressure. The residue was triturated with PE:EA=1:1 (10 mL) and the solid was collected by filtration to afford the title compound (crude, 450 mg, white solid, yield: 90 %). MS(ESI, m/z): 316.05 [M+H]⁺.
e) Preparation of 7-(bromomethyl)-1,5-dihydro-4H-pyrazolo[4,3-c]quinolin-4-one: A solution of tert-butyl 7-(hydroxymethyl)-4-oxo-4,5-dihydro-1H-pyrazolo[4,3-c]quinoline-1-carboxylate (450 mg, 1.3 mmol) in HBr (48% in water,10 mL) was stirred at 85°C for 3 hours. The reaction mixture was concentrated under reduced pressure. Acetonitrile (3 ml) was added to the crude product and the mixture was concentrated under reduced pressure to give the title compound (crude, off-white solid, 300 mg). MS(ESI, m/z): 275.85 [M+H]⁺.
f) Preparation of tert-butyl 7-(bromomethyl)-4-oxo-4,5-dihydro-1H-pyrazolo[4,3-c]quinoline-1-carboxylate: To a solution of 7-(bromomethyl)-1,5-dihydro-4H-pyrazolo[4,3-c]quinolin-4-one (353 mg, 1.2 mmol) in DMF (4 mL) was added di-tert-butyl dicarbonate (287 mg, 1.3mmol) and DMAP (20 mg, 0.14 mmol) at room temperature. The mixture was stirred at room temperature 2 hours. The mixture was added water (20 ml) and extracted with EA (40 mL × 2). The combined organic phase was washed with brine, dried over Na₂SO₄ and concentrated under reduced pressure. The residue was purified by silica gel (DCM/MeOH=10:1) to afford the title compound (131 mg, yellow solid, 33 %). MS(ESI, m/z): 378.00 [M+H]⁺.
g) Preparation of tert-butyl 7-((4-(2-methyl-6-(methylcarbamoyl)pyridin-3-yl)piperazin-1-yl)rnethyl)-4-oxo-4,5-dihydro-1H-pyrazolo[4,3-c]quinoline-1-carboxylate: To a solution of tert-butyl 7-(bromomethyl)-4-oxo-4,5-dihydro-1H-pyrazolo[4,3-c]quinoline-1-carboxylate (13 mg, 0.34 mmol), KI (10.0 mg, 0.1 mmol) and N,6-dimethyl-5-(piperazin-1-yl)picolinamide (81 mg, 0.34 mmol) in acetonitrile (3 mL) was added DIEA (140.0 mg, 1.0 mmol) at room temperature under N₂. The resulting suspension was stirred at 80°C for 30 min. After completion, the solvent was removed under reduced pressure. The organic phase was dried over anhydrous sodium sulfate and concentrated to afford residue, which was washed with acetonitrile to give the title compound (95.0 mg, white solid, yield: 52 %). MS(ESI, m/z): 532.25 [M+H]⁺.
h) Preparation of 7-((4-(2-methyl-6-(methylcarbamoyl)quinolin-3-yl)piperazin-1-yl)methyl)-1,5-dihydro-4H-pyrazolo[4,3-c]quinoline-4-one: To solution of tert-butyl 7-((4-(2-methyl-6-(methylcarbamoyl)pyridin-3-yl)piperazin-1-yl)methyl)-4-oxo-4,5-dihydro-1H-pyrazolo[4,3-c]quinoline-1-carboxylate (95 mg, 0.17 mmol) in dioxane (5 mL) was added HCl/dioxane (5 mL) under N₂. The resulting suspension was stirred at room temperature for 2 h. After completion, the solvent was removed under reduced pressure. The residue was purified by Prep-TLC (DCM/MeOH=10/1) to afford the target compound (5.0 mg, white solid, yield: 6 %).

The following compounds of Examples 107-108, 121-122, 138-139, 143 and 149 were prepared using a synthesis method similar to that described in Example 83.

The following compounds of Examples 109-117, 123-130, 133-137, 140-142, 144, and 146-148 were prepared using a synthesis method similar to that described in Example 73.

The compound of Example 118 was prepared using a synthesis method similar to that described in Example 48.

The following compounds of Examples 119-120 were prepared using a synthesis method similar to that described in Example 31.

The following compounds of Examples 131-132, and 150 were prepared using a synthesis method similar to that described in Example 48.

The compound of Example 145 was prepared using a synthesis method similar to that described in Example 24.

| Example | Compound | MW | LC-MS (ESI) | ¹H NMR (400MHz) |
|---|---|---|---|---|
| 105 | 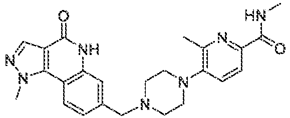 | 445.52 | 446.15 [M+H]⁺ | DMSO-d₆: δ 11.39 (s, 1H), δ 8.43 (q, J = 4.8 Hz, 1H), 8.17 (d, J = 8.3 Hz, 1H), 8.07 (s, 1H), 7.79 (d, J = 8.3 Hz, 1H), 7.56 - 7.44 (m, 2H), 7.28 (d, J = 8.2 Hz, 1H), 4.35 (s, 3H), 3.64 (s, 2H), 3.40-3.31(m, 4H), 2.99-2.92 (m, 4H), 2.79 (d, J = 4.8 Hz, 3H), 2.59 (s, 3H). |
| 106 | 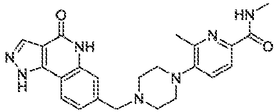 | 431.49 | 432.15 [M+H]⁺ | DMSO-d₆: δ 11.29 (s, 1H), 8.44 (s, 1H), 8.03 (s, 1H), 7.78 (s, 1H). 7.44 (d, J = 30.3 Hz, 2H), 7.24 (s, 1H), 3.62 (s, 2H), 3.00-2.90 (m, 4H), 2.80 (s, 3H), 2.65-2.55 (s, 4H), 2.50 (s, 3H). |
| 107 | 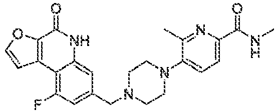 | 449.48 | 450.20 [M+H]⁺ | DMSO-d₆: δ 12.06 (s, 1H), 8.39 (q, J = 5.3, 4.6 Hz, 1H), 8.27 (d, J = 1.9 Hz, 1H), 7.76 (d, J = 8.3 Hz, 1H), 7.45 (d, J = 8.3 Hz, 1H), 7.28 - 7.18 (m, 2H), 7.10 (d, J = 11.5 Hz, 1H), 3.61 (s, 2H), 2.93 (dd, J = 6.1, 3.4 Hz, 4H), 2.76 (d, J = 4.8 Hz, 3H), 2.61 - 2.53 (m, 4H), 2.45 (s, 3H). |
| 108 | 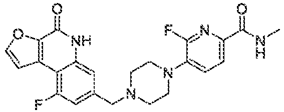 | 453.44 | 454.30 [M+H]⁺ | DMSO-d₆: δ 12.05 (s, 1H), 8.37 (s, 1H), 8.27 (d, J = 1.9 Hz, 1H), 7.81 (d, J = 8.1 Hz, 1H), 7.62 - 7.45 (m, 1H), 7.29 - 7.18 (m, 2H), 7.09 (d, J = 11.3 Hz, 1H), 3.60 (s, 2H), 3.19 - 3.11 (m, 4H), 2.72 (d, J = 4.7 Hz, 3H), 2.59 - 2.50 (m, 4H). |
| 109 | 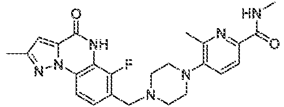 | 463.52 | 464.35 [M+H]⁺ | DMSO-d₆: δ 11.87 (br, 1H), 8.40-8.38 (m, 1H), 7.82 (d, J = 8.0 Hz, 1H) , 7.74 (d, J = 8.0 Hz, 1H), 7.43 (d,J = 8.0 Hz, 1H), 7.28 (t, J = 8.0 Hz, 1H), 6.94 (s, 1H), 3.65 (s. 2H), 2.94-2.86 (m, 4H), 2.75 (d, J= 4.0 Hz, 3H), 2.62-2.54 (m, 4H), 2.44 (s, 3H), 2.39 (s,3H). |
| 110 | 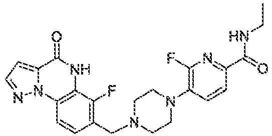 | 467.48 | 468.15 [M+H]⁺ | DMSO-d₆: δ 8.42-8.39 (m,1H), 8.06 (d, J=2.0 Hz, 1H), 7.91 (d, J = 8.4 Hz, 1H), 7.80 (d, J = 7.6 Hz, 1H), 7.55-7.50 (m, 1H), 7.34-7.30 (m, 1H), 7.15 (d, J = 1.6 Hz, 1H), 3.66 (s, 2H), 3.26-3.21 (m, 2H), 3.16-3.10 (m, 4H), 2.59-2.53 (m, 4H), 1.04 (t, J = 6.8 Hz, 3H). |
| 111 | 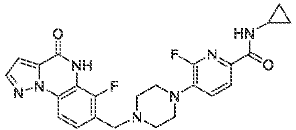 | 479.49 | 480.35 [M+H]⁺ | DMSO-d₆: δ 8.31 (d, J = 4.4 Hz, 1H), 8.06 (d, J=2.0 Hz, 1H), 7.91 (d, J = 8.4 Hz, 1H), 7.79 (d, J = 8.0 Hz, 1H), 7.54-7.49 (m, 1H), 7.33-7.30 (m, 1H), 7.15 (d, J = 2.0 Hz, 1H), 3.66 (s, 2H), 3.15-3.09 (m, 4H), 2.81-2.80 (m, 1 H), 2.58-2.52 (m, 4H), 0.62-0.62 (m, 4H). |
| 112 | 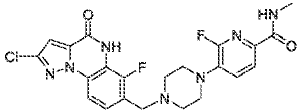 | 487.90 | 488.30 [M+H]⁺ | DMSO-d₆: δ 12.13 (br, 1H), 8.41-8.39 (m, 1H), 7.87-7.79 (m, 2H), 7.54-7.50 (m, 1H), 7.34-7.30 (m, 1H), 7.27 (s, 1H), 3.66 (s, 2H), 3.16-3.10 (m, 4H), 2.72 (d, J= 4.8 Hz, 3H), 2.59-2.53 (m, 4H). |
| 113 | 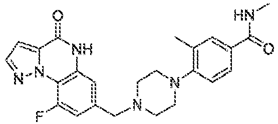 | 448.50 | 449.20 [M+H]⁺ | CD₃OD: δ 8.04 (d, J = 2.1 Hz, 1H), 7.61 (d, J = 12.8 Hz, 2H), 7.23 (d, J = 13.9 Hz, 3H), 7.07 (d. J = 8.3 Hz, 1H), 3.67 (s, 2H), 3.07 - 2.95 (m, 4H), 2.88 (s, 3H), 2.75 - 2.52 (m, 4H), 2.32 (s, 3H). |
| 114 | 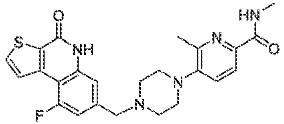 | 465.55 | 466.15 [M+H]⁺ | DMSO-d₆: δ 12.09 (s, 1H), 8.44 (q, J = 4.3 Hz, 1H), 8.24 (d, J = 5.1 Hz, 1H), 7.92 - 7.86 (m, 1H), 7.79 (d, J = 8.2 Hz, 1H), 7.48 (d, J = 8.3 Hz, 1H), 7.31 (s, 1H), 7.13 (d, J = 12.0 Hz, 1H), 3.65 (s, 2H), 3.00 - 2.93 (m, 4H), 2.79 (d, J = 4.5 Hz, 3H), 2.65 - 2.53 (m, 4H), 2.48 (s, 3H). |
| 115 | 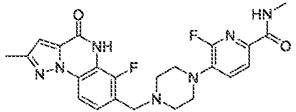 | 467.48 | 468.30 [M+H]⁺ | DMSO-d₆: δ 11.87 (br, 1H), 8.40-8.38 (m, 1H), 7.81 (t, J = 8.0 Hz, 2H), 7.54-7.49 (m, 1H), 7.28 (t, J = 8.0 Hz, 1H), 6.94 (s, 1H), 3.64 (s, 2H), 3.14-3.12 (m, 4H), 2.72 (d, J= 8.0 Hz. 3H), 2.58-2.56 (m, 4H), 2.39 (s, 3H). |
| 116 | 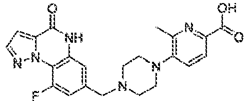 | 436.45 | 437.10 [M+H]⁺ | DMSO-d₆: δ 7.87 (d, J = 1.6 Hz, 1H), 7.70 (d, J = 8.4 Hz, 1H), 7.42 (d, J = 8.0 Hz, 1H), 7.03 (s, 1H), 6.84 (d, J = 12.0 Hz, 1H), 6.78 (s, 1 H), 3.53 (s, 2H), 2.91-2.85 (m, 4H), 2.58-2.52 (m, 4H), 2.37 (s, 3H). |
| 117 | 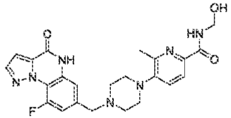 | 465.49 | 466.15 [M+H]⁺ | DMSO-d₆: δ 8.95 (t, J = 6.6 Hz, 1H), 8.09 (s, 1H), 7.85 (d, J = 8.2 Hz, 1H), 7.50 (d, J = 8.3 Hz, 1H), 7.23 (s, 1H), 7.20 - 7.11 (m, 2H), 4.74 (d, J = 6.6 Hz, 2H), 3.61 (s, 2H), 3.01 - 2.94 (m, 4H), 2.64 - 2.57 (m, 4H), 2.50 (s, 3H). |
| 118 | 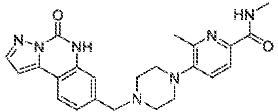 | 431.50 | 432.15 [M+H]⁺ | DMSO-d₆: δ 11.78 (s, 1H), 8.39 (q, J = 5.8, 5.2 Hz, 1H), 8.04 (d, J = 1.8 Hz, 1H), 7.98 (d, J = 8.0 Hz, 1H), 7.76 (d, J = 8.2 Hz, 1H), 7.45 (d, J = 8.3 Hz, 1H), 7.31 (s, 1H), 7.24 (d, J = 8.1 Hz, 1H), 7.15 (d, J = 1.8 Hz, 1H), 3.60 (s, 2H), 2.97 - 2.88 (m, 4H), 2.76 (d, J = 4.8 Hz, 3H), 2.60 - 2.53 (m, 4H), 2.45 (s, 3H). |
| 119 | 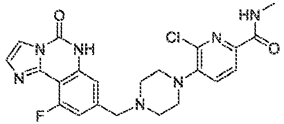 | 469.91 | 470.10 [M+H]⁺ | DMSO-d₆: δ 12.40 (br, 1H), 7.94-7.92 (m, 2H), 7.72-7.70 (m, 1H), 7.54-7.50 (m, 1H), 7.36-7.29 (m, 2H), 4.37 (s, 2H), 3.38-3.31 (m, 8H), 2.76-2.72 (m, 3H). |
| 120 | 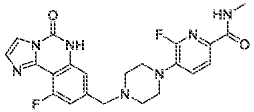 | 453.45 | 454.10 [M+H]⁺ | DMSO-d₆: δ 8.41-8.39 (m,1H), 7.86 (s, 1H), 7.81 (d, J = 7.6 Hz, 1H), 7.57-7.52 (m, 1H), 7.43 (s, 1 H), 7.17 (s, 1H), 7.08 (d, J = 10.0 Hz, 1H), 3.59 (s, 2H), 3.19-3.12 (m, 4H), 2.72 (d, J= 4.4 Hz, 3H), 2.58-2.52 (m, 4H). |
| 121 | 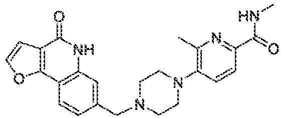 | 431.50 | 432.10 [M+H]⁺ | DMSO-d₆: δ 11.67 (br, 1H), 8.41- 8.37 (m, 1H). 8.04 (d, J = 4.0 Hz, 1H), 7.85 (d, J = 8.0 Hz, 1H), 7.76 (d, J = 8.0 Hz, 1H),7.44 -7.42 (m, 2H), 7.24 (d, J = 12.0 Hz, 1H), 7.02 (d, J = 4.0 Hz, 1H), 3.61 (s, 2H), 2.93-2.92 (m, 4H), 2.75 (d, J = 4.0 Hz, 3H), 2.56-2.53 (m, 4H), 2.46 (s, 3H). |
| 122 | 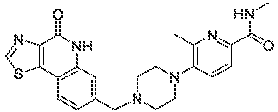 | 448.55 | 449.15 [M+H]⁺ | DMSO-d₆: δ 11.93 (s, 1H), 9.27 (s, 1H), 8.40 - 8.37 (m, 1H), 7.78 (dd, J = 18.7. 8.2 Hz, 2H), 7.47 - 7.42 (m, 2H), 7.24 (d, J = 8.1 Hz, 1H), 3.62 (s, 2H), 2.95-2.89 (m, 4H), 2.76 (d, J = 4.8 Hz, 3H), 2.59-2.53 (m, 4H), 2.50 (s, 3H). |
| 123 | 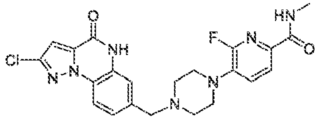 | 469.91 | 470.10 [M+H]⁺ | DMSO-d₆: δ 12.01 (s, 1H), 8.37 (d, J = 5.1 Hz, 1H), 7.99 (d, J = 8.3 Hz, 1H), 7.81 (d, J = 8.0 Hz, 1H), 7.58 - 7.49 (m, 1H), 7.36 (s, 1H), 7.26 (d, J = 8.5 Hz, 1H), 7.22 (s, 1H), 3.58 (s, 2H), 3.18 - 3.11 (m, 4H), 2.72 (d, J = 4.6 Hz, 3H), 2.57 - 2.51 (m, 4H). |
| 124 | 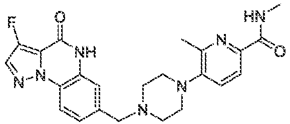 | 449.49 | 450.10 [M+H]⁺ | DMSO-d₆: δ 8.43 (d, J = 4.9 Hz, 1H), 8.20 (s, 1H), 8.17 (d, J = 3.7 Hz, 1H), 8.07 (d, J = 8.3 Hz, 1H), 7.79 (d, J= 8.2 Hz, 1H), 7.48 (d, J = 8.3 Hz, 1H), 7.37 (s, 1H), 7.29 (d, J = 8.5 Hz, 1H), 3.62 (s, 2H), 2.98 - 2.92 (m, 4H), 2.79 (d, J = 4.9 Hz, 3H), 2.63 - 2.55 (m, 4H), 2.54 (s, 3H). |
| 125 | 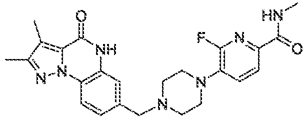 | 463.52 | 464.15 [M+H]⁺ | DMSO-d₆: δ 8.40 (d, J = 5.4 Hz, 1H), 7.97 (d, J = 8.4 Hz, 1H), 7.84 (d, J = 8.1 Hz, 1H), 7.62 - 7.52 (m, 1H), 7.31 (s, 1H), 7.22 (d, J = 8.3 Hz, 1H), 3.58 (s, 2H), 3.23 - 3.14 (m, 4H), 2.76 (d, J = 4.7 Hz, 3H), 2.61 - 2.53 (m, 4H), 2.36 (s, 3H), 2.33 (s, 3H). |
| 126 | 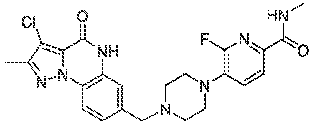 | 483.93 | 484.15 [M+H]⁺ | DMSO-d₆: δ 11.86 (s,1H),8.37 (d, J = 4.7 Hz, 1H), 7.97 (d, J = 8.4 Hz, 1H), 7.80 (d, J = 87.5 Hz, 1H), 7.53 (dd, J = 10.6, 8.2 Hz, 1H), 7.30 (s, 1H), 7.22 (d, J = 8.5 Hz, 1H), 3.56 (s, 2H), 3.20 - 3.10 (m, 4H), 2.72 (d, J = 4.8 Hz, 3H), 2.61 - 2.50 (m, 4H), 2.34 (s, 3H) |
| 127 | 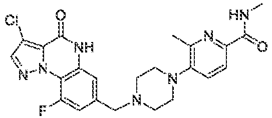 | 483.93 | 484.10 [M+H]⁺ | DMSO-d₆: δ 8.39 (d, J = 4.6 Hz, 1H), 8.20 (s, 1H), 7.76 (d, J = 8.2 Hz, 1H), 7.45 (d, J = 8.2 Hz, 1H), 7.14 (d, J = 13.0 Hz, 1H), 3.57 (s, 2H), 2.95-2.86 (m, 4H), 2.76 (d, J = 4.8 Hz, 3H), 2.60-2.46 (m, 4H), 2.42 (s, 3H). |
| 128 | 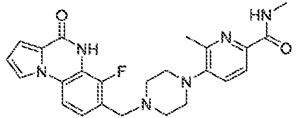 | 448.50 | 449.15 [M+H]⁺ | DMSO-d₆: δ 11.24 (s, 1H), 8.43 - 8.34 (m, 1H), 8.16 (d, J = 2.7 Hz, 1H), 7.85 (d, J = 8.6 Hz, 1H), 7.75 (d, J = 8.4 Hz, 1H), 7.44 (d, J = 8.3 Hz, 1H), 7.21 (t, J = 7.8 Hz, 1H), 7.09 - 7.01 (m, 1H), 6.70 - 6.66 (m, 1H), 3.65 (s, 2H), 2.96 - 2.87 (m, 4H), 2.76 (d, J = 4.8 Hz, 3H), 2.63 - 2.55 (m, 4H), 2.45 (s, 3H). |
| 129 | 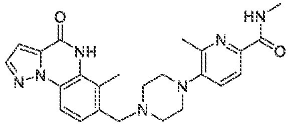 | 445.53 | 446.15 [M+H]⁺ | DMSO-d₆: δ 8.41-8.37 (m, 1H), 8.03 (d, J = 4.0 Hz, 1H), 7.97 (d, J = 8.0 Hz, 1H), 7.74 (d, J = 8.0 Hz, 1H), 7.41 (d, J = 8.0 Hz, 1H), 7.25 (d, J = 8.0 Hz, 1H), 7.12 (d, J= 4.0 Hz, 1H), 3.59 (s, 2H), 2.91 - 2.88 (m, 4H), 2.76 (d, J= 4.0 Hz, 3H), 2.57-2.55 (m, 4H), 2.48 (s, 3H), 2.46 (s, 3H). |
| 130 | 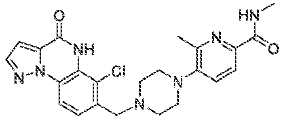 | 465.94 | 466.10 [M+H]⁺ | DMSO-d₆: δ 8.41-8.37 (m, 1H), 8.06-8.05 (m, 2H), 7.77 (d, J = 8.0 Hz, 1H), 7.59 (s, 1H), 7.45 (d, J = 8.0 Hz, 1H), 7.13 (s, 1H), 3.68 (s, 2H), 2.97 - 2.94 (m, 4H), 2.76 (d, J= 4.0 Hz, 3H), 2.65-2.63 (m, 4H), 2.48(s, 3H). |
| 131 | 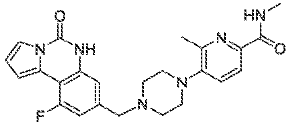 | 448.50 | 449.35 [M+H]⁺ | DMSO-d₆: δ 11.72 (s, 1H), 8.43 (q, J = 5.3, 4.8 Hz, 1H), 7.80 (d, J = 8.0 Hz, 1H), 7.68 (dd, J = 3.2, 1.4 Hz, 1H), 7.49 (d, J = 8.3 Hz, 1H), 7.19 - 7.04 (m, 2H), 6.91 - 6.83 (m, 1H), 6.73 (t, J = 3.3 Hz, 1H), 3.62 (s, 2H), 3.01 - 2.93 (m, 4H), 2.80 (d, J = 4.8 Hz, 3H), 2.64 - 2.56 (m, 4H), 2.50 (s, 3H). |
| 132 | 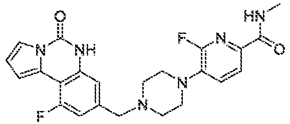 | 452.47 | 453.15 [M+H]⁺ | DMSO-d₆: δ 11.71 (s, 1H), 8.41 (d, J = 4.9 Hz, 1H), 7.85 (d. J = 8.0 Hz, 1H), 7.67 (d, J = 3.5 Hz, 1H), 7.58 (dd, J = 10.6, 8.1 Hz, 1H), 7.14 - 7.04 (m, 2H), 6.90 - 6.83 (m, 1H). 6.73 (t, J = 3.3 Hz. 1H), 3.59 (s. 2H), 3.23 - 3.15 (m, 4H), 2.76 (d, J = 4.7 Hz, 3H), 2.61 - 2.55 (m, 4H). |
| 133 | 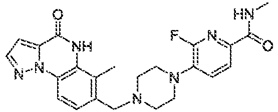 | 449.49 | 450.15 [M+H]⁺ | DMSO-d₆: δ 10.99 (br, 1H), 8.41-8.37 (m, 1H), 8.03 (d, J = 2.0 Hz, 1H), 7.96 (d, J = 8.8 Hz, 1H), 7.79 (d, J = 8.0 Hz, 1H), 7.54-7.50 (m, 1H), 7.23 (d, J = 8.4 Hz, 1H), 7.11 (d, J= 1.6 Hz, 1H), 3.57 (s, 2H), 3.11 - 3.10 (m, 4H), 2.72 (d, J= 4.4 Hz, 3H), 2.57-2.54 (m, 4H), 2.46 (s, 3H). |
| 134 | 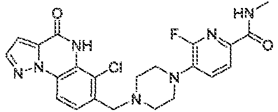 | 469.91 | 470.10 [M+H]⁺ | DMSO-d₆: δ 8.41-8.37 (m, 1H), 8.05-8.04 (m, 2H), 7.81 (d, J = 8.0 Hz, 1H), 7.51-7.52 (m, 2H), 7.13 (s, 1H), 3.64 (s, 2H), 3.16 - 3.12 (m, 4H), 2.72 (d, J= 4.0 Hz, 3H), 2.61-2.59 (m, 4H). |
| 135 | 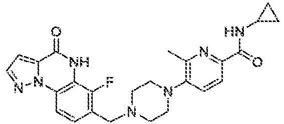 | 475.53 | 476.20 [M+H]⁺ | DMSO-d₆: δ 8.28 (d, J = 5.2 Hz, 1H), 8.06 (d, J=2.0 Hz, 1H), 7.91 (d, J = 8.0 Hz, 1H), 7.74 (d, J = 8.4 Hz, 1H), 7.43 (d, J = 8.8 Hz, 1H), 7.32(t, J = 8.0 Hz, 1H), 7.15 (d, J = 2.0 Hz, 1H), 3.67 (s, 2H), 2.91-2.90 (m, 4H), 2.81-2.80 (m, 1 H), 2.58-2.56 (m, 4H), 2.43 (s, 3H), 0.65-0.64 (m, 2H), 0.60-0.59 (m, 2H). |
| 136 | 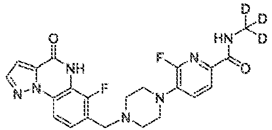 | 456.47 | 457.10 [M+H]⁺ | DMSO-d₆: δ 8.33 (s, 1H), 8.07 (d, J = 2.0 Hz, 1H), 7.91 (d, J = 8.8 Hz, 1H), 7.79 (d, J = 8.4 Hz, 1H), 7.54-7.49 (m, 1H), 7.34-7.30 (m, 1H), 7.15 (d, J= 2.0 Hz, 1H), 3.66 (s, 2H), 3.14 -3.12 (m, 4H), 2.57-2.55 (m, 4H). |
| 137 | 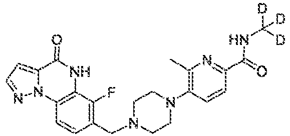 | 452.51 | 453.20 [M+H]⁺ | DMSO-d₆: δ 8.36 (s, 1H), 8.07 (d, J = 2.0 Hz, 1H). 7.91 (d, J = 8.8 Hz, 1H), 7.75 (d, J = 8.0 Hz, 1H), 7.41 (d, J = 8.0 Hz, 1H), 7.34-7.30 (m, 1H), 7.15 (d, J= 2.0 Hz, 1H), 3.67 (s, 2H), 2.92 - 2.90 (m, 4H), 2.59-2.55 (m, 4H), 2.44 (s,3H). |
| 138 | 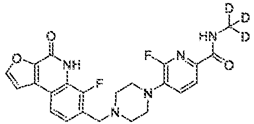 | 457.67 | 457.10 [M+H]⁺ | DMSO-d₆: δ 11.84 (s, 1H), 8.36 (d, J = 5.0 Hz, 1H), 8.24 (s, 1H), 7.79 (d, J = 8.1 Hz, 2H), 7.55 - 7.48 (m, 1H), 7.46 (s, 1H), 7.32 - 7.25 (m, 1H). 3.68 (s, 2H), 3.17-3.08 (m, 4H), 2.72 (d, J = 4.6 Hz, 3H), 2.60-2.52 (s. 4H). |
| 139 | 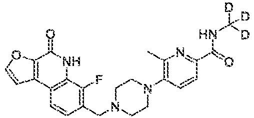 | 452.50 | 453.15 [M+H]⁺ | DMSO-d₆: δ 11.85 (s, 1H), 8.35 (s, 1H), 8.24 (d, J = 1.8 Hz, 1H), 7.80 (d, J = 8.0 Hz, 1H), 7.75 (d, J = 8.2 Hz, 1H), 7.47 (d, J = 1.9 Hz, 1H), 7.43 (d, J = 8.2 Hz, 1H), 7.32 -7.26 (m, 1H), 3.69 (s, 2H), 2.93-2.86 (m, 4H), 2.62-2.53 (m, 4H), 2.44 (s, 3H). |
| 140 | 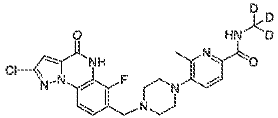 | 486.95 | 487.35 [M+H]⁺ | DMSO-d₆: δ 8.36 (s, 1H), 7.82 (d, J = 8.4 Hz, 1H), 7.74 (d, J = 8.2 Hz, 1H), 7.43 (d, J = 8.4 Hz, 1H), 7.34 - 7.28 (m, 1H), 7.24 (s, 1H), 3.67 (s. 2H), 2.93-2.87 (m, 4H), 2.60-2.55 (s, 4H), 2.44 (s, 3H). |
| 141 | 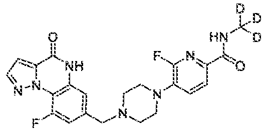 | 456.47 | 457.15 [M+H]⁺ | DMSO-d₆: δ 11.99 (s, 1H), 8.35 (s, 1H), 8.07 (d, J = 2.1 Hz, 1H), 7.81 (d, J = 8.2 Hz, 1H), 7.60 - 7.49 (m, 1H), 7.20 (s, 1H), 7.18 - 7.12 (m, 2H), 3.57 (s, 2H), 3.20 - 3.11 (m, 4H), 2.59 - 2.51 (m, 4H). |
| 142 | 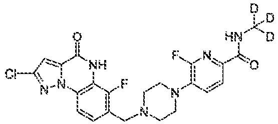 | 490.91 | 491.30 [M+H]⁺ | DMSO-d₆: δ 8.34 (s, 1H), 7.81 (t, J = 8.7 Hz, 2H), 7.56 - 7.48 (m, 1H), 7.33 - 7.26 (m, 1H), 7.23 (s, 1H), 3.65 (s, 2H), 3.16-3.08 (m, 4H), 2.59-2.52 (m, 4H). |
| 143 | 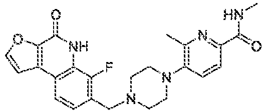 | 449.49 | 450.15 [M+H]⁺ | DMSO-d₆: δ 11.82(s 1H), 8.39-8.37 (m, 1H), 8.24 (d, J = 2.0 Hz, 1H), 7.79 (d, J = 7.6 Hz, 1H), 7.74 (d, J = 8.4 Hz, 1H), 7.47 (d, J = 4.0 Hz, 1H), 7.43 (d, J = 8.0 Hz. 1H), 7.29 (t, J = 8.0 Hz, 1H), 3.69 (s, 2H). 2.91-2.90 (m, 4H), 2.75 (d, J = 4.8 Hz, 3H), 2.59 - 2.58 (m, 4H), 2.44 (s, 3H). |
| 144 | 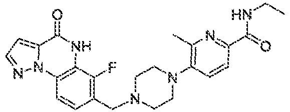 | 463.52 | 464.15 [M+H]⁺ | DMSO-d₆: δ 11.95 (br, 1H), 8.42-8.37 (m, 1H), 8.07 (d, J = 1.6 Hz, 1H), 7.91 (d, J = 8.4 Hz, 1H), 7.75 (d, J = 7.6 Hz, 1H), 7.43 (d, J = 8.0 Hz. 1H), 7.32-7.31 (m, 1H), 7.15 (d, J= 2.0 Hz, 1H), 3.67 (s, 2H), 3.26-3.24 (m, 2H), 2.91 - 2.88 (m, 4H), 2.59-2.55 (m, 4H), 2.48 (s. 3H), 1.06 (t, J = 8.0 Hz, 3H). |
| 145 | 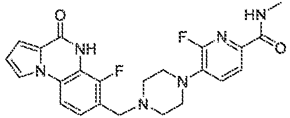 | 452.47 | 453.20 [M+H]⁺ | DMSO-d₆: δ 8.37 (d, J = 5.0 Hz, 1H), 8.30 (s, 1H), 8.15 (dd, J = 2.8, 1.5 Hz, 1H), 7.87 - 7.76 (m, 2H), 7.52 (dd, J = 10.6, 8.2 Hz, 1H), 7.19 (1, J = 7.7 Hz, 1H), 7.04 (dd, J = 3.8, 1.4 Hz, 1H), 6.71 - 6.64 (m, 1H), 3.62 (s, 2H), 3.16 - 3.09 (m, 4H), 2.72 (d, J = 4.8 Hz, 3H), 2.59 - 2.52 (m, 4H). |
| 146 | 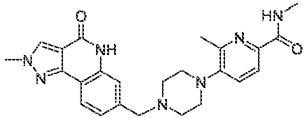 | 445.52 | 446.15 [M+H]⁺ | DMSO-d₆: δ 11.08 (s, 1H), 8.52 (s, 1H), 8.41 (d, J = 5.5 Hz, 1H), 7.95 (d, J = 8.0 Hz, 1H). 7.79 (d, J = 8.3 Hz, 1H), 7.48 (d, J = 8.4 Hz, 1H). 7.35 (s, 1H), 7.18 (d, J = 8.0 Hz, 1H), 4.08 (s, 3H), 3.60 (s, 2H), 2.99 - 2.92 (m, 4H), 2.79 (d, J = 4.8 Hz, 3H), 2.62 - 2.53 (m, 4H), 2.49 (s, 3H). |
| 147 | 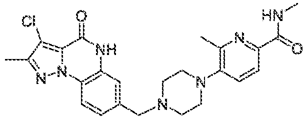 | 479.96 | 480.20 [M+H]⁺ | DMSO-d₆: δ 11.86 (s, 1H), 8.38 (d, J = 4.9 Hz, 1H), 7.97 (d, J = 8.3 Hz, 1H), 7.76 (d, J = 8.2 Hz, 1H), 7.44 (d, J = 8.3 Hz, 1H), 7.32 (s, 1H), 7.23 (d, J = 8.3 Hz, 1H), 3.62 (s, 2H), 3.00 - 2.87 (m, 4H), 2.76 (d, J = 4.8 Hz, 3H), 2.64 - 2.55 (m, 4H), 2.46 (s, 3H), 2.34 (s, 3H). |
| 148 | 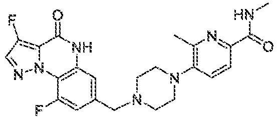 | 467.48 | 468.15 [M+H]⁺ | DMSO-d₆: δ 12.00(br,1H),8.45-8.39 (m, 1H), 8.21 (d, J = 3.7 Hz, 1H). 7.80 (d, J = 8.2 Hz, 1H), 7.49 (d, J = 8.1 Hz, 1H), 7.20 (s, 1H), 7.16 (s, 1H), 3.61 (s, 2H), 2.99-2.92 (m, 4H), 2.80 (d, J= 4.8 Hz, 3H), 2.63-2.55 (m, 4H), 2.49 (s, 3H). |
| 149 | 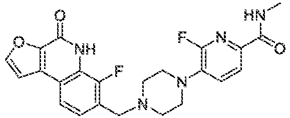 | 453.45 | 454.15 [M+H]⁺ | DMSO-d₆: δ 11.84 (s, 1H), 8.36 (d, J = 5.0 Hz, 1H), 8.24 (s, 1H), 7.79 (d, J = 8.1 Hz, 2H), 7.56 - 7.48 (m, 1H). 7.46 (s, 1H), 7.32 - 7.25 (m, 1H), 3.68 (s, 2H), 3.19-3.06 (m, 4H), 2.72 (d, J = 4.6 Hz, 3H), 2.61-2.51 (s, 4H). |
| 150 | 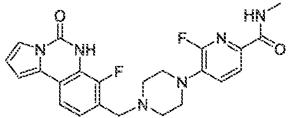 | 452.47 | 453.15 [M+H]⁺ | DMSO-d₆: δ 11.53 (s, 1H), 8.37 (d, J = 4.7 Hz, 1H), 7.80 (d, J = 8.0 Hz, 1H), 7.69 (d, J = 8.2 Hz, 1H), 7.60 (dd, J = 3.0, 1.4 Hz, 1H), 7.51 (dd, J = 10.6, 8.2 Hz, 1H), 7.23 - 7.15 (m, 1H), 7.01 (dd, J = 3.5, 1.3 Hz, 1H), 6.66 (t, J = 3.3 Hz, 1H), 3.62 (s, 2H), 3.16-3.09 (m, 4H), 2.72 (d, J = 4.8 Hz, 3H), 2.59-2.51(m, 4H). |

### Example 151

### PARP1 and PARP2 chemiluminescent Assay

The solution of recombinant poly(ADP-ribose) polymerase 1 and 2 (PARP1 and PARP2) (40 ng enzyme/well) and the compounds to be tested were mixed, respectively. The solutions were added to a 96-well plate coated with histone mixture, incubated at room temperature for 1 h, then 50 µL 0.3ng/mL Streptavidin-HRP was added to each well. The plates were incubated for 30 minutes at room temperature. Finally, the plates were treated with streptavidin-HRP followed by addition of the ELISA ECL substrate to produce chemiluminescence that can be measured using a chemiluminescence reader. Inhibition of the tested compound to PARP1/2 enzyme activity was calculated according to the following formula. $Inhibition \left(%\right) = \frac{Readings of positive control - X}{Readings of positive control - Readings of negative control}$ IC₅₀ value is obtained by fitting the s-shaped dose response curve equation by using XL Fit software. The curve equation is Y=100 / (1+10^(logC-logIC₅₀)), C is the compound concentration.

Table 1 summarize the inhibitory effects of compounds on PARP1 and PARP2 enzyme activity (IC₅₀).

**Table 1**

| Example | IC₅₀, nM | | Example | IC₅₀, nM | | Example | IC₅₀, | nM |
|---|---|---|---|---|---|---|---|---|
| | PARP1 | PARP2 | | PARP1 | PARP2 | | PARP1 | PARP2 |
| 1 | 1.57 | 252.5 | 61 | 0.37 | 19.56 | 102 | 6.91 | 100.62 |
| 2 | 1.91 | 128.50 | 62 | 0.29 | 149.84 | 103 | 0.76 | 1250.26 |
| 3 | 1.33 | 467.50 | 65 | 1.21 | 20.92 | 104 | 1.42 | 598.05 |
| 4 | 1.59 | 188 | 66 | 1.63 | 66.06 | 105 | 1.16 | 910.27 |
| 6 | 1.86 | / | 67 | 2.02 | 48.21 | 106 | 1.16 | 910.27 |
| 7 | 1.90 | / | 68 | 1.61 | 90.15 | 107 | 0.60 | 459.06 |
| 16 | 1.99 | / | 69 | 2.32 | 49.9 | 108 | 0.66 | 353.02 |
| 17 | 1.76 | 29.79 | 70 | 1.37 | 103.11 | 109 | 1.15 | 1267.50 |
| 24 | 0.83 | 31.54 | 71 | 1.35 | 66.44 | 110 | 1.95 | 965.40 |
| 25 | 1.93 | / | 72 | 1.12 | 109.89 | 111 | 1.27 | 454.75 |
| 28 | 0.77 | >3000 | 73 | 1.96 | 78.12 | 112 | 0.57 | 222.86 |
| 30 | 1.54 | 2594.4 | 74 | 1.52 | 186.05 | 113 | 1.90 | 370.20 |
| 31 | 1.37 | >3000 | 75 | 2.91 | 228.13 | 114 | 0.67 | 16.90 |
| 34 | 0.20 | 2.15 | 76 | 2.10 | 64.57 | 115 | 1.45 | 288.25 |
| 35 | 0.81 | 26.34 | 77 | 1.51 | 241.08 | 117 | 0.91 | 644.98 |
| 36 | 0.44 | 41.45 | 78 | 1.38 | 153.41 | 118 | 0.60 | 1216.56 |
| 37 | 0.47 | 46.51 | 79 | 1.24 | 25.61 | 119 | 1.83 | 9497.80 |
| 38 | 0.30 | 59.88 | 80 | 2.26 | 32.00 | 120 | 2.34 | 1950.74 |
| 39 | 0.19 | 119.95 | 83 | 1.08 | 18.32 | 121 | 0.70 | 168.25 |
| 40 | 1.54 | 38.70 | 84 | 0.77 | 10.1 | 123 | 0.85 | 22.28 |
| 41 | 1.02 | 19.88 | 85 | 1.52 | 78.02 | 124 | 14.19 | 1451.54 |
| 42 | 1.50 | 95.03 | 86 | 0.66 | 13.26 | 125 | 1.05 | 3.70 |
| 43 | 1.43 | 1167.04 | 87 | 1.22 | 84.05 | 126 | 0.79 | 1.03 |
| 44 | 1.76 | 317.60 | 88 | 1.72 | 80.32 | 127 | 0.85 | 82.97 |
| 45 | 2.26 | 593.42 | 89 | 1.72 | 860.54 | 128 | 0.85 | 251.68 |
| 46 | 0.72 | 80.26 | 90 | 1.57 | 517.05 | 129 | 1.48 | 368.66 |
| 47 | 0.69 | 19.26 | 91 | 1.39 | 626.63 | 130 | 0.79 | 1.03 |
| 48 | 0.43 | 118.40 | 92 | 1.38 | 237.59 | 131 | 0.78 | 110.68 |
| 53 | 2.34 | 1951 | 93 | 1.53 | 487.92 | 132 | 0.72 | 64.64 |
| 54 | 1.32 | 12.67 | 94 | 2.69 | >10000 | 133 | 1.50 | 221.94 |
| 55 | 2.24 | 48.92 | 95 | 1.82 | 886.24 | 134 | 1.73 | 129.46 |
| 56 | 2.32 | 37.91 | 96 | 3.27 | 3299.69 | 135 | 1.16 | 1991.65 |
| 57 | 1.77 | 67.86 | 98 | 1.91 | 40.97 | | | |
| 58 | 2.72 | 247.81 | 99 | 1.09 | 15.14 | | | |
| 59 | 1.39 | 21.90 | 100 | 0.95 | 65.65 | | | |
| 60 | 0.74 | 19.78 | 101 | 4.43 | 35.56 | | | |

Relative to PARP2 enzyme, most of the compounds tested have potent and selective inhibitory effect on PARP1 enzyme.

### Example 152

### Growth inhibition assays against BRCA mutant human breast cancer MDA-MB-436 cell line

The cells were cultured in complete medium (DMEM medium +10% FBS+ Insulin + glutathione). When the confluence reached about 80%, cells were digested and gently dispensed from the bottom of the dish with a 1 mL pipette. Cell suspension was collected and centrifuged at 500rpm for 3min. The supernatant was discarded, and the cell pellet were re-suspended in complete medium. The cells were seeded into a culture dish at an appropriate proportion, and then cultured in a 5% CO₂ incubator at 37°C. The assay was carried out when the cells were in optimum condition and the confluence was reached 80%. Cells in the logarithmic growth phase were taken to centrifugate, and the culture supernatant was removed. The cells were resuspended in refresh complete medium and counted. The resuspended cells were seeded at 3000/well in a 96-well plate and incubated at 37°C, 5% CO₂ incubator overnight. The compound was prepared as below: 1000× dilution tested compound solution to 40× test compound solution by adding 5µL 1000× compound solution to 120 µL Medium (25 fold dilution). The solution was mixed by oscillation. 0.1% DMSO was used as the control.

The next day, the 96-well plate inoculated with cells was taken out from the incubator, and the culture supernatant was removed. Then fresh medium of 195 uL/well and 5µL/ well of 40× test compound solution as mentioned above were added into the 96 well plate, respectively. Finally, the plate was incubated for 7 days in a 37°C 5% CO₂ incubator. The medium containing compound was changed on the fourth day. After 7 days, 20µL of CCK-8 was added to each well and shaken gently, then was cultured for 4 hours. The plate was shaken for 5min after incubation. the absorbance values of 450nm or 650nm wavelengths were recorded respectively (OD = absorbance value of 450nm - absorbance value of 650nm) by using the multifunction readout instrument.

Data were analyzed by software GraphPad Prism 6.0. The inhibitory activity of compounds on cell proliferation was plotted using cell survival rate against the compound concentration as coordinates. Cell survival rate %= (OD_{compound} - OD_{background})/(OD_{DMSO}-OD_{background}) ×100. The IC₅₀ value was fitted by the s-shaped dose response curve equation: Y=100 / (1+10^(logC-logIC₅₀)), and C was the compound concentration.

Table 2 summarizes the inhibitory effect data (IC₅₀) of the compounds on the proliferation of human breast cancer cells MDA-MB-436.

**Table 2**

| | | | | | | |
|---|---|---|---|---|---|---|
| Example | **1** | **2** | **3** | **4** | **6** | **7** |
| IC₅₀ (nM) | 170.22 | 2.65 | 5.01 | 4.22 | 17.7 | 4.36 |
| Example | **16** | **17** | **24** | **25** | **26** | **28** |
| IC₅₀ (nM) | 1.71 | 2.59 | 0.78 | 44.87 | 23.93 | 0.98 |
| Example | **29** | **31** | **34** | **35** | **36** | **37** |
| IC₅₀ (nM) | 28.18 | 2.08 | 0.75 | 0.39 | 1.11 | 1.07 |
| Example | **38** | **39** | **40** | **41** | **42** | **43** |
| IC₅₀ (nM) | 4.06 | 3.56 | 1.15 | 1.41 | 0.93 | 1.20 |
| Example | **44** | **45** | **46** | **47** | **48** | **49** |
| IC₅₀ (nM) | 2.17 | 2.24 | 2.27 | 2.60 | 0.65 | 2.18 |
| Example | **50** | **54** | **55** | **56** | **57** | **58** |
| IC₅₀ (nM) | 5.70 | 1.85 | 1.44 | 0.65 | 1.43 | 1.87 |
| Example | **59** | **60** | **61** | **62** | **63** | **65** |
| IC₅₀ (nM) | 0.99 | 0.63 | 0.98 | 3.42 | 3.32 | 1.12 |
| Example | **66** | **67** | **68** | **69** | **70** | **71** |
| IC₅₀ (nM) | 1.66 | 2.11 | 2.92 | 1.08 | 1.41 | 0.77 |
| Example | **72** | **73** | **74** | **75** | **76** | **77** |
| IC₅₀ (nM) | 2.09 | 1.77 | 1.58 | 3.31 | 1.37 | 2.39 |
| Example | **78** | **79** | **80** | **81** | **82** | **83** |
| IC₅₀ (nM) | 1.00 | 1.04 | 2.37 | 4.67 | 3.66 | 0.74 |
| Example | **84** | **85** | **86** | **87** | **88** | **89** |
| IC₅₀ (nM) | 0.74 | 2.46 | 0.87 | 0.94 | 0.38 | 2.22 |
| Example | **90** | **91** | **92** | **93** | **94** | **95** |
| IC₅₀ (nM) | 2.44 | 2.81 | 5.05 | 5.68 | 5.05 | 6.78 |
| Example | **96** | **97** | **98** | **99** | **100** | **101** |
| IC₅₀ (nM) | 1.93 | 110.66 | 0.62 | 0.54 | 0.68 | 2.48 |
| Example | **102** | **103** | **104** | **105** | **114** | **115** |
| IC₅₀ (nM) | 2.24 | 1.17 | 1.15 | 0.77 | 3.72 | 1.98 |
| Example | **116** | **117** | **118** | **119** | | |
| IC₅₀ (nM) | >100 | 4.74 | 10.67 | 16.18 | | |

The compounds tested have good inhibitory effect on the proliferation of BRCA mutated human breast cancer cells MDA-MB-436.

Having now fully described this disclosure, it will be understood by those of ordinary skill in the art that the same can be performed within a wide and equivalent range of conditions, formulations and other parameters without affecting the scope of the disclosure or any embodiment thereof. All patents, patent applications and publications cited herein are fully incorporated by reference herein in their entirety.

The disclosure also comprises the following clauses, which may be claimed:
1. A compound of Formula I: 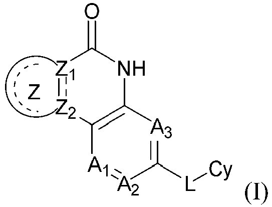 or stereoisomers, tautomers, N-oxides, hydrates, solvates, isotope-substituted derivatives, or pharmaceutically acceptable salts thereof, or prodrugs thereof, or mixtures thereof, wherein:
   A₁, A₂ and A₃ each are independently selected from N and CR₁;
   The Z ring shown as 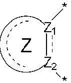 is an optionally substituted 5-7 membered carbocyclic group, an optionally substituted 5-7 membered heterocyclic group or an optionally substituted 5 membered heteroaryl group, * indicates the position at which the Z ring is attached to the rest of the compound, the dashed lines indicate the optional presence of unsaturated bonds, wherein Z₁ and Z₂ are each independently C or N, wherein Z₁ and Z₂ are not N at the same time; in addition, Z₁ and Z₂ satisfy the following conditions: (1) when the Z ring is an optionally substituted 5-7 membered carbocyclic group or an optionally substituted 5-7 membered heterocyclic group, both of Z₁ and Z₂ are C, and among the bonds of the Z ring, only the bond between Z₁ and Z₂ is a double bond; (2) when the Z ring is an optionally substituted 5-7 membered heterocyclic group and Z₂ is C, the ring atom connected to Z₂ of the Z ring is not N; (3) when the Z ring is an optionally substituted 5 membered heteroaryl group and Z₁ is N, in addition to the N at the Z₁ position, the 5 membered heteroaryl further contains 1-3 N heteroatoms; or when Z ring is an optionally substituted 5 membered heteroaryl, Z₁ is N, and there are no other heteroatoms in the Z ring, A₁ is CR₁;
   L is selected from a bond and alkylene optionally substituted by R₂ and/or R₃;
   Cy is selected from a group consisting of an optionally substituted heterocyclic group, an optionally substituted aryl, and an optionally substituted heteroaryl;
   R₁ is selected from a group consisting of hydrogen, halogen, an optionally substituted alkyl, an optionally substituted alkoxy and an optionally substituted carbocyclic group;
   R₂ and R₃ are each independently selected from a group consisting of halogen, cyano, an optionally substituted alkyl, an optionally substituted alkoxy, an optionally substituted cycloalkyl, an optionally substituted alkenyl, and an optionally substituted alkynyl; or R₂ and R₃ together with the attached C form a ring.
2. The compound of clause 1, or stereoisomers, tautomers, N-oxides, hydrates, solvates, isotope-substituted derivatives, or pharmaceutically acceptable salts thereof, or prodrugs thereof, or any mixture thereof, wherein the Z ring is optionally substituted by 1-3 groups selected from a group consisting of hydroxy, halogen, C₁₋₄ alkyl, C₁₋₄ alkoxy, halogenated C₁₋₄ alkyl, halogenated C₁₋₄ alkoxy, C₁₋₄ alkyl substituted with hydroxy, C₁₋₄ alkoxy substituted with hydroxy and amino (-NR'R"), wherein R' and R" each are independently H or C₁₋₄ alkyl; preferably, the Z ring is optionally substituted by 1-3 groups selected from a group consisting of halogen or C₁₋₄ alkyl;
   preferably, the Z ring is selected from the following groups: 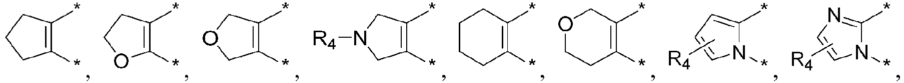 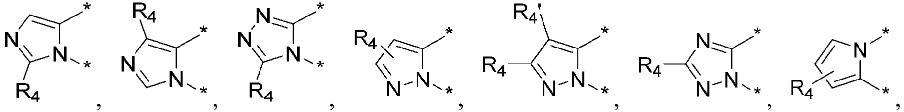 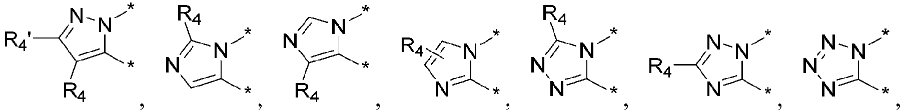 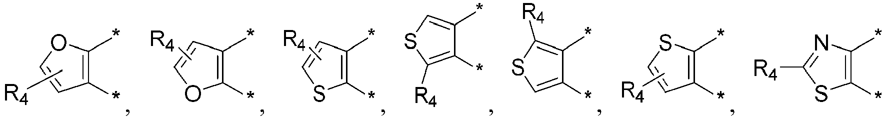 and 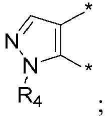
   preferably, the Z ring is selected from the following groups: 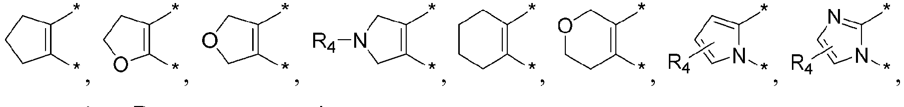 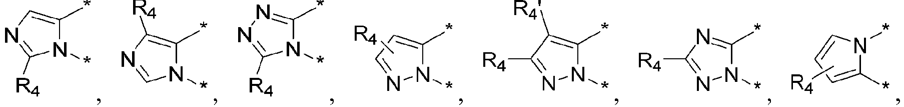 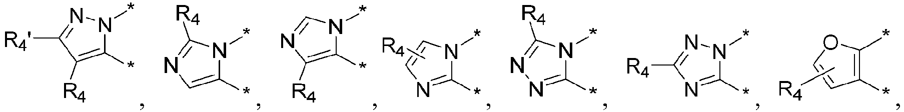 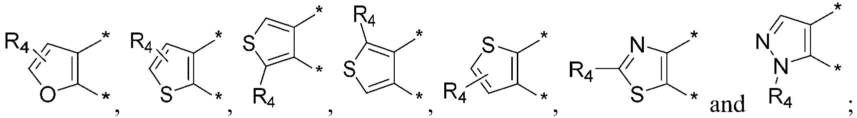
   more preferably, the Z ring is selected from the following groups: 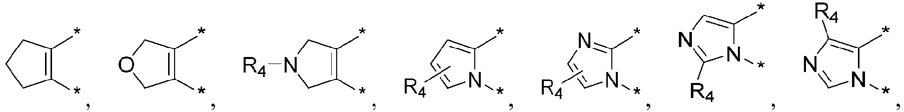 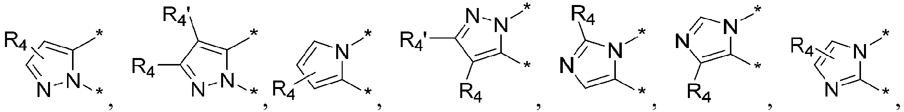 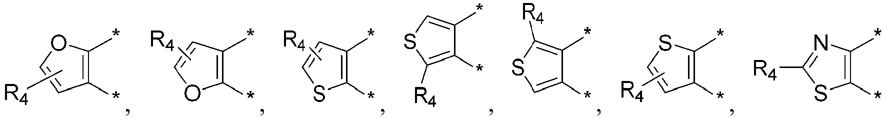 and 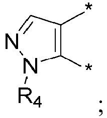
   wherein * indicates the position at which the Z ring is attached to the rest of the compound; each R₄ is independently selected from a group consisting of hydrogen, halogen and an optionally substituted alkyl, preferably hydrogen, halogen and an optionally substituted C₁₋₃ alkyl; each R₄' is independently selected from a group consisting of hydrogen, halogen and an optionally substituted alkyl, preferably hydrogen, halogen and an optionally substituted C₁₋₃ alkyl.
3. The compound of clause 1, or stereoisomers, tautomers, N-oxides, hydrates, solvates, isotope-substituted derivatives, or pharmaceutically acceptable salts thereof, or prodrugs thereof, or any mixture thereof, wherein the Z ring is selected from the following groups: 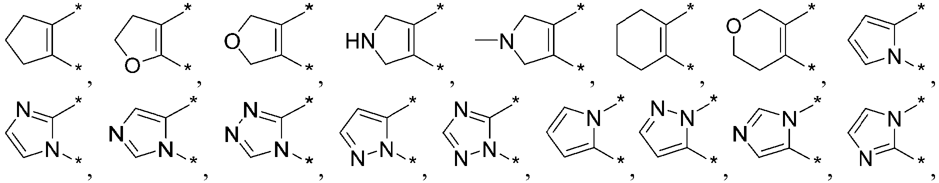 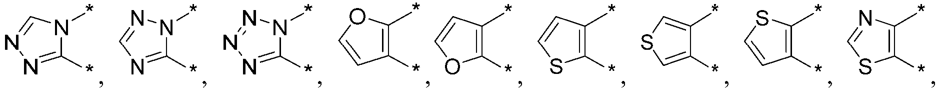 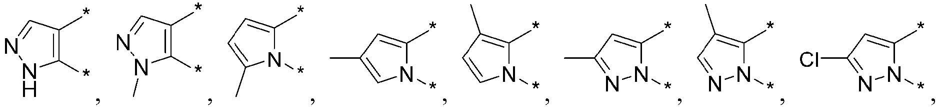 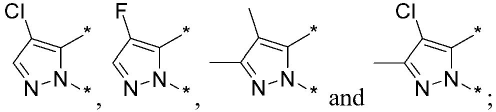 preferably, the Z ring is selected from the following groups: 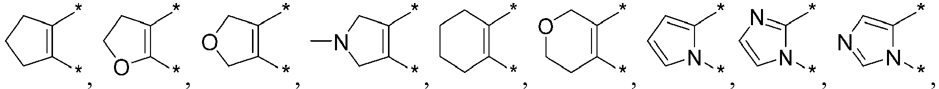 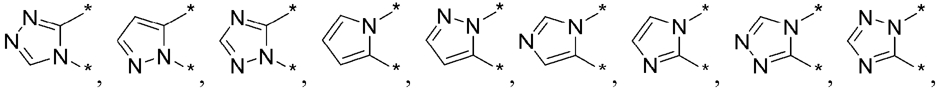 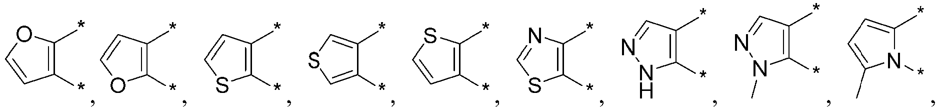 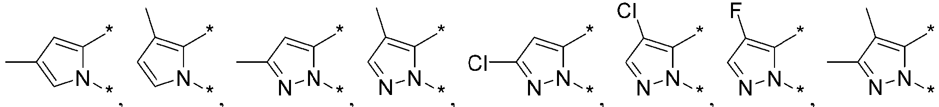 and 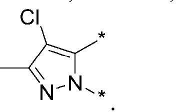
4. The compound of any one of clauses 1-3, or stereoisomers, tautomers, N-oxides, hydrates, solvates, isotope-substituted derivatives, or pharmaceutically acceptable salts thereof, or prodrugs thereof, or any mixture thereof, wherein:
   only one of A₁, A₂ and A₃ is N, and the other two are independently CR₁; or A₃ is CH, one of A₁ and A₂ is N and the other is CR₁; A₁ is N, both of A₂ and A₃ are CH; or A₂ is N, both A₁ and A₃ are CH; or all of A₁, A₂ and A₃ are CR₁; and/or
   each R₁ is independently hydrogen, halogen, or C₁₋₃ alkyl or C₁₋₃ alkoxy optionally substituted by 1-5 groups selected from a group consisting of halogen, hydroxy and -NR'R", wherein R' and R" each are independently H, C₁₋₄ alkyl or C₃₋₆ cycloalkyl; preferably, R₁ is hydrogen, halogen, C₁₋₃ alkyl or halogenated C₁₋₃ alkyl; and/or
   L is an unsubstituted C₁₋₃ alkylene, preferably a methylene, optionally substituted by R₂ and/R₃, wherein R₂ and R₃ are each independently halogen or C₁₋₃ alkyl or R₂ and R₃ together with the attached C form a 3-6 membered cycloalkyl.
5. The compound of any one of clauses 1-4, or stereoisomers, tautomers, N-oxides, hydrates, solvates, isotope-substituted derivatives, or pharmaceutically acceptable salts thereof, or prodrugs thereof, or any mixture thereof, wherein Cy is a 5-7 membered nitrogen-containing heterocyclic group optionally substituted by 1-3 groups selected from a group consisting of: halogen; C₁₋₄ alkyl; C₁₋₄ alkoxy; halogenated C₁₋₄ alkyl; halogenated C₁₋₄ alkoxy; and 6-14 membered aryl, 5-10 membered heteroaryl, 4-10 membered heterocyclic group and C₃₋₈ cycloalkyl, which are each independently optionally substituted by 1-5 substituents selected from a group consisting of halogen, an optionally substituted alkyl, an optionally substituted alkoxy, an optionally substituted carbocyclic group, an optionally substituted alkenyl, an optionally substituted alkynyl, -NR'R", -C(O)-NR'R" and carboxyl, wherein the said R' and R" each are independently H, an optionally substituted C₁₋₁₀ alkyl, an optionally substituted C₃₋₈ cycloalkyl, an optionally substituted aryl or an optionally substituted heteroaryl;
   preferably, the 6-14 membered aryl, 5-10 membered heteroaryl, 4-10 membered heterocyclic group and C₃₋₈ cycloalkyl are each at least substituted by -C(O)-NR'R", and optionally further substituted by one or two substituents selected from a group consisting of halogen, C₁₋₄ alkyl and halogenated C₁₋₄ alkyl;
   more preferably, Cy is piperazinyl substituted by a 5-10 membered heteroaryl, preferably a 5-10 membered nitrogen-containing heteroaryl, more preferably pyridyl, which is at least substituted by -C(O)-NR'R" and optionally further substituted by one or two substituents selected from a group consisting of halogen, C₁₋₄ alkyl and halogenated C₁₋₄ alkyl.
6. The compound of any one of clauses 1, or stereoisomers, tautomers, N-oxides, hydrates, solvates, isotope-substituted derivatives, or pharmaceutically acceptable salts thereof, or prodrugs thereof, or any mixture thereof, wherein A₁, A₂ and A₃ are each independently CR₁; each R₁ is independently H, C₁₋₃ alkyl or halogen; L is -CH₂- or -CH₂CH₂-; Cy is piperazinyl substituted with pyridyl, and the said pyridyl is substituted with -C(O)-NR'R" and optionally further substituted by one or two substituents selected from a group consisting of halogen, C₁₋₄ alkyl and halogenated C₁₋₄ alkyl; R' and R" each are independently H, C₁₋₄ alkyl optionally substituted by hydroxy, or C₃₋₈ cycloalkyl.
7. The compound of clause 1, or stereoisomers, tautomers, N-oxides, hydrates, solvates, isotope-substituted derivatives, or pharmaceutically acceptable salts thereof, or prodrugs thereof, or mixtures thereof, wherein the compound of Formula I is represented by Formulae IIa and IIb as shown below: 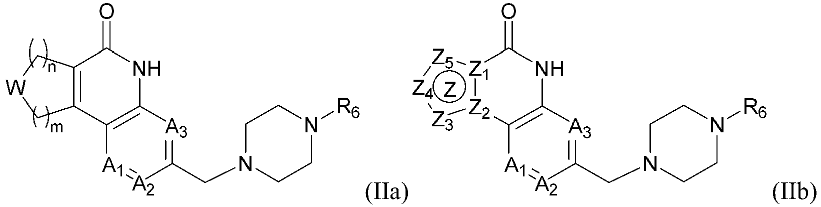 wherein:
   W is selected form CR₄R₅, O, S and NR₄; preferably, W is O, CH₂ or N-CH₃;
   Z₁ and Z₂ each are independently C or N, with the proviso that Z₁ and Z₂ are not N at the same time; Z₃, Z₄ and Z₅ are independently selected from a group consisting of CR₄, O, S, N, and NR₄; and when Z₁ is N, at least one of Z₃, Z₄ and Z₅ is N; or when Z₁ is N and all of Z₃, Z₄ and Z₅ are CR₄, A₁ is CR₁; preferably, the Z ring is selected from the following groups: 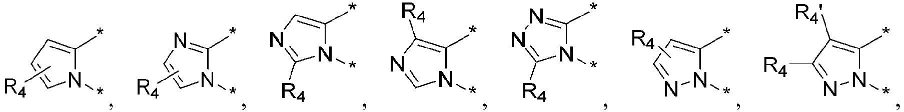 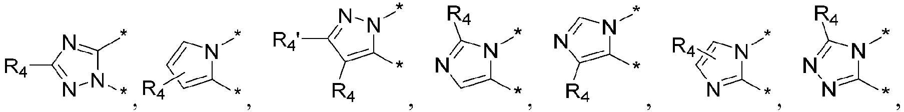 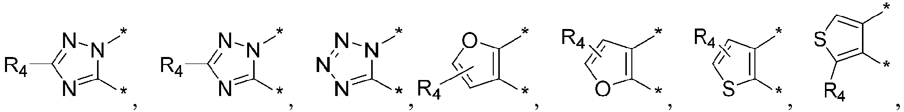 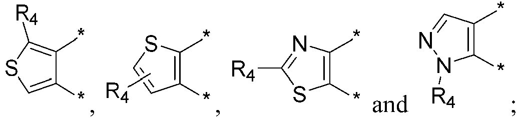 more preferably, the Z ring is the 5 membered heteroaryl group as defined in clause 3;
   A₁, A₂, A₃ and R₁ are as defined in any one of clauses 1-6;
   R₄, R₄' and R₅ each are independently selected from hydrogen, halogen and an optionally substituted alkyl;
   R₆ is selected from an optionally substituted aryl and an optionally substituted heteroaryl; preferably, R₆ is: 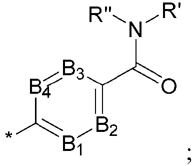 wherein, B₁, B₂, B₃ and B₄ are independently selected from a group consisting of N and CR₇; R₇ is selected from a group consisting of hydrogen, halogen, an optionally substituted alkyl, an optionally substituted alkoxy, an optionally substituted carbocyclic group, an optionally substituted alkenyl and an optionally substituted alkynyl; wherein R' and R" each are independently hydrogen, an optionally substituted C₁₋₁₀ alkyl, an optionally substituted C₃₋₈ cycloalkyl, an optionally substituted aryl or an optionally substituted heteroaryl; * indicates the position at which the said group is attached to the rest of the compound;
   n and m each are independently selected from a group consisting of 0, 1, 2 and 3; and 2≤n+m≤4; preferably, n is 1 or 2; preferably, m is 1 or 2;
   when m is 0 and n is 3, W is not NH;
   preferably, W is O, CH₂ or N-CH₃; A₁, A₂ and A₃ each are independently selected from N and CR₁, wherein each R₁ is independently hydrogen, C₁₋₃ alkyl or halogen; R₆ is phenyl, pyridyl, pyrimidinyl or pyridazinyl substituted at the para position with an optionally substituted aminoacyl group; n is 1; m is 1 or 2.
8. The compound of clause 1, or stereoisomers, tautomers, N-oxides, hydrates, solvates, isotope-substituted derivatives, or pharmaceutically acceptable salts thereof, or prodrugs thereof, or mixtures thereof, wherein the compound of Formula I is represented by Formulae IIIa and IIIb as shown below: 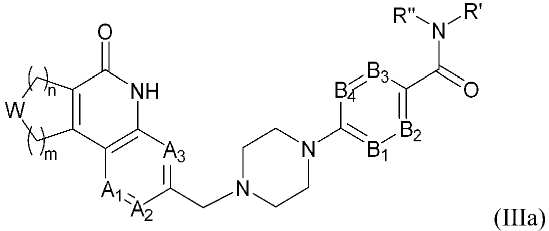 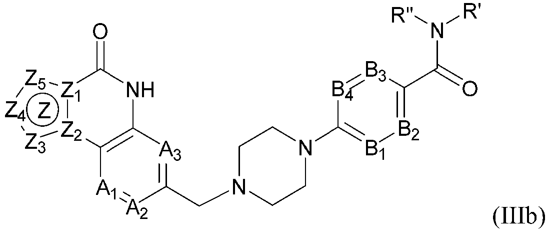 wherein:
   W, Z₁, Z₂, Z₃, Z₄, Z₅, A₁, A₂, A₃, B₁, B₂, B₃, B₄, n and m are as described in clause 7;
   R' and R" each are independently H, an optionally substituted C₁₋₁₀ alkyl, an optionally substituted cycloalkyl, an optionally substituted aryl or an optionally substituted heteroaryl; or
   B₃ and R" together with the attached aminoacyl group form a 6-membered heterocyclic group;
   preferably, A₁, A₂ and A₃ each are independently selected from N and CR₁; and when the Z ring is 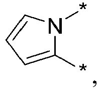 A₁ is CR₁, A₂ and A₃ each are independently N or CR₁; wherein R₁ is hydrogen, C₁₋₃ alkyl or halogen;
   preferably, B₁, B₂, B₃ and B₄ are independently selected from a group consisting of N and CR₇; wherein R₇ is hydrogen, halogen, C₁₋₃ alkyl, C₁₋₃ alkoxy or halogenated C₁₋₃ alkyl;
   preferably, R' and R" each are independently hydrogen, an optionally substituted C₁₋₃ alkyl, an optionally substituted C₃₋₆ cycloalkyl;
   preferably, B₃ and R" together with the attached amido group form a 6-membered heterocyclic group.
9. The compound of clause 1, or stereoisomers, tautomers, N-oxides, hydrates, solvates, isotope-substituted derivatives, or pharmaceutically acceptable salts thereof, or prodrugs thereof, or mixtures thereof, wherein the compound of Formula I is represented by Formulae IVa, IVb and IVc as shown below: 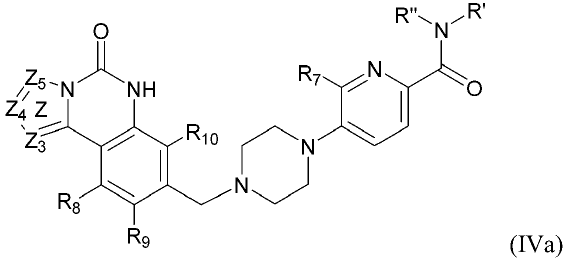 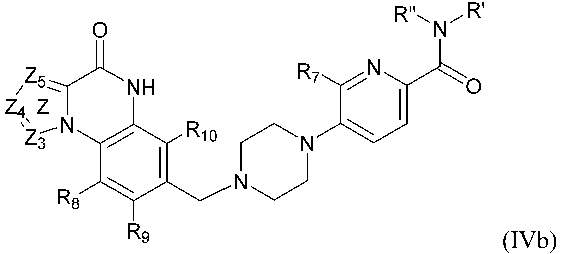 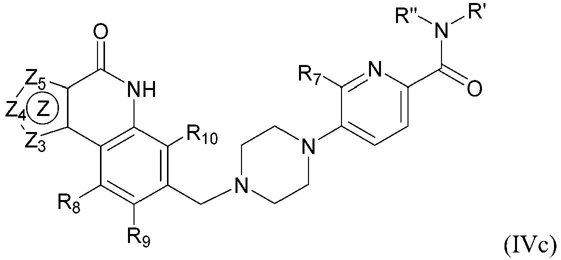 wherein:
   Z₃, Z₄, and Z₅ are as described in clause 8; preferably, the 5-membered ring containing Z₃, Z₄ and Z₅ is selected from the following groups: 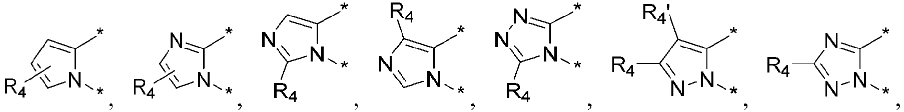 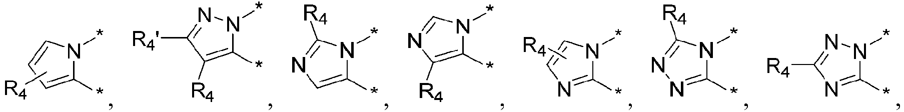 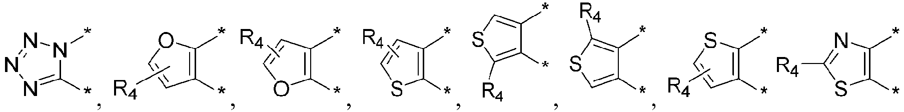 and 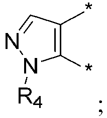
   R₇, R' and R" are as described in clause 7 or 8;
   R₈, R₉ and R₁₀ each are independently selected from a group consisting of hydrogen, halogen, an optionally substituted alkyl, and an optionally substituted alkoxy.
10. The compound of clause 1, or stereoisomers, tautomers, N-oxides, hydrates, solvates, isotope-substituted derivatives, or pharmaceutically acceptable salts thereof, or prodrugs thereof, or mixtures thereof, wherein:
   the 5-membered ring containing Z₃, Z₄ and Z₅ is selected from the following groups: 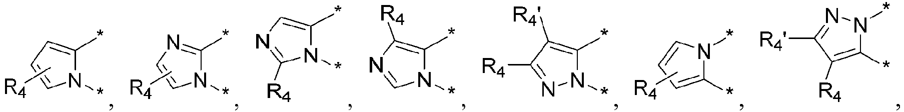 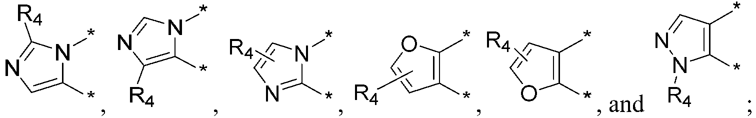 wherein each R₄ is independently selected from a group consisting of hydrogen and C₁₋₃ alkyl; each R₄' is independently selected from a group consisting of hydrogen and C₁₋₃ alkyl; preferably, the 5-membered ring containing Z₃, Z₄ and Z₅ is selected from the following groups: 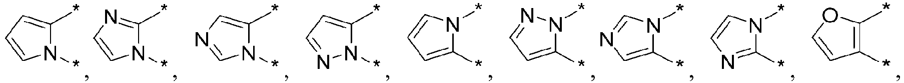 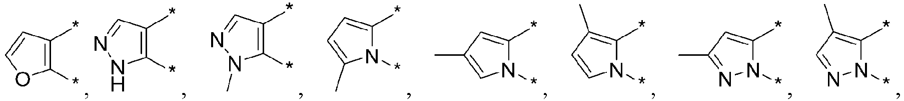 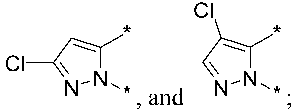
   R₇ is H, C₁₋₃ alkyl or halogen;
   R₈, R₉ and R₁₀ each are hydrogen, halogen or C₁₋₃ alkyl; preferably, R₈ is C₁₋₃ alkyl or halogen and both of R₉ and R₁₀ are H; or R₉ is C₁₋₃ alkyl or halogen and both of R₈ and R₁₀ are H; or both of R₈ and R₉ are H and R₁₀ is C₁₋₃ alkyl or halogen; or all of R₈, R₉ and R₁₀ are H; and
   R' and R' are each independently H, C₁₋₃ alkyl or C₃₋₆ cycloalkyl.
11. The compound of clause 1, wherein the compound is selected from a group consisting of a group consisting of:
   7-((4-(6-(methylcarbamoyl)pyridin-3-yl)piperazin-1-yl)methyl)-3,5-dihydrofuro[3,4-c]quinolin-4(1H)-one;
   7-((4-(2-fluoro-6-(methylcarbamoyl)pyridin-3-yl)piperazin-1-yl)methyl)-3,5-dihydrofuro[3,4-c]quinolin-4(1H)-one;
   7-((4-(2-chloro-6-(methylcarbamoyl)pyridin-3-yl)piperazin-1-yl)methyl)-3,5-dihydrofuro[3,4-c]quinolin-4(1H)-one;
   7-((4-(2-methyl-6-(methylcarbamoyl)pyridin-3-yl)piperazin-1-yl)methyl)-3,5-dihydrofuro[3,4-c]quinolin-4(1H)-one;
   7-((4-(2-trifluoromethyl-6-(methylcarbamoyl)pyridin-3-yl)piperazin-1-yl)methyl)-3,5-dihydrofuro[3,4-c]quinolin-4(1H)-one;
   7-((4-(2-chloro-6-(ethylcarbamoyl)pyridin-3-yl)piperazin-1-yl)methyl)-3,5-dihydrofuro[3,4-c]quinolin-4(1H)-one;
   7-((4-(2-methyl-6-(ethylcarbamoyl)pyridin-3-yl)piperazin-1-yl)methyl)-3,5-dihydrofuro[3,4-c]quinolin-4(1H)-one;
   7-((4-(2-trifluoromethyl-6-(ethylcarbamoyl)pyridin-3-yl)piperazin-1-yl)methyl)-3,5-dihydrofuro[3,4-c]quinolin-4(1H)-one;
   7-((4-(2-chloro-6-(methylcarbamoyl)pyridin-3-yl)piperazin-1-yl)methyl)-9-fluoro-3,5-dihydrofuro[3,4-c]quinolin-4(1H)-one;
   7-((4-(2-methyl-6-(methylcarbamoyl)pyridin-3-yl)piperazin-1-yl)methyl)-9-fluoro-3,5-dihydrofuro[3,4-c]quinolin-4(1H)-one;
   7-((4-(2-trifluoromethyl-6-(methylcarbamoyl)pyridin-3-yl)piperazin-1-yl)methyl)-9-fluoro-3,5-dihydrofuro[3,4-c]quinolin-4(1H)-one;
   7-((4-(2-chloro-6-(ethylcarbamoyl)pyridin-3-yl)piperazin-1-yl)methyl)-9-fluoro-3,5-dihydrofuro[3,4-c]quinolin-4(1H)-one;
   7-((4-(2-methyl-6-(ethylcarbamoyl)pyridin-3-yl)piperazin-1-yl)methyl)-9-fluoro-3,5-dihydrofuro[3,4-c]quinolin-4(1H)-one;
   7-((4-(2-trifluoromethyl-6-(ethylcarbamoyl)pyridin-3-yl)piperazin-1-yl)methyl)-9-fluoro-3,5-dihydrofuro[3,4-c]quinolin-4(1H)-one;
   3-((4-(2-methyl-6-(methylcarbamoyl)pyridin-3-yl)piperazin-1-yl)methyl)-7,8,9,10-tetrahydrophenanthridin-6(5H)-one;
   7-((4-(2-chloro-6-(methylcarbamoyl)pyridin-3-yl)piperazin-1-yl)methyl)-1,2,3,5-tetrahydro-4H-cyclopenta[c]quinolin-4-one;
   7-((4-(2-methyl-6-(methylcarbamoyl)pyridin-3-yl)piperazin-1-yl)methyl)-1,2,3,5-tetrahydro-4H-cyclopenta[c]quinolin-4-one;
   7-((4-(2-chloro-6-(methylcarbamoyl)pyridin-3-yl)piperazin-1-yl)methyl)-3,5-dihydrofuro[3,2-c]quinolin-4(2H)-one;
   7-((4-(2-methyl-6-(methylcarbamoyl)pyridin-3-yl)piperazin-1-yl)methyl)-3,5-dihydrofuro[3,2-c]quinolin-4(2H)-one;
   3-((4-(2-methyl-6-(methylcarbamoyl)pyridin-3-yl)piperazin-1-yl)methyl)-7,9-dihydrofuro[3,4-c][1,5]naphthyridin-6(5H)-one;
   7-((4-(2-chloro-6-(methylcarbamoyl)pyridin-3-yl)piperazin-1-yl)methyl)-2-methyl-1,2,3,5-tetrahydro-4H-pyrrolo[3,4-c]quinolin-4-one;
   8-((4-(2-chloro-6-(methylcarbamoyl)pyridin-3-yl)piperazin-1-yl)methyl)-1,2,4,6-tetrahydro-5H-pyrano[3,4-c]quinolin-5-one;
   8-((4-(2-methyl-6-(methylcarbamoyl)pyridin-3-yl)piperazin-1-yl)methyl)-1,2,4,6-tetrahydro-5H-pyrano[3,4-c]quinolin-5-one;
   7-((4-(2-methyl-6-(methylcarbamoyl)pyridin-3-yl)piperazin-1-yl)methyl)pyrrolo[1,2-a]quinoxalin-4(5H)-one;
   7-((4-(2-methyl-6-(methylcarbamoyl)pyridin-3-yl)piperazin-1-yl)methyl)imidazo[1,2-a]quinoxalin-4(5H)-one;
   7-((4-(2-methyl-6-(methylcarbamoyl)pyridin-3-yl)piperazin-1-yl)methyl)imidazo[1,5-a]quinoxalin-4(5H)-one;
   7-((4-(2-methyl-6-(methylcarbamoyl)pyridin-3-yl)piperazin-1-yl)methyl)-[1,2,4]triazolo[4,3-a]quinoxalin-4(5H)-one;
   7-((4-(2-methyl-6-(methylcarbamoyl)pyridin-3-yl)piperazin-1-yl)methyl)pyrazolo[1,5-a]quinoxalin-4(5H)-one;
   7-((4-(2-methyl-6-(methylcarbamoyl)pyridin-3-yl)piperazin-1-yl)methyl)-[1,2,4]triazolo[1,5-a]quinoxalin-4(5H)-one;
   8-((4-(2-methyl-6-(methylcarbamoyl)pyridin-3-yl)piperazin-1-yl)methyl)imidazo[1,5-c]quinazolin-5(6H)-one;
   8-((4-(2-methyl-6-(methylcarbamoyl)pyridin-3-yl)piperazin-1-yl)methyl)imidazo[1,2-c]quinazolin-5(6H)-one;
   8-((4-(2-methyl-6-(methylcarbamoyl)pyridin-3-yl)piperazin-1-yl)methyl)-[1,2,4]triazolo[4,3-c]quinazolin-5(6H)-one;
   8-((4-(2-methyl-6-(methylcarbamoyl)pyridin-3-yl)piperazin-1-yl)methyl)-[1,2,4]triazolo[1,5-c]quinazolin-5(6H)-one;
   7-((4-(2-fluoro-6-(methylcarbamoyl)pyridin-3-yl)piperazin-1-yl)methyl)pyrrolo[1,2-a]quinoxalin-4(5H)-one;
   7-((4-(2-chloro-6-(methylcarbamoyl)pyridin-3-yl)piperazin-1-yl)methyl)pyrrolo[1,2-a]quinoxalin-4(5H)-one;
   7-((4-(2-fluoro-6-(methylcarbamoyl)pyridin-3-yl)piperazin-1-yl)methyl)pyrazolo[1,5-a]quinoxalin-4(5H)-one;
   7-((4-(2-chloro-6-(methylcarbamoyl)pyridin-3-yl)piperazin-1-yl)methyl)pyrazolo[1,5-a]quinoxalin-4(5H)-one;
   7-((4-(2-fluoro-6-(methylcarbamoyl)pyridin-3-yl)piperazin-1-yl)methyl)imidazo[1,5-a]quinoxalin-4(5H)-one;
   7-((4-(2-chloro-6-(methylcarbamoyl)pyridin-3-yl)piperazin-1-yl)methyl)imidazo[1,5-a]quinoxalin-4(5H)-one;
   7-((4-(2-methyl-6-(methylcarbamoyl)pyridin-3-yl)piperazin-1-yl)methyl)-9-fluoropyrrolo[1,2-a]quinoxalin-4(5H)-one;
   7-((4-(2-fluoro-6-(methylcarbamoyl)pyridin-3-yl)piperazin-1-yl)methyl)-9-fluoropyrrolo[1,2-a]quinoxalin-4(5H)-one;
   7-((4-(2-chloro-6-(methylcarbamoyl)pyridin-3-yl)piperazin-1-yl)methyl)-9-fluoropyrrolo[1,2-a]quinoxalin-4(5H)-one;
   7-((4-(2-methyl-6-(methylcarbamoyl)pyridin-3-yl)piperazin-1-yl)methyl)-9-fluoropyrazolo[1,5-a]quinoxalin-4(5H)-one;
   7-((4-(2-fluoro-6-(methylcarbamoyl)pyridin-3-yl)piperazin-1-yl)methyl)-9-fluoropyrazolo[1,5-a]quinoxalin-4(5H)-one;
   7-((4-(2-chloro-6-(methylcarbamoyl)pyridin-3-yl)piperazin-1-yl)methyl)-9-fluoropyrazolo[1,5-a]quinoxalin-4(5H)-one;
   7-((4-(2-methyl-6-(methylcarbamoyl)pyridin-3-yl)piperazin-1-yl)methyl)-9-chloropyrazolo[1,5-a]quinoxalin-4(5H)-one;
   7-((4-(2-fluoro-6-(methylcarbamoyl)pyridin-3-yl)piperazin-1-yl)methyl)-9-chloropyrazolo[1,5-a]quinoxalin-4(5H)-one;
   8-((4-(2-methyl-6-(methylcarbamoyl)pyridin-3-yl)piperazin-1-yl)methyl)pyrrolo[1,2-c]quinazolin-5(6H)-one;
   7-((4-(2-methyl-6-(ethylcarbamoyl)pyridin-3-yl)piperazin-1-yl)methyl)-1,2,3,5-tetrahydro-4H-cyclopenta[c]quinolin-4-one;
   7-((4-(2-chloro-6-(ethylcarbamoyl)pyridin-3-yl)piperazin-1-yl)methyl)-1,2,3,5-tetrahydro-4H-cyclopenta[c]quinolin-4-one;
   8-((4-(2-fluoro-6-(methylcarbamoyl)pyridin-3-yl)piperazin-1-yl)methyl)imidazo[1,2-c]quinazolin-5(6H)-one;
   8-((4-(2-chloro-6-(methylcarbamoyl)pyridin-3-yl)piperazin-1-yl)methyl)imidazo[1,2-c]quinazolin-5(6H)-one;
   8-((4-(2-fluoro-6-(methylcarbamoyl)pyridin-3-yl)piperazin-1-yl)methyl)-10-fluoroimidazo[1,2-c]quinazolin-5(6H)-one;
   7-((4-(2-methyl-6-(methylcarbamoyl)pyridin-3-yl)piperazin-1-yl)methyl)-1-methylpyrrolo[1,2-a]quinoxalin-4(5H)-one;
   7-((4-(2-methyl-6-(methylcarbamoyl)pyridin-3-yl)piperazin-1-yl)methyl)-2-methylpyrrolo[1,2-a]quinoxalin-4(5H)-one;
   7-((4-(2-methyl-6-(methylcarbamoyl)pyridin-3-yl)piperazin-1-yl)methyl)-3-methylpyrrolo[1,2-a]quinoxalin-4(5H)-one;
   7-((4-(2-methyl-6-(methylcarbamoyl)pyridin-3-yl)piperazin-1-yl)methyl)-2-methylpyrazolo[1,5-a]quinoxalin-4(5H)-one;
   7-((4-(2-methyl-6-(methylcarbamoyl)pyridin-3-yl)piperazin-1-yl)methyl)-3-methylpyrazolo[1,5-a]quinoxalin-4(5H)-one;
   7-((4-(2-methyl-6-(cyclopropylcarbamoyl)pyridin-3-yl)piperazin-1-yl)methyl)pyrrolo[1,2-a]quinoxalin-4(5H)-one;
   7-((4-(2-fluoro-6-(cyclopropylcarbamoyl)pyridin-3-yl)piperazin-1-yl)methyl)pyrrolo[1,2-a]quinoxalin-4(5H)-one;
   7-((4-(2-chloro-6-(cyclopropylcarbamoyl)pyridin-3-yl)piperazin-1-yl)methyl)pyrrolo[1,2-a]quinoxalin-4(5H)-one;
   7-((4-(2-methyl-6-(cyclopropylcarbamoyl)pyridin-3-yl)piperazin-1-yl)methyl)pyrazolo[1,5-a]quinoxalin-4(5H)-one;
   7-((4-(2-methyl-6-(cyclopropylcarbamoyl)pyridin-3-yl)piperazin-1-yl)methyl)-3,5-dihydrofuro[3,4-c]quinolin-4(1H)-one;
   7-((4-(2-fluoro-6-(methylcarbamoyl)pyridin-3-yl)piperazin-1-yl)methyl)-9-fluoro-3,5-dihydrofuro[3,4-c]quinolin-4(1H)-one;
   7-((4-(6-(methylcarbamoyl)pyridin-3-yl)piperazin-1-yl)methyl)pyrrolo[1,2-a]quinoxalin-4(5H)-one;
   7-((4-(2-methyl-6-(cyclopropylcarbamoyl)pyridin-3-yl)piperazin-1-yl)methyl)-9-fluoropyrrolo[1,2-a]quinoxalin-4(5H)-one;
   7-((4-(2-fluoro-6-(cyclopropylcarbamoyl)pyridin-3-yl)piperazin-1-yl)methyl)-9-fluoropyrrolo[1,2-a]quinoxalin-4(5H)-one;
   7-((4-(2-chloro-6-(cyclopropylcarbamoyl)pyridin-3-yl)piperazin-1-yl)methyl)-9-fluoropyrrolo[1,2-a]quinoxalin-4(5H)-one;
   7-((4-(2-fluoro-6-(methylcarbamoyl)pyridin-3-yl)piperazin-1-yl)methyl)-2-methylpyrazolo[1,5-a]quinoxalin-4(5H)-one;
   7-((4-(2-chloro-6-(methylcarbamoyl)pyridin-3-yl)piperazin-1-yl)methyl)-2-methylpyrazolo[1,5-a]quinoxalin-4(5H)-one;
   7-((4-(2-fluoro-6-(methylcarbamoyl)pyridin-3-yl)piperazin-1-yl)methyl)-3-methylpyrazolo[1,5-a]quinoxalin-4(5H)-one;
   7-((4-(2-chloro-6-(methylcarbamoyl)pyridin-3-yl)piperazin-1-yl)methyl)-3-methylpyrazolo[1,5-a]quinoxalin-4(5H)-one;
   7-((4-(2-fluoro-6-(methylcarbamoyl)pyridin-3-yl)piperazin-1-yl)methyl)-9-fluoro-2-methylpyrazolo[1,5-a]quinoxalin-4(5H)-one;
   7-((4-(2-chloro-6-(methylcarbamoyl)pyridin-3-yl)piperazin-1-yl)methyl)-9-fluoro-2-methylpyrazolo[1,5-a]quinoxalin-4(5H)-one;
   7-((4-(2-methyl-6-(methylcarbamoyl)pyridin-3-yl)piperazin-1-yl)methyl)-9-fluoro-2-methylpyrazolo[1,5-a]quinoxalin-4(5H)-one;
   7-((4-(2-fluoro-6-(methylcarbamoyl)pyridin-3-yl)piperazin-1-yl)methyl)-9-fluoro-3-methylpyrazolo[1,5-a]quinoxalin-4(5H)-one;
   7-((4-(2-chloro-6-(methylcarbamoyl)pyridin-3-yl)piperazin-1-yl)methyl)-9-fluoro-3-methylpyrazolo[1,5-a]quinoxalin-4(5H)-one;
   7-((4-(2-methyl-6-(methylcarbamoyl)pyridin-3-yl)piperazin-1-yl)methyl)-9-fluoro-3-methylpyrazolo[1,5-a]quinoxalin-4(5H)-one;
   7-((4-(2-methyl-6-(methylcarbamoyl)pyridin-3-yl)piperazin-1-yl)methyl)-9-fluoro-1,2,3,5-tetrahydro-4H-cyclopenta[c]quinolin-4-one;
   7-((4-(2-fluoro-6-(methylcarbamoyl)pyridin-3-yl)piperazin-1-yl)methyl)-9-fluoro-1,2,3,5-tetrahydro-4H-cyclopenta[c]quinolin-4-one;
   7-((4-(2-methyl-6-(cyclopropylcarbamoyl)pyridin-3-yl)piperazin-1-yl)methyl)-9-fluoro-1,2,3,5-tetrahydro-4H-cyclopenta[c]quinolin-4-one;
   7-((4-(2-fluoro-6-(cyclopropylcarbamoyl)pyridin-3-yl)piperazin-1-yl)methyl)-9-fluoro-1,2,3,5-tetrahydro-4H-cyclopenta[c]quinolin-4-one;
   7-((4-(2-methyl-6-(methylcarbamoyl)pyridin-3-yl)piperazin-1-yl)methyl)thieno[3,4-c]quinolin-4(5H)-one;
   7-((4-(2-methyl-6-(methylcarbamoyl)pyridin-3-yl)piperazin-1-yl)methyl)thieno[2,3-c]quinolin-4(5H)-one;
   7-((4-(2-methyl-6-(methylcarbamoyl)pyridin-3-yl)piperazin-1-yl)methyl)thieno[3,2-c]quinolin-4(5H)-one;
   7-((4-(2-methyl-6-(methylcarbamoyl)pyridin-3-yl)piperazin-1-yl)methyl)-9-fluorothieno[3,4-c]quinolin-4(5H)-one;
   7-((4-(2-methyl-6-(methylcarbamoyl)pyridin-3-yl)piperazin-1-yl)methyl)-2-chloro-9-fluoropyrazolo[1,5-a]quinoxalin-4(5H)-one;
   7-((4-(2-fluoro-6-(methylcarbamoyl)pyridin-3-yl)piperazin-1-yl)methyl)-2-chloro-9-fluoropyrazolo[1,5-a]quinoxalin-4(5H)-one;
   7-((4-(2-methyl-6-(carbamoyl)pyridin-3-yl)piperazin-1-yl)methyl)-9-fluoropyrazolo[1,5-a]quinoxalin-4(5H)-one;
   7-((4-(2-methyl-6-((methyl-d3)-carbamoyl)pyridin-3-yl)piperazin-1-yl)methyl)-9-fluoropyrazolo[1,5-a]quinoxalin-4(5H)-one;
   7-((4-(2-methyl-6-(ethylcarbamoyl)pyridin-3-yl)piperazin-1-yl)methyl)-9-fluoropyrazolo[1,5-a]quinoxalin-4(5H)-one;
   7-((4-(2-methyl-6-(cyclopropylcarbamoyl)pyridin-3-yl)piperazin-1-yl)methyl)-9-fluoropyrazolo[1,5-a]quinoxalin-4(5H)-one;
   7-((4-(2-methyl-6-(methylcarbamoyl)pyridin-3-yl)piperazin-1-yl)methyl)-8-fluoropyrazolo[1,5-a]quinoxalin-4(5H)-one;
   7-((4-(2-methyl-6-(methylcarbamoyl)pyridin-3-yl)piperazin-1-yl)methyl)-6-fluoropyrazolo[1,5-a]quinoxalin-4(5H)-one;
   7-((4-(2-fluoro-6-(methylcarbamoyl)pyridin-3-yl)piperazin-1-yl)methyl)-6-fluoropyrazolo[1,5-a]quinoxalin-4(5H)-one;
   8-((4-(2-methyl-6-(methylcarbamoyl)pyridin-3-yl)piperazin-1-yl)methyl)-10-fluoroimidazo[1,2-c]quinazolin-5(6H)-one;
   7-((4-(6-(methylcarbamoyl)pyridin-3-yl)piperazin-1-yl)methyl)-9-fluoropyrazolo[1,5-a]quinoxalin-4(5H)-one;
   7-((4-(2-methyl-6-(methylcarbamoyl)pyridin-3-yl)piperazin-1-yl)methyl)-3-chloropyrazolo[1,5-a]quinoxalin-4(5H)-one;
   7-((4-(2-fluoro-6-(methylcarbamoyl)pyridin-3-yl)piperazin-1-yl)methyl)-3-chloropyrazolo[1,5-a]quinoxalin-4(5H)-one;
   7-((4-(2-methyl-6-(methylcarbamoyl)pyridin-3-yl)piperazin-1-yl)methyl)-2-chloropyrazolo[1,5-a]quinoxalin-4(5H)-one;
   7-((4-(2-methyl-6-(methylcarbamoyl)pyridin-3-yl)piperazin-1-yl)methyl)-2,3-dimethylpyrazolo[1,5-a]quinoxalin-4(5H)-one;
   7-((4-(2-methyl-6-(methylcarbamoyl)pyridin-3-yl)piperazin-1-yl)methyl)-9-methylpyrazolo[1,5-a]quinoxalin-4(5H)-one;
   7-((4-(2-methyl-6-(methylcarbamoyl)pyridin-3-yl)piperazin-1-yl)methyl)-6-fluoro-2-chloropyrazolo[1,5-a]quinoxalin-4(5H)-one;
   7-((4-(2-methyl-6-(methylcarbamoyl)pyridin-3-yl)piperazin-1-yl)methyl)furo[2,3-c]quinolin-4(5H)-one;
   7-((4-(2-methyl-6-(methylcarbamoyl)pyridin-3-yl)piperazin-1-yl)methyl)-1-methyl-1,5-dihydro-4H-pyrazolo[4,3-c]quinolin-4-one;
   7-((4-(2-methyl-6-(methylcarbamoyl)pyridin-3-yl)piperazin-1-yl)methyl)-1,5-dihydro-4H-pyrazolo[4,3-c]quinolin-4-one;
   7-((4-(2-methyl-6-(methylcarbamoyl)pyridin-3-yl)piperazin-1-yl)methyl)-9-fluoro-furo[2,3-c]quinolin-4(5H)-one;
   7-((4-(2-fluoro-6-(methylcarbamoyl)pyridin-3-yl)piperazin-1-yl)methyl)-9-fluoro-furo[2,3-c]quinolin-4(5H)-one;
   7-((4-(2-methyl-6-(methylcarbamoyl)pyridin-3-yl)piperazin-1-yl)methyl)-6-fluoro-2-methylpyrazolo[1,5-a]quinoxalin-4(5H)-one;
   7-((4-(2-fluoro-6-(ethylcarbamoyl)pyridin-3-yl)piperazin-1-yl)methyl)-6-fluoropyrazolo[1,5-a]quinoxalin-4(5H)-one;
   7-((4-(2-fluoro-6-(cyclopropylcarbamoyl)pyridin-3-yl)piperazin-1-yl)methyl)-6-fluoropyrazolo[1,5-a]quinoxalin-4(5H)-one;
   7-((4-(2-fluoro-6-(methylcarbamoyl)pyridin-3-yl)piperazin-1-yl)methyl)-6-fluoro-2-chloropyrazolo[1,5-a]quinoxalin-4(5H)-one;
   7-((4-(2-methyl-4-(methylcarbamoyl)phenyl)piperazin-1-yl)methyl)-9-fluoropyrazolo[1,5-a]quinoxalin-4(5H)-one;
   7-((4-(2-methyl-6-(methylcarbamoyl)pyridin-3-yl)piperazin-1-yl)methyl)-9-fluorothieno[2,3-c]quinolin-4(5H)-one;
   7-((4-(2-fluoro-6-(methylcarbamoyl)pyridin-3-yl)piperazin-1-yl)methyl)-6-fluoro-2-methylpyrazolo[1,5-a]quinoxalin-4(5H)-one;
   7-((4-(2-methyl-6-carboxypyridin-3-yl)piperazin-1-yl)methyl)-9-fluoropyrazolo[1,5-a]quinoxalin-4(5H)-one;
   7-((4-(2-methyl-6-(N-(hydroxymethyl)carbamoyl)pyridin-3-yl)piperazin-1-yl)methyl)-9-fluoropyrazolo[1,5-a]quinoxalin-4(5H)-one;
   8-((4-(2-methyl-6-(methylcarbamoyl)pyridin-3-yl)piperazin-1-yl)methyl)pyrazolo[1,5-c]quinazolin-5(6H)-one;
   8-((4-(2-chloro-6-(methylcarbamoyl)pyridin-3-yl)piperazin-1-yl)methyl)-10-fluoroimidazo[1,2-c]quinazolin-5(6H)-one;
   8-((4-(2-fluoro-6-(methylcarbamoyl)pyridin-3-yl)piperazin-1-yl)methyl)-10-fluoroimidazo[1,2-c]quinazolin-5(6H)-one;
   7-((4-(2-methyl-6-(methylcarbamoyl)pyridin-3-yl)piperazin-1-yl)methyl)furo[3,2-c]quinolin-4(5H)-one;
   7-((4-(2-methyl-6-(methylcarbamoyl)pyridin-3-yl)piperazin-1-yl)methyl)thiazolo[4,5-c]quinolin-4(5H)-one;
   7-((4-(2-fluoro-6-(methylcarbamoyl)pyridin-3-yl)piperazin-1-yl)methyl)-2-chloropyrazolo[1,5-a]quinoxalin-4(5H)-one;
   7-((4-(2-methyl-6-(methylcarbamoyl)pyridin-3-yl)piperazin-1-yl)methyl)-3-fluoropyrazolo[1,5-a]quinoxalin-4(5H)-one;
   7-((4-(2-fluoro-6-(methylcarbamoyl)pyridin-3-yl)piperazin-1-yl)methyl)-2,3-dimethylpyrazolo[1,5-a]quinoxalin-4(5H)-one;
   7-((4-(2-fluoro-6-(methylcarbamoyl)pyridin-3-yl)piperazin-1-yl)methyl)-3-chloro-2-methylpyrazolo[1,5-a]quinoxalin-4(5H)-one;
   7-((4-(2-methyl-6-(methylcarbamoyl)pyridin-3-yl)piperazin-1-yl)methyl)-3-chloro-9-fluoropyrazolo[1,5-a]quinoxalin-4(5H)-one;
   7-((4-(2-methyl-6-(methylcarbamoyl)pyridin-3-yl)piperazin-1-yl)methyl)-6-fluoropyrrolo[1,2-a]quinoxalin-4(5H)-one;
   7-((4-(2-methyl-6-(methylcarbamoyl)pyridin-3-yl)piperazin-1-yl)methyl)-6-methylpyrazolo[1,5-a]quinoxalin-4(5H)-one;
   7-((4-(2-methyl-6-(methylcarbamoyl)pyridin-3-yl)piperazin-1-yl)methyl)-6-chloropyrazolo[1,5-a]quinoxalin-4(5H)-one;
   8-((4-(2-methyl-6-(methylcarbamoyl)pyridin-3-yl)piperazin-1-yl)methyl)-10-fluoropyrrolo[1,2-c]quinazolin-5(6H)-one;
   8-((4-(2-fluoro-6-(methylcarbamoyl)pyridin-3-yl)piperazin-1-yl)methyl)-10-fluoropyrrolo[1,2-c]quinazolin-5(6H)-one;
   7-((4-(2-fluoro-6-(methylcarbamoyl)pyridin-3-yl)piperazin-1-yl)methyl)-6-methylpyrazolo[1,5-a]quinoxalin-4(5H)-one;
   7-((4-(2-fluoro-6-(methylcarbamoyl)pyridin-3-yl)piperazin-1-yl)methyl)-6-chloropyrazolo[1,5-a]quinoxalin-4(5H)-one;
   7-((4-(2-methyl-6-(cyclopropylcarbamoyl)pyridin-3-yl)piperazin-1-yl)methyl)-6-fluoropyrazolo[1,5-a]quinoxalin-4(5H)-one;
   6-fluoro-7-((4-(2-fluoro-6-((methyl-d3)carbamoyl)pyridin-3-yl)piperazin-1-yl)methyl)pyrazolo[1,5-a]quinoxalin-4(5H)-one;
   6-fluoro-7-((4-(2-methyl-6-((methyl-d3)carbamoyl)pyridin-3-yl)piperazin-1-yl)methyl)pyrazolo[1,5-a]quinoxalin-4(5H)-one;
   6-fluoro-7-((4-(2-fluoro-6-((methyl-d3)carbamoyl)pyridin-3-yl)piperazin-1-yl)methyl)furo[2,3-c]quinolin-4(5H)-one;
   6-fluoro-7-((4-(2-methyl-6-((methyl-d3)carbamoyl)pyridin-3-yl)piperazin-1-yl)methyl)furo[2,3-c]quinolin-4(5H)-one;
   6-fluoro-7-((4-(2-methyl-6-((methyl-d3)carbamoyl)pyridin-3-yl)piperazin-1-yl)methyl)-2-chloropyrazolo[1,5-a]quinoxalin-4(5H)-one;
   9-fluoro-7-((4-(2-fluoro-6-((methyl-d3)carbamoyl)pyridin-3-yl)piperazin-1-yl)methyl)pyrazolo[1,5-a]quinoxalin-4(5H)-one;
   6-fluoro-7-((4-(2-fluoro-6-((methyl-d3)carbamoyl)pyridin-3-yl)piperazin-1-yl)methyl)-2-chloropyrazolo[1,5-a]quinoxalin-4(5H)-one;
   7-((4-(2-methyl-6-(methylcarbamoyl)pyridin-3-yl)piperazin-1-yl)methyl)-6-fluoro-furo[2,3-c]quinolin-4(5H)-one;
   7-((4-(2-methyl-6-(ethylcarbamoyl)pyridin-3-yl)piperazin-1-yl)methyl)-6-fluoropyrazolo[1,5-a]quinoxalin-4(5H)-one
   7-((4-(2-fluoro-6-(methylcarbamoyl)pyridin-3-yl)piperazin-1-yl)methyl)-6-fluoropyrrolo[1,2-a]quinoxalin-4(5H)-one;
   7-((4-(2-methyl-6-(methylcarbamoyl)pyridin-3-yl)piperazin-1-yl)methyl)-2-methyl-2,5-dihydro-4H-pyrazolo[4,3-c]quinolin-4-one;
   7-((4-(2-methyl-6-(methylcarbamoyl)pyridin-3-yl)piperazin-1-yl)methyl)-3-chloro-2-methylpyrazolo[1,5-a]quinoxalin-4(5H)-one;
   7-((4-(2-methyl-6-(methylcarbamoyl)pyridin-3-yl)piperazin-1-yl)methyl)-3,9-difluoropyrazolo[1,5-a]quinoxalin-4(5H)-one;
   7-((4-(2-fluoro-6-(methylcarbamoyl)pyridin-3-yl)piperazin-1-yl)methyl)-6-fluoro-furo[2,3-c]quinolin-4(5H)-one;
   8-((4-(2-fluoro-6-(methylcarbamoyl)pyridin-3-yl)piperazin-1-yl)methyl)-7-fluoropyrrolo[1,2-c]quinazolin-5(6H)-one;
   or stereoisomers, tautomers, N-oxides, hydrates, isotope-substituted derivatives, solvates or pharmaceutically acceptable salts thereof, or prodrugs thereof, or mixtures thereof.
12. Use of the compound of any one of clauses 1-11, or stereoisomers, tautomers, N-oxides, hydrates, isotope-substituted derivatives, solvates or pharmaceutically acceptable salts thereof, or prodrugs thereof, or mixtures thereof in the manufacture of a medicament for treatment or prevention of a disease or condition responsive to the inhibition of PARP activity; preferably, the disease or condition is cancer.
13. The use of clause 12, wherein:
   the cancer is selected from liver cancer, melanoma, Hodgkin's disease, non-Hodgkin's lymphomas, acute lymphocytic leukemia, chronic lymphocytic leukemia, multiple myeloma, neuroblastoma, breast cancer, ovarian cancer, lung cancer (such as small cell lung cancer), Wilms' tumor, cervical cancer, testicular cancer, soft-tissue sarcoma, primary macroglobulinemia, bladder cancer, chronic myeloid leukemia, primary brain cancer, malignant melanoma, gastric cancer, colon cancer, malignant pancreatic islet tumor, malignant carcinoid cancer, choriocarcinoma, mycosis fungoide, head and neck cancer, osteogenic sarcoma, pancreatic cancer, acute myeloid leukemia, hairy cell leukemia, rhabdomyosarcoma, Kaposi's sarcoma, urogenital tumors, thyroid cancer, esophageal cancer, malignant hypercalcemia, cervical hyperplasia, renal cell carcinoma, endometrial cancer, polycythemia vera, idiopathic thrombocythemia, adrenocortical carcinoma, skin cancer, or prostatic cancer; and/or
   the medicament further comprises at least one known anticancer drug or a pharmaceutically acceptable salt thereof; preferably, the anticancer drug is selected from a group consisting of a group consisting of: busulfan, melphalan, chlorambucil, cyclophosphamide, ifosfamide, temozolomide, bendamustine, cis-platin, mitomycin C, bleomycin, carboplatin, camptothecin, irinotecan, topotecan, doxorubicin, epirubicin, aclarubicin, mitoxantrone, methylhydroxy ellipticine, etoposide, 5-azacytidine, gemcitabine, 5-fluorouracil, capecitabine, methotrexate, S-fluoro-2'-deoxy-uridine, fludarabine, nelarabine, ara-C, pralatrexate, pemetrexed, hydroxyurea, thioguanine, colchicine, vinblastine, vincristine, vinorelbine, paclitaxel, ixabepilone, cabazitaxel, docetaxel, mAb, panitumumab, necitumumab, nivolumab, pembrolizumab, ramucirumab, bevacizumab, pertuzumab, trastuzumab, cetuximab, obinutuzumab, ofatumumab, rituximab, alemtuzumab, ibritumomab, tositumomab, brentuximab, daratumumab, elotuzumab, T-DM1, Ofatumumab, Dinutuximab, Blinatumomab, ipilimumab, avastin, herceptin, mabthera, T-DM1, Trastuzumab Deruxtecan, Trastuzumab Emtansine, Datopotamab Deruxtecan, Gemtuzumab Ozogamicin, Brentuximab Vedotin, Inotuzumab Ozogamicin, Sacituzumab govitecan, Enfortumab Vedotin, Belantamab Mafodotin, imatinib, gefitinib, erlotinib, osimertinib, afatinib, ceritinib, alectinib, crizotinib, erlotinib, lafenib, sorafenib, regorafenib, vemurafenib, dabrafenib, aflibercept, sunitinib, nilotinib, dasatinib, bosutinib, ponatinib, ibrutinib, cabozantinib, lenvatinib, vandetanib, trametinib, cobimetinib, axitinib, temsirolimus, idelalisib, pazopanib, Torisel, everolimus, tamoxifen, letrozole, fulvestrant, mitoguazone, octreotide, retinoic acid, arsenic, zoledronic acid, bortezomib, carfilzomib, Ixazomib, vismodegib, sonidegib, denosumab, thalidomide, lenalidomide, Venetoclax, Aldesleukin (recombinant human interleukin-2), sipueucel-T (prostate cancer therapeutic vaccine); and/or
   the medicament is used in combination with radiotherapy.
14. A pharmaceutical composition comprising the compound of any one of clauses 1-11, or stereoisomers, tautomers, N-oxides, hydrates, isotope-substituted derivatives, solvates or pharmaceutically acceptable salts thereof, or prodrugs thereof or mixtures thereof and a pharmaceutically acceptable carrier.
15. The pharmaceutical composition of clause 14, wherein the composition further includes at least one known anticancer drug or pharmaceutically acceptable salts thereof; preferably, the at least one known anticancer drug is selected from a group consisting of the group consisting of: busulfan, melphalan, chlorambucil, cyclophosphamide, ifosfamide, temozolomide, bendamustine, cis-platin, mitomycin C, bleomycin, carboplatin, camptothecin, irinotecan, topotecan, doxorubicin, epirubicin, aclarubicin, mitoxantrone, methylhydroxy ellipticine, etoposide, 5-azacytidine, gemcitabine, 5-fluorouracil, capecitabine, methotrexate, 5-fluoro-2'-deoxy-uridine, fludarabine, nelarabine, ara-C, pralatrexate, pemetrexed, hydroxyurea, thioguanine, colchicine, vinblastine, vincristine, vinorelbine, paclitaxel, ixabepilone, cabazitaxel, docetaxel, mAb, panitumumab, necitumumab, nivolumab, pembrolizumab, ramucirumab, bevacizumab, pertuzumab, trastuzumab, cetuximab, obinutuzumab, ofatumumab, rituximab, alemtuzumab, ibritumomab, tositumomab, brentuximab, daratumumab, elotuzumab, T-DM1, Ofatumumab, Dinutuximab, Blinatumomab, ipilimumab, avastin, herceptin, mabthera, imatinib, gefitinib, erlotinib, ostinib, afatinib, ceritinib, alectinib, crizotinib, erlotinib, lapatinib, solutinib lafenib, regorafenib, vemurafenib, dabrafenib, aflibercept, sunitinib, nilotinib, dasatinib, bosutinib, pratinib, brutinib, cabozantinib, lenvatinib, vandetanib, trametinib, cabitinib, axitinib, temsirolimus, idelalisib, pazopanib, everolimus, tamoxifen, letrozole, fulvestrant, mitoguanhydrazone, octreotide, retinoic acid, arsenic, zoledronic acid, bortezomib, carfilzomib, Ixazomib, vismodegib, sonidegib, denosumab, thalidomide, lenalidomide, Venetoclax, Aldesleukin (recombinant human interleukin-2), sipueucel-T (prostate cancer therapeutic vaccine).

## Claims

1. A compound of Formula I: 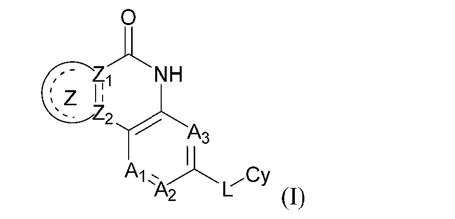 or stereoisomers, tautomers, N-oxides, hydrates, solvates, isotope-substituted derivatives, or pharmaceutically acceptable salts thereof, or prodrugs thereof, or mixtures thereof, wherein:
A₁, A₂ and A₃ each are independently selected from N and CR₁;
The Z ring shown as 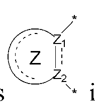 is an optionally substituted 5-7 membered carbocyclic group, an optionally substituted 5-7 membered heterocyclic group or an optionally substituted 5 membered heteroaryl group, * indicates the position at which the Z ring is attached to the rest of the compound, the dashed lines indicate the optional presence of unsaturated bonds, wherein Z₁ and Z₂ are each independently C or N, wherein Z₁ and Z₂ are not N at the same time; in addition, Z₁ and Z₂ satisfy the following conditions: (1) when the Z ring is an optionally substituted 5-7 membered carbocyclic group or an optionally substituted 5-7 membered heterocyclic group, both of Z₁ and Z₂ are C, and among the bonds of the Z ring, only the bond between Z₁ and Z₂ is a double bond; (2) when the Z ring is an optionally substituted 5-7 membered heterocyclic group and Z₂ is C, the ring atom connected to Z₂ of the Z ring is not N; (3) when the Z ring is an optionally substituted 5 membered heteroaryl group and Z₁ is N, in addition to the N at the Z₁ position, the 5 membered heteroaryl further contains 1-3 N heteroatoms; or when Z ring is an optionally substituted 5 membered heteroaryl, Z₁ is N, and there are no other heteroatoms in the Z ring, A₁ is CR₁;
L is selected from a bond and alkylene optionally substituted by R₂ and/or R₃;
Cy is selected from a group consisting of an optionally substituted heterocyclic group, an optionally substituted aryl, and an optionally substituted heteroaryl;
R₁ is selected from a group consisting of hydrogen, halogen, an optionally substituted alkyl, an optionally substituted alkoxy and an optionally substituted carbocyclic group;
R₂ and R₃ are each independently selected from a group consisting of halogen, cyano, an optionally substituted alkyl, an optionally substituted alkoxy, an optionally substituted cycloalkyl, an optionally substituted alkenyl, and an optionally substituted alkynyl; or R₂ and R₃ together with the attached C form a ring.

2. The compound of claim 1, wherein the compound is of Formula IIIb: 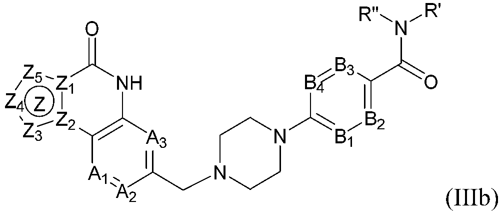 or stereoisomers, tautomers, N-oxides, hydrates, solvates, isotope-substituted derivatives, or pharmaceutically acceptable salts thereof, or prodrugs thereof, or mixtures thereof, wherein:
B₁, B₂, B₃ and B₄ are independently selected from a group consisting of N and CR₇; R₇ is selected from a group consisting of hydrogen, halogen, an optionally substituted alkyl, an optionally substituted alkoxy, an optionally substituted carbocyclic group, an optionally substituted alkenyl and an optionally substituted alkynyl;
Z₁ and Z₂ each are independently C or N, with the proviso that Z₁ and Z₂ are not N at the same time; Z₃, Z₄ and Z₅ are independently selected from a group consisting of CR₄, O, S, N, and NR₄; and when Z₁ is N, at least one of Z₃, Z₄ and Z₅ is N; or when Z₁ is N and all of Z₃, Z₄ and Z₅ are CR₄, A₁ is CR₁;
R₄ is selected from hydrogen, halogen and an optionally substituted alkyl; and
R' and R" each are independently hydrogen, an optionally substituted C₁₋₁₀ alkyl, an optionally substituted C₃₋₈ cycloalkyl, an optionally substituted aryl or an optionally substituted heteroaryl.

3. The compound of claim 2, wherein the Z ring is selected from following groups: 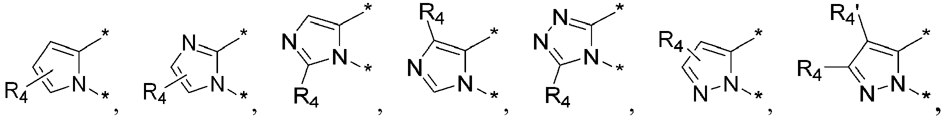 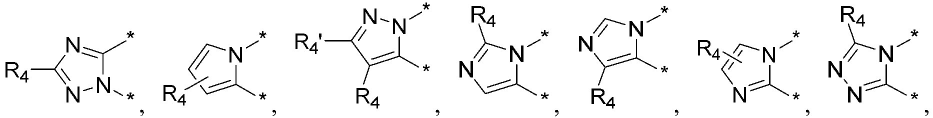 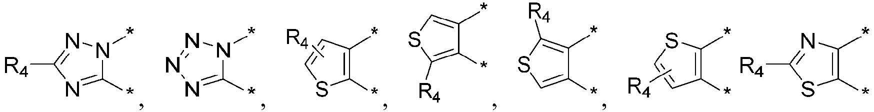 and 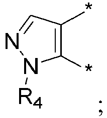 wherein R₄ and R₄' each are independently selected from hydrogen, halogen and an optionally substituted alkyl.

4. The compound of claim 3, wherein the Z ring is selected from the following groups:

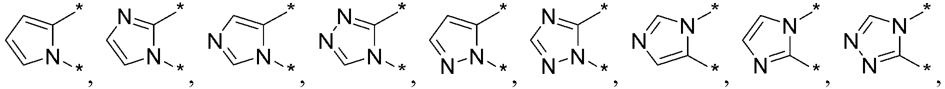

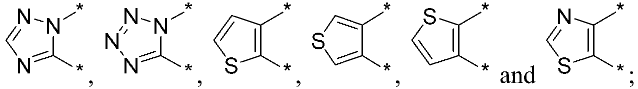

5. The compound of claim 3, wherein the Z ring is selected from the following groups: 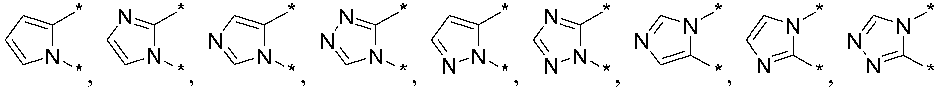 and

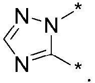

6. The compound of claim 3, wherein the Z ring is: 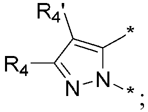 R₄ and R₄' each are independently selected from hydrogen, halogen and an optionally substituted alkyl.

7. The compound of claim 1, wherein the compound is of Formula IVb: 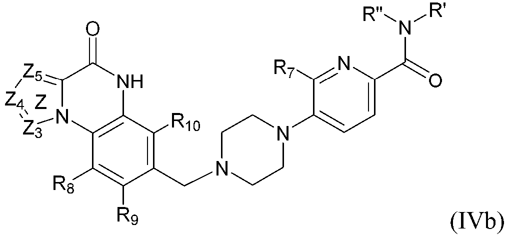 or stereoisomers, tautomers, N-oxides, hydrates, solvates, isotope-substituted derivatives, or pharmaceutically acceptable salts thereof, or mixtures thereof, or prodrugs thereof, wherein:
Z₃, Z₄, Z₅, R₇, R' and R" are as defined in any of the foregoing embodiments;
R₈, R₉ and R₁₀ each are independently selected from a group consisting of hydrogen, halogen, an optionally substituted alkyl, and an optionally substituted alkoxy.

8. The compound of claim 7, wherein the 5-membered ring containing Z₃, Z₄ and Z₅ is selected from the following groups: 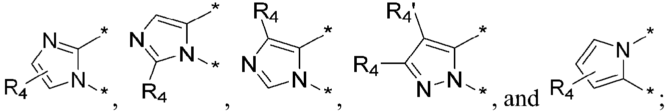 wherein each R₄ is independently selected from a group consisting of hydrogen, halogen and C₁₋₃ alkyl; each R₄' is independently selected from a group consisting of hydrogen, halogen and C₁₋₃ alkyl.

9. The compound of claim 7, wherein the 5-membered ring containing Z₃, Z₄ and Z₅ is selected from the following groups: 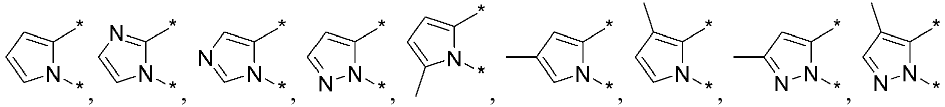 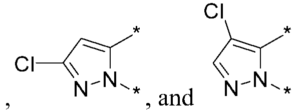
R₇ is H, C₁₋₃ alkyl or halogen;
R₈, R₉ and R₁₀ each are hydrogen, halogen or C₁₋₃ alkyl; preferably, R₈ is C₁₋₃ alkyl or halogen and both of R₉ and R₁₀ are H; or R₉ is C₁₋₃ alkyl or halogen and both of R₈ and R₁₀ are H; or both of R₈ and R₉ are H and R₁₀ is C₁₋₃ alkyl or halogen; or all of R₈, R₉ and R₁₀ are H; and
R' and R" are each independently H or C₁₋₃ alkyl or C₃₋₆ cycloalkyl.

10. The compound of claim 1, wherein the compound is selected from a group consisting of a group consisting of:
7-((4-(6-(methylcarbamoyl)pyridin-3-yl)piperazin-1-yl)methyl)-3,5-dihydrofuro[3,4-c]quinolin-4(1H)-one;
7-((4-(2-fluoro-6-(methylcarbamoyl)pyridin-3-yl)piperazin-1-yl)methyl)-3,5-dihydrofuro[3,4-c]quinolin-4(1H)-one;
7-((4-(2-chloro-6-(methylcarbamoyl)pyridin-3-yl)piperazin-1-yl)methyl)-3,5-dihydrofuro[3,4-c]quinolin-4(1H)-one;
7-((4-(2-methyl-6-(methylcarbamoyl)pyridin-3-yl)piperazin-1-yl)methyl)-3,5-dihydrofuro[3,4-c]quinolin-4(1H)-one;
7-((4-(2-trifluoromethyl-6-(methylcarbamoyl)pyridin-3-yl)piperazin-1-yl)methyl)-3,5-dihydrofuro[3,4-c]quinolin-4(1H)-one;
7-((4-(2-chloro-6-(ethylcarbamoyl)pyridin-3-yl)piperazin-1-yl)methyl)-3,5-dihydrofuro[3,4-c]quinolin-4(1H)-one;
7-((4-(2-methyl-6-(ethylcarbamoyl)pyridin-3-yl)piperazin-1-yl)methyl)-3,5-dihydrofuro[3,4-c]quinolin-4(1H)-one;
7-((4-(2-trifluoromethyl-6-(ethylcarbamoyl)pyridin-3-yl)piperazin-1-yl)methyl)-3,5-dihydrofuro[3,4-c]quinolin-4(1H)-one;
7-((4-(2-chloro-6-(methylcarbamoyl)pyridin-3-yl)piperazin-1-yl)methyl)-9-fluoro-3,5-dihydrofuro[3,4-c]quinolin-4(1H)-one;
7-((4-(2-methyl-6-(methylcarbamoyl)pyridin-3-yl)piperazin-1-yl)methyl)-9-fluoro-3,5-dihydrofuro[3,4-c]quinolin-4(1H)-one;
7-((4-(2-trifluoromethyl-6-(methylcarbamoyl)pyridin-3-yl)piperazin-1-yl)methyl)-9-fluoro-3,5-dihydrofuro[3,4-c]quinolin-4(1H)-one;
7-((4-(2-chloro-6-(ethylcarbamoyl)pyridin-3-yl)piperazin-1-yl)methyl)-9-fluoro-3,5-dihydrofuro[3,4-c]quinolin-4(1H)-one;
7-((4-(2-methyl-6-(ethylcarbamoyl)pyridin-3-yl)piperazin-1-yl)methyl)-9-fluoro-3,5-dihydrofuro[3,4-c]quinolin-4(1H)-one;
7-((4-(2-trifluoromethyl-6-(ethylcarbamoyl)pyridin-3-yl)piperazin-1-yl)methyl)-9-fluoro-3,5-dihydrofuro[3,4-c]quinolin-4(1H)-one;
3-((4-(2-methyl-6-(methylcarbamoyl)pyridin-3-yl)piperazin-1-yl)methyl)-7,8,9,10-tetrahydrophenanthridin-6(5H)-one;
7-((4-(2-chloro-6-(methylcarbamoyl)pyridin-3-yl)piperazin-1-yl)methyl)-1,2,3,5-tetrahydro-4H-cyclopenta[c]quinolin-4-one;
7-((4-(2-methyl-6-(methylcarbamoyl)pyridin-3-yl)piperazin-1-yl)methyl)-1,2,3,5-tetrahydro-4H-cyclopenta[c]quinolin-4-one;
7-((4-(2-chloro-6-(methylcarbamoyl)pyridin-3-yl)piperazin-1-yl)methyl)-3,5-dihydrofuro[3,2-c]quinolin-4(2H)-one;
7-((4-(2-methyl-6-(methylcarbamoyl)pyridin-3-yl)piperazin-1-yl)methyl)-3,5-dihydrofuro[3,2-c]quinolin-4(2H)-one;
3-((4-(2-methyl-6-(methylcarbamoyl)pyridin-3-yl)piperazin-1-yl)methyl)-7,9-dihydrofuro[3,4-c] [1,5]naphthyridin-6(5H)-one;
7-((4-(2-chloro-6-(methylcarbamoyl)pyridin-3-yl)piperazin-1-yl)methyl)-2-methyl-1,2,3,5-tetrahydro-4H-pyrrolo[3,4-c]quinolin-4-one;
8-((4-(2-chloro-6-(methylcarbamoyl)pyridin-3-yl)piperazin-1-yl)methyl)-1,2,4,6-tetrahydro-5H-pyrano[3,4-c]quinolin-5-one;
8-((4-(2-methyl-6-(methylcarbamoyl)pyridin-3-yl)piperazin-1-yl)methyl)-1,2,4,6-tetrahydro-5H-pyrano[3,4-c]quinolin-5-one;
7-((4-(2-methyl-6-(methylcarbamoyl)pyridin-3-yl)piperazin-1-yl)methyl)pyrrolo[1,2-a]quinoxalin-4(5H)-one;
7-((4-(2-methyl-6-(methylcarbamoyl)pyridin-3-yl)piperazin-1-yl)methyl)imidazo[1,2-a]quinoxalin-4(5H)-one;
7-((4-(2-methyl-6-(methylcarbamoyl)pyridin-3-yl)piperazin-1-yl)methyl)imidazo[1,5-a]quinoxalin-4(5H)-one;
7-((4-(2-methyl-6-(methylcarbamoyl)pyridin-3-yl)piperazin-1-yl)methyl)-[1,2,4]triazolo[4,3-a]quinoxalin-4(5H)-one;
7-((4-(2-methyl-6-(methylcarbamoyl)pyridin-3-yl)piperazin-1-yl)methyl)pyrazolo[1,5-a]quinoxalin-4(5H)-one;
7-((4-(2-methyl-6-(methylcarbamoyl)pyridin-3-yl)piperazin-1-yl)methyl)-[1,2,4]triazolo[1,5-a]quinoxalin-4(5H)-one;
8-((4-(2-methyl-6-(methylcarbamoyl)pyridin-3-yl)piperazin-1-yl)methyl)imidazo[1,5-c]quinazolin-5(6H)-one;
8-((4-(2-methyl-6-(methylcarbamoyl)pyridin-3-yl)piperazin-1-yl)methyl)imidazo[1,2-c]quinazolin-5(6H)-one;
8-((4-(2-methyl-6-(methylcarbamoyl)pyridin-3-yl)piperazin-1-yl)methyl)-[1,2,4]triazolo[4,3-c]quinazolin-5(6H)-one;
8-((4-(2-methyl-6-(methylcarbamoyl)pyridin-3-yl)piperazin-1-yl)methyl)-[1,2,4]triazolo[1,5-c]quinazolin-5(6H)-one;
7-((4-(2-fluoro-6-(methylcarbamoyl)pyridin-3-yl)piperazin-1-yl)methyl)pyrrolo[1,2-a]quinoxalin-4(5H)-one;
7-((4-(2-chloro-6-(methylcarbamoyl)pyridin-3-yl)piperazin-1-yl)methyl)pyrrolo[1,2-a]quinoxalin-4(5H)-one;
7-((4-(2-fluoro-6-(methylcarbamoyl)pyridin-3-yl)piperazin-1-yl)methyl)pyrazolo[1,5-a]quinoxalin-4(5H)-one;
7-((4-(2-chloro-6-(methylcarbamoyl)pyridin-3-yl)piperazin-1-yl)methyl)pyrazolo[1,5-a]quinoxalin-4(5H)-one;
7-((4-(2-fluoro-6-(methylcarbamoyl)pyridin-3-yl)piperazin-1-yl)methyl)imidazo[1,5-a]quinoxalin-4(5H)-one;
7-((4-(2-chloro-6-(methylcarbamoyl)pyridin-3-yl)piperazin-1-yl)methyl)imidazo[1,5-a]quinoxalin-4(5H)-one;
7-((4-(2-methyl-6-(methylcarbamoyl)pyridin-3-yl)piperazin-1-yl)methyl)-9-fluoropyrrolo[1,2-a]quinoxalin-4(5H)-one;
7-((4-(2-fluoro-6-(methylcarbamoyl)pyridin-3-yl)piperazin-1-yl)methyl)-9-fluoropyrrolo[1,2-a]quinoxalin-4(5H)-one;
7-((4-(2-chloro-6-(methylcarbamoyl)pyridin-3-yl)piperazin-1-yl)methyl)-9-fluoropyrrolo[1,2-a]quinoxalin-4(5H)-one;
7-((4-(2-methyl-6-(methylcarbamoyl)pyridin-3-yl)piperazin-1-yl)methyl)-9-fluoropyrazolo[1,5-a]quinoxalin-4(5H)-one;
7-((4-(2-fluoro-6-(methylcarbamoyl)pyridin-3-yl)piperazin-1-yl)methyl)-9-fluoropyrazolo[1,5-a]quinoxalin-4(5H)-one;
7-((4-(2-chloro-6-(methylcarbamoyl)pyridin-3-yl)piperazin-1-yl)methyl)-9-fluoropyrazolo[1,5-a]quinoxalin-4(5H)-one;
7-((4-(2-methyl-6-(methylcarbamoyl)pyridin-3-yl)piperazin-1-yl)methyl)-9-chloropyrazolo[1,5-a]quinoxalin-4(5H)-one;
7-((4-(2-fluoro-6-(methylcarbamoyl)pyridin-3-yl)piperazin-1-yl)methyl)-9-chloropyrazolo[1,5-a]quinoxalin-4(5H)-one;
8-((4-(2-methyl-6-(methylcarbamoyl)pyridin-3-yl)piperazin-1-yl)methyl)pyrrolo[1,2-c]quinazolin-5(6H)-one;
7-((4-(2-methyl-6-(ethylcarbamoyl)pyridin-3-yl)piperazin-1-yl)methyl)-1,2,3,5-tetrahydro-4H-cyclopenta[c]quinolin-4-one;
7-((4-(2-chloro-6-(ethylcarbamoyl)pyridin-3-yl)piperazin-1-yl)methyl)-1,2,3,5-tetrahydro-4H-cyclopenta[c]quinolin-4-one;
8-((4-(2-fluoro-6-(methylcarbamoyl)pyridin-3-yl)piperazin-1-yl)methyl)imidazo[1,2-c]quinazolin-5(6H)-one;
8-((4-(2-chloro-6-(methylcarbamoyl)pyridin-3-yl)piperazin-1-yl)methyl)imidazo[1,2-c]quinazolin-5(6H)-one;
8-((4-(2-fluoro-6-(methylcarbamoyl)pyridin-3-yl)piperazin-1-yl)methyl)-10-fluoroimidazo[1,2-c]quinazolin-5(6H)-one;
7-((4-(2-methyl-6-(methylcarbamoyl)pyridin-3-yl)piperazin-1-yl)methyl)-1-methylpyrrolo[1,2-a]quinoxalin-4(5H)-one;
7-((4-(2-methyl-6-(methylcarbamoyl)pyridin-3-yl)piperazin-1-yl)methyl)-2-methylpyrrolo[1,2-a]quinoxalin-4(5H)-one;
7-((4-(2-methyl-6-(methylcarbamoyl)pyridin-3-yl)piperazin-1-yl)methyl)-3-methylpyrrolo[1,2-a]quinoxalin-4(5H)-one;
7-((4-(2-methyl-6-(methylcarbamoyl)pyridin-3-yl)piperazin-1-yl)methyl)-2-methylpyrazolo[1,5-a]quinoxalin-4(5H)-one;
7-((4-(2-methyl-6-(methylcarbamoyl)pyridin-3-yl)piperazin-1-yl)methyl)-3-methylpyrazolo[1,5-a]quinoxalin-4(5H)-one;
7-((4-(2-methyl-6-(cyclopropylcarbamoyl)pyridin-3-yl)piperazin-1-yl)methyl)pyrrolo[1,2-a]quinoxalin-4(5H)-one;
7-((4-(2-fluoro-6-(cyclopropylcarbamoyl)pyridin-3-yl)piperazin-1-yl)methyl)pyrrolo[1,2-a]quinoxalin-4(5H)-one;
7-((4-(2-chloro-6-(cyclopropylcarbamoyl)pyridin-3-yl)piperazin-1-yl)methyl)pyrrolo[1,2-a]quinoxalin-4(5H)-one;
7-((4-(2-methyl-6-(cyclopropylcarbamoyl)pyridin-3-yl)piperazin-1-yl)methyl)pyrazolo[1,5-a]quinoxalin-4(5H)-one;
7-((4-(2-methyl-6-(cyclopropylcarbamoyl)pyridin-3-yl)piperazin-1-yl)methyl)-3,5-dihydrofuro[3,4-c]quinolin-4(1H)-one;
7-((4-(2-fluoro-6-(methylcarbamoyl)pyridin-3-yl)piperazin-1-yl)methyl)-9-fluoro-3,5-dihydrofuro[3,4-c]quinolin-4(1H)-one;
7-((4-(6-(methylcarbamoyl)pyridin-3-yl)piperazin-1-yl)methyl)pyrrolo[1,2-a]quinoxalin-4(5H)-one;
7-((4-(2-methyl-6-(cyclopropylcarbamoyl)pyridin-3-yl)piperazin-1-yl)methyl)-9-fluoropyrrolo[1,2-a]quinoxalin-4(5H)-one;
7-((4-(2-fluoro-6-(cyclopropylcarbamoyl)pyridin-3-yl)piperazin-1-yl)methyl)-9-fluoropyrrolo[1,2-a]quinoxalin-4(5H)-one;
7-((4-(2-chloro-6-(cyclopropylcarbamoyl)pyridin-3-yl)piperazin-1-yl)methyl)-9-fluoropyrrolo[1,2-a]quinoxalin-4(5H)-one;
7-((4-(2-fluoro-6-(methylcarbamoyl)pyridin-3-yl)piperazin-1-yl)methyl)-2-methylpyrazolo[1,5-a]quinoxalin-4(5H)-one;
7-((4-(2-chloro-6-(methylcarbamoyl)pyridin-3-yl)piperazin-1-yl)methyl)-2-methylpyrazolo[1,5-a]quinoxalin-4(5H)-one;
7-((4-(2-fluoro-6-(methylcarbamoyl)pyridin-3-yl)piperazin-1-yl)methyl)-3-methylpyrazolo[1,5-a]quinoxalin-4(5H)-one;
7-((4-(2-chloro-6-(methylcarbamoyl)pyridin-3-yl)piperazin-1-yl)methyl)-3-methylpyrazolo[1,5-a]quinoxalin-4(5H)-one;
7-((4-(2-fluoro-6-(methylcarbamoyl)pyridin-3-yl)piperazin-1-yl)methyl)-9-fluoro-2-methylpyrazolo[1,5-a]quinoxalin-4(5H)-one;
7-((4-(2-chloro-6-(methylcarbamoyl)pyridin-3-yl)piperazin-1-yl)methyl)-9-fluoro-2-methylpyrazolo[1,5-a]quinoxalin-4(5H)-one;
7-((4-(2-methyl-6-(methylcarbamoyl)pyridin-3-yl)piperazin-1-yl)methyl)-9-fluoro-2-methylpyrazolo[1,5-a]quinoxalin-4(5H)-one;
7-((4-(2-fluoro-6-(methylcarbamoyl)pyridin-3-yl)piperazin-1-yl)methyl)-9-fluoro-3-methylpyrazolo[1,5-a]quinoxalin-4(5H)-one;
7-((4-(2-chloro-6-(methylcarbamoyl)pyridin-3-yl)piperazin-1-yl)methyl)-9-fluoro-3-methylpyrazolo[1,5-a]quinoxalin-4(5H)-one;
7-((4-(2-methyl-6-(methylcarbamoyl)pyridin-3-yl)piperazin-1-yl)methyl)-9-fluoro-3-methylpyrazolo[1,5-a]quinoxalin-4(5H)-one;
7-((4-(2-methyl-6-(methylcarbamoyl)pyridin-3-yl)piperazin-1-yl)methyl)-9-fluoro-1,2,3,5-tetrahydro-4H-cyclopenta[c]quinolin-4-one;
7-((4-(2-fluoro-6-(methylcarbamoyl)pyridin-3-yl)piperazin-1-yl)methyl)-9-fluoro-1,2,3,5-tetrahydro-4H-cyclopenta[c]quinolin-4-one;
7-((4-(2-methyl-6-(cyclopropylcarbamoyl)pyridin-3-yl)piperazin-1-yl)methyl)-9-fluoro-1,2,3,5-tetrahydro-4H-cyclopenta[c]quinolin-4-one;
7-((4-(2-fluoro-6-(cyclopropylcarbamoyl)pyridin-3-yl)piperazin-1-yl)methyl)-9-fluoro-1,2,3,5-tetrahydro-4H-cyclopenta[c]quinolin-4-one;
7-((4-(2-methyl-6-(methylcarbamoyl)pyridin-3-yl)piperazin-1-yl)methyl)thieno[3,4-c]quinolin-4(5H)-one;
7-((4-(2-methyl-6-(methylcarbamoyl)pyridin-3-yl)piperazin-1-yl)methyl)thieno[2,3-c]quinolin-4(5H)-one;
7-((4-(2-methyl-6-(methylcarbamoyl)pyridin-3-yl)piperazin-1-yl)methyl)thieno[3,2-c]quinolin-4(5H)-one;
7-((4-(2-methyl-6-(methylcarbamoyl)pyridin-3-yl)piperazin-1-yl)methyl)-9-fluorothieno[3,4-c]quinolin-4(5H)-one;
7-((4-(2-methyl-6-(methylcarbamoyl)pyridin-3-yl)piperazin-1-yl)methyl)-2-chloro-9-fluoropyrazolo[1,5-a]quinoxalin-4(5H)-one;
7-((4-(2-fluoro-6-(methylcarbamoyl)pyridin-3-yl)piperazin-1-yl)methyl)-2-chloro-9-fluoropyrazolo[1,5-a]quinoxalin-4(5H)-one;
7-((4-(2-methyl-6-(carbamoyl)pyridin-3-yl)piperazin-1-yl)methyl)-9-fluoropyrazolo[1,5-a]quinoxalin-4(5H)-one;
7-((4-(2-methyl-6-((methyl-d3)-carbamoyl)pyridin-3-yl)piperazin-1-yl)methyl)-9-fluoropyrazolo[1,5-a]quinoxalin-4(5H)-one;
7-((4-(2-methyl-6-(ethylcarbamoyl)pyridin-3-yl)piperazin-1-yl)methyl)-9-fluoropyrazolo[1,5-a]quinoxalin-4(5H)-one;
7-((4-(2-methyl-6-(cyclopropylcarbamoyl)pyridin-3-yl)piperazin-1-yl)methyl)-9-fluoropyrazolo[1,5-a]quinoxalin-4(5H)-one;
7-((4-(2-methyl-6-(methylcarbamoyl)pyridin-3-yl)piperazin-1-yl)methyl)-8-fluoropyrazolo[1,5-a]quinoxalin-4(5H)-one;
7-((4-(2-methyl-6-(methylcarbamoyl)pyridin-3-yl)piperazin-1-yl)methyl)-6-fluoropyrazolo[1,5-a]quinoxalin-4(5H)-one;
7-((4-(2-fluoro-6-(methylcarbamoyl)pyridin-3-yl)piperazin-1-yl)methyl)-6-fluoropyrazolo[1,5-a]quinoxalin-4(5H)-one;
8-((4-(2-methyl-6-(methylcarbamoyl)pyridin-3-yl)piperazin-1-yl)methyl)-10-fluoroimidazo[1,2-c]quinazolin-5(6H)-one;
7-((4-(6-(methylcarbamoyl)pyridin-3-yl)piperazin-1-yl)methyl)-9-fluoropyrazolo[1,5-a]quinoxalin-4(5H)-one;
7-((4-(2-methyl-6-(methylcarbamoyl)pyridin-3-yl)piperazin-1-yl)methyl)-3-chloropyrazolo[1,5-a]quinoxalin-4(5H)-one;
7-((4-(2-fluoro-6-(methylcarbamoyl)pyridin-3-yl)piperazin-1-yl)methyl)-3-chloropyrazolo[1,5-a]quinoxalin-4(5H)-one;
7-((4-(2-methyl-6-(methylcarbamoyl)pyridin-3-yl)piperazin-1-yl)methyl)-2-chloropyrazolo[1,5-a]quinoxalin-4(5H)-one;
7-((4-(2-methyl-6-(methylcarbamoyl)pyridin-3-yl)piperazin-1-yl)methyl)-2,3-dimethylpyrazolo[1,5-a]quinoxalin-4(5H)-one;
7-((4-(2-methyl-6-(methylcarbamoyl)pyridin-3-yl)piperazin-1-yl)methyl)-9-methylpyrazolo[1,5-a]quinoxalin-4(5H)-one;
7-((4-(2-methyl-6-(methylcarbamoyl)pyridin-3-yl)piperazin-1-yl)methyl)-6-fluoro-2-chloropyrazolo[1,5-a]quinoxalin-4(5H)-one;
7-((4-(2-methyl-6-(methylcarbamoyl)pyridin-3-yl)piperazin-1-yl)methyl)furo[2,3-c]quinolin-4(5H)-one;
7-((4-(2-methyl-6-(methylcarbamoyl)pyridin-3-yl)piperazin-1-yl)methyl)-1-methyl-1,5-dihydro-4H-pyrazolo[4,3-c]quinolin-4-one;
7-((4-(2-methyl-6-(methylcarbamoyl)pyridin-3-yl)piperazin-1-yl)methyl)-1,5-dihydro-4H-pyrazolo[4,3-c]quinolin-4-one;
7-((4-(2-methyl-6-(methylcarbamoyl)pyridin-3-yl)piperazin-1-yl)methyl)-9-fluoro-furo[2,3-c]quinolin-4(5H)-one;
7-((4-(2-fluoro-6-(methylcarbamoyl)pyridin-3-yl)piperazin-1-yl)methyl)-9-fluoro-furo[2,3-c]quinolin-4(5H)-one;
7-((4-(2-methyl-6-(methylcarbamoyl)pyridin-3-yl)piperazin-1-yl)methyl)-6-fluoro-2-methylpyrazolo[1,5-a]quinoxalin-4(5H)-one;
7-((4-(2-fluoro-6-(ethylcarbamoyl)pyridin-3-yl)piperazin-1-yl)methyl)-6-fluoropyrazolo[1,5-a]quinoxalin-4(5H)-one;
7-((4-(2-fluoro-6-(cyclopropylcarbamoyl)pyridin-3-yl)piperazin-1-yl)methyl)-6-fluoropyrazolo[1,5-a]quinoxalin-4(5H)-one;
7-((4-(2-fluoro-6-(methylcarbamoyl)pyridin-3-yl)piperazin-1-yl)methyl)-6-fluoro-2-chloropyrazolo[1,5-a]quinoxalin-4(5H)-one;
7-((4-(2-methyl-4-(methylcarbamoyl)phenyl)piperazin-1-yl)methyl)-9-fluoropyrazolo[1,5-a]quinoxalin-4(5H)-one;
7-((4-(2-methyl-6-(methylcarbamoyl)pyridin-3-yl)piperazin-1-yl)methyl)-9-fluorothieno[2,3-c]quinolin-4(5H)-one;
7-((4-(2-fluoro-6-(methylcarbamoyl)pyridin-3-yl)piperazin-1-yl)methyl)-6-fluoro-2-methylpyrazolo[1,5-a]quinoxalin-4(5H)-one;
7-((4-(2-methyl-6-carboxypyridin-3-yl)piperazin-1-yl)methyl)-9-fluoropyrazolo[1,5-a]quinoxalin-4(5H)-one;
7-((4-(2-methyl-6-(N-(hydroxymethyl)carbamoyl)pyridin-3-yl)piperazin-1-yl)methyl)-9-fluoropyrazolo[1,5-a]quinoxalin-4(5H)-one;
8-((4-(2-methyl-6-(methylcarbamoyl)pyridin-3-yl)piperazin-1-yl)methyl)pyrazolo[1,5-c]quinazolin-5(6H)-one;
8-((4-(2-chloro-6-(methylcarbamoyl)pyridin-3-yl)piperazin-1-yl)methyl)-10-fluoroimidazo[1,2-c]quinazolin-5(6H)-one;
8-((4-(2-fluoro-6-(methylcarbamoyl)pyridin-3-yl)piperazin-1-yl)methyl)-10-fluoroimidazo[1,2-c]quinazolin-5(6H)-one;
7-((4-(2-methyl-6-(methylcarbamoyl)pyridin-3-yl)piperazin-1-yl)methyl)furo[3,2-c]quinolin-4(5H)-one;
7-((4-(2-methyl-6-(methylcarbamoyl)pyridin-3-yl)piperazin-1-yl)methyl)thiazolo[4,5-c]quinolin-4(5H)-one;
7-((4-(2-fluoro-6-(methylcarbamoyl)pyridin-3-yl)piperazin-1-yl)methyl)-2-chloropyrazolo[1,5-a]quinoxalin-4(5H)-one;
7-((4-(2-methyl-6-(methylcarbamoyl)pyridin-3-yl)piperazin-1-yl)methyl)-3-fluoropyrazolo[1,5-a]quinoxalin-4(5H)-one;
7-((4-(2-fluoro-6-(methylcarbamoyl)pyridin-3-yl)piperazin-1-yl)methyl)-2,3-dimethylpyrazolo[1,5-a]quinoxalin-4(5H)-one;
7-((4-(2-fluoro-6-(methylcarbamoyl)pyridin-3-yl)piperazin-1-yl)methyl)-3-chloro-2-methylpyrazolo[1,5-a]quinoxalin-4(5H)-one;
7-((4-(2-methyl-6-(methylcarbamoyl)pyridin-3-yl)piperazin-1-yl)methyl)-3-chloro-9-fluoropyrazolo[1,5-a]quinoxalin-4(5H)-one;
7-((4-(2-methyl-6-(methylcarbamoyl)pyridin-3-yl)piperazin-1-yl)methyl)-6-fluoropyrrolo[1,2-a]quinoxalin-4(5H)-one;
7-((4-(2-methyl-6-(methylcarbamoyl)pyridin-3-yl)piperazin-1-yl)methyl)-6-methylpyrazolo[1,5-a]quinoxalin-4(5H)-one;
7-((4-(2-methyl-6-(methylcarbamoyl)pyridin-3-yl)piperazin-1-yl)methyl)-6-chloropyrazolo[1,5-a]quinoxalin-4(5H)-one;
8-((4-(2-methyl-6-(methylcarbamoyl)pyridin-3-yl)piperazin-1-yl)methyl)-10-fluoropyrrolo[1,2-c]quinazolin-5(6H)-one;
8-((4-(2-fluoro-6-(methylcarbamoyl)pyridin-3-yl)piperazin-1-yl)methyl)-10-fluoropyrrolo[1,2-c]quinazolin-5(6H)-one;
7-((4-(2-fluoro-6-(methylcarbamoyl)pyridin-3-yl)piperazin-1-yl)methyl)-6-methylpyrazolo[1,5-a]quinoxalin-4(5H)-one;
7-((4-(2-fluoro-6-(methylcarbamoyl)pyridin-3-yl)piperazin-1-yl)methyl)-6-chloropyrazolo[1,5-a]quinoxalin-4(5H)-one;
7-((4-(2-methyl-6-(cyclopropylcarbamoyl)pyridin-3-yl)piperazin-1-yl)methyl)-6-fluoropyrazolo[1,5-a]quinoxalin-4(5H)-one;
6-fluoro-7-((4-(2-fluoro-6-((methyl-d3)carbamoyl)pyridin-3-yl)piperazin-1-yl)methyl)pyrazolo[1,5-a]quinoxalin-4(5H)-one;
6-fluoro-7-((4-(2-methyl-6-((methyl-d3)carbamoyl)pyridin-3-yl)piperazin-1-yl)methyl)pyrazolo[1,5-a]quinoxalin-4(5H)-one;
6-fluoro-7-((4-(2-fluoro-6-((methyl-d3)carbamoyl)pyridin-3-yl)piperazin-1-yl)methyl)furo[2,3-c]quinolin-4(5H)-one;
6-fluoro-7-((4-(2-methyl-6-((methyl-d3)carbamoyl)pyridin-3-yl)piperazin-1-yl)methyl)furo[2,3-c]quinolin-4(5H)-one;
6-fluoro-7-((4-(2-methyl-6-((methyl-d3)carbamoyl)pyridin-3-yl)piperazin-1-yl)methyl)-2-chloropyrazolo[1,5-a]quinoxalin-4(5H)-one;
9-fluoro-7-((4-(2-fluoro-6-((methyl-d3)carbamoyl)pyridin-3-yl)piperazin-1-yl)methyl)pyrazolo[1,5-a]quinoxalin-4(5H)-one;
6-fluoro-7-((4-(2-fluoro-6-((methyl-d3)carbamoyl)pyridin-3-yl)piperazin-1-yl)methyl)-2-chloropyrazolo[1,5-a]quinoxalin-4(5H)-one;
7-((4-(2-methyl-6-(methylcarbamoyl)pyridin-3-yl)piperazin-1-yl)methyl)-6-fluoro-furo[2,3-c]quinolin-4(5H)-one;
7-((4-(2-methyl-6-(ethylcarbamoyl)pyridin-3-yl)piperazin-1-yl)methyl)-6-fluoropyrazolo[1,5-a]quinoxalin-4(5H)-one
7-((4-(2-fluoro-6-(methylcarbamoyl)pyridin-3-yl)piperazin-1-yl)methyl)-6-fluoropyrrolo[1,2-a]quinoxalin-4(5H)-one;
7-((4-(2-methyl-6-(methylcarbamoyl)pyridin-3-yl)piperazin-1-yl)methyl)-2-methyl-2,5-dihydro-4H-pyrazolo[4,3-c]quinolin-4-one;
7-((4-(2-methyl-6-(methylcarbamoyl)pyridin-3-yl)piperazin-1-yl)methyl)-3-chloro-2-methylpyrazolo[1,5-a]quinoxalin-4(5H)-one;
7-((4-(2-methyl-6-(methylcarbamoyl)pyridin-3-yl)piperazin-1-yl)methyl)-3,9-difluoropyrazolo[1,5-a]quinoxalin-4(5H)-one;
7-((4-(2-fluoro-6-(methylcarbamoyl)pyridin-3-yl)piperazin-1-yl)methyl)-6-fluoro-furo[2,3-c]quinolin-4(5H)-one;
8-((4-(2-fluoro-6-(methylcarbamoyl)pyridin-3-yl)piperazin-1-yl)methyl)-7-fluoropyrrolo[1,2-c]quinazolin-5(6H)-one;
or stereoisomers, tautomers, N-oxides, hydrates, isotope-substituted derivatives, solvates or pharmaceutically acceptable salts thereof, or prodrugs thereof, or mixtures thereof.

11. A pharmaceutical composition comprising the compound of any one of claims 1-10, or stereoisomers, tautomers, N-oxides, hydrates, isotope-substituted derivatives, solvates or pharmaceutically acceptable salts thereof, or mixtures thereof and a pharmaceutically acceptable carrier.

12. The pharmaceutical composition of claim 11, wherein the composition further includes at least one known anticancer drug or pharmaceutically acceptable salts thereof; preferably, the at least one known anticancer drug is selected from a group consisting of the group consisting of: busulfan, melphalan, chlorambucil, cyclophosphamide, ifosfamide, temozolomide, bendamustine, cis-platin, mitomycin C, bleomycin, carboplatin, camptothecin, irinotecan, topotecan, doxorubicin, epirubicin, aclarubicin, mitoxantrone, methylhydroxy ellipticine, etoposide, 5-azacytidine, gemcitabine, 5-fluorouracil, capecitabine, methotrexate, 5-fluoro-2'-deoxy-uridine, fludarabine, nelarabine, ara-C, pralatrexate, pemetrexed, hydroxyurea, thioguanine, colchicine, vinblastine, vincristine, vinorelbine, paclitaxel, ixabepilone, cabazitaxel, docetaxel, mAb, panitumumab, necitumumab, nivolumab, pembrolizumab, ramucirumab, bevacizumab, pertuzumab, trastuzumab, cetuximab, obinutuzumab, ofatumumab, rituximab, alemtuzumab, ibritumomab, tositumomab, brentuximab, daratumumab, elotuzumab, T-DM1, trastuzumab deruxtecan, trastuzumab emtansine, datopotamab deruxtecan, gemtuzumab ozogamicin, brentuximab vedotin, inotuzumab ozogamicin, sacituzumab govitecan, enfortumab vedotin, belantamab mafodotin, imatinib, gefitinib, erlotinib, ostinib, afatinib, ceritinib, alectinib, crizotinib, erlotinib, lapatinib, solutinib lafenib, regorafenib, vemurafenib, dabrafenib, aflibercept, sunitinib, nilotinib, dasatinib, bosutinib, pratinib, ibrutinib, cabozantinib, lenvatinib, vandetanib, trametinib, cabitinib, axitinib, temsirolimus, idelalisib, pazopanib, everolimus, tamoxifen, letrozole, fulvestrant, mitoguanhydrazone, octreotide, retinoic acid, arsenic, zoledronic acid, bortezomib, carfilzomib, Ixazomib, vismodegib, sonidegib, denosumab, thalidomide, lenalidomide, Venetoclax, Aldesleukin (recombinant human interleukin-2), sipuleucel-T (prostate cancer therapeutic vaccine).

13. A compound of claims 1-10, or stereoisomers, tautomers, N-oxides, hydrates, solvates, isotope-substituted derivatives, or pharmaceutically acceptable salts thereof, or prodrugs thereof, or mixtures thereof, and a pharmaceutically acceptable carrier, or a pharmaceutical composition of claim 11, or stereoisomers, tautomers, N-oxides, hydrates, solvates, isotope-substituted derivatives, or pharmaceutically acceptable salts thereof, or prodrugs thereof, or mixtures thereof, and a pharmaceutically acceptable carrier, for use in a method of treating or preventing a disease or condition responsive to the inhibition of PARP activity.

14. The compound for use of claim 13, wherein the disease or condition responsive to the inhibition of PARP activity is cancer.

15. The compound for use of claim 14, wherein the cancer is selected from liver cancer, melanoma, Hodgkin's disease, non-Hodgkin's lymphomas, acute lymphocytic leukemia, chronic lymphocytic leukemia, multiple myeloma, neuroblastoma, breast cancer, ovarian cancer, lung cancer (such as small cell lung cancer), Wilms' tumor, cervical cancer, testicular cancer, soft-tissue sarcoma, primary macroglobulinemia, bladder cancer, chronic myeloid leukemia, primary brain cancer, malignant melanoma, gastric cancer, colon cancer, malignant pancreatic islet tumor, malignant carcinoid cancer, choriocarcinoma, mycosis fungoide, head and neck cancer, osteogenic sarcoma, pancreatic cancer, acute myeloid leukemia, hairy cell leukemia, rhabdomyosarcoma, Kaposi's sarcoma, urogenital tumors, thyroid cancer, esophageal cancer, malignant hypercalcemia, cervical hyperplasia, renal cell carcinoma, endometrial cancer, polycythemia vera, idiopathic thrombocythemia, adrenocortical carcinoma, skin cancer, and prostatic cancer.
